# EUROPEAN PATENT SPECIFICATION

(11) **EP 2 505 652 B1**
(45) Date of publication and mention of the grant of the patent: **14.10.2015**
(21) Application number: 11192693.7
(22) Date of filing: 02.05.2008
(51) Int. Cl.: C12N 15/82, C07K 14/415

(54) **Plants having enhanced yield-related traits and a method for making the same**
Pflanzen mit verbesserten Ertragseigenschaften und Verfahren zu ihrer Herstellung
Plantes dotées de caractéristiques de rendement améliorées et procédé de fabrication de celles-ci

(30) Priority: 08.05.2007 US 91657507 P; 06.06.2007 US 94221407 P; 29.06.2007 US 93798907 P; 05.07.2007 US 94803607 P; 03.05.2007 EP 07107448; 29.05.2007 EP 07109052; 29.05.2007 EP 07109068; 11.06.2007 EP 07109961; 19.06.2007 EP 07110548; 19.06.2007 EP 07110557
(43) Date of publication of application: 03.10.2012
(62) Divisional of application: 08767542.7
(73) Proprietor: BASF Plant Science Company GmbH, 67056 Ludwigshafen (DE)
(72) Inventor: Sanz Molinero, Ana Isabel, B-9050 Gentbrugge (BE); Hatzfeld, Yves, 59000 Lille (FR); Vandenabeele, Steven, B-9700 Oudenaarde (BE); Shirley, Amber, Durham, NC North Carolina 27703 (US); Darnielle, Lalitree, Durham, NC North Carolina 27707 (US); McKersie, Bryan, Raleigh, NC North Carolina 27617 (US); Frankard, Valerie, B-1410 Waterloo (BE)

(56) References cited:
- WO-A2-03/007699
- WO-A2-2006/056590
- US-A1- 2007 033 671
- ITO Y ET AL: "Radial axis differentiation in a globular embryo is marked by HAZ1, a PHD-finger homeobox gene of rice", GENE, ELSEVIER, AMSTERDAM, NL, vol. 331, 28 April 2004 (2004-04-28), pages 9-15, XP004503512, ISSN: 0378-1119, DOI: 10.1016/J.GENE.2004.02.040
- UEBERLACKER BAERBEL ET AL: "Ectopic expression of the maize homeobox genes ZmHox1a or ZmHox1b causes pleiotropic alterations in the vegetative and floral development of transgenic tobacco", PLANT CELL, vol. 8, no. 3, 1996, pages 349-362, XP002682055, ISSN: 1040-4651
- HALBACH THORSTEN ET AL: "Transcriptional activation by the PHD finger is inhibited through an adjacent leucine zipper that binds 14-3-3 proteins", NUCLEIC ACIDS RESEARCH, OXFORD UNIVERSITY PRESS, SURREY, GB, vol. 28, no. 18, 1 September 2000 (2000-09-01), pages 3542-3550, XP009091974, ISSN: 0305-1048, DOI: 10.1093/NAR/28.18.3542

## Description

The present invention relates generally to the field of molecular biology and concerns a method for enhancing seed yield-related traits by increasing expression in a plant of a nucleic acid encoding a plant homeodomain finger-homeodomain (PHDf-HD) polypeptide. The present invention also concerns plants having increased expression of a nucleic acid encoding a PHDf-HD polypeptide, which plants have enhanced seed yield-related traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigor has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

Surprisingly, it has now been found that increasing expression of a nucleic acid encoding a plant homeodomain finger-homeodomain (PHDf-HD) polypeptide gives plants having enhanced seed yield-related traits relative to control plants.

According to one embodiment, there is provided a method for improving or enhancing seed yield related traits of a plant relative to control plants, comprising increasing expression of a nucleic acid encoding a plant homeodomain finger-homeodomain (PHDf-HD) polypeptide in a plant.

### Background

Transcription factors are usually defined as proteins that show sequence-specific DNA binding affinity and that are capable of activating and/or repressing transcription. The *Arabidopsis thaliana* genome codes for at least 1533 transcriptional regulators, accounting for ∼5.9% of its estimated total number of genes (Riechmann et al. (2000) Science 290: 2105-2109). The Database of Rice Transcription Factors (DRTF) is a collection of known and predicted transcription factors of *Oryza sativa L. ssp. indica* and *Oryza sativa L. ssp. japonica,* and currently contains 2,025 putative transcription factors (TF) gene models in *indica* and 2,384 in *japonica,* distributed in 63 families (Gao et al. (2006) Bioinformatics 2006, 22(10):1286-7).

One of these families is the superfamily of homeodomain (HD) transcription factors involved in many aspects of developmental processes. HD transcription factors are characterized by the presence of a homeodomain (HD), which is a 60-amino acid DNA-binding domain (BD). *Arabidopsis thaliana* and rice contain approximately 100 HD transcription factors, which can be further classified into subfamilies based on amino acid sequence identity (Richardt et al. (2007) Plant Phys 143(4): 1452-1466). Some of these subfamilies are characterized by the presence of additional conserved domains that facilitate DNA binding and/or protein- protein interactions.

One of these domains is the PHD finger, named plant homeodomain finger (PHDf) due to its association on a same polypeptide with a DNA-binding HD, that was originally identified by amino acid sequence identity between a maize homeobox transcription factor ZmHOX1a (Bellman & Werr (1992) EMBO J 11:3367-3374) and its *Arabidopsis* relative ATHAT3.1 (Schindler et al. (1993) Plant J 4: 137-150). The PHDf is a Cys₄-His-Cys₃ zinc-finger-like motif capable of chelating two zinc ions. PHDfs are found in nuclear proteins and are thought to be involved in chromatin-mediated transcriptional regulation (Halbach et al. (2000) Nucleic Acid Res 28(18): 3542-3550).

Transcriptional factors combining a PHDf and a HD are therefore named PHDf-HDs (Halbach *et al.* (2000) *supra).* In plants, such PHDf-HDs are further characterized by the presence of a leucine zipper (ZIP) upstream of the PHDf. Both domains (the ZIP and the PHDf) together form a highly conserved 180 amino acid region called the ZIP/PHDf domain (Halbach *et al.* (2000) *supra*).

Transgenic tobacco plants strongly overexpressing either of two maize PHDf-HD polypeptides (ZmHOX1a or ZmHOX1b) using a cauliflower mosaic virus 35S promoter combined with an omega enhancer, showed identical morphological changes: size reduction, adventitious root formation, and homeotic floral transformations (Uberlacker et al. (1996) Plant Cell 8: 349-362). Transgenic rice and tobacco plants strongly overexpressing Oryza sativa HAZ1 PHDF-HD polypeptide using cauliflower mosaic virus 35S promoter showed no abnormal growth or phenotypic change compared to wild types (Ito et al. (2004) Gene 331: 9-15).

In US patent 7,196,245, an *Arabidopsis thaliana* PHDf-HD polypeptide (identified as G416) was transformed into Arabidopsis, and shown to promote early flowering in the transgenic plants compared to control plants, with no impact on seed yield.

Surprisingly, it has now been found that increasing expression of a nucleic acid sequence encoding a PHDf-HD polypeptide gives plants having enhanced seed yield-related traits, relative to control plants.

According to one embodiment, there is provided a method for enhancing seed yield-related traits in plants, relative to control plants, comprising increasing expression of a nucleic acid sequence encoding a PHDf-HD polypeptide in a plant. The enhanced seed yield-related traits comprise one or more of: increased number of primary panicles, increased total seed yield per plant, increased number of (filled) seeds, increased thousand kernel weight (TKW), increased harvest index.

### Definitions

### Polypeptide(s)/Protein(s)

The terms "polypeptide" and "protein" are used interchangeably herein and refer to amino acids in a polymeric form of any length, linked together by peptide bonds.

### Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

### Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

### Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG^{®}-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1: Examples of conserved amino acid substitutions**

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

### Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

### Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

### Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

### Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

### Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:
1) DNA-DNA hybrids (Meinkoth and Wahl, Anal. Biochem., 138: 267-284, 1984):
   Tₘ= 81.5°C + 16.6xlog₁₀[Na⁺]^{a} + 0.41x%[G/C^{b}] - 500x[L^{c}]⁻¹ - 0.61x% formamide
2) DNA-RNA or RNA-RNA hybrids:
   Tm= 79.8 + 18.5 (log₁₀[Na⁺]^{a}) + 0.58 (%G/C^{b}) + 11.8 (%G/C^{b})² - 820/L^{c}
3) oligo-DNA or oligo-RNA^{d} hybrids:
   For <20 nucleotides: Tₘ= 2 (Iₙ)
   For 20-35 nucleotides: Tₘ= 22 + 1.46 (Iₙ)
^{a} or for other monovalent cation, but only accurate in the 0.01-0.4 M range.
^{b} only accurate for %GC in the 30% to 75% range.
^{c} L = length of duplex in base pairs.
^{d} oligo, oligonucleotide; Iₙ, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 µg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

### Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

### Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

### Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

### Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

### Operably linked

The term "operably linked" as used herein refers to a functional linkage between the promoter sequence and the gene of interest, such that the promoter sequence is able to initiate transcription of the gene of interest.

### Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a: Examples of constitutive promoters**

| **Gene Source** | **Reference** |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

### Ubiquitous promoter

A ubiquitous promoter is active in substantially all tissues or cells of an organism.

### Developmentally-requlated promoter

A developmentally-regulated promoter is active during certain developmental stages or in parts of the plant that undergo developmental changes.

### Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

### Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b: Examples of root-specific promoters**

| **Gene Source** | **Reference** |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2d, 2e, 2f. Further examples of seed-specific promoters are given in Qing Qu and Takaiwa (Plant Biotechnol. J. 2, 113-125, 2004), which disclosure is incorporated by reference herein as if fully set forth.

**Table 2c: Examples of seed-specific promoters**

| **Gene source** | **Reference** |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PR00117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175 rice RAB21 | WO 2004/070039 |
| PRO005 | WO 2004/070039 |
| PRO009 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d: examples of endosperm-specific promoters**

| **Gene source** | **Reference** |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e: Examples of embryo specific promoters:**

| **Gene source** | **Reference** |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f: Examples of aleurone-specific promoters:**

| **Gene source** | **Reference** |
|---|---|
| α-amylase (Amy32b) | Lanahan et al, Plant Cell 4:203-211, 1992; |
| | Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g.

**Table 2g: Examples of green tissue-specific promoters**

| **Gene** | **Expression** | **Reference** |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h: Examples of meristem-specific promoters**

| **Gene source** | **Expression pattern** | **Reference** |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

### Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

### Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

### Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

### Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

### Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

### Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an α-anomeric nucleic acid sequence. An α-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

### Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta^{®}; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

### Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
(a) the nucleic acid sequences encoding proteins useful in the methods of the invention, or
(b) genetic control sequence(s) which is operably linked with the nucleic acid sequence according to the invention, for example a promoter, or
(c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

### Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et al. (Nat. Biotechnol 14(6): 745-50, 1996) or Frame et al. (Plant Physiol 129(1): 13-22, 2002), Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

### T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

### TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

### Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8), and approaches exist that are generally applicable regardless of the target organism (Miller et al, Nature Biotechnol. 25, 778-785, 2007).

### Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

### Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

### Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

### Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

### Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

### Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., Olea spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., Poa spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

### Detailed description of the invention

Surprisingly, it has now been found that increasing expression in a plant of a nucleic acid sequence encoding a PHDf-HD polypeptide gives plants having enhanced seed yield-related traits, relative to control plants.

According to a first embodiment, the present invention provides a method for enhancing seed yield-related traits in plants relative to control plants, comprising increasing expression in a plant of a nucleic acid sequence encoding a PHDf-HD polypeptide, which PHDf-HD polypeptide comprises:
(i) a domain having at least 25%, 30%, 35%, 40%, 45%, 50%, 55%, 60%, 65%, 70%, 75%, 80%, 85%, 90%, 95%, 98%, 99% or more amino acid sequence identity to a leucine zipper/plant homeodomain finger (ZIP/PHDf) domain as represented by SEQ ID NO: 233; and
(ii) a domain having at least 25%, 30%, 35%, 40%, 45%, 50%, 55%, 60%, 65%, 70%, 75%, 80%, 85%, 90%, 95%, 98%, 99% or more amino acid sequence identity to a homeodomain (HD) as represented by SEQ ID NO: 234; and
(iii) wherein said PHDf-HD polypeptide, when used in the construction of a HD phylogenetic tree, such as the one depicted in Figure 13, clusters with the PHDf-HD group of polypeptides comprising the polypeptide sequence as represented by SEQ ID NO: 180, rather than with any other HD group; and/or wherein said PHDf-HD polypeptide has in increasing order of preference at least 30%, 35%, 40%, 45%, 50%, 55%, 60%, 65%, 70%, 75%, 80%, 85%, 90%, 95%, 98%, 99% or more sequence identity to the PHDf-HD polypeptide as represented by SEQ ID NO: 180 or to any of the polypeptide sequences given in Table D1 herein, and optionally selecting for plants having enhanced yield-related traits.

A preferred method for increasing expression of a nucleic acid sequence encoding a PHDf-HD polypeptide is by introducing and expressing in a plant a nucleic acid sequence encoding a PHDf-HD polypeptide.

Any reference hereinafter to a "protein useful in the methods of the invention" is taken to mean a PHDf-HD polypeptide as defined herein. Any reference hereinafter to a "nucleic acid sequence useful in the methods of the invention" is taken to mean a nucleic acid sequence capable of encoding such a PHDf-HD polypeptide. The nucleic acid sequence to be introduced into a plant (and therefore useful in performing the methods of the invention) is any nucleic acid sequence encoding the type of polypeptide, which will now be described, hereafter also named *"PHDf-HD* nucleic acid sequence" or *"PHDf-HD* gene".

A "PHDf-HD polypeptide" as defined herein refers to any polypeptide comprising: (i) a domain having at least 25%, 30%, 35%, 40%, 45%, 50%, 55%, 60%, 65%, 70%, 75%, 80%, 85%, 90%, 95%, 98%, 99% or more amino acid sequence identity to a leucine zipper/plant homeodomain finger (ZIP/PHDf) domain as represented by SEQ ID NO: 233; and (ii) a domain having at least 25%, 30%, 35%, 40%, 45%, 50%, 55%, 60%, 65%, 70%, 75%, 80%, 85%, 90%, 95%, 98%, 99% or more amino acid sequence identity to a homeodomain (HD) as represented by SEQ ID NO: 234.

Alternatively or additionally, a "PHDf-HD polypeptide" as defined herein refers to any polypeptide comprising: (i) a PHD domain as represented by PFAM00628; and (ii) an HD as represented by PFAM00046.

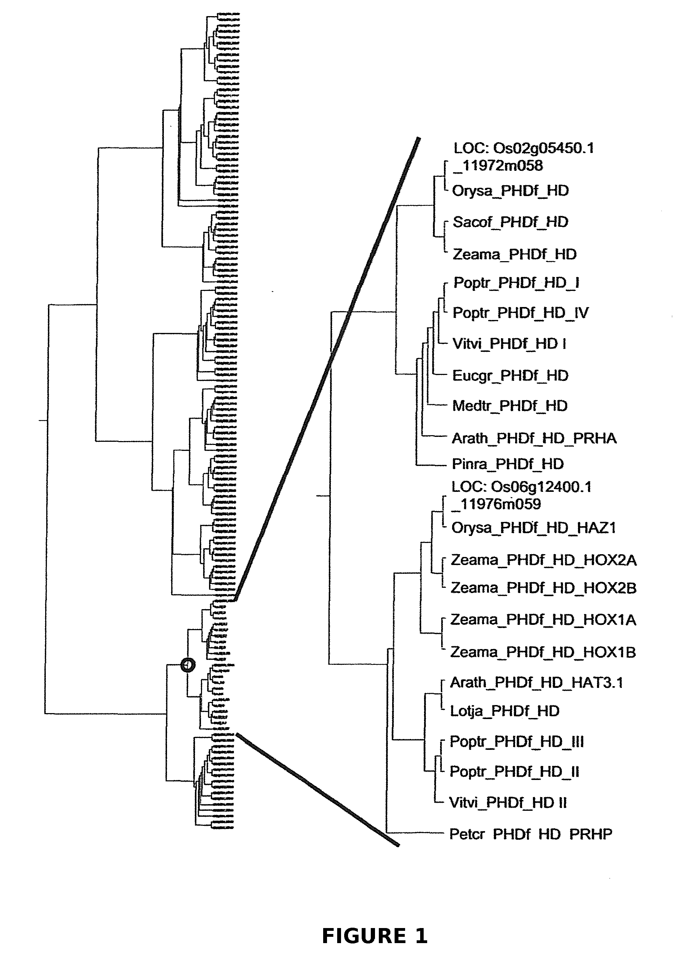

Additionally the "PHDf-HD polypeptide" as defined herein refers to any polypeptide sequence which when used in the construction of a HD phylogenetic tree, such as the one depicted in Figure 1, clusters with the PHDf-HD group of polypeptides comprising the polypeptide sequence as represented by SEQ ID NO: 180 or to any of the polypeptide sequences given in Table D1, rather than with any other HD group.

Alternatively or additionally, a "PHDf-HD polypeptide" as defined herein refers to any polypeptide having in increasing order of preference at least 25%, 30%, 35%, 40%, 45%, 50%, 55%, 60%, 65%, 70%, 75%, 80%, 85%, 90%, 95%, 98%, 99% or more amino acid sequence identity to the PHDf-HD polypeptide as represented by SEQ ID NO: 180 or to any of the polypeptide sequences given in Table D1 herein.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004), or Pfam (Bateman et al., Nucleic Acids Research 30(1): 276-280 (2002). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains may also be identified using routine techniques, such as by sequence alignment. Analysis of the polypeptide sequence of SEQ ID NO: 180 is presented below in Examples 2 and 4 herein. For example, a PHDf-HD polypeptide as represented by SEQ ID NO: 180 comprises a PHD finger with a Pfam entry PF00628, and a HD with Pfam entry PF00046.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid sequence or polypeptide sequence or over selected domains or conserved motif(s), using the programs mentioned above using the default parameters. Example 3 herein describes in Table D2 the percentage identity between the ZIP/PHDf as represented by SEQ ID NO: 233 and ZIP/PHDf of the PHDf-HD polypeptides listed in Table D1, and in Table D3 the percentage identity between the HD as represented by SEQ ID NO: 234 and the HD of the PHDf-HD polypeptides listed in Table D1 of Example 1.

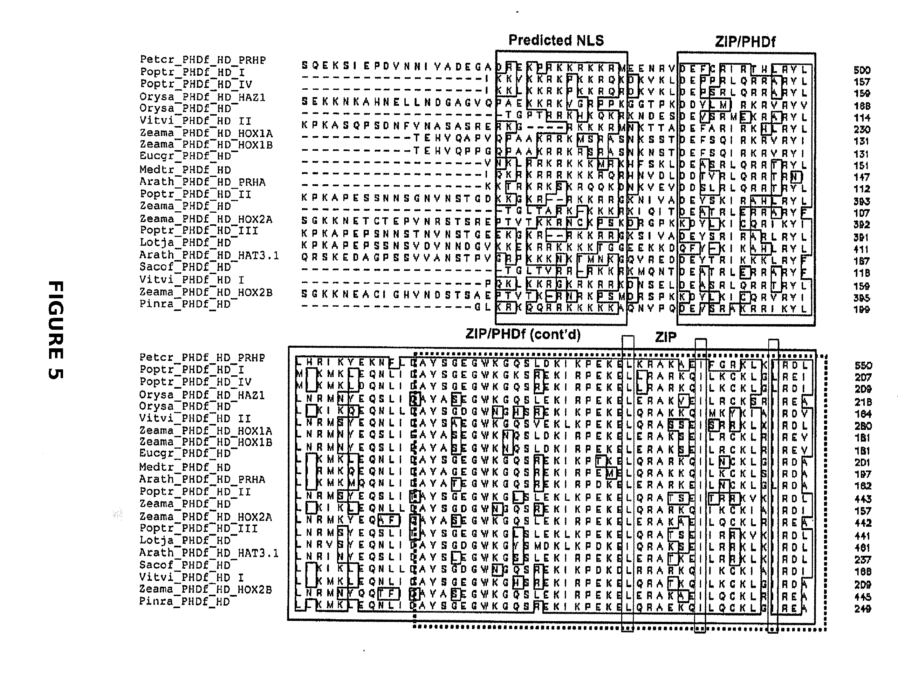

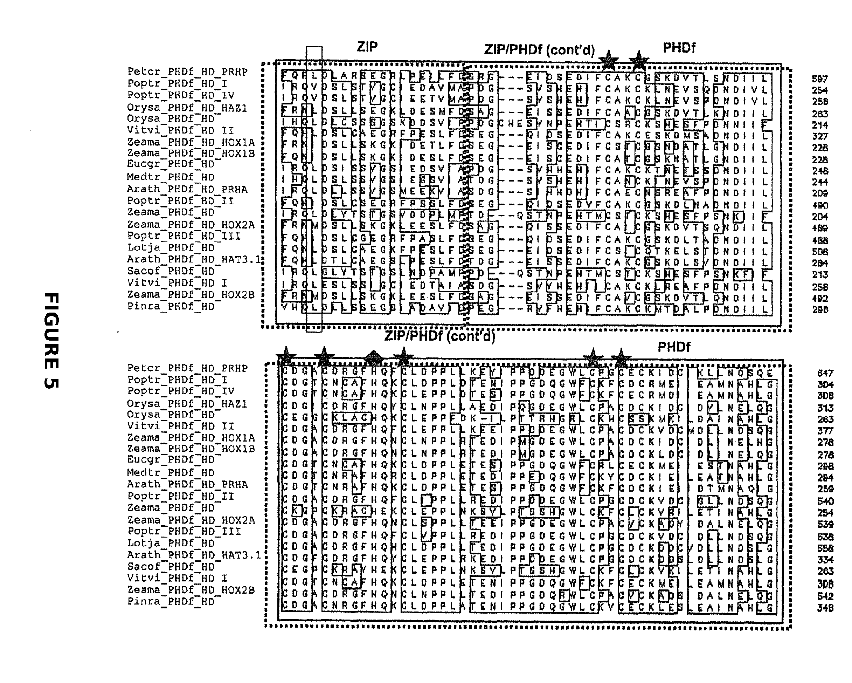

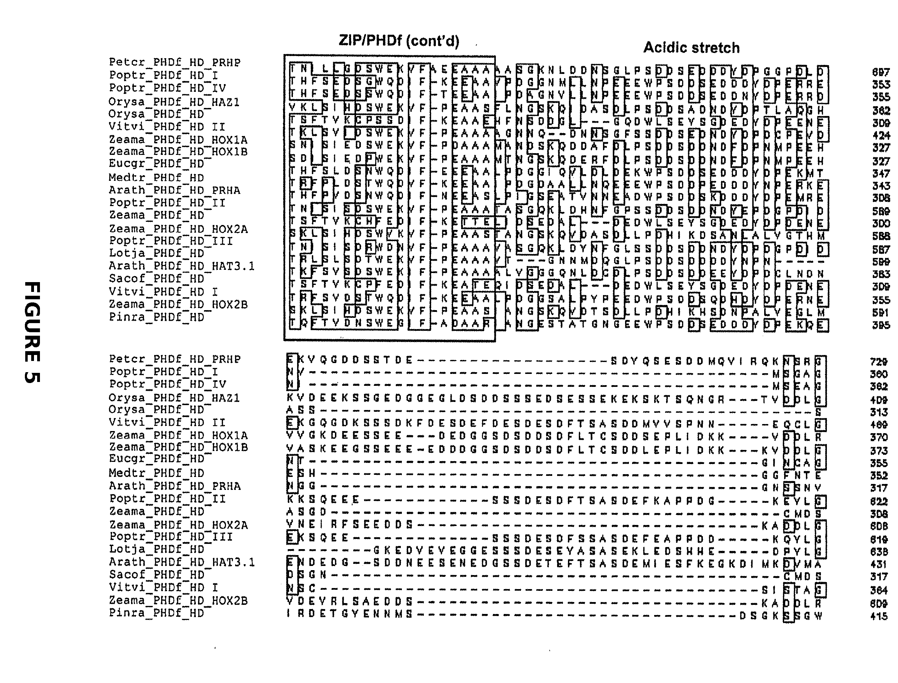

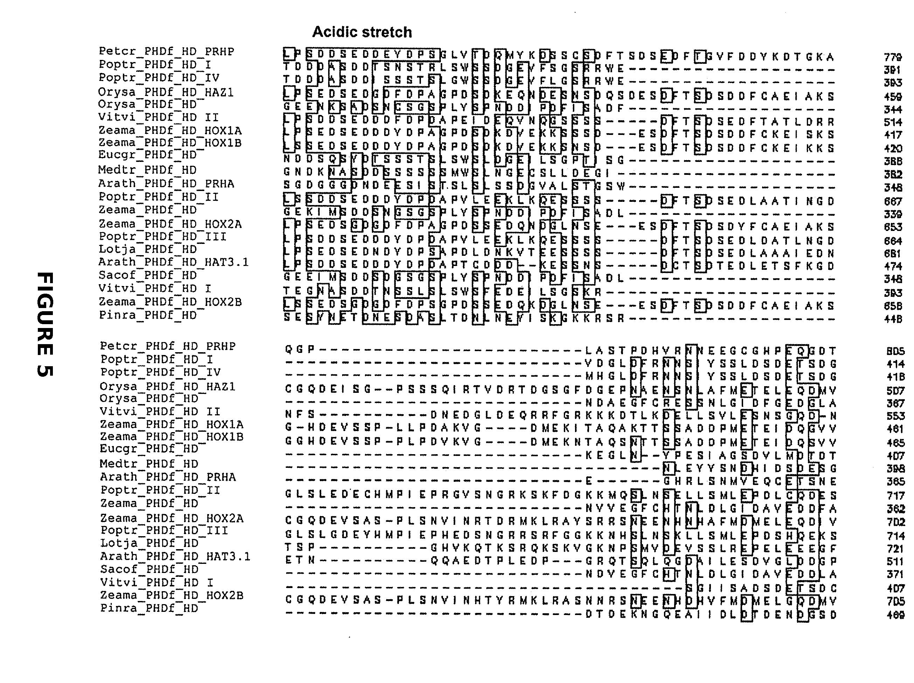

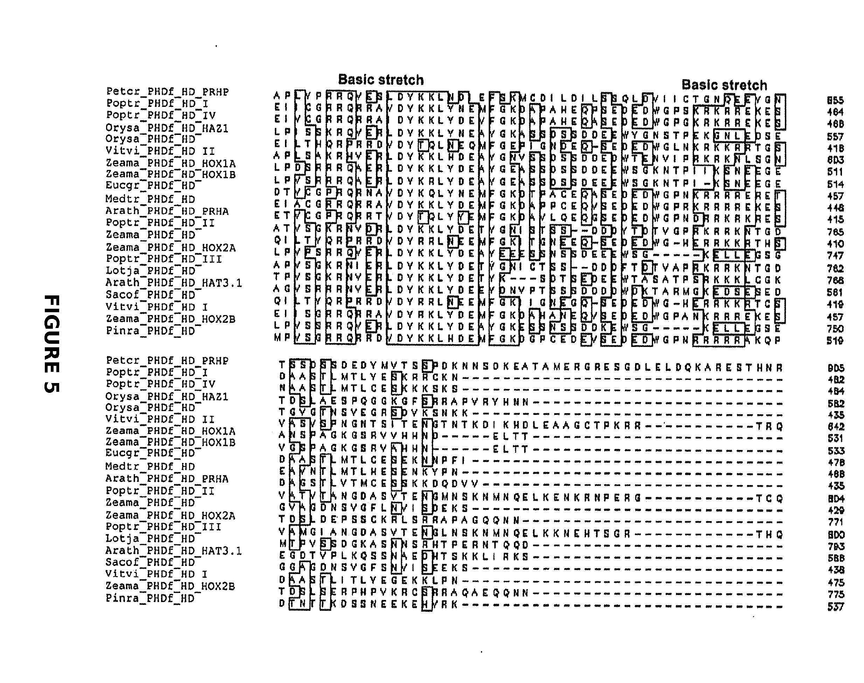

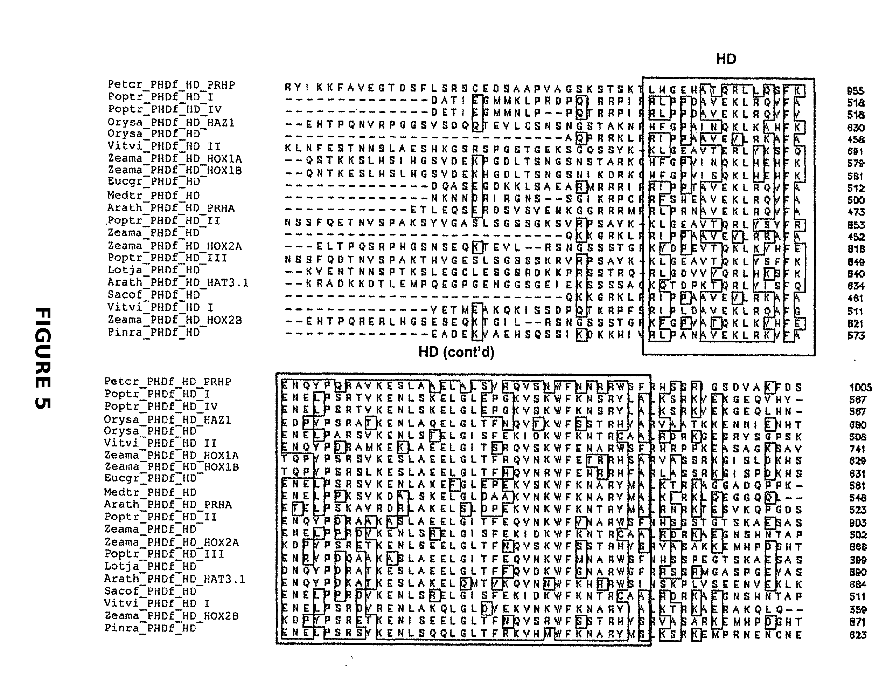

Furthermore, amino acid sequences enriched in basic (Lys and Arg) or acidic (Glu and Asp) amino acids, respectively called basic and acidic stretches, may also readily be identified simply by eye inspection (Figure 5). Alternatively primary amino acid composition (in %) may be calculated to determine if a polypeptide domain is rich in specific amino acids using software programs from the ExPASy server, in particular the ProtParam tool (Gasteiger E et al. (2003) ExPASy: the proteomics server for in-depth protein knowledge and analysis. Nucleic Acids Res 31:3784-3788). The composition of the protein of interest may then be compared to the average amino acid composition (in %) in the Swiss-Prot Protein Sequence data bank.

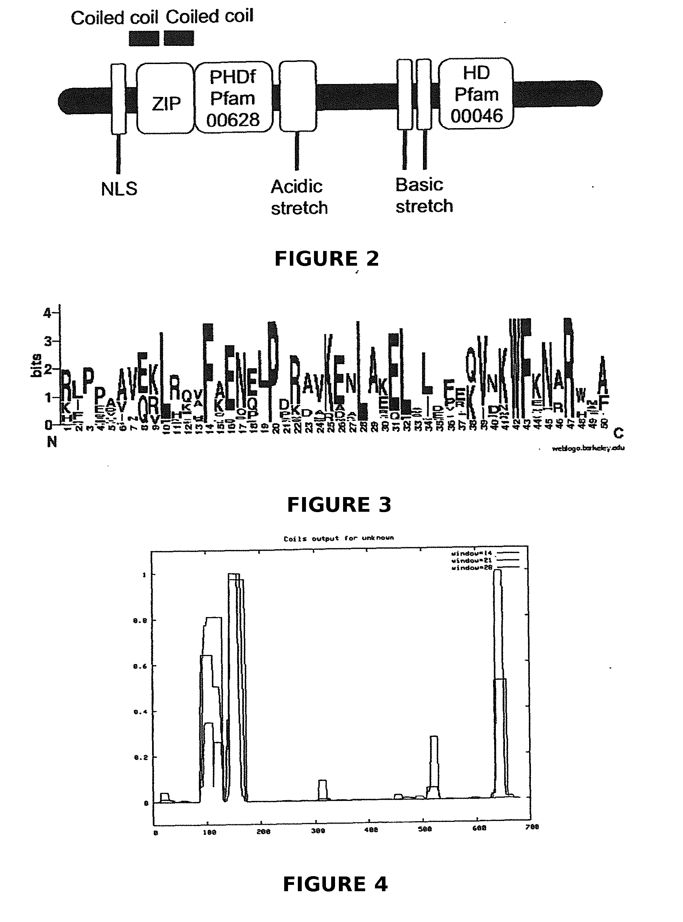

Coiled coils are important to identify for protein-protein interactions, such as oligomerization, either of identical proteins, of proteins of the same family, or of unrelated proteins. A PHDf-HD polypeptide presents at least one predicted coiled coil region. Recently much progress has been made in computational prediction of coiled coils from sequence data. Among algorithms well known to a person skilled in the art are available at the ExPASy Proteomics tools COILS, PAIRCOIL, PAIRCOIL2, MULTICOIL, or MARCOIL, hosted by the Swiss Institute for Bioinformatics. In Example 5 and Figure 4, are shown respectively the numerical and graphical results of SEQ ID NO: 180 as produced by the COILS algorithm analysis. Two N-terminal predicted coiled coil domains are identified in a PHDf-HD polypeptide sequence as represented by SEQ ID NO: 180, with a strong probability. A C-terminal coiled coil is also predicted, with a lower probability.

The task of protein subcellular localisation prediction is important and well studied. Knowing a protein's localisation helps elucidate its function. Experimental methods for protein localization range from immunolocalization to tagging of proteins using green fluorescent protein (GFP). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP and others. The identification of subcellular localisation of the polypeptide of the invention is shown in Example 6. In particular SEQ ID NO: 180 of the present invention is assigned to the nuclear compartment of eucaryotic cells.

Furthermore, PHDf-HD polypeptides useful in the methods of the present invention (at least in their native form) typically, but not necessarily, have transcriptional regulatory activity and capacity to interact with other proteins. Therefore, PHDf-HD polypeptides with reduced transcriptional regulatory activity, without transcriptional regulatory activity, with reduced protein-protein interaction capacity, or with no protein-protein interaction capacity, may equally be useful in the methods of the present invention. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). To determine the DNA binding activity of PHDf-HD polypeptides, several assays are available, such as DNA binding gel-shift assays (or gel retardation assays; Korfhage et al. (1994) Plant C 6: 695-708), in vitro DNA binding assays (Schindler et al. (1993) Plant J 4(1): 137-150), or transcriptional activation of PHDf-HD polypeptides in yeast, animal and plant cells (Halbach et al. (2000) Nucleic Acid Res 28(18): 3542-3550). Specific DNA binding sequences can be determined using the random oligonucleotide selection technique (Viola & Gonzalez (May 26, 2007) Biochemistry ).

The present invention is illustrated by transforming plants with the nucleic acid sequence represented by SEQ ID NO: 179, encoding the polypeptide sequence of SEQ ID NO: 180. However, performance of the invention is not restricted to these sequences; the methods of the invention may advantageously be performed using any nucleic acid sequence encoding PHDf-HD or PHDf-HD polypeptide as defined herein.

Examples of nucleic acid sequences encoding PHDf-HD polypeptides are given in Table D1 of Example 1 herein. Such nucleic acid sequences are useful in performing the methods of the invention. The polypeptide sequences given in Table D1 of Example 1 are example sequences of orthologues and paralogues of the PHDf-HD polypeptide represented by SEQ ID NO: 180, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table D1 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 179 or SEQ ID NO: 180, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues. Any sequence clustering within the group comprising SEQ ID NO: 180 (encircled in Figure 1) would be considered to fall within the aforementioned definition of a PHDf-HD polypeptide, and would be considered suitable for use in the methods of the invention.

Nucleic acid variants may also be useful in practising the methods of the invention. Examples of such variants include nucleic acid sequences encoding homologues and derivatives of any one of the polypeptide sequences given in Table D1 of Example 1, the terms "homologue" and "derivative" being as defined herein. Also useful in the methods of the invention are nucleic acid sequences encoding homologues and derivatives of orthologues or paralogues of any one of the polypeptide sequences given in Table D1 of Example 1. Homologues and derivatives useful in the methods of the present invention have substantially the same biological and functional activity as the unmodified protein from which they are derived.

Further nucleic acid variants useful in practising the methods of the invention include portions of nucleic acid sequences encoding PHDf-HD polypeptides, nucleic acid sequences hybridising to nucleic acid sequences encoding PHDf-HD polypeptides, splice variants of nucleic acid sequences encoding PHDf-HD polypeptides, allelic variants of nucleic acid sequences encoding PHDf-HD polypeptides and variants of nucleic acid sequences encoding PHDf-HD polypeptides obtained by gene shuffling. The terms hybridising sequence, splice variant, allelic variant and gene shuffling are as described herein.

Nucleic acid sequences encoding PHDf-HD polypeptides need not be full-length nucleic acid sequences, since performance of the methods of the invention does not rely on the use of full-length nucleic acid sequences. According to the present invention, there is provided a method for enhancing seed yield-related traits in plants, comprising introducing and expressing in a plant a portion of any one of the nucleic acid sequences given in Table D1 of Example 1, or a portion of a nucleic acid sequence encoding an orthologue, paralogue or homologue of any of the polypeptide sequences given in Table D1 of Example 1.

A portion of a nucleic acid sequence may be prepared, for example, by making one or more deletions to the nucleic acid sequence. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a PHDf-HD polypeptide as defined herein, and have substantially the same biological activity as the polypeptide sequences given in Table D1 of Example 1. Preferably, the portion is a portion of any one of the nucleic acid sequences given in Table D1 of Example 1, or is a portion of a nucleic acid sequence encoding an orthologue or paralogue of any one of the polypeptide sequences given in Table D1 of Example 1. Preferably the portion is, in increasing order of preference at least 600, 800, 1000, 1200, 1400, 1600, 1800, 2000, 2200, 2400, 2600, 2800 consecutive nucleotides in length, the consecutive nucleotides being of any one of the nucleic acid sequences given in Table D1 of Example 1, or of a nucleic acid sequence encoding an orthologue or paralogue of any one of the polypeptide sequences given in Table D1 of Example 1. Most preferably the portion is a portion of the nucleic acid sequence of SEQ ID NO: 179. Preferably, the portion encodes a polypeptide sequence which when used in the construction of a HD phylogenetic tree, such as the one depicted in Figure 1, clusters with the group of PHDf-HD polypeptides comprising the polypeptide sequence represented by SEQ ID NO: 180 rather than with any other HD group.

Another nucleic acid sequence variant useful in the methods of the invention is a nucleic acid sequence capable of hybridising, under reduced stringency conditions, preferably under stringent conditions, with a nucleic acid sequence encoding a PHDf-HD polypeptide as defined herein, or with a portion as defined herein.

According to the present invention, there is provided a method for enhancing seed yield-related traits comprising introducing and expressing in a plant a nucleic acid sequence capable of hybridizing to any one of the nucleic acid sequences given in Table D1 of Example 1, or comprising introducing and expressing in a plant a nucleic acid sequence capable of hybridising to a nucleic acid sequence encoding an orthologue, paralogue or homologue of any of the nucleic acid sequences given in Table D1 of Example 1.

Hybridising sequences useful in the methods of the invention encode a PHDf-HD polypeptide as defined herein, and have substantially the same biological activity as the polypeptide sequences given in Table D1 of Example 1. Preferably, the hybridising sequence is capable of hybridising to any one of the nucleic acid sequences given in Table D1 of Example 1, or to a portion of any of these sequences, a portion being as defined above, or wherein the hybridising sequence is capable of hybridising to a nucleic acid sequence encoding an orthologue or paralogue of any one of the polypeptide sequences given in Table D1 of Example 1. Most preferably, the hybridising sequence is capable of hybridising to a nucleic acid sequence as represented by SEQ ID NO: 179 or to a portion thereof.

Preferably, the hybridising sequence encodes a polypeptide sequence which when used in the construction of a HD phylogenetic tree, such as the one depicted in Figure 1, clusters with the group of PHDf-HD polypeptides comprising the polypeptide sequence represented by SEQ ID NO: 180 rather than with any other HD group.

Another nucleic acid sequence variant useful in the methods of the invention is a splice variant encoding a PHDf-HD polypeptide as defined hereinabove, a splice variant being as defined herein.

According to the present invention, there is provided a method for enhancing seed yield-related traits, comprising introducing and expressing in a plant a splice variant of any one of the nucleic acid sequences given in Table D1 of Example 1, or a splice variant of a nucleic acid sequence encoding an orthologue, paralogue or homologue of any of the polypeptide sequences given in Table D1 of Example 1.

Preferred splice variants are splice variants of a nucleic acid sequence represented by SEQ ID NO: 179, or a splice variant of a nucleic acid sequence encoding an orthologue or paralogue of SEQ ID NO: 180. Preferably, the polypeptide sequence encoded by the splice variant, when used in the construction of a HD phylogenetic tree, such as the one depicted in Figure 1, clusters with the group of PHDf-HD polypeptides comprising the polypeptide sequence represented by SEQ ID NO: 180 rather than with any other HD group.

Another nucleic acid sequence variant useful in performing the methods of the invention is an allelic variant of a nucleic acid sequence encoding a PHDf-HD polypeptide as defined hereinabove, an allelic variant being as defined herein.

According to the present invention, there is provided a method for enhancing seed yield-related traits, comprising introducing and expressing in a plant an allelic variant of any one of the nucleic acid sequences given in Table D1 of Example 1, or comprising introducing and expressing in a plant an allelic variant of a nucleic acid sequence encoding an orthologue, paralogue or homologue of any of the polypeptide sequences given in Table D1 of Example 1. The allelic variants useful in the methods of the present invention have substantially the same biological activity as the polypeptide of SEQ ID NO: 180 and any of the polypeptide sequences depicted in Table D1 of Example 1. Allelic variants exist in nature, and encompassed within the methods of the present invention is the use of these natural alleles. Preferably, the allelic variant is an allelic variant of SEQ ID NO: 179 or an allelic variant of a nucleic acid sequence encoding an orthologue or paralogue of SEQ ID NO: 180. Preferably, the polypeptide sequence encoded by the allelic variant, when used in the construction of a HD phylogenetic tree, such as the one depicted in Figure 1, clusters with the PHDf-HD polypeptides comprising the polypeptide sequence represented by SEQ ID NO: 180 rather than with any other HD group.

Gene shuffling or directed evolution may also be used to generate variants of nucleic acid sequences encoding PHDf-HD polypeptides as defined above; the term "gene shuffling" being as defined herein.

According to the present invention, there is provided a method for enhancing yield-related traits in plants, preferably enhancing seed yield-related traits, comprising introducing and expressing in a plant a variant of any one of the nucleic acid sequences given in Table D1 of Example 1, or comprising introducing and expressing in a plant a variant of a nucleic acid sequence encoding an orthologue, paralogue or homologue of any of the polypeptide sequences given in Table D1 of Example 1, which variant nucleic acid sequence is obtained by gene shuffling.

Advantageously, the present invention provides hitherto unknown PHDf-HD nucleic acid and polypeptide sequences.

According to a further embodiment of the present invention, there is provided an isolated nucleic acid molecule comprising:
(i) a nucleic acid represented by SEQ ID NO: 242;
(ii) a nucleic acid or fragment thereof that is complementary to SEQ ID NO: 242;
(iii) a nucleic acid encoding an PHDf-HD polypeptide having, in increasing order of preference, at least 70%, 75%, 80%, 85%, 90%, 95%, 96%, 97%, 98%, 99% or more sequence identity to the amino acid sequence given in SEQ ID NO: 243;
(iv) a nucleic acid capable of hybridizing under stringent conditions to any one of the nucleic acids given in (i), (ii) or (iii) above.

According to a further embodiment of the present invention, there is therefore provided an isolated polypeptide comprising:
(i) a nucleic acid represented by SEQ ID NO: 242;
(ii) a nucleic acid or fragment thereof that is complementary to SEQ ID NO: 242;
(iii) a nucleic acid encoding an PHDf-HD polypeptide having, in increasing order of preference, at least 70%, 75%, 80%, 85%, 90%, 95%, 96%, 97%, 98%, 99% or more sequence identity to the amino acid sequence given in SEQ ID NO: 243;
(iv) a nucleic acid capable of hybridizing under stringent conditions to any one of the nucleic acids given in (i), (ii) or (iii) above.

Preferably, the polypeptide sequence encoded by the variant nucleic acid sequence obtained by gene shuffling, when used in the construction of a HD phylogenetic tree, such as the one depicted in Figure 1, clusters with the group of PHDf-HD polypeptides comprising the polypeptide sequence represented by SEQ ID NO: 180 rather than with any other HD group.

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acid sequences encoding PHDf-HD polypeptides may be derived from any natural or artificial source. The nucleic acid sequence may be modified from its native form in composition and/or genomic environment through deliberate human manipulation. Preferably the PHDf-HD polypeptide-encoding nucleic acid sequence is from a plant, further preferably from a monocotyledonous plant, more preferably from the family Poaceae, most preferably the nucleic acid sequence is from *Oryza sativa.*

Performance of the methods of the invention gives plants having increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing plant seed yield , relative to control plants, which method comprises increasing expression in a plant of a nucleic acid sequence encoding a PHDf-HD polypeptide as defined herein.

Since the transgenic plants according to the present invention have enhanced seed yield-related traits it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced (early) vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others. The growth rate as defined herein is not taken to mean earlier flowering.

According to a preferred feature of the present invention, performance of the methods of the invention gives plants having an increased growth rate relative to control plants. Therefore, according to the present invention, there is provided a method for increasing the growth rate of plants, which method comprises increasing expression in a plant of a nucleic acid sequence encoding a PHDf-HD polypeptide as defined herein.

Enhanced seed yield-related traits occur whether the plant is under non-stress conditions or whether the plant is exposed to various stresses compared to control plants grown under comparable conditions. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes, and insects. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions having enhanced seed yield-related traits, relative to control plants grown under comparable conditions. Therefore, according to the present invention, there is provided a method for enhancing seed yield-related traits in plants grown under non-stress conditions or under mild drought conditions, which method comprises modulating, preferably increasing, expression in a plant of a nucleic acid sequence encoding a PHDf-HD polypeptide. Plants with optimal growth conditions, cultivated under non-stress conditions, typically yield in increasing order of preference at least 90%, 87%, 85%, 83%, 80%, 77% or 75% of the average production of such plant in a given environment. Average production may be calculated on harvest and/or season basis on a given location. Persons skilled in the art are aware of average yield productions of a crop.

Performance of the methods according to the present invention results in plants grown under abiotic stress conditions having enhanced seed yield-related traits, relative to control plants grown under comparable stress conditions. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. Since diverse environmental stresses activate similar pathways, the exemplification of the present invention with drought stress should not be seen as a limitation to drought stress, but more as a screen to indicate the involvement of PHDf-HD polypeptides as defined above, in enhancing seed yield-related traits, relative to control plants grown in comparable stress conditions, in abiotic stresses in general.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, MgCl2, CaCl2, amongst others.

Performance of the methods of the invention gives plants having enhanced seed yield-related traits, under abiotic stress conditions relative to control plants grown in comparable stress conditions. Therefore, according to the present invention, there is provided a method for enhancing seed yield-related traits, in plants grown under abiotic stress conditions, which method comprises increasing expression in a plant of a nucleic acid sequence encoding a PHDf-HD polypeptide. According to one aspect of the invention, the abiotic stress is an osmotic stress, selected from one or more of the following: water stress, salt stress, oxidative stress and ionic stress.

Another example of abiotic environmental stress is the reduced availability of one or more nutrients that need to be assimilated by the plants for growth and development. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor for crop plant growth and production (Frink et al. (1999) Proc Natl Acad Sci USA 96(4): 1175-1180), and has as well a major impact on protein accumulation and amino acid composition. Therefore, of great interest are crop plants with enhanced yield-related traits, preferably enhanced seed yield-related traits, when grown under nitrogen-limiting conditions.

Performance of the methods of the invention gives plants grown under conditions of nutrient deficiency, particularly under conditions of nitrogen deficiency, enhanced seed yield-related traits, relative to control plants grown under comparable conditions. Therefore, according to the present invention, there is provided a method for enhancing seed yield-related traits in plants grown under conditions of nutrient deficiency, which method comprises increasing expression in a plant of a nucleic acid sequence encoding a PHDf-HD polypeptide. Nutrient deficiency may result from a lack or excess of nutrients such as nitrogen, phosphates and other phosphorous-containing compounds, potassium, calcium, cadmium, magnesium, manganese, iron and boron, amongst others.

The present invention encompasses plants or parts thereof (including seeds) or cells thereof obtainable by the methods according to the present invention. The plants or parts thereof or cells thereof comprise a nucleic acid transgene encoding a PHDf-HD polypeptide as defined above.

The invention also provides genetic constructs and vectors to facilitate introduction and/or increased expression in plants of nucleic acid sequences encoding PHDf-HD polypeptides. The gene constructs may be inserted into vectors, which may be commercially available, suitable for transforming into plants and for expression of the gene of interest in the transformed cells. The invention also provides use of a gene construct as defined herein in the methods of the invention.

More specifically, the present invention provides a construct comprising:
(a) a nucleic acid sequence encoding a PHDf-HD polypeptide as defined above;
(b) one or more control sequences capable of increasing, expression of the nucleic acid sequence of (a) comprising at least a GOS2 promoter; and optionally
(c) a transcription termination sequence.

Preferably, the nucleic acid sequence encoding a PHDf-HD polypeptide is as defined above. The term "control sequence" and "termination sequence" are as defined herein.

One of the control sequences of the construct according to the invention is a constitutive promoter GOS2 isolated from a plant genome. Preferably, the GOS2 promoteris from rice, more preferably a GOS2 promoter as represented by SEQ ID NO: 235.

Plants are transformed with a vector comprising any of the nucleic acid sequences described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to modulate, preferably increase, expression of the nucleic acid sequence. A constitutive promoter is particularly useful in the methods, preferably a constitutive promoter isolated from a plant genome. The plant constitutive promoter drives expression of a coding sequence at a level that is in all instances below that obtained under the control of a 35S CaMV promoter.

Other organ-specific promoters, for example for preferred expression in leaves, stems, tubers, meristems, seeds (embryo and/or endosperm), are useful in performing the methods of the invention. See the "Definitions" section herein for definitions of the various promoter types.

It should be clear that the applicability of the present invention is not restricted to the PHDf-HD polypeptide-encoding nucleic acid sequence represented by SEQ ID NO: 179, nor is the applicability of the invention restricted to expression of a PHDf-HD polypeptide-encoding nucleic acid sequence when driven by a constitutive promoter.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acid sequences, it is advantageous to use marker genes (or reporter genes). Therefore, the genetic construct may optionally comprise a selectable marker gene. Selectable markers are described in more detail in the "definitions" section herein.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having enhanced seed yield-related traits, relative to control plants, comprising introduction and expression in a plant of any nucleic acid sequence encoding a PHDf-HD polypeptide as defined hereinabove.

More specifically, the present invention provides a method for the production of transgenic plants having enhanced seed yield-related traits, relative to control plants, which method comprises:
(i) introducing and expressing in a plant, plant part, or plant cell a nucleic acid sequence encoding a PHDf-HD polypeptide, under the control of plant constitutive promoter; and
(ii) cultivating the plant cell under conditions promoting plant growth and development.

The nucleic acid sequence of (i) may be any of the nucleic acid sequences capable of encoding a PHDf-HD polypeptide as defined herein.

The nucleic acid sequence may be introduced directly into a plant cell or into the plant itself (including introduction into a tissue, organ or any other part of a plant). According to a preferred feature of the present invention, the nucleic acid sequence is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid sequence encoding a PHDf-HD polypeptide as defined hereinabove, operably linked to a plant constitutive promoter. Preferred host cells according to the invention are plant cells. Host plants for the nucleic acid sequences or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants, which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant comprising an isolated nucleic acid sequence encoding a PHDf-HD (as defined hereinabove) operably linked to a plant constitutive promoter, such as, but not limited to seeds, leaves, fruits, flowers, stems, rhizomes, tubers and bulbs. Also disclosed are products derived, preferably directly derived, from a harvestable part of such a plant, such as dry pellets or powders, oil, fat and fatty acids, starch or proteins.

According to the invention, the expression is increased. Methods for increasing expression of nucleic acid sequences or genes, or gene products, are well documented in the art and examples are provided in the definitions section.

As mentioned above, a preferred method for increasing expression of a nucleic acid sequence encoding a PHDf-HD polypeptide is by introducing and expressing in a plant a nucleic acid sequence encoding a PHDf-HD polypeptide; however the effects of performing the method, i.e. enhancing seed yield-related traits, may also be achieved using other well known techniques, including but not limited to T-DNA activation tagging, TILLING, homologous recombination. A description of these techniques is provided in the definitions section.

The present invention also encompasses use of nucleic acid sequences encoding PHDf-HD polypeptides as described herein and use of these PHDf-HD polypeptides in enhancing any of the aforementioned seed yield-related traits in plants.

Nucleic acid sequences encoding PHDf-HD polypeptide described herein, or the PHDf-HD polypeptides themselves, may find use in breeding programmes in which a DNA marker is identified that may be genetically linked to a PHDf-HD polypeptide-encoding gene. The genes/ nucleic acid sequences, or the PHDf-HD polypeptides themselves may be used to define a molecular marker. This DNA or protein marker may then be used in breeding programmes to select plants having enhanced yield-related traits, preferably enhanced seed yield-related traits, as defined hereinabove in the methods of the invention.

Allelic variants of a gene/nucleic acid sequence encoding a PHDf-HD polypeptide may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give enhanced yield-related traits. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acid sequences encoding PHDf-HD polypeptides may also be used as probes for genetically and physically mapping the genes that they are a part of, and as markers for traits linked to those genes. Such information may be useful in plant breeding in order to develop lines with desired phenotypes. Such use of nucleic acid sequences encoding a PHDf-HD polypeptide requires only a nucleic acid sequence of at least 15 nucleotides in length. The nucleic acid sequences encoding a PHDf-HD polypeptide may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acid sequences encoding a PHDf-HD polypeptide. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid sequence encoding a PHDf-HD polypeptide in the genetic map previously obtained using this population (Botstein et al. (1980) Am. J. Hum. Genet. 32: 314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid sequence probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid sequence probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid sequence amplification-based methods for genetic and physical mapping may be carried out using the nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic acid sequence Res. 18:3671), Radiation Hybrid Mapping (Walter et al. (1997) Nat. Genet. 7:22-28) and Happy Mapping (Dear and Cook (1989) Nucleic acid sequence Res. 17:6795-6807). For these methods, the sequence of a nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced seed yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

### Description of figures

The present invention will now be described with reference to the following figures in which:

### I. Plant homeodomain finger-homeodomain (PHDf-HD) polypeptide

**Figure 1** represents a neighbour-joining tree constructed after an alignment of all the transcription factors belonging to the HD family (downloaded from the rice TFDB database hosted at the server of the University of Potsdam) and all of the polypeptide sequences of Table D1 (when full length), performed using the Clustal algorithm (1.83) of progressive alignment, using default values. The group of interest, comprising the two rice paralogs (SEQ ID NO: 180 or Os02g05450.1, and SEQ ID NO: 202 or Os06g12400.1) has been circled. Any polypeptide falling within this HD group (after a new multiple alignment step as described hereinabove) is considered to be useful in performing the methods of the invention as described herein.
**Figure 2** represents a cartoon of a PHDf-HD polypeptide as represented by SEQ ID NO: 180, which comprises one or more of the following features: a predicted nuclear localisation signal (NLS), a leucine zipper (ZIP), a PHD finger (PHDf, Pfam00628), an acidic stretch, two basic stretches, a homeodomain (HD, Pfam00046).
**Figure 3** shows the sequence logo of the homeodomain (HD) of the PHDf-HD polypeptides of Table D1, where the overall height of the stack indicates the sequence conservation at that position, while the height of symbols within the stack indicates the relative frequency of each amino or nucleic acid at that position. The HD as represented by SEQ ID NO: 234, and comprised in SEQ ID NO: 180, is in accordance with the sequence logo as represented in this figure.
**Figure 4** shows the graphical output of the COILS algorithm predicting two coiled coil domains in the N-terminal half of the polypeptide as represented by SEQ ID NO: 180. The X axis represents the amino acid residue coordinates, the Y axis the probability (ranging from 0 to 1) that a coiled coil domain is present, and the three lines, the three windows (14, 21, 28) examined.
**Figure 5** shows a CLUSTAL W (1;83) multiple sequence alignment of PHDf-HD polypeptides from Table D1 (when full length), where a number of features are identified. From the N-terminus to the C-terminus of the polypeptides are: (i) a predicted nuclear localisation signal (NLS); (ii) a leucine zipper (ZIP), with four heptads (boxed, in which usually a leucine (occasionally an isoleucine, a valine, or a methionine) appears every seventh amino acid); (iii) a PHD finger (PHDf), with the typical C4HC3 (four cysteines, one histidine, three cysteines) with a characteristic cysteine spacing; (iv) an acidic stretch (rich in acidic amino acids D and E); (v) basic stretches (rich in basic amino acids K and R); (vi) a homeodomain (HD).

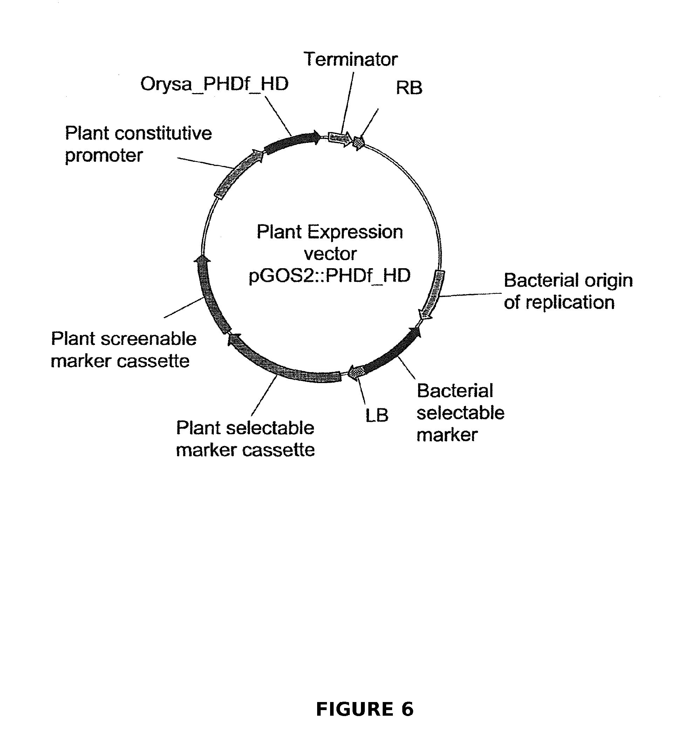

**Figure 6** shows the binary vector for modulated, preferably increased, expression in *Oryza sativa* of a nucleic acid sequence encoding a PHDf-HD polypeptide under the control of a rice GOS2 promoter (pGOS2)

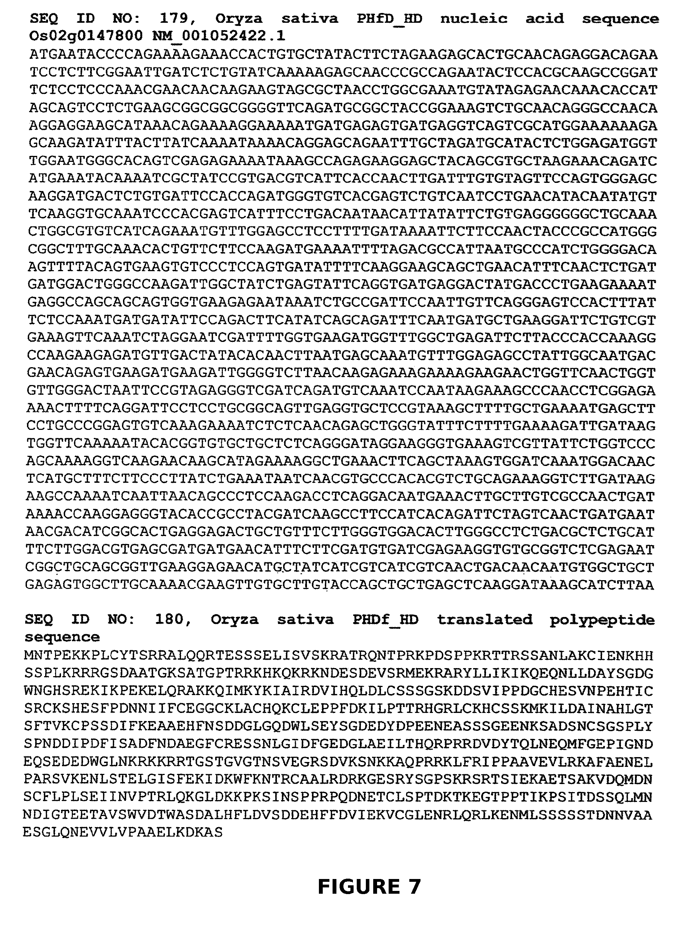

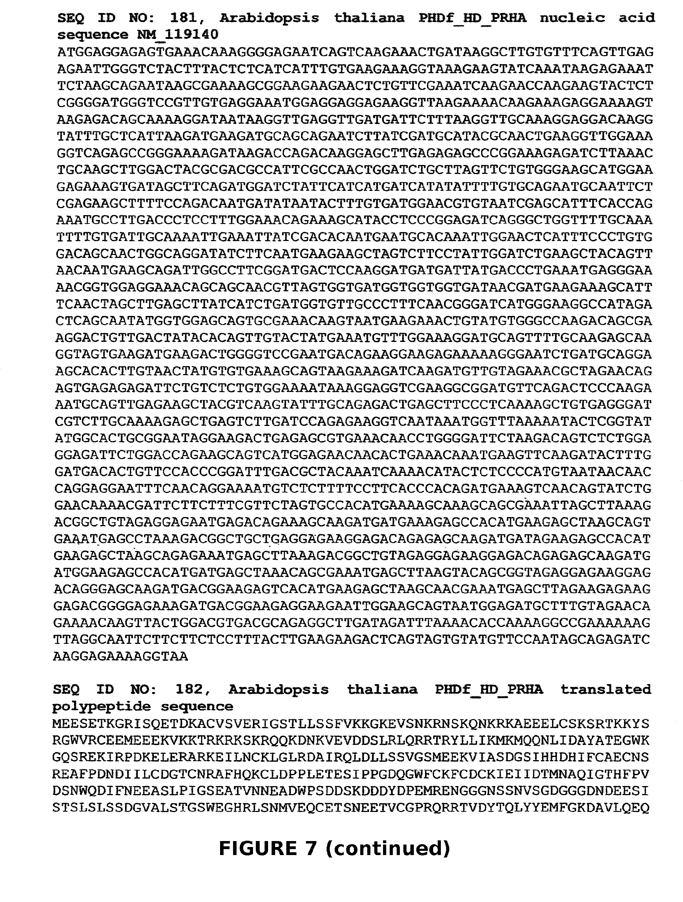

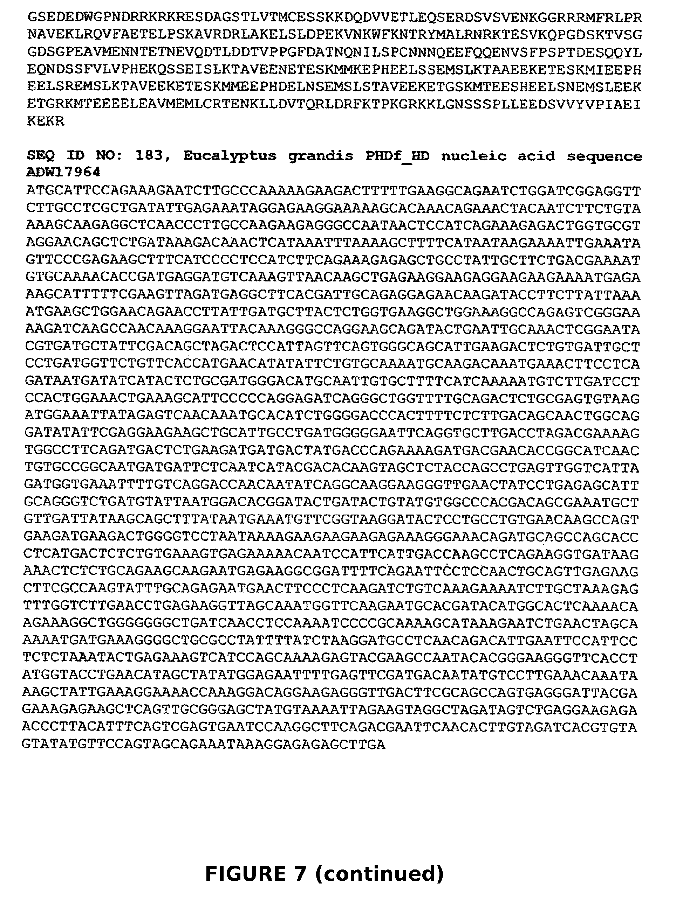

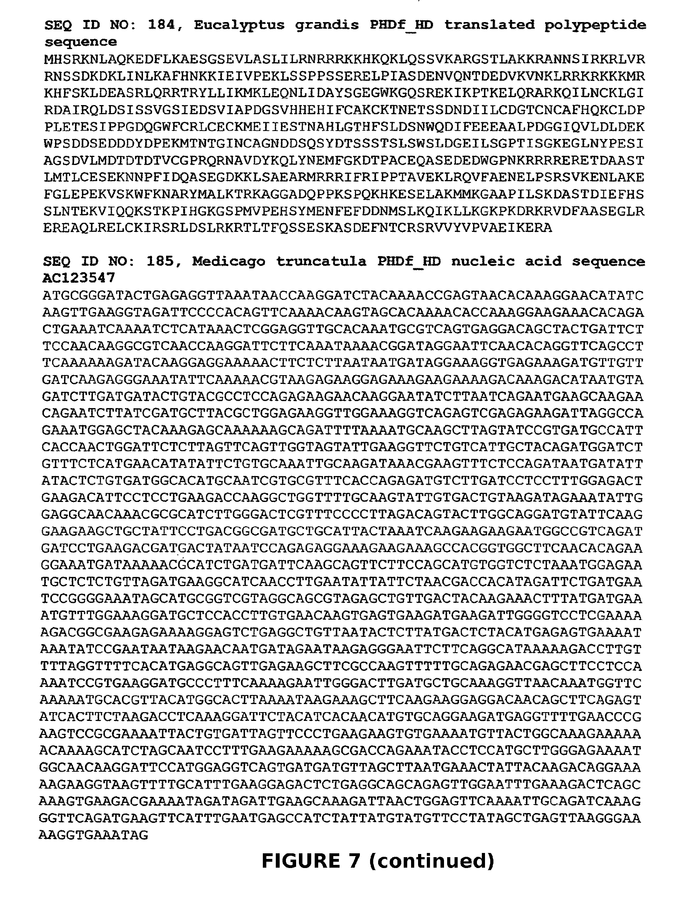

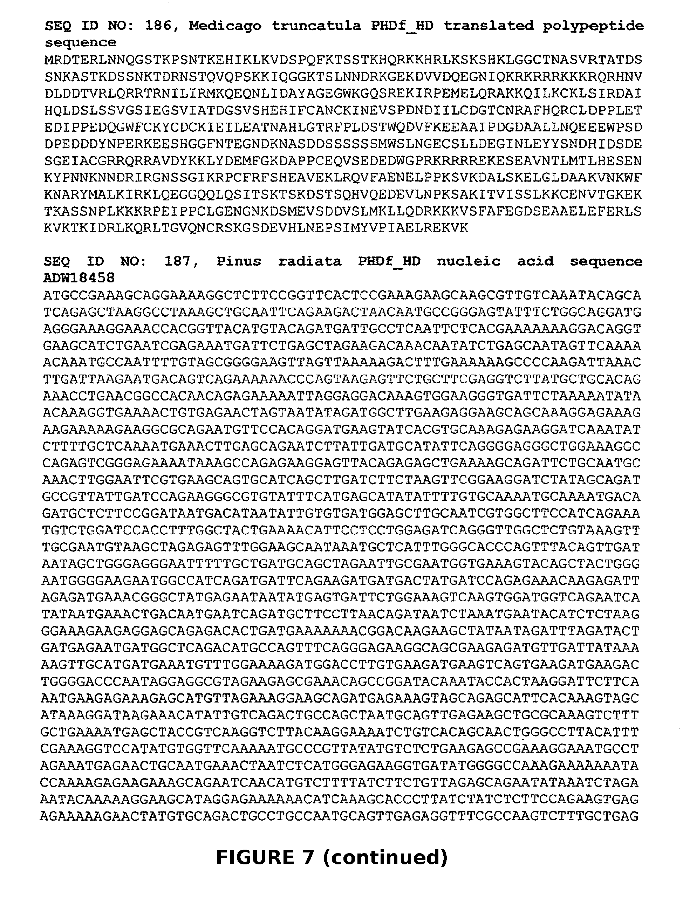

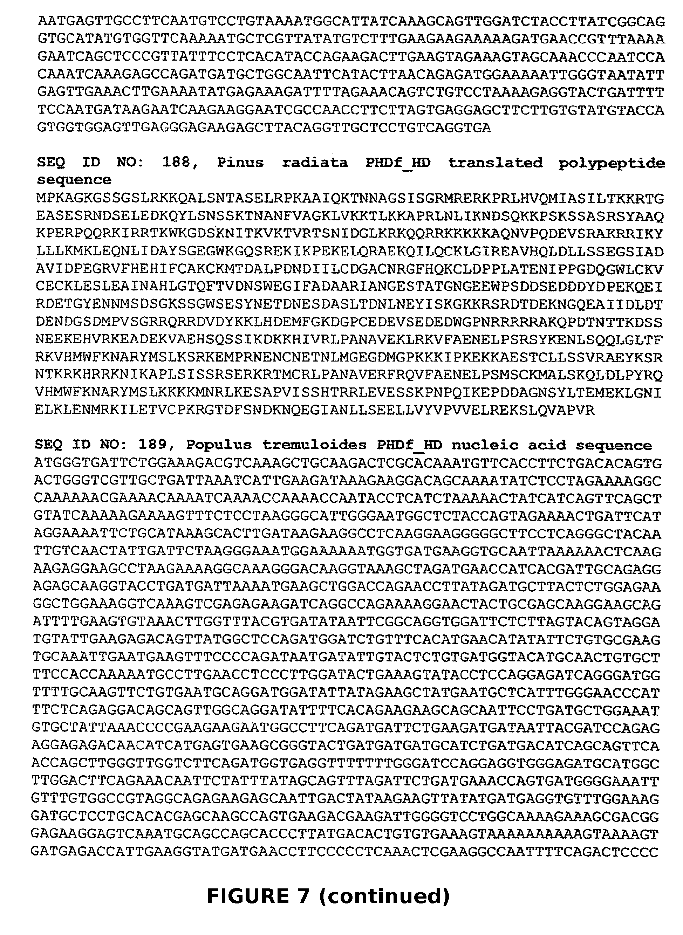

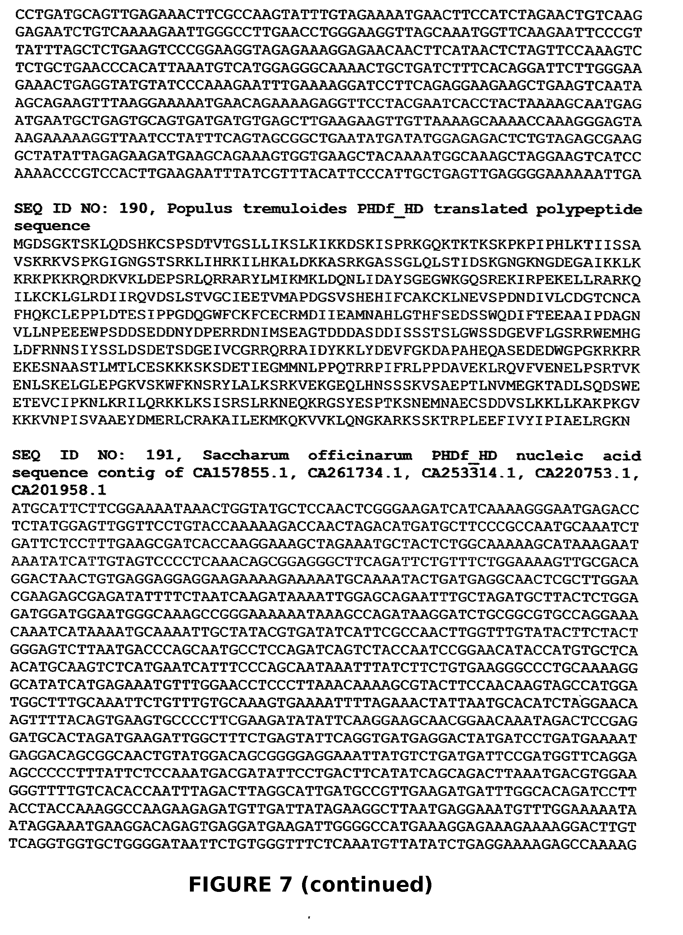

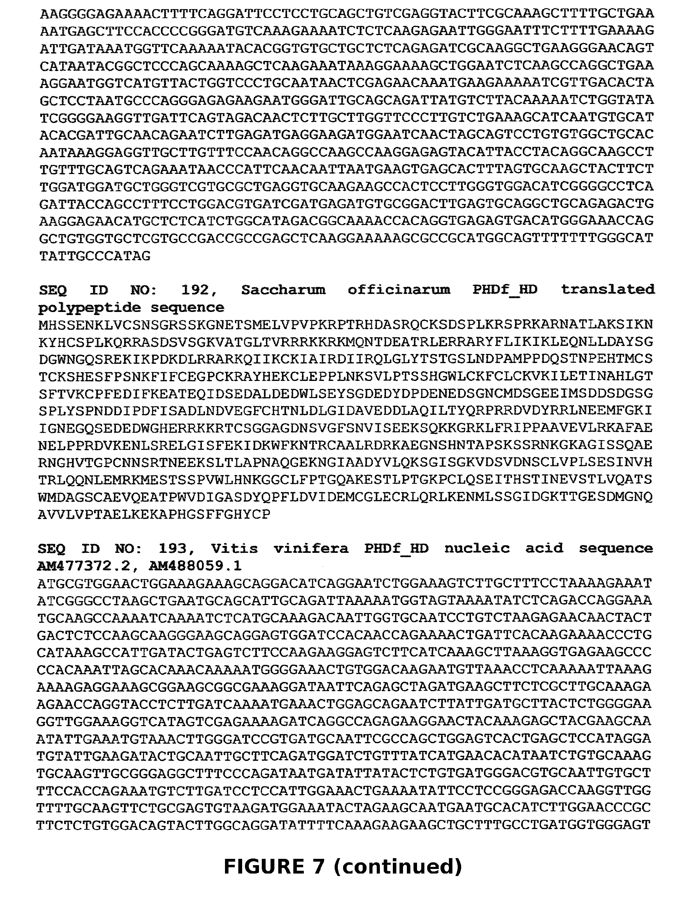

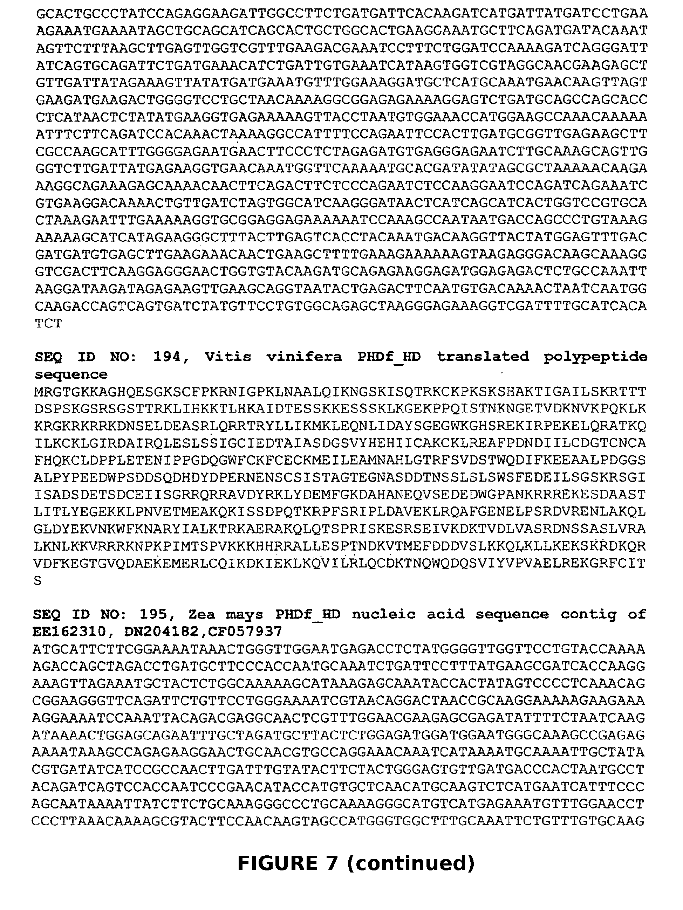

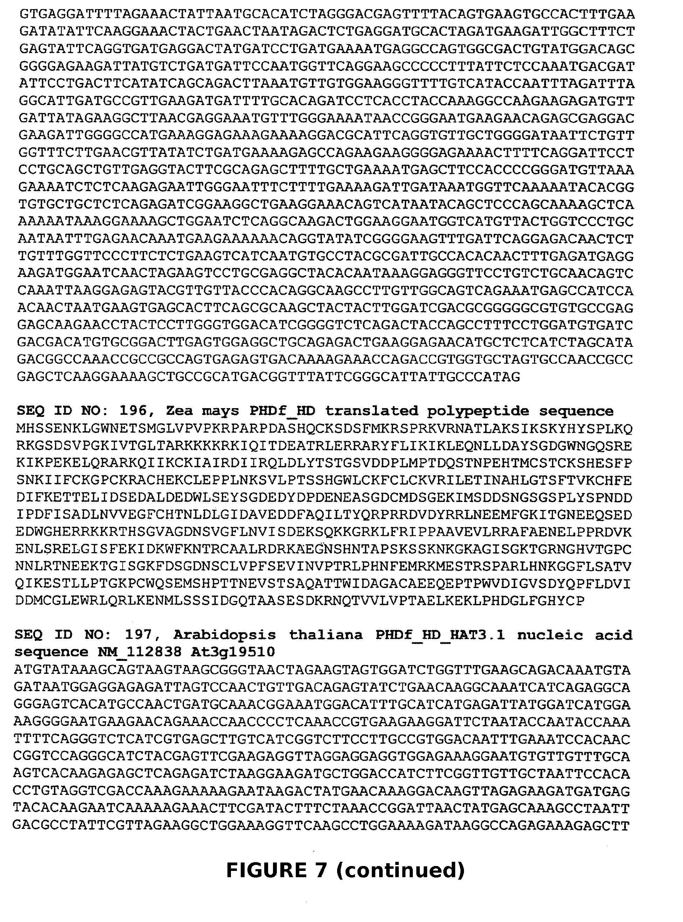

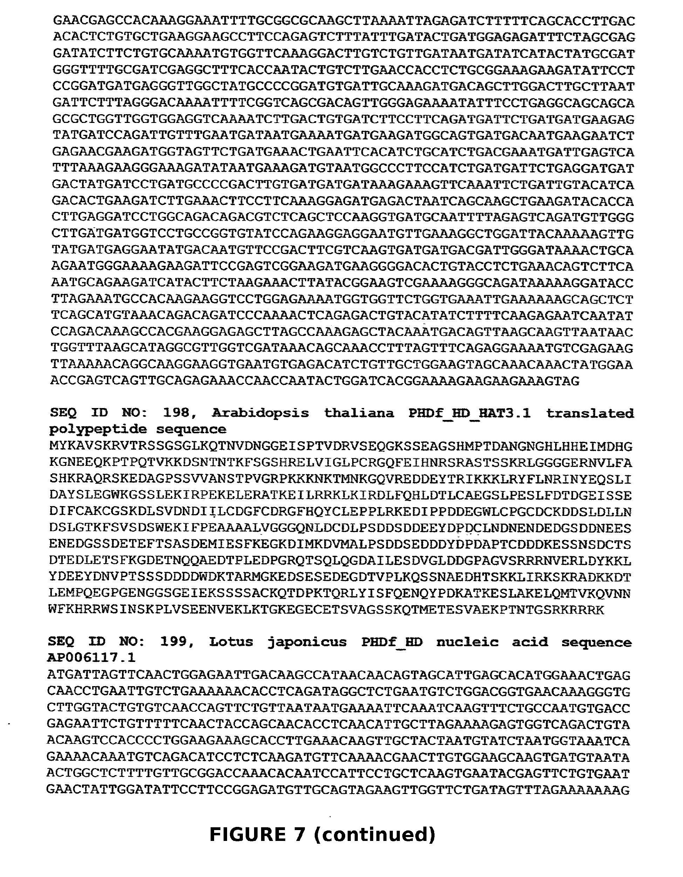

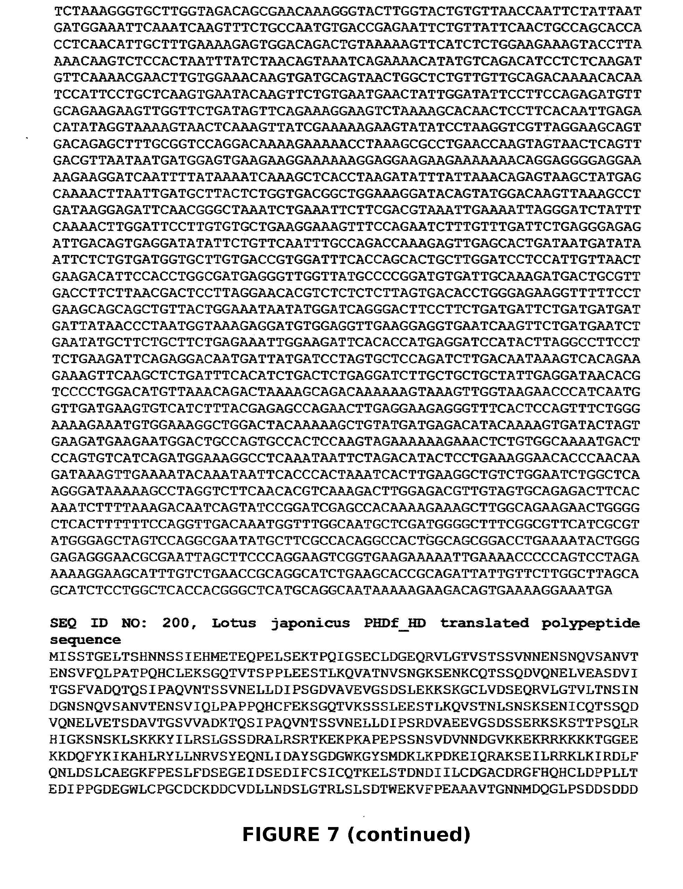

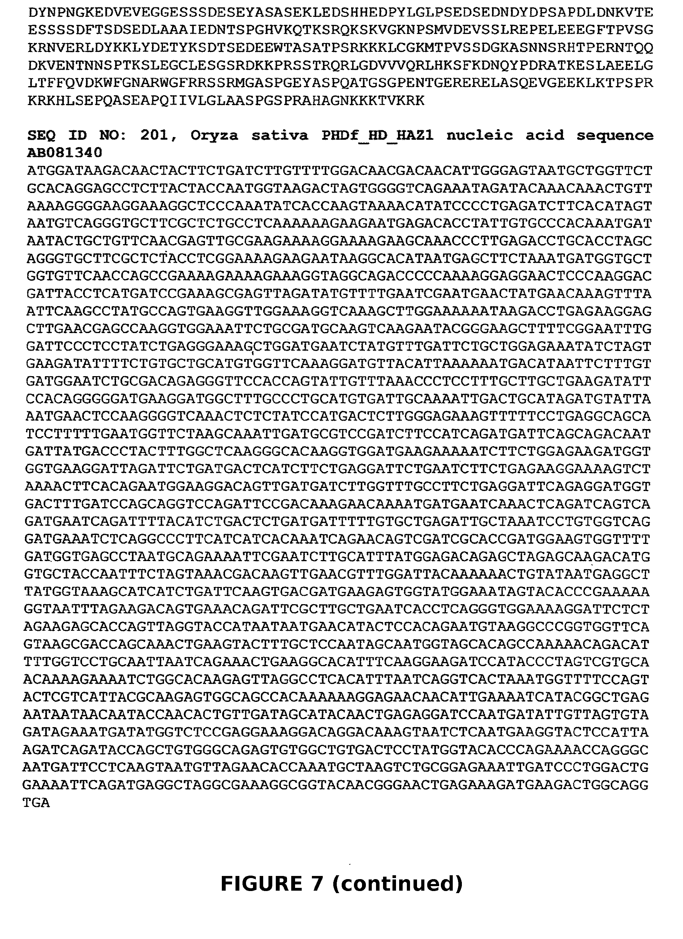

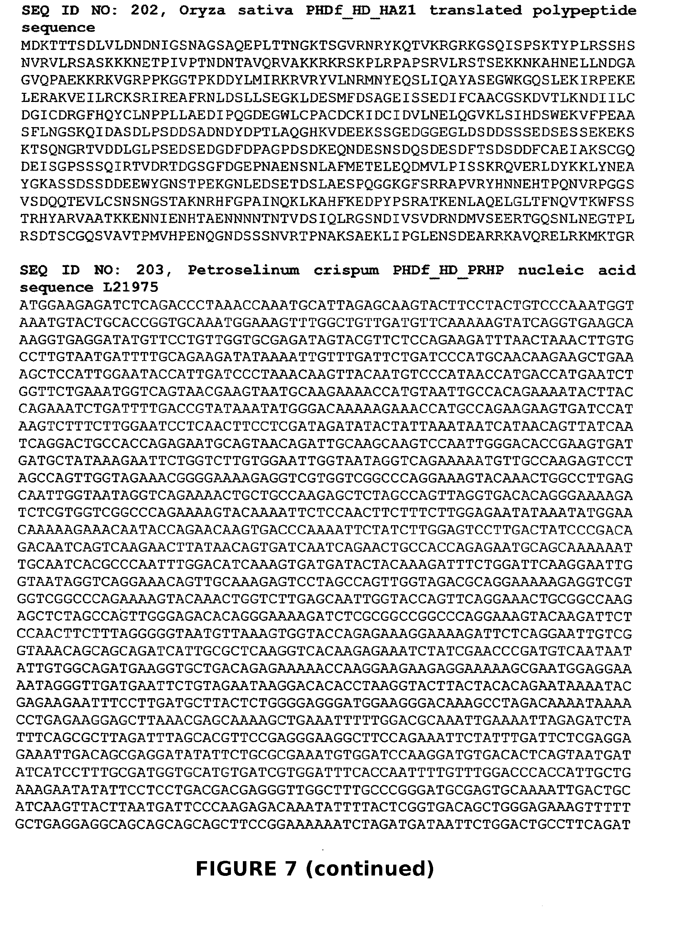

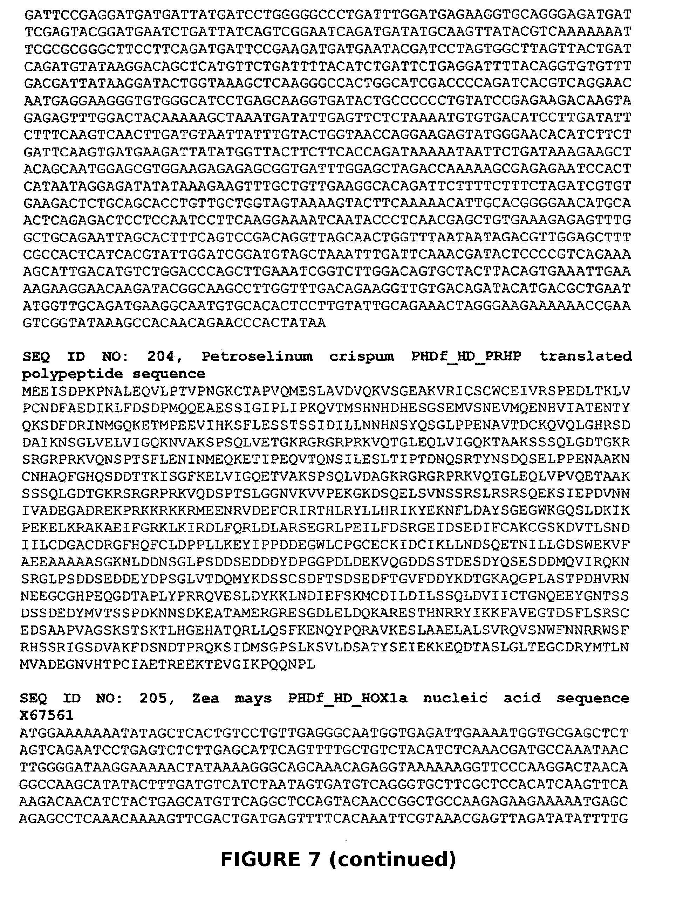

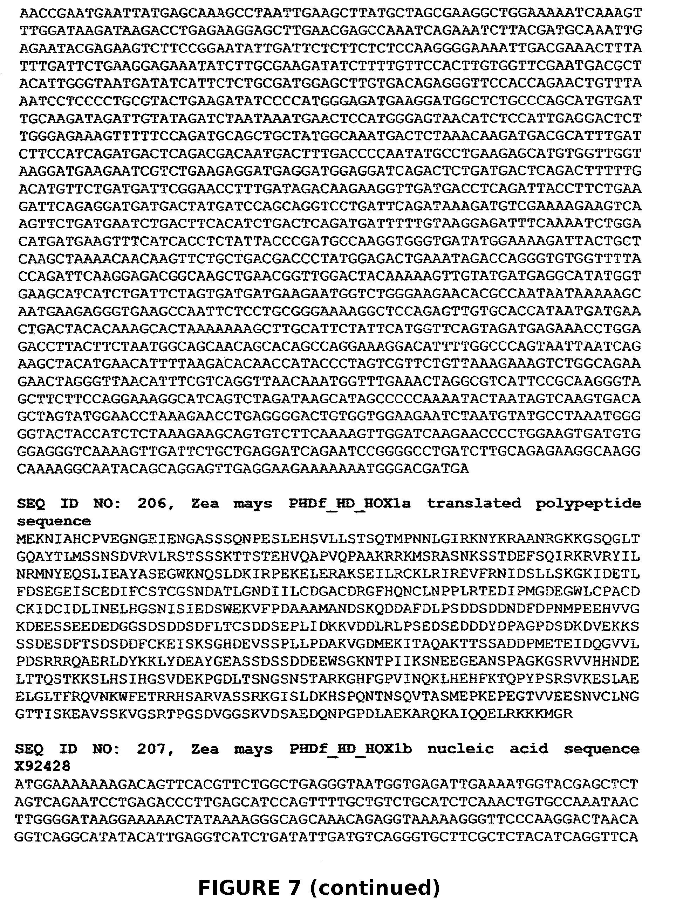

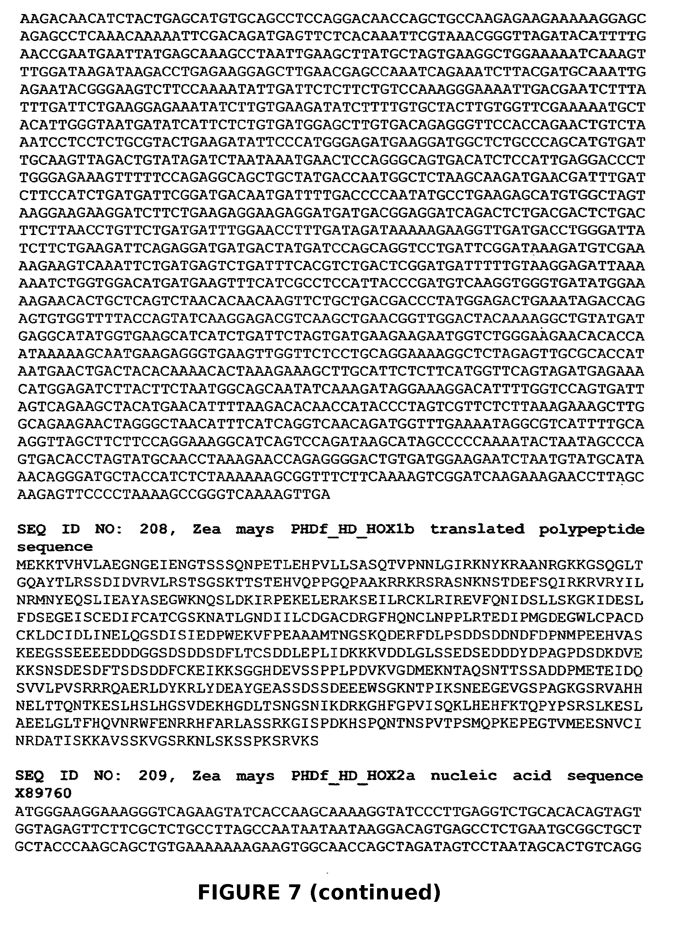

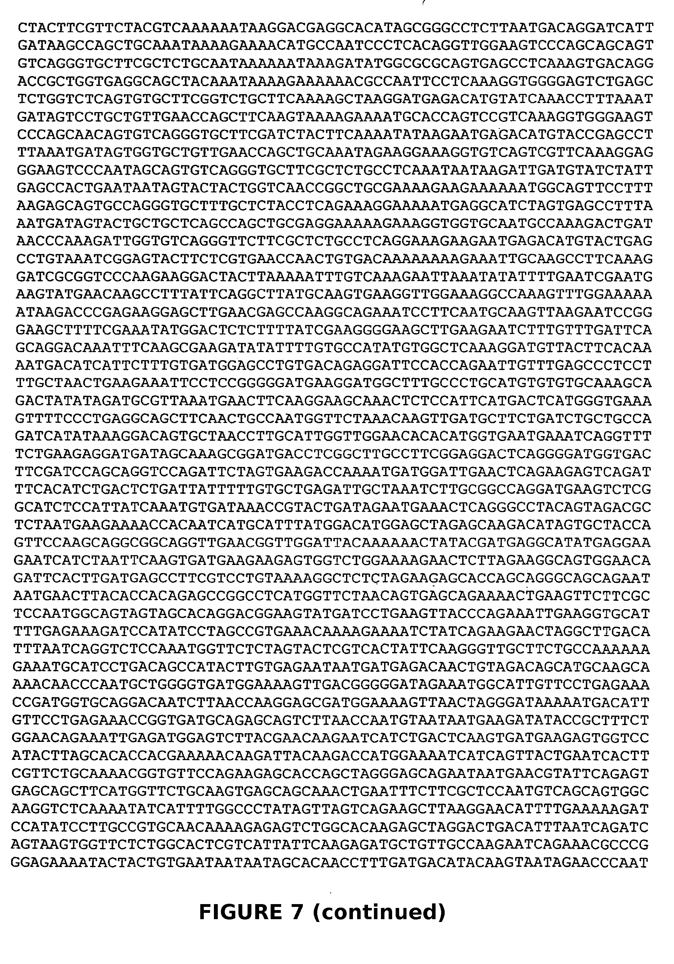

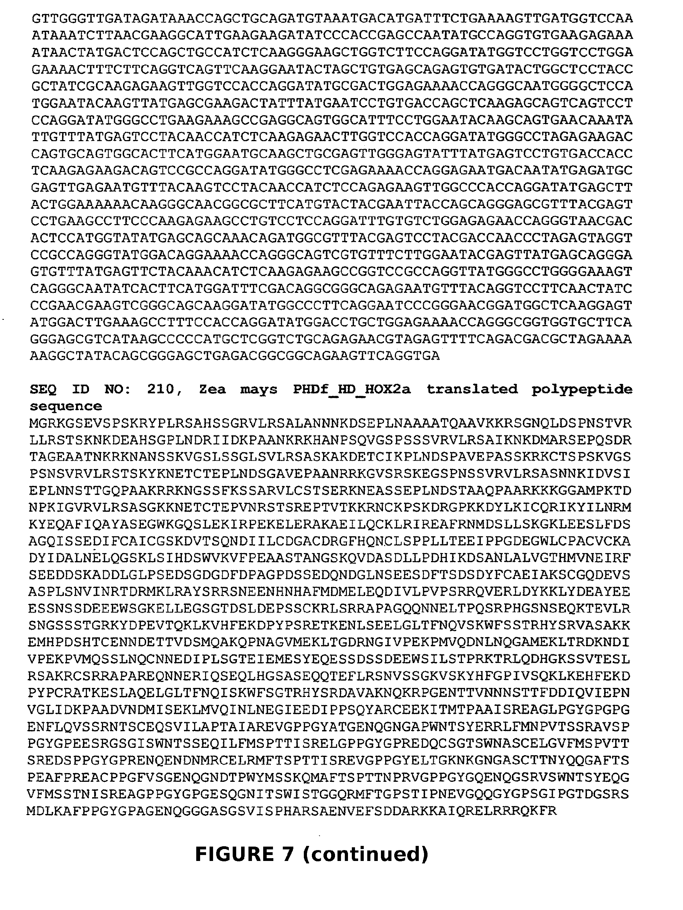

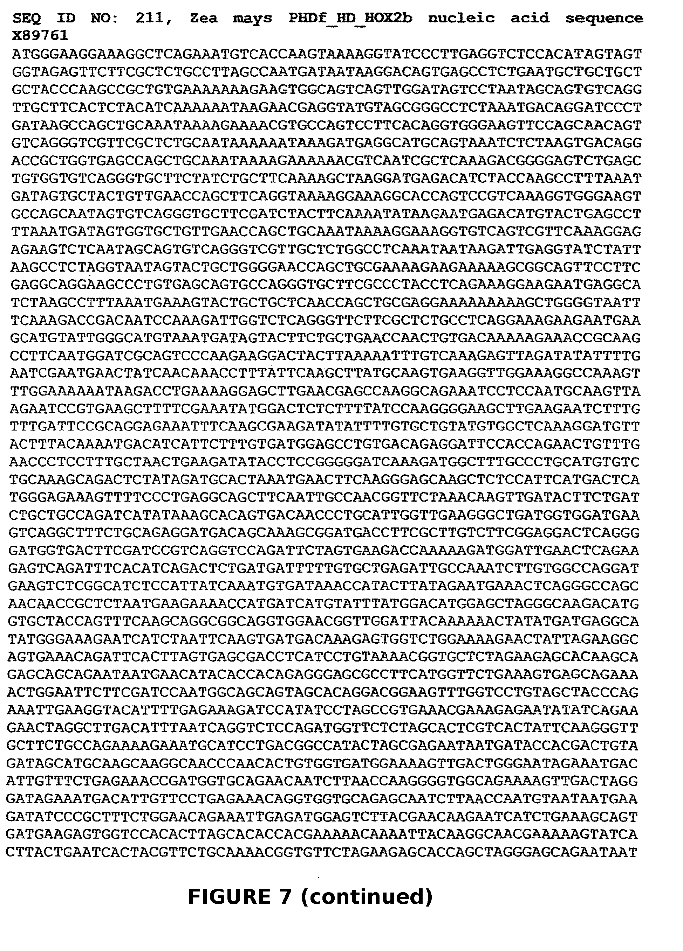

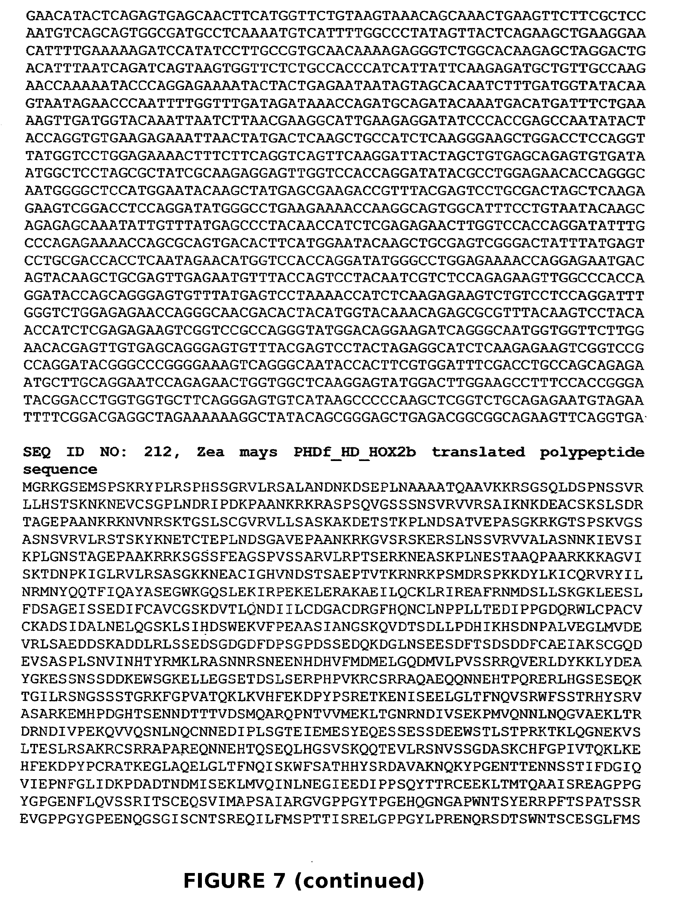

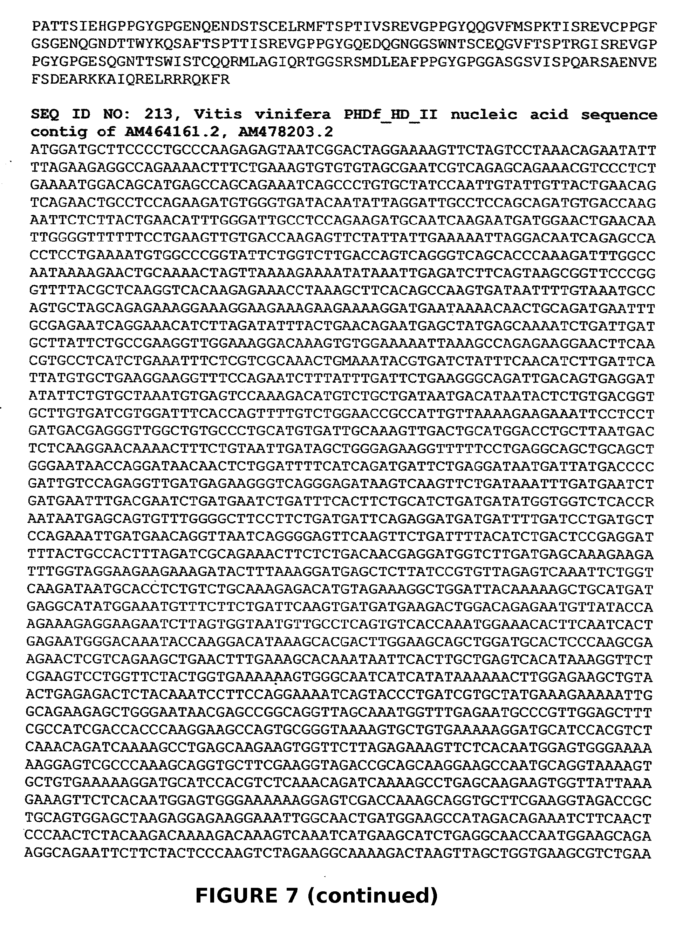

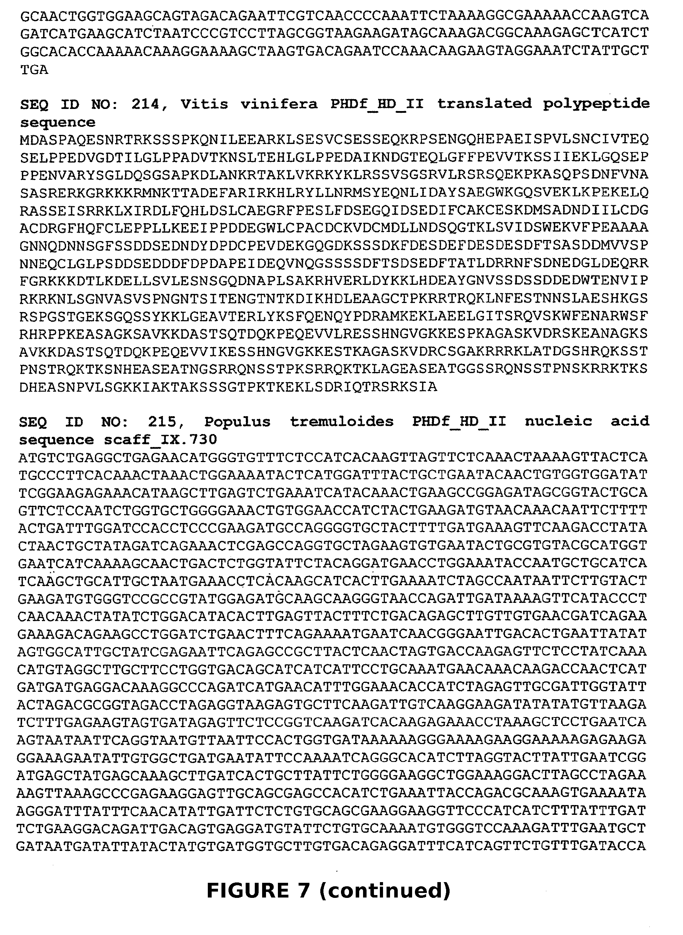

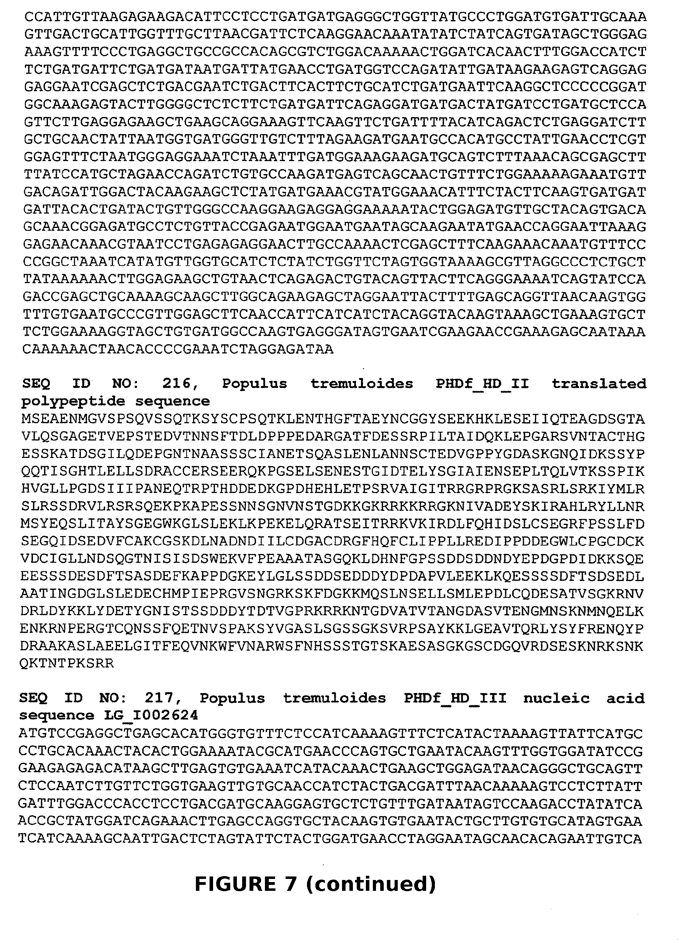

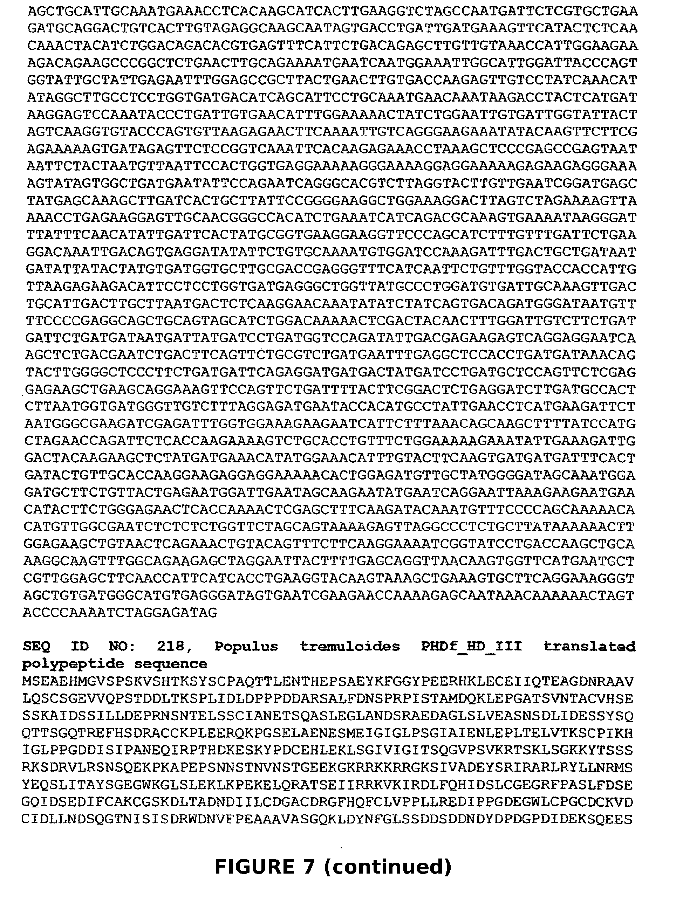

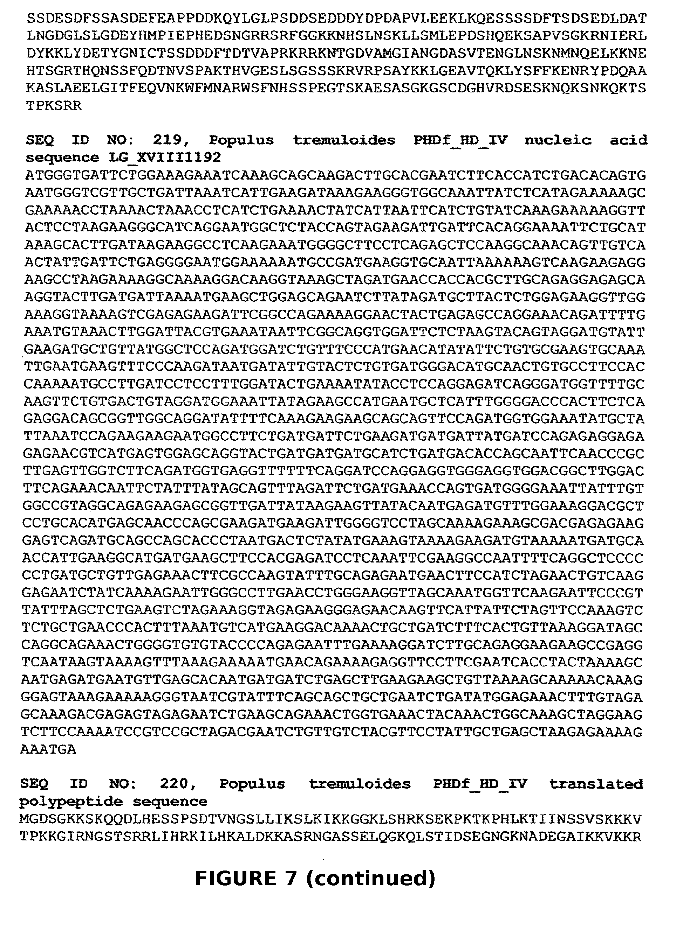

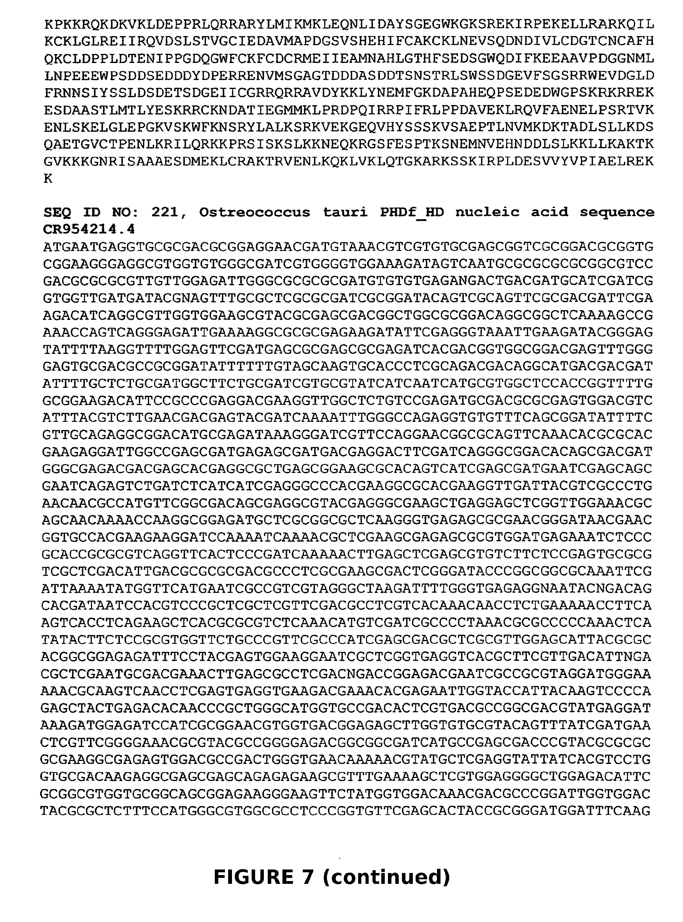

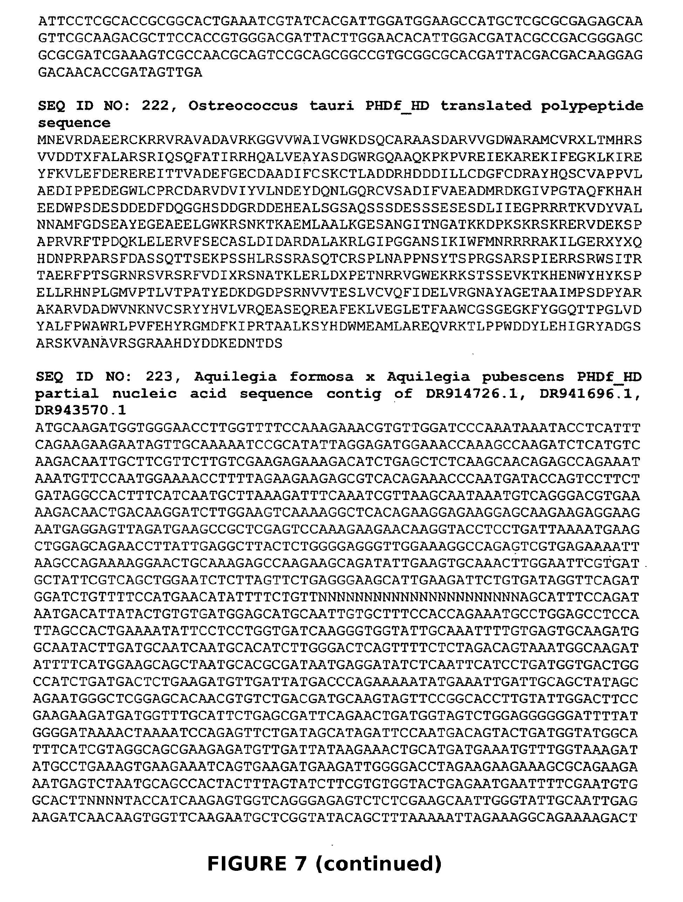

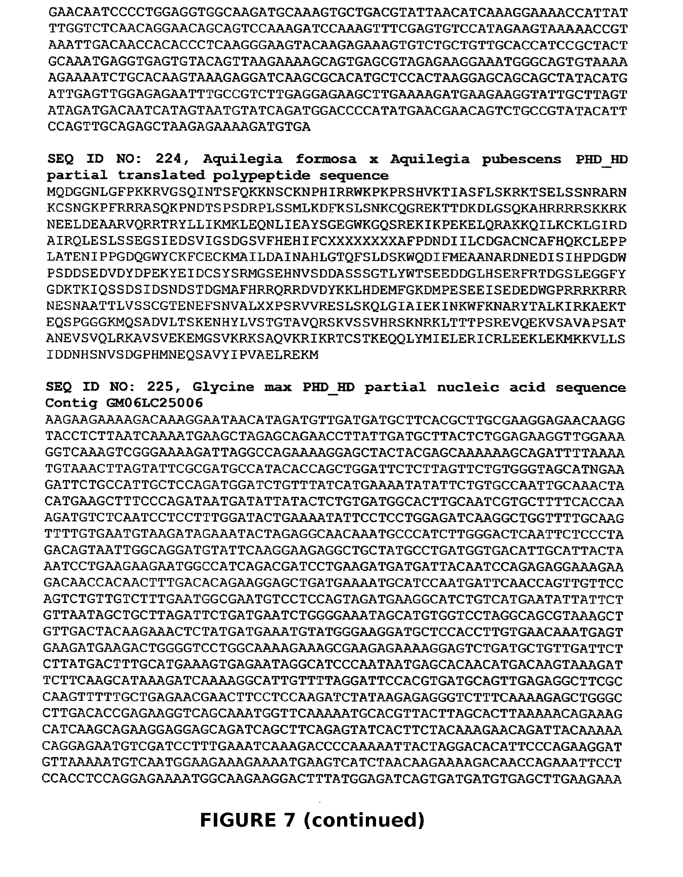

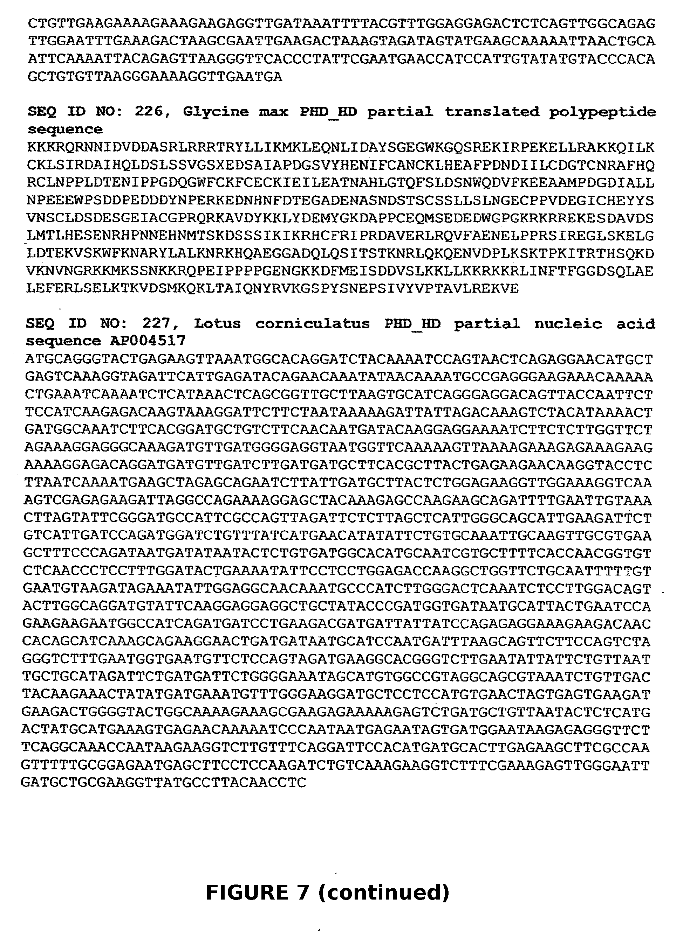

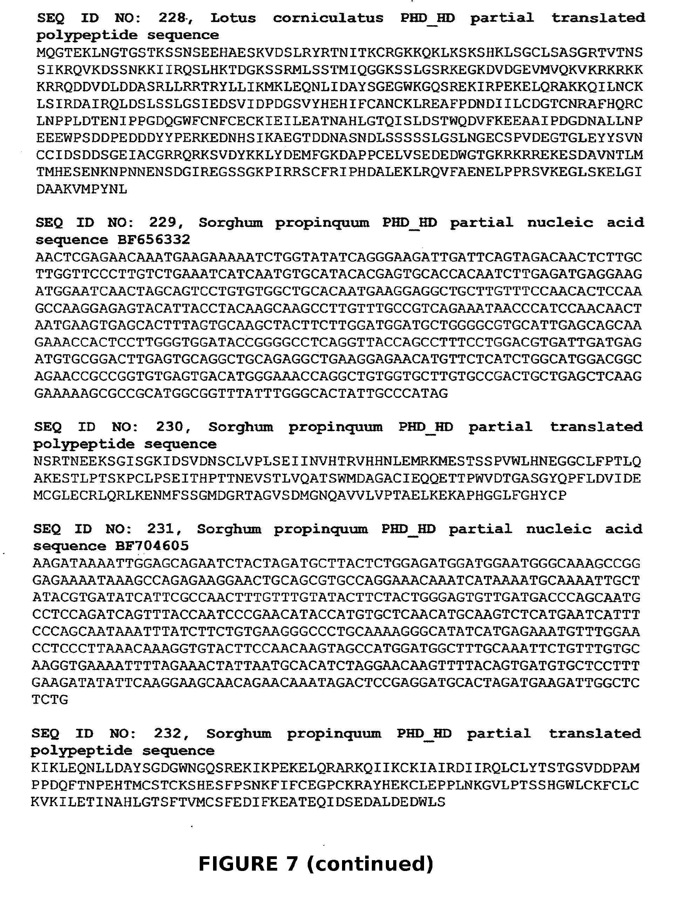

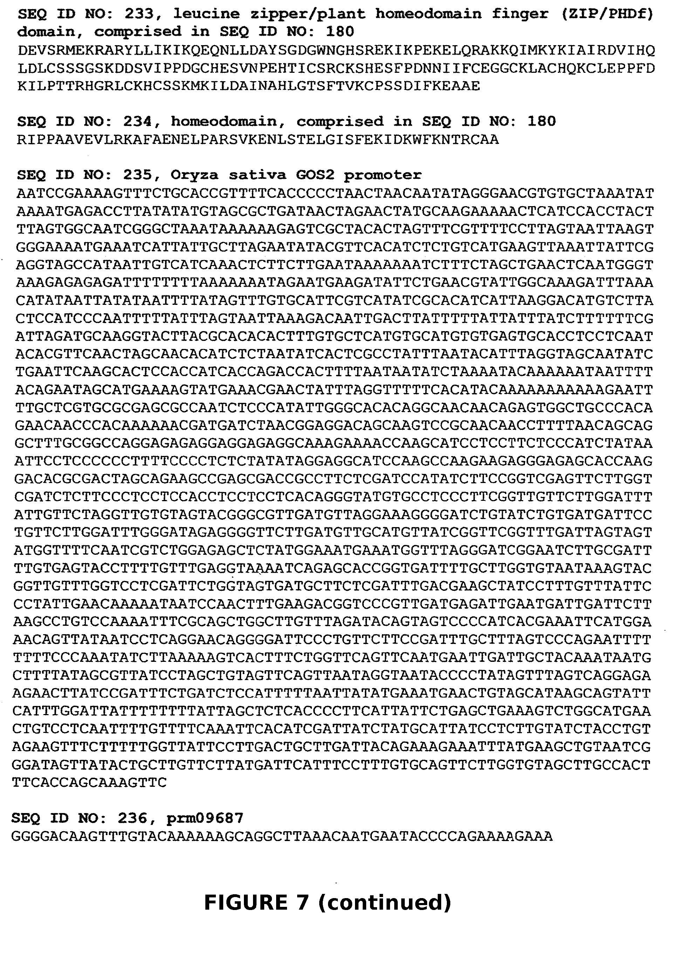

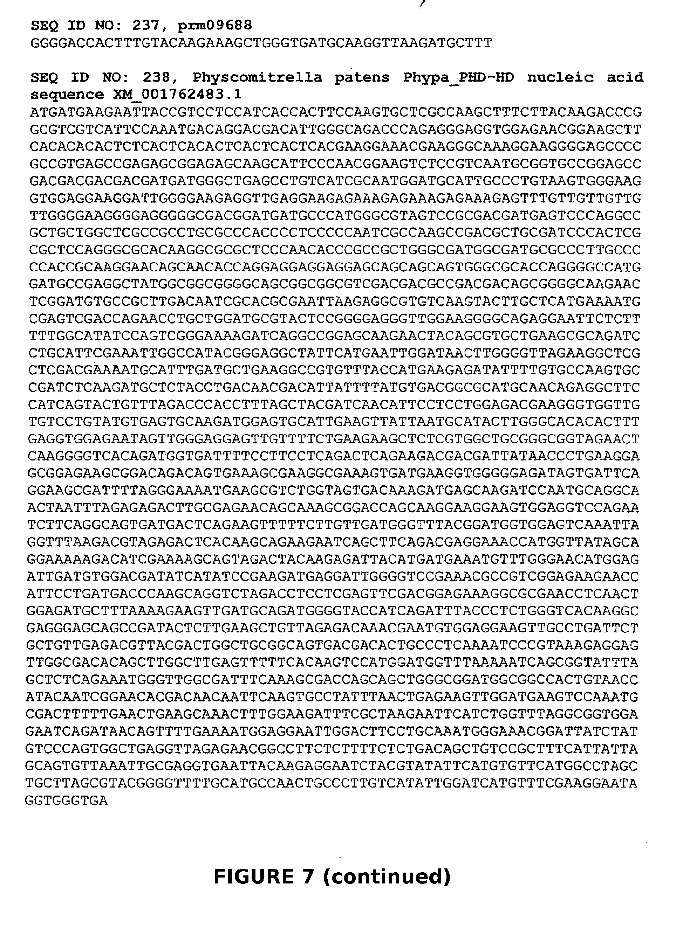

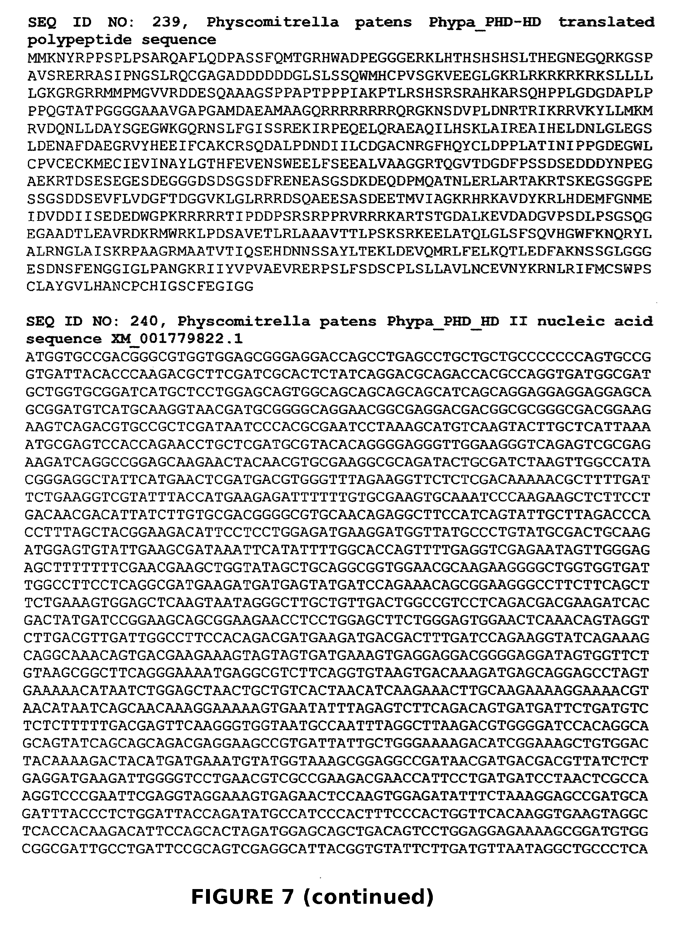

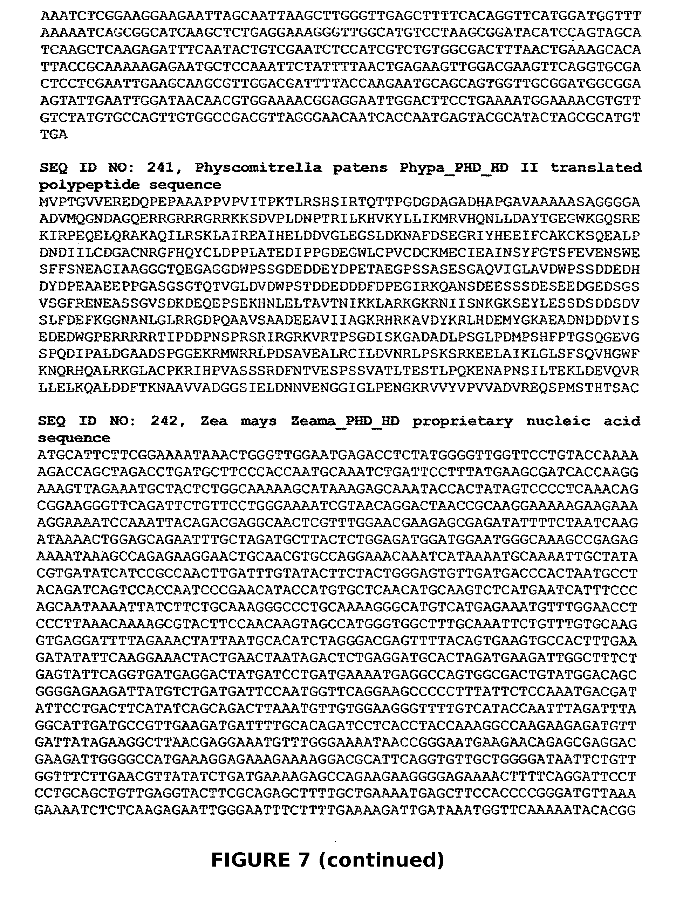

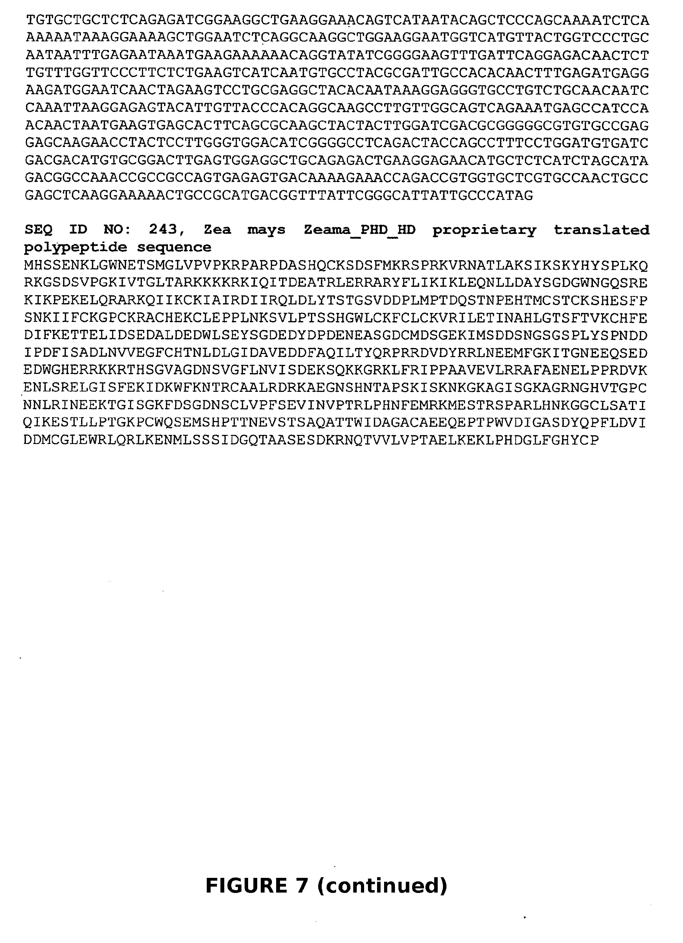

**Figure 7** details examples of a PHDf-HD sequences useful in performing the methods according to the present invention.

### Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in Sambrook (2001) Molecular Cloning: a laboratory manual, 3rd Edition Cold Spring Harbor Laboratory Press, CSH, New York, or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### Example 1: Identification of sequences related to the nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid sequence or polypeptide sequences to sequence databases and by calculating the statistical significance of matches. For example, the polypeptide encoded by the nucleic acid sequence of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid sequence (or polypeptide) sequences over a particular length. In some instances, the default parameters may be adjusted to modify the stringency of the search. For example the E-value may be increased to show less stringent matches. This way, short nearly exact matches may be identified.

Table D1 provides a list of nucleic acid sequences related to the PHDf-HDnucleic acid sequence used in the methods of the present invention.

**Table D1: Examples of PHDf-HD polypeptides:**

| **Name** | **Source organism** | **Nucleic acid SEQ ID NO:** | **Polypeptide SEQ ID NO:** | **Database accession #** | **Status** |
|---|---|---|---|---|---|
| Orysa_PHDf HD | *Oryza sativa* | 179 | 180 | Os02g05450.1 NM_001052422 | FL |
| Arath_PHDf_HD_PRHA | *Arabidopsis thaliana* | 181 | 182 | At4g029940 | FL |
| | | | | NM_119140 | |
| Eucgr_PHDf_HD | *Eucalyptus grandis* | 183 | 184 | ADW17964 | FL |
| Medtr_PHDf_HD | *Medicago truncatula* | 185 | 186 | AC 123547 | FL |
| Pinra_PHDf_HD | *Pinus radiata* | 187 | 188 | ADW18458 | FL |
| Poptr_PHDf_HD | *Populus tremuloides* | 189 | 190 | scaff_VI.625 | FL |
| Sacof_PHDf_HD | *Saccharum officinarum* | 191 | 192 | CA157855.1, CA261734.1, CA253314.1, CA220753.1, CA201958.1 | FL |
| Vitvi_PHDf_HD | *Vitis vinifera* | 193 | 194 | AM477372.2, AM488059.1 | FL |
| Zeama_PHDf_HD | *Zea mays* | 195 | 196 | EE162310, DN204182, CF057937 | FL |
| Arath_PHDf_HD_HAT3.1 | *Arabidopsis thaliana* | 197 | 198 | AT3G19510 NM_112838 | FL |
| Lotja_PHDf_HD | *Lotusjaponicus* | 199 | 200 | AP006117.1 | FL |
| Orysa_PHDf HD_HAZ1 | *Oryza sativa* | 201 | 202 | AB081340 Os06g12400.1 | FL |
| Petcr_PHDf_HD_PRHP | *Petroselinum crispum* | 203 | 204 | L21975 | FL |
| Zeama_PHDf_HD_HOX1a | *Zea mays* | 205 | 206 | X67561 | FL |
| Zeama_PHDf_HD_HOX1b | *Zea mays* | 207 | 208 | X92428 | FL |
| Zeama_PHDf_HD_HOX2a | *Zea mays* | 209 | 210 | X89760.1 | FL |
| Zeama_PHDf_HD_HOX2b | *Zea mays* | 211 | 212 | X89761 | FL |
| Vitvi_PHDf_HD_II | *Vitis vinifera* | 213 | 214 | AM464161.2 AM478203.2 | FL |
| Poptr_PHDf_HD_II | *Populus tremuloides* | 215 | 216 | scaff_IX.730 | FL |
| Poptr_PHDf_HD_III | *Populus tremuloides* | 217 | 218 | LG_I002624 | FL |
| Poptr_PHDf_HD_IV | *Populus tremuloides* | 219 | 220 | LG_XVIII1192 | FL |
| Ostta_PHDf_HD | *Ostreococcus tauri* | 221 | 222 | CR954214.4 | FL |
| Aqufor_PHDf_HD partial | *Aquilegia formosa x Aquilegia pubescens* | 223 | 224 | DR914726.1, DR941696.1, DR943570.1 | Partial |
| Glyma_PHDf_HD | *Glycine max* | 225 | 226 | Contig GM06LC25006 | Partial |
| Lotco_PHDf_HD | *Lotus corniculatus* | 227 | 228 | AP004517 | Partial |
| Sorpr_PHDf_HD 3' | *Sorghum propinquum* | 229 | 230 | BF656332 | Partial |
| Sorpr_PHDf_HD 5' | *Sorghum propinquum* | 231 | 232 | BF704605 | Partial |
| Phypa_PHDf HD | *Physcomitrella patens* | 238 | 239 | XM_001762483 | FL |
| Phypa_PHDf_HD | *Physcomitrella patens* | 240 | 241 | XM_001779822 | FL |
| Zeama_PHDf_HD | *Zea mays* | 242 | 243 | | FL |

### Example 2: Alignment of PHDf-HD polypeptide sequences

The University of Potsdam, Germany, has created plant transcription factor databases, including for Orysa sativa named riceTFDB. The polypeptide sequences corresponding to the transcription factors belonging to the HD family (120 gene models (91 loci) identified so far) were all downloaded, including the two PHDf-HD polypeptides (Os02g05450.1 and Os06g12400.1) identified to date. The polypeptide sequences of Table D1 of the present application were added (when full length, i.e. 21 polypeptide sequences) to the set of the HD family.

Alignment of all the polypeptide sequences was performed the Clustal algorithm (1.83) of progressive alignment, using default values (Thompson et al. (1997) Nucleic Acids Res 25:4876-4882; Chenna et al. (2003). Nucleic Acids Res 31:3497-3500). A neighbour-joining tree was constructed thereafter, and is represented in Figure 1 of the present application. The group of interest, comprising the two rice paralogs (Os02g05450.1 and Os06g12400.1) has been circled. Any polypeptide falling within this HD group (after a new multiple alignment step as described hereinabove) is considered to be useful in performing the methods of the invention as described herein.

In a multiple sequence alignment of the full length PHDf-HD polypeptides of Table D1, a number of features can be identified, and are marked in Figure 5. From the N-terminus to the C-terminus of the polypeptides are: (i) a predicted nuclear localisation signal (NLS); (ii) a leucine zipper (ZIP), with four heptads (boxed in which usually a leucine (occasionally an isoleucine, a valine, or a methionine)) appears every seventh amino acid; (iii) a PHD finger (PHDf), with the typical C4HC3 (four cysteines, one histidine, three cysteines) with a characteristics cysteine spacing; (iv) an acidic stretch (rich in acidic amino acids D and E); (v) basic stretches (rich in basic amino acids K and R); (vi) a homeodomain (HD).

### Example 3: Calculation of global percentage identity between polypeptide sequences useful in performing the methods of the invention

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:
Scoring matrix: Blosum62
First Gap: 12
Extending gap: 2

Results of the software analysis are shown in Table D2 for the global similarity and identity over the full length of the polypeptide sequences (excluding the partial polypeptide sequences). Percentage identity is given above the diagonal and percentage similarity is given below the diagonal.

The percentage identity between the PHDf-HD polypeptide sequences useful in performing the methods of the invention can be as low as 15 % amino acid identity compared to SEQ ID NO: 180.

**Table D2: MatGAT results for global similarity and identity over the full length of the polypeptide sequences.**

| | **1** | **2** | **3** | **4** | **5** | **6** | **7** | **8** | **9** | **10** | **11** | **12** | **13** | **14** | **15** | **16** | **17** | **18** | **19** | **20** | **21** |
|---|---|---|---|---|---|---|---|---|---|---|---|---|---|---|---|---|---|---|---|---|---|
| 1. Arath_PHDf_HD_PRHA | | **25** | 31 | 40 | 19 | 38 | 26 | 17 | 32 | 40 | 20 | 19 | 40 | 30 | 40 | 23 | 31 | 25 | 25 | 18 | 19 |
| 2. Arath_PHDf-HD_HAT3 | 44 | | 23 | 27 | 33 | 26 | 34 | 28 | 27 | 26 | 34 | 35 | 27 | 23 | 28 | 34 | 24 | 36 | 35 | 18 | 20 |
| **3. Orysa_PHDf_HD** | **46** | **39** | | **35** | **18** | **34** | **20** | **18** | **31** | **34** | **17** | **18** | **33** | **56** | **36** | **21** | **57** | **21** | **23** | **15** | **15** |
| 4. Eucgr_PHDf_HD | 57 | 45 | 51 | | 21 | 47 | 27 | 20 | 36 | 50 | 22 | 21 | 51 | 32 | 52 | 25 | 32 | 26 | 27 | 17 | 17 |
| 5. Lotja_PHDf-HD | 36 | 47 | 31 | 35 | | 22 | 29 | 32 | 23 | 20 | 39 | 40 | 20 | 18 | 21 | 34 | 18 | 31 | 32 | 20 | 22 |
| 6. Medtr_PHDf_HD | 55 | 44 | 51 | 64 | 36 | | 26 | 19 | 35 | 50 | 20 | 20 | 49 | 30 | 50 | 25 | 32 | 27 | 26 | 17 | 17 |
| 7.Orysa_PHDf-HD_HAZ1 | 42 | 50 | 35 | 47 | 45 | 43 | | 26 | 25 | 25 | 29 | 29 | 26 | 21 | 26 | 32 | 21 | 50 | 46 | 29 | 30 |
| 8. Petcr_PHDf_HD_PRHP | 31 | 39 | 28 | 32 | 48 | 31 | 38 | | 22 | 20 | 32 | 33 | 20 | 18 | 20 | 30 | 18 | 26 | 25 | 19 | 21 |
| 9. Pinra_PHDf_HD | 53 | 44 | 47 | 51 | 42 | 50 | 44 | 39 | | 36 | 23 | 23 | 37 | 28 | 39 | 26 | 30 | 25 | 27 | 19 | 19 |
| 10. Poptr_PHDf_HD_I | 58 | 45 | 52 | 67 | 36 | 66 | 45 | 33 | 52 | | 20 | 21 | 84 | 30 | 55 | 25 | 31 | 25 | 25 | 17 | 17 |
| 11. Poptr_PH_Df_HD_III | 36 | 47 | 30 | 34 | 56 | 35 | 44 | 46 | 41 | 36 | | 80 | 21 | 19 | 22 | 37 | 18 | 31 | 31 | 20 | 20 |
| 12. Poptr_PHDf_HD_II | 36 | 49 | 31 | 34 | 57 | 34 | 46 | 47 | 42 | 35 | 86 | | 21 | 18 | 22 | 38 | 18 | 30 | 30 | 20 | 21 |
| 13. Poptr_PHDf_HD_IV | 59 | 46 | 51 | 68 | 37 | 67 | 44 | 33 | 54 | 91 | 35 | 35 | | 30 | 57 | 25 | 32 | 25 | 25 | 17 | 17 |
| 14. Sacof_PHDf_HD | 48 | 43 | 69 | 51 | 31 | 50 | 38 | 29 | 45 | 51 | 34 | 33 | 51 | | 34 | 22 | 83 | 21 | 22 | 15 | 16 |
| 15. Vitvi_PHDf_HD_I | 58 | 47 | 52 | 69 | 36 | 67 | 43 | 32 | 52 | 74 | 36 | 35 | 75 | 52 | | 23 | 34 | 27 | 25 | 16 | 17 |
| 16. Vitvi_PHDf_HD_II | 39 | 47 | 36 | 39 | 56 | 39 | 45 | 47 | 43 | 40 | 56 | 57 | 40 | 36 | 41 | | 22 | 32 | 31 | 23 | 24 |
| 17. Zeama_PHDf_HD | 48 | 41 | 70 | 51 | 32 | 52 | 38 | 30 | 45 | 54 | 33 | 32 | 53 | 87 | 53 | 34 | | 22 | 22 | 15 | 15 |
| 18. Zeama_hox1a | 43 | 54 | 39 | 44 | 43 | 44 | 63 | 38 | 43 | 44 | 44 | 43 | 45 | 42 | 43 | 46 | 42 | | 83 | 24 | 24 |
| 19. Zeama_hox1b | 42 | 53 | 40 | 44 | 44 | 42 | 60 | 36 | 43 | 44 | 44 | 43 | 45 | 41 | 44 | 45 | 41 | 88 | | 23 | 23 |
| 20. Zeama_hox2a | 27 | 27 | 24 | 25 | 32 | 25 | 36 | 31 | 31 | 26 | 30 | 31 | 27 | 25 | 26 | 34 | 24 | 31 | 30 | | 81 |
| 21. Zeama_Hox2b | 29 | 28 | 23 | 27 | 33 | 27 | 37 | 34 | 31 | 27 | 32 | 33 | 26 | 25 | 27 | 35 | 25 | 32 | 30 | 86 | |

The percentage identity can be substantially increased if the identity calculation is performed between the conserved ZIP/PHDf domain (conserved leucine zipper/plant homeodomain finger domain, comprising the Zip and PHDf domains) of SEQ ID NO: 180 (as represented by SEQ ID NO: 233) and the conserved ZIP/PHDf domain of the polypeptides useful in performing the invention. The conserved ZIP/PHDf of SEQ ID NO: 233 is in total 180 contiguous amino acids long. Percentage identity over the conserved ZIP/PHDf domain amongst the polypeptide sequences useful in performing the methods of the invention ranges between 30 % and 75% amino acid identity, as shown in Table D3.

**Table D3: MatGAT results for global similarity and identity over the conserved ZIP/PHDf domain amongst of the polypeptide sequences.**

| | **1** | **2** | **3** | **4** | **5** | **6** | **7** | **8** | **9** | **10** | **11** | **12** | **13** | **14** | **15** | **16** | **17** | **18** | **19** | **20** | **21** |
|---|---|---|---|---|---|---|---|---|---|---|---|---|---|---|---|---|---|---|---|---|---|
| 1. ZIP/PHDf_Arath_PHDf_HD_PRHA | | 52 | **50** | 77 | 48 | 74 | 53 | 48 | 64 | 73 | 48 | 46 | 72 | 51 | 76 | 50 | 52 | 50 | 50 | 53 | 53 |
| 2. ZIP/PHDf_Arath_PHDf-HD_HAT3 | 70 | | **44** | 52 | 73 | 54 | 70 | 69 | 56 | 52 | 75 | 75 | 51 | 43 | 53 | 78 | 46 | 65 | 67 | 64 | 65 |
| 3. ZIP/PHDf_Orysa_PHDf_HD | 69 | 60 | | **56** | **39** | **54** | **37** | **39** | **56** | **53** | **39** | **38** | **53** | **70** | **59** | **41** | **71** | **37** | **38** | **34** | **33** |
| 4. ZIP/PHDf_Eucgr_PHDf_HD | 90 | 70 | 73 | | 52 | 79 | 48 | 50 | 71 | 82 | 51 | 49 | 81 | 55 | 84 | 52 | 56 | 48 | 48 | 49 | 48 |
| 5. ZIP/Lotja_PHDf-HD | 67 | 89 | 59 | 69 | | 52 | 64 | 69 | 52 | 47 | 76 | 76 | 47 | 37 | 50 | 78 | 38 | 64 | 66 | 61 | 61 |
| 6. ZIP/Medtr_PHDf_HD | 86 | 70 | 72 | 87 | 68 | | 52 | 49 | 66 | 74 | 49 | 48 | 75 | 50 | 79 | 51 | 53 | 51 | 51 | 52 | 52 |
| 7. ZIP/PHDf_Orysa_PHDf-HD_HAZ1 | 69 | 83 | 54 | 67 | 83 | 68 | | 64 | 51 | 47 | 68 | 67 | 47 | 35 | 47 | 68 | 37 | 79 | 80 | 82 | 84 |
| 8. ZIP/PHDf_Petcr_PHDf_HD_PRHP | 67 | 80 | 54 | 66 | 83 | 65 | 78 | | 53 | 47 | 71 | 73 | 45 | 39 | 48 | 74 | 42 | 62 | 63 | 62 | 62 |
| 9. ZIP/PHDf_Pinra_PHDf_HD | 82 | 74 | 72 | 83 | 73 | 82 | 70 | 69 | | 66 | 53 | 52 | 67 | 52 | 73 | 55 | 54 | 53 | 52 | 53 | 52 |
| 10. ZIP/PHDf_Poptr_PHDf_HD_I | 87 | 70 | 72 | 92 | 69 | 88 | 67 | 66 | 83 | | 48 | 47 | 93 | 53 | 82 | 50 | 55 | 49 | 48 | 49 | 49 |
| 11. ZIP/PHDf_Poptr_PHDf_HD_III | 66 | 87 | 57 | 67 | 90 | 65 | 82 | 82 | 70 | 67 | | 93 | 48 | 36 | 51 | 82 | 39 | 67 | 70 | 65 | 64 |
| 12. ZIP/PHDf_Poptr_PHDf_HD_II | 65 | 88 | 56 | 66 | 90 | 65 | 81 | 84 | 69 | 66 | 96 | | 46 | 35 | 49 | 83 | 38 | 67 | 69 | 64 | 64 |
| 13. ZIP/PHDf_Poptr_PHDf_HD_IV | 86 | 70 | 72 | 91 | 69 | 88 | 67 | 66 | 82 | 97 | 67 | 66 | | 52 | 82 | 48 | 54 | 47 | 47 | 48 | 47 |
| 14. ZIP/PHDf_Sacof_PHDf_HD | 71 | 60 | 82 | 73 | 57 | 69 | 55 | 57 | 70 | 72 | 56 | 55 | 72 | | 57 | 39 | 92 | 35 | 35 | 33 | 33 |
| 15. ZIP/PHDf_Vitvi_PHDf_HD_I | 89 | 71 | 73 | 90 | 70 | 88 | 67 | 64 | 84 | 91 | 67 | 66 | 89 | 73 | | 51 | 58 | 48 | 48 | 48 | 48 |
| 16. ZIP/PHDf_Vitvi_PHDf_HD_II | 67 | 88 | 58 | 69 | 91 | 68 | 84 | 84 | 73 | 69 | 90 | 93 | 67 | 57 | 69 | | 42 | 65 | 67 | 65 | 64 |
| 17. ZIP/PHDf_Zeama_PHDf_HD | 74 | 61 | 83 | 73 | 58 | 70 | 56 | 58 | 69 | 73 | 57 | 55 | 72 | 96 | 73 | 57 | | 38 | 37 | 35 | 34 |
| 18. ZIP/PHDf_Zeama_hox1a | 71 | 84 | 59 | 71 | 85 | 70 | 88 | 79 | 74 | 72 | 83 | 82 | 71 | 61 | 70 | 85 | 62 | | 94 | 74 | 76 |
| 19. ZIP/PHDf_Zeama_hox1b | 70 | 84 | 58 | 69 | 85 | 68 | 89 | 79 | 74 | 70 | 84 | 83 | 70 | 60 | 70 | 85 | 60 | 98 | | 75 | 78 |
| 20. ZIP/PHDf_Zeama_hox2a | 67 | 80 | 54 | 67 | 83 | 67 | 89 | 76 | 70 | 67 | 80 | 79 | 66 | 54 | 66 | 82 | 56 | 87 | 87 | | 92 |
| 21. ZIP/PHDf_Zeama_Hox2b | 67 | 82 | 53 | 66 | 83 | 67 | 90 | 77 | 71 | 66 | 80 | 79 | 67 | 53 | 67 | 82 | 55 | 88 | 88 | 97 | |

The percentage identity can also be calculated between the conserved HD of SEQ ID NO: 180 (as represented by SEQ ID NO: 234) and the conserved HD of the polypeptides useful in performing the invention. Percentage identity over the conserved HD amongst the polypeptide sequences useful in performing the methods of the invention ranges between 25 % and 70% amino acid identity, as shown in Table D4.

**Table D4: MatGAT results for global similarity and identity over the conserved HD amongst of the polypeptide sequences.**

| | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| 1. HD_Arath_PHDCHD_PRHA | | 34 | **54** | 72 | 36 | 72 | 38 | 36 | 58 | 68 | 38 | 36 | 70 | 54 | 70 | 34 | 54 | 36 | 33 | 29 | 27 |
| 2. HD_Arath_PHDf-HD_HAT3 | 56 | | **28** | 32 | 54 | 34 | 40 | 58 | 30 | 32 | 54 | 58 | 32 | 28 | 32 | 58 | 28 | 42 | 39 | 40 | 39 |
| 3. HD_Orysa_PHDf_HD | 76 | 60 | | **68** | **42** | **58** | **42** | **36** | **46** | **64** | **38** | **40** | **66** | **94** | **62** | **42** | **92** | **38** | **35** | **35** | **33** |
| 4. HD_Eucgr_PHDf_HD | 86 | 58 | 78 | | 42 | 72 | 44 | 42 | 60 | 82 | 38 | 38 | 84 | 66 | 76 | 42 | 66 | 44 | 41 | 35 | 33 |
| 5. HD_Lotja_PHDf-HD | 60 | 72 | 66 | 64 | | 38 | 52 | 58 | 36 | 42 | 64 | 68 | 42 | 42 | 40 | 68 | 40 | 56 | 55 | 50 | 46 |
| 6. HD_Medtr_PHDf_HD | 78 | 60 | 70 | 82 | 66 | | 36 | 36 | 62 | 68 | 38 | 36 | 70 | 58 | 66 | 36 | 58 | 40 | 37 | 27 | 27 |
| 7. HD_Orysa_PHDf-HD_HAZ1 | 56 | 54 | 64 | 62 | 66 | 58 | | 44 | 26 | 44 | 50 | 48 | 44 | 42 | 40 | 46 | 42 | 70 | 65 | 73 | 71 |
| 8. HD_Petcr_PHDf_HD_PRHP | 58 | 74 | 58 | 56 | 66 | 58 | 58 | | 36 | 42 | 54 | 58 | 42 | 36 | 38 | 62 | 36 | 50 | 45 | 40 | 39 |
| 9. HD_Pinra_PHDf_HD | 74 | 54 | 64 | 72 | 58 | 72 | 50 | 52 | | 58 | 30 | 32 | 60 | 44 | 56 | 36 | 44 | 28 | 28 | 21 | 19 |
| 10. HD_Poptr_PHDf_HD_I | 86 | 56 | 78 | 92 | 64 | 84 | 64 | 56 | 74 | | 36 | 36 | 98 | 64 | 72 | 42 | 64 | 42 | 39 | 35 | 33 |
| 11. HD_Poptr_PHDf_HD_III | 54 | 72 | 62 | 60 | 82 | 58 | 66 | 66 | 54 | 58 | | 88 | 36 | 38 | 38 | 72 | 38 | 52 | 49 | 46 | 44 |
| 12. HD_Poptr_PHDf_HD_II | 56 | 72 | 64 | 62 | 82 | 60 | 68 | 66 | 56 | 60 | 100 | | 36 | 40 | 38 | 78 | 40 | 50 | 47 | 46 | 44 |
| 13. HD_Poptr_PHDf_HD_IV | 88 | 56 | 80 | 94 | 64 | 86 | 64 | 56 | 76 | 98 | 58 | 60 | | 66 | 72 | 42 | 66 | 42 | 39 | 35 | 33 |
| 14. HD_Sacof_PHDf_HD | 74 | 60 | 94 | 76 | 64 | 72 | 62 | 56 | 62 | 76 | 60 | 62 | 78 | | 64 | 42 | 98 | 36 | 33 | 35 | 33 |
| 15. HD_Vitvi_PHDf_HD_I | 82 | 60 | 80 | 86 | 70 | 80 | 60 | 58 | 74 | 88 | 62 | 64 | 88 | 82 | | 38 | 64 | 40 | 37 | 29 | 27 |
| 16. HD_Vitvi_PHDf_HD_II | 64 | 76 | 66 | 66 | 82 | 68 | 64 | 74 | 56 | 66 | 84 | 84 | 66 | 64 | 68 | | 40 | 48 | 43 | 44 | 44 |
| 17. HD_Zeama_PHDf_HD | 74 | 60 | 94 | 76 | 64 | 72 | 62 | 56 | 62 | 76 | 60 | 62 | 78 | 100 | 82 | 64 | | 36 | 33 | 35 | 33 |
| 18. HD_Zeama_hox1a | 56 | 60 | 62 | 60 | 66 | 60 | 78 | 60 | 52 | 62 | 62 | 64 | 62 | 58 | 60 | 68 | 56 | | 86 | 60 | 56 |
| 19. HD_Zeama_hox1b | 57 | 63 | 61 | 61 | 69 | 61 | 80 | 63 | 51 | 63 | 67 | 69 | 63 | 57 | 61 | 71 | 55 | 92 | | 59 | 53 |
| 20. HD_Zeama_hox2a | 58 | 60 | 60 | 60 | 65 | 64 | 89 | 60 | 52 | 62 | 64 | 64 | 62 | 60 | 62 | 64 | 58 | 77 | 81 | | 90 |
| 21. HD_Zeama_Hox2b | 56 | 56 | 56 | 56 | 62 | 58 | 85 | 54 | 48 | 58 | 60 | 60 | 58 | 56 | 58 | 62 | 54 | 71 | 77 | 92 | |

### Example 4: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 180 are presented in Table D5.

**Table D5: InterPro scan results of the polypeptide sequence as represented by SEQ ID NO: 180**

| **InterPro accession number** | **Integrated database name** | **Integrated database accession number** | **Integrated database accession name** | **Amino acid coordinates on SEQ ID NO: 180** |
|---|---|---|---|---|
| IPR0013556 Homeobox | ProDom | PD000010 | PRH_ARATH_P48785 | 439-497 |
| IPR0013556 Homeobox | PFAM | PF00046 | Homeobox | 439-495 |
| IPR0013556 Homeobox | SMART | SM00389 | HOX | 438-500 |
| IPR0013556 Homeobox | Profile | PS50071 | Homeobox_2 | 436-496 |
| IPR00195 Zinc-finger, PHD-type | PFAM | PF00628 | PHD | 197-251 |
| IPR00195 Zinc-finger, PHD-type | SMART | SM00249 | PHD | 197-249 |
| IPR00195 Zinc-finger, PHD-type | Profile | PS50016 | ZF_PHD_2 | 195-251 |
| IPR012287 Homeodomain-related | Gene3D | G3DSA:1.10.10.60 | No description | 436-501 |
| IPR013256 Chromatin SPT2 | SMART | SM00784 | No description | 78-154 |
| Untegrated IPR | PANTHER | PTHR19418 | Homeobox protein | 397-414 |
| | | | | 431-590 |

The results of the InterPro scan clearly identifes the essential features of a PHDf-HD polypeptide, i.e., a PHDf zinc finger and homeobox, as represented for example respectively by Pfam entries PF00628 and PF00046.

HDs are known to have canonical residues. The HD of PHDf-HD polypeptides is highly divergent in sequence even at positions that are almost invariable among homeodomains. The sequence logo of the HD of the PHDf-HD polypeptides of Table D1, is shown in Figure 3. Sequence logos are a graphical representation of an amino acid or nucleic acid multiple sequence alignment. Each logo consists of stacks of symbols, one stack for each position in the sequence. The overall height of the stack indicates the sequence conservation at that position, while the height of symbols within the stack indicates the relative frequency of each amino or nucleic acid at that position. In general, a sequence logo provides a richer and more precise description of, for example, a binding site, than would a consensus sequence. The algorithm (WebLogo) to produce such logos is available at the server of the University of California, Berkeley. The HD as represented by SEQ ID NO: 234, and comprised in SEQ ID NO: 180, is in accordance with the sequence logo as represented in Figure 3. Polypeptides useful in performing the methods according to the invention comprise an HD comprised in the sequence logo as shown in Figure 3.

### Example 5: Prediction of secondary structure features of the polypeptide sequences useful in performing the methods of the invention

Coiled coils usually contain a repeated seven amino acid residue pattern called heptad repeats. Coiled coils are important to identify for protein-protein interactions, such as oligomerization, either of identical proteins, of proteins of the same family, or of unrelated proteins. Recently much progress has been made in computational prediction of coiled coils from sequence data. Many algorithms well known to a person skilled in the art are available at the ExPASy Proteomics tools. One of them, COILS, is a program that compares a sequence to a database of known parallel two-stranded coiled-coils and derives a similarity score. By comparing this score to the distribution of scores in globular and coiled-coil proteins, the program then calculates the probability that the sequence will adopt a coiled-coil conformation.

The PHDf-HD polypeptide as represented by SEQ ID NO: 180, has two N-terminal predicted coiled coil domains, with a high probability, in all three windows (14, 21 and 28) examined. In Table D6, the residue coordinates, residues, the three windows and corresponding probability values are shown. In Figure 4, is the graphical output of the COILS algorithm on the polypeptide as represented by SEQ ID NO: 180, where the two predicted coiled coils are clearly visible in the N-terminal half of the polypeptide, in all three windows (as represented by the three lines).

**Table D6: Numerical output of the COILS algorithm on the polypeptide as represented by SEQ ID NO: 180. The residue coordinates (#), residues, the three windows and corresponding probability values are shown. Probabilities above 0.09 are shown in grey.**

| **#** | *Residue* | **Window=14** | **Prob** | **Window=21** | **Prob** | **Window=28** | **Prob** |
|---|---|---|---|---|---|---|---|
| 87 | P | d | 0.001 | e | 0.001 | E | 0.001 |
| 88 | T | e | 0.063 | f | 0.274 | F | 0.099 |
| 89 | R | f | 0.063 | g | 0.567 | G | 0.099 |
| 90 | R | g | 0.063 | a | 0.567 | A | 0.099 |
| 91 | K | b | 0.066 | b | 0.642 | B | 0.188 |
| 92 | H | c | 0.066 | c | 0.642 | C | 0.188 |
| 93 | K | d | 0.069 | d | 0.642 | D | 0.591 |
| 94 | Q | e | 0.178 | e | 0.642 | E | 0.672 |
| 95 | K | f | 0.335 | f | 0.642 | F | 0.672 |
| 96 | R | g | 0.335 | g | 0.642 | G | 0.772 |
| 97 | K | a | 0.335 | a | 0.642 | A | 0.772 |
| 98 | N | b | 0.347 | b | 0.642 | B | 0.772 |
| 99 | D | c | 0.347 | c | 0.642 | C | 0.772 |
| 100 | E | d | 0.347 | d | 0.642 | D | 0.772 |
| 101 | S | e | 0.347 | e | 0.642 | E | 0.808 |
| 102 | D | f | 0.347 | f | 0.642 | F | 0.808 |
| 103 | E | g | 0.347 | g | 0.642 | g | 0.808 |
| 104 | V | a | 0.347 | a | 0.642 | a | 0.808 |
| 105 | S | b | 0.347 | b | 0.642 | b | 0.808 |
| 106 | R | c | 0.347 | c | 0.642 | C | 0.808 |
| 107 | M | d | 0.347 | d | 0.642 | d | 0.808 |
| 108 | E | e | 0.347 | e | 0.642 | e | 0.808 |
| 109 | K | f | 0.347 | f | 0.642 | F | 0.808 |
| 110 | R | g | 0.347 | g | 0.642 | g | 0.808 |
| 111 | A | a | 0.347 | a | 0.642 | a | 0.808 |
| 112 | R | b | 0.307 | b | 0.514 | b | 0.808 |
| 113 | Y | c | 0.066 | c | 0.503 | c | 0.808 |
| 114 | L | d | 0.262 | d | 0.503 | d | 0.808 |
| 115 | L | e | 0.262 | e | 0.503 | e | 0.808 |
| 116 | I | f | 0.262 | f | 0.503 | F | 0.808 |
| 117 | K | g | 0.262 | g | 0.503 | g | 0.808 |
| 118 | I | a | 0.262 | a | 0.503 | a | 0.808 |
| 119 | K | b | 0.262 | b | 0.503 | b | 0.808 |
| 120 | Q | c | 0.262 | c | 0.503 | c | 0.808 |
| 121 | E | d | 0.262 | d | 0.503 | d | 0.808 |
| 122 | Q | e | 0.262 | e | 0.503 | e | 0.808 |
| 123 | N | f | 0.262 | f | 0.503 | F | 0.808 |
| 124 | L | g | 0.262 | g | 0.434 | g | 0.808 |
| 125 | L | a | 0.262 | a | 0.434 | a | 0.808 |
| 126 | D | b | 0.262 | b | 0.434 | b | 0.808 |
| 127 | A | c | 0.262 | c | 0.304 | c | 0.808 |
| 128 | Y | d | 0.145 | d | 0.304 | d | 0.808 |
| 129 | S | e | 0.145 | e | 0.044 | e | 0.808 |
| 130 | G | f | 0.119 | f | 0.009 | F | 0.367 |
| 131 | D | g | 0.078 | g | 0.007 | g | 0.136 |
| 132 | G | a | 0.002 | a | 0.001 | a | 0.005 |
| 133 | W | b | 0 | a | 0.001 | a | 0.001 |
| 134 | N | b | 0.001 | b | 0.045 | b | 0.057 |
| 135 | G | c | 0.001 | c | 0.045 | c | 0.057 |
| 136 | H | d | 0.003 | d | 0.07 | d | 0.219 |
| 137 | S | e | 0.008 | e | 0.132 | e | 0.231 |
| 138 | R | f | 0.025 | f | 0.357 | F | 0.231 |
| 139 | E | g | 0.025 | g | 0.357 | g | 0.231 |
| 140 | K | a | 0.025 | a | 0.357 | a | 0.231 |
| 141 | I | b | 0.028 | b | 0.357 | b | 0.231 |
| 142 | K | c | 0.07 | c | 0.357 | c | 0.462 |
| 143 | P | d | 0.07 | d | 0.357 | d | 0.462 |
| 144 | E | e | 0.998 | e | 0.997 | e | 0.974 |
| 145 | K | f | 0.998 | f | 0.997 | f | 0.974 |
| 146 | E | g | 0.998 | g | 0.997 | g | 0.974 |
| 147 | L | a | 0.998 | a | 0.997 | a | 0.974 |
| 148 | Q | b | 0.998 | b | 0.997 | b | 0.974 |
| 149 | R | c | 0.998 | c | 0.997 | c | 0.974 |
| 150 | A | d | 0.998 | d | 0.997 | d | 0.974 |
| 151 | K | e | 0.998 | e | 0.997 | e | 0.974 |
| 152 | K | f | 0.998 | f | 0.997 | f | 0.974 |
| 153 | Q | g | 0.998 | g | 0.997 | g | 0.974 |
| 154 | I | a | 0.998 | a | 0.997 | a | 0.974 |
| 155 | M | b | 0.998 | b | 0.997 | b | 0.974 |
| 156 | K | c | 0.998 | c | 0.997 | c | 0.974 |
| 157 | Y | d | 0.998 | d | 0.997 | d | 0.974 |
| 158 | K | e | 0.989 | e | 0.997 | e | 0.974 |
| 159 | I | f | 0.896 | f | 0.997 | f | 0.974 |
| 160 | A | g | 0.486 | g | 0.997 | g | 0.974 |
| 161 | I | a | 0.409 | a | 0.997 | a | 0.974 |
| 162 | R | b | 0.274 | b | 0.997 | b | 0.974 |
| 163 | D | c | 0.255 | c | 0.997 | c | 0.974 |
| 164 | V | d | 0.095 | d | 0.997 | d | 0.974 |
| 165 | I | e | 0.025 | e | 0.871 | e | 0.974 |
| 166 | H | f | 0.025 | f | 0.622 | f | 0.974 |
| 167 | Q | g | 0.025 | g | 0.496 | g | 0.974 |
| 168 | L | a | 0.025 | a | 0.496 | a | 0.974 |
| 169 | D | b | 0.025 | b | 0.468 | b | 0.974 |
| 170 | L | c | 0.005 | c | 0.111 | c | 0.974 |
| 171 | C | d | 0.002 | d | 0.02 | d | 0.974 |
| 172 | S | e | 0.001 | e | 0.007 | e | 0.719 |
| 173 | S | f | 0.001 | f | 0.003 | f | 0.482 |
| 174 | S | g | 0.001 | g | 0.001 | g | 0.066 |
| 175 | G | a | 0 | a | 0 | a | 0.001 |

Another coiled coil is predicted in the C-terminal half of the PHDf-HD polypeptide as represented by SEQ ID NO: 180, with a lower probability than the two coiled domains comprised in the N-terminal half of the polypeptide.

### Example 6: Subcellular localisation prediction of the polypeptide sequences useful in performing the methods of the invention

LOCtree is an algorithm that can predict the subcellular localization and DNA-binding propensity of non-membrane proteins in non-plant and plant eukaryotes as well as prokaryotes. LOCtree classifies eukaryotic animal proteins into one of five subcellular classes, while plant proteins are classified into one of six classes and prokaryotic proteins are classified into one of three classes.

Whenever available, LOCtree also reports predictions based on the following: 1) Nuclear localization signals found by the PredictNLS algorithm, 2) Localization inferred using Prosite motifs and Pfam domains found in the protein, and 3) SWISS-PROT keywords associated with a protein. Localization is inferred in the last two cases using the entropy-based LOCkey algorithm. The software is hosted at the University of Columbia, USA.

Motif and keyword based prediction of subcellular localization of a PHDf-HD polypeptide as represented by SEQ ID NO: 180, using LOCkey:

| **Predicted Localization** | **Confidence** | **Alternative prediction** | **SWISS-PROT keywords used to assign localization** |
|---|---|---|---|
| Nuclear | 100 | - | Homeobox, DNA-binding, Transcription regulation, Nuclear protein, Transcription, Zinc-finger |

Prediction of a nuclear localisation signal (NLS) is done using PredictNLS algorithm, for example. The algorithm is also hosted by the server at the University of Columbia, USA. In the Table below, prediction of NLS on the polypeptide of SEQ ID NO: 180 using the PredictNLS algorithm, is shown.

| **Generalized motif ([KR]{3,5} between 3 and 5 times K or R)** | **Hit on polypeptide of SEQ ID NO: 180** | **Coordinates on polypeptide of SEQ ID NO: 180** |
|---|---|---|
| K[RK]{3,5}x{11,18}[RK]Kx{2,3}K | KRRRGSDAATGKSATGPTRRKHKQK | 71-95 |
| [KR]{4}x{20,24}K{1,4}xK | KRRRGSDAATGKSATGPTRRKHKQKRK | 71-97 |

In the polypeptide sequence below (SEQ ID NO: 180), the position of the predicted NLS is shown in bold and underlined twice:

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

### Example 7: Cloning of nucleic acid sequence as represented by SEQ ID NO: 179

Unless otherwise stated, recombinant DNA techniques were performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The *Oryza sativa* PHDf-HD gene was amplified by PCR using as template a rice cDNA bank synthesized from mRNA extracted from mixed plant tissues. Primer prm09687 (SEQ ID NO: 236; sense,: 5'-ggggacaagtttgtacaaaaaagcaggcttaaacaatgaataccccagaaaa gaaa-3') and primer prm09688 SEQ ID NO: 237; reverse, complementary,: 5'-ggggaccactttgtacaagaaagctgggtgatgcaaggttaagatgcttt-3'), which include the AttB sites for Gateway recombination, were used for PCR amplification. PCR was performed using Hifi Taq DNA polymerase in standard conditions. A PCR fragment of the expected length (including attB sites) was amplified and purified also using standard methods. The first step of the Gateway procedure, the BP reaction, was then performed, during which the PCR fragment recombined *in vivo* with the pDONR201 plasmid to produce, according to the Gateway terminology, an "entry clone". Plasmid pDONR201 was purchased from Invitrogen, as part of the Gateway^{®} technology.

### Example 8: Expression vector construction using the nucleic acid sequence as represented by SEQ ID NO: 179

The entry clone comprising SEQ ID NO: 179 was subsequently used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 235) for constitutive expression was located upstream of this Gateway cassette.

After the LR recombination step, the resulting expression vector pGOS2::PHDf-HD and (Figure 6) was transformed into *Agrobacterium* strain LBA4044 according to methods well known in the art.

### Example 9: Plant transformation

### Rice transformation

The *Agrobacterium* containing the expression vectors were used independently to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing each individual expression vector was used independently for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for each construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Example 10: Phenotypic evaluation procedure

### 10.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions are supplied with water at regular intervals to ensure that water and nutrients are not limiting to satisfy plant needs to complete growth and development.

From the stage of sowing until the stage of maturity the plants were passed several times through a digital imaging cabinet. At each time point digital images (2048x1536 pixels, 16 million colours) were taken of each plant from at least 6 different angles.

### 10.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

### 10.3 Parameters measured

### Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

### Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed weight per plant was measured by weighing all filled husks harvested from one plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed weight per plant and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### Reduced nutrient (nitrogen) availability screen

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters were recorded as detailed for growth under normal conditions.

### Example 11: Results of the phenotypic evaluation of the transgenic rice plants

The results of the evaluation of transgenic rice plants expressing the nucleic acid sequence encoding a PHDf-HD polypeptide as represented by SEQ ID NO: 180, under the control of the GOS2 promoter for constitutive expression, are presented below.

There was a significant increase in the number of panicles, in the total seed yield per plant, in the total number of filled seeds, in the total number of seeds, in the Thousand Kernel Weight (TKW), and in the harvest index of the transgenic plants compared to corresponding nullizygotes (controls), as shown in Table D7.

**Table D7: Results of the evaluation of transgenic rice plants expressing the nucleic acid sequence encoding a PHDf-HD polypeptide as represented by SEQ ID NO: 180, under the control of the GOS2 promoter for constitutive expression.**

| | Average % increase in 3 events in the T1 generation |
|---|---|
| Number of first panicles | 22% |
| Total seed yield per plant | 30% |
| Total number of filled seeds | 24% |
| Total number of seeds | 24% |
| TKW | 4% |
| Harvest index | 17% |

### Example 12: Results of the phenotypic evaluation of the transgenic rice plants grown under reduced nutrient availability

The results of the evaluation of transgenic rice plants expressing the nucleic acid sequence encoding a PHDf-HD polypeptide as represented by SEQ ID NO: 180, under the control of the GOS2 promoter for constitutive expression, and grown under reduced nutrient availability, are presented below.

There was a significant increase in biomass, in emergeance vigor, in the total seed yield per plant, in the total number of filled seeds, in the seed filling rate, in the total number of seeds, in the Thousand Kernel Weight (TKW), and in the harvest index of the transgenic plants compared to corresponding nullizygotes (controls), as shown in Table D8.

**Table D8: Results of the evaluation of transgenic rice plants expressing the nucleic acid sequence encoding a PHDf-HD polypeptide as represented by SEQ ID NO: 180, under the control of the GOS2 promoter for constitutive expression, and grown under reduced nutrient availability.**

| | Overall average % increase (6 events in the T2 generation) |
|---|---|
| Biomass | 8% |
| Emergeance vigor | 20% |
| Total seed yield per plant | 17% |
| Total number of filled seeds | 12% |
| Seed filling rate | 3% |
| Total number of seeds | 9% |
| TKW | 5% |
| Harvest index | 9% |

### Example 13: Examples of transformation of other crops

Transformation of corn, wheat, soybean, rapeseed/canola, alfalfa and cotton was carried out as described below.

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

### Corn transformation

Transformation of maize (Zea *mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium*, the petiole explants are transferred to MSBAP-3 medium containing 3 mg/l BAP, cefotaxime, carbenicillin, or timentin (300 mg/1) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Cotton transformation

Cotton (*Gossypium hirsutum* L.) transformation is performed using *Agrobacterium tumefaciens*, on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then co-cultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

### Example 14: Examples of abiotic stress screens

### Drought screen

Plants from a selected number of events are grown in potting soil under normal conditions until they approached the heading stage. They are then transferred to a "dry" section where irrigation is withheld. Humidity probes are inserted in randomly chosen pots to monitor the soil water content (SWC). When SWC go below certain thresholds, the plants are automatically re-watered continuously until a normal level is reached again. The plants are then retransferred to normal conditions. The rest of the cultivation (plant maturation, seed harvest) is the same as for plants not grown under abiotic stress conditions. Growth and yield parameters are recorded as detailed for growth under normal conditions.

### Salt stress screen

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants were harvested. Growth and yield parameters are recorded as detailed for growth under normal conditions.

## Claims

1. A method for enhancing seed-yield related-traits, in plants relative to control plants, comprising increasing, expression in a plant of a nucleic acid sequence encoding a plant homeodomain finger-homeodomain (PHDf-HD) polypeptide, which PHDf-HD polypeptide comprises:
(i) a domain having at least 25%, 30%, 35%, 40%, 45%, 50%, 55%, 60%, 65%, 70%, 75%, 80%, 85%, 90%, 95%, 98%, 99% or more amino acid sequence identity to a leucine zipper/plant homeodomain finger (ZIP/PHDf) domain as represented by SEQ ID NO: 233; and
(ii) a domain having at least 25%, 30%, 35%, 40%, 45%, 50%, 55%, 60%, 65%, 70%, 75%, 80%, 85%, 90%, 95%, 98%, 99% or more amino acid sequence identity to a homeodomain (HD) as represented by SEQ ID NO: 234; and
(iii) wherein said PHDf-HD polypeptide, when used in the construction of a HD phylogenetic tree, such as the one depicted in Figure 1 clusters with the PHDf-HD group of polypeptides comprising the polypeptide sequence as represented by SEQ ID NO: 180, rather than with any other HD group; and/or wherein said PHDf-HD polypeptide has in increasing order of preference at least 30%, 35%, 40%, 45%, 50%, 55%, 60%, 65%, 70%, 75%, 80%, 85%, 90%, 95%, 98%, 99% or more sequence identity to the PHDf-HD polypeptide as represented by SEQ ID NO: 180 or to any of the polypeptide sequences given in Table D1 herein, and optionally selecting for plants having enhanced yield-related traits.

2. Method according to claim 1, wherein said PHDf-HD polypeptide comprises: (i) a PHD domain as represented by PFAM00628; and (ii) an HD as represented by PFAM00046.

3. Method according to claim 1 or 2, wherein said nucleic acid sequence encoding a PHDf-HD polypeptide is represented by any one of the nucleic acid sequence SEQ ID NOs given in Table D1 or a portion thereof, or a sequence capable of hybridising with any one of the nucleic acid sequences SEQ ID NOs given in Table D1; and/or wherein said nucleic acid sequence encodes an orthologue or paralogue of any of the SEQ ID NOs given in Table D1.

4. Method according to any one of claims 1 to 3, wherein said increased, expression is effected by any one or more of: T-DNA activation tagging, TILLING, or homologous recombination.

5. Method according to any one of claims 1 to 4, wherein said increased expression is effected by introducing and expressing in a plant a nucleic acid sequence encoding a PHDf-HD polypeptide.

6. Method according to any one of claims 1 to 5, wherein said seed yield-related traits, comprising one or more of: (i) increased number of primary panicle; (ii) increased total seed weight per plant; (iii) increased number of (filled) seeds; (iv) increased TKW; or (v) increased harvest index.

7. Method according to any one of claims 1 to 6, wherein said nucleic acid sequence is operably linked to a constitutive promoter, preferably to a plant constitutive promoter, more preferably to a GOS2 promoter, most preferably to a GOS2 promoter from rice; and/or wherein said nucleic acid sequence encoding a PHDf-HD polypeptide is of plant origin, preferably from a monocotyledonous plant, further preferably from the family Poacae more preferably from the genus Oryza, most preferably from *Oryza sativa.*

8. Plants having enhanced seed yield related traits or parts thereof (including seeds), or plant cells obtainable by a method according to any one of claims 1 to 7, wherein said plant, part or cell thereof comprises an isolated nucleic acid transgene encoding a PHDf-HD polypeptide operably linked to a GOS2 promoter.

9. Construct comprising:
(i) A nucleic acid sequence encoding a PHDf-HD polypeptide as defined in any one of claims 1 to 3;
(ii) one or more control sequences capable of driving expression of the nucleic acid sequence of (i); and optionally
(iii) a transcription termination sequence.
wherein at least one of the control sequences is a GOS2 promoter.

10. Use of a construct according to claim 9 in a method for making plants having enhanced seed yield-related traits, preferably one or more of: (i) increased number of primary panicle; (ii) increased total seed weight per plant; (iii) increased number of (filled) seeds; (iv) increased TKW; or (v) increased harvest index; or use of a nucleic acid sequence encoding a PHDf-HD polypeptide as defined in any one of claims 1 to 4 in enhancing seed yield-related traits, comprising one or more of: (i) increased number of primary panicles; (ii) increased total seed weight per plant; (iii) increased number of (filled) seeds; (iv) increased TKW; or (v) increased harvest index.

11. Plant having enhanced seed yield related traits relative to control plants or plant part or plant cell thereof transformed with a construct according to claim 9.

12. Method for the production of transgenic plants having enhanced seed yield-related traits relative to control plants, comprising:
(i) introducing and expressing in a plant, plant part, or plant cell, a nucleic acid sequence encoding a PHDf-HD polypeptide as defined in any one of claims 1 to 3, under the control of plant constitutive promoter; and
(ii) cultivating the plant cell under conditions promoting plant growth and development.

13. Transgenic plant having enhanced seed yield-related traits relative to control plants, resulting from increased expression of a nucleic acid transgene encoding a PHDf-HD polypeptide as defined in any one of claims 1 to 3, operably linked to a GOS2 promoter, or a transgenic plant cell derived from said transgenic plant comprising said nucleic acid transgene; or harvestable parts of said plant comprising said nucleic acid transgene encoding a PHDf-HD polypeptide, wherein said harvestable parts are preferably seeds.

14. Transgenic plant according to claim 8, 11 or 13, wherein said plant is a crop plant or a monocot or a cereal, such as rice, maize, wheat, barley, millet, rye, triticale, sorghum and oats, or a transgenic plant cell derived from said transgenic plant.

## Patentansprüche

1. Verfahren zum Verstärken von mit dem Samenertrag in Zusammenhang stehenden Merkmalen in Pflanzen im Vergleich zu Kontrollpflanzen, bei dem man die Expression einer Nukleinsäuresequenz, die für ein Pflanzenhomöodomänefinger-Homöodomäne (PHDf-HD) -Polypeptid codiert, erhöht, wobei das PHDf-HD-Polypeptid Folgendes umfasst:
(i) eine Domäne mit mindestens 25%, 30%, 35%, 40%, 45%, 50%, 55%, 60%, 65%, 70%, 75%, 80%, 85%, 90%, 95%, 98%, 99% oder mehr Aminosäuresequenzidentität zu einer Leucin-Zipper/Pflanzenhomöodomänefinger (ZIP/PHDf) -Domäne gemäß SEQ ID NO: 233; und
(ii) eine Domäne mit mindestens 25%, 30%, 35%, 40%, 45%, 50%, 55%, 60%, 65%, 70%, 75%, 80%, 85%, 90%, 95%, 98%, 99% oder mehr Aminosäuresequenzidentität zu einer Homöodomäne (HD) gemäß SEQ ID NO: 234; und
(iii) wobei das PHDf-HD-Polypeptid, wenn es in der Konstruktion eines phylogenetischen HD-Baums, wie dem in Fig. 1 dargestellten, verwendet wird, eher mit der Gruppe der PHDf-HD-Polypeptide umfassend die Polypeptidsequenz gemäß SEQ ID NO: 180 und nicht mit einer anderen HD-Gruppe Cluster bilden; und/oder wobei das PHDf-HD-Polypeptid, mit steigender Bevorzugung, mindestens 30%, 35%, 40%, 45%, 50%, 55%, 60%, 65%, 70%, 75%, 80%, 85%, 90%, 95%, 98%, 99% oder mehr Sequenzidentität mit dem PHDf-HD-Polypeptid gemäß SEQ ID NO: 180 oder mit einer der hierin in Tabelle D1 angegebenen Polypeptidsequenzen aufweist, und gegebenenfalls auf Pflanzen mit verstärkten mit dem Samenertrag in Zusammenhang stehenden Merkmalen selektiert.

2. Verfahren nach Anspruch 1, wobei das PHDf-HD-Polypeptid Folgendes umfasst: (i) eine PHD-Domäne gemäß PFAM00628; und (ii) eine HD gemäß PFAM00046.

3. Verfahren nach Anspruch 1 oder 2, wobei die Nukleinsäuresequenz, die für ein PHDf-HD-Polypeptid codiert, durch eine der in Tabelle D1 angegebenen Nukleinsäuresequenz SEQ ID Nos oder einen Teil davon, oder einer Sequenz, die fähig ist, die mit einer der in Tabelle D1 angegebenen Nukleinsäuresequenz SEQ ID Nos zu hybridisieren, repräsentiert wird; und/oder wobei die Nukleinsäuresequenz für ein Ortholog oder Paralog von einer der in Tabelle D1 angegebenen SEQ ID Nos codiert.

4. Verfahren nach einem der Ansprüche 1 bis 3, wobei die erhöhte Expression durch einen oder mehrere der folgenden Vorgänge bewirkt wird: T-DNA-Aktivierungs-Tagging, TILLING oder homologe Rekombination.

5. Verfahren nach einem der Ansprüche 1 bis 4, wobei die erhöhte Expression durch Einführen und Exprimieren einer Nukleinsäuresequenz, die für ein PHDf-HD-Polypeptid codiert, in eine(r) Pflanze bewirkt wird.

6. Verfahren nach einem der Ansprüche 1 bis 5, wobei die mit dem Samenertrag in Zusammenhang stehenden Merkmale eines oder mehrere der folgenden Merkmale umfassen: (i) erhöhte Anzahl an Primärrispen; (ii) erhöhtes Samengesamtgewicht pro Pflanze; (iii) erhöhte Anzahl (gefüllte) Samen; (iv) erhöhtes TKG; oder (v) erhöhter Harvest Index.

7. Verfahren nach einem der Ansprüche 1 bis 6, wobei die Nukleinsäuresequenz operativ mit einem konstitutiven Promoter, vorzugsweise mit einem pflanzlichen konstitutiven Promoter, stärker bevorzugt mit einem GOS2-Promoter, am stärksten bevorzugt mit einem GOS2-Promoter aus Reis, verknüpft ist; und/oder wobei die Nukleinsäuresequenz, die für ein PHDf-HD-Polypeptid codiert, pflanzlichen Ursprungs ist und vorzugsweise von einer monokotylen Pflanze, stärker bevorzugt von der Familie Poacae, stärker bevorzugt von der Gattung Oryza, am stärksten bevorzugt von *Oryza sativa* stammt.

8. Pflanzen mit verstärkten mit dem Samenertrag in Zusammenhang stehenden Merkmalen oder Teile davon (einschließlich Samen), oder Pflanzenzellen, die nach einem Verfahren nach einem der Ansprüche 1 bis 7 erhältlich sind, wobei die Pflanze, der Teil oder die Zelle davon ein isoliertes Nukleinsäuretransgen, das für ein PHDf-HD-Polypeptid codiert, in operativer Verknüpfung mit einem GOS2-Promoter umfasst.

9. Konstrukt, das Folgendes umfasst:
(i) eine Nukleinsäuresequenz, die für ein wie in einem der Ansprüche 1 bis 3 definiertes PHDf-HD-Polypeptid codiert;
(ii) eine oder mehrere Kontrollsequenzen, die fähig sind, die Expression der Nukleinsäuresequenz von (i) voranzutreiben; und gegebenenfalls
(iii) eine Transkriptionsterminationssequenz,
wobei es sich bei mindestens einer der Kontrollsequenzen um einen GOS2-Promoter handelt.

10. Verwendung eines Konstrukts nach Anspruch 9 in einem Verfahren zur Herstellung von Pflanzen mit mit dem Samenertrag in Zusammenhang stehenden Merkmalen, vorzugsweise einem oder mehreren der folgenden Merkmale: (i) erhöhte Anzahl Primärrispen; (ii) erhöhtes Samengesamtgewicht pro Pflanze; (iii) erhöhte Anzahl (gefüllte) Samen; (iv) erhöhtes TKG; oder (v) erhöhter Harvest Index; oder Verwendung einer Nukleinsäuresequenz, die für ein wie in einem der Ansprüche 1 bis 4 definiertes PHDf-HD-Polypeptid codiert, zum Verstärken von mit dem Samenertrag in Zusammenhang stehenden Merkmalen, das eines oder mehrere der folgenden Merkmale umfasst: (i) erhöhte Anzahl Primärrispen; (ii) erhöhtes Samengesamtgewicht pro Pflanze; (iii) erhöhte Anzahl (gefüllte) Samen; (iv) erhöhtes TKG; oder (v) erhöhter Harvest Index.

11. Pflanze mit im Vergleich zu Kontrollpflanzen verstärkten mit dem Samenertrag in Zusammenhang stehenden Merkmalen, oder Pflanzenteil oder Pflanzenzelle davon, die/der mit einem Konstrukt nach Anspruch 9 transformiert ist.

12. Verfahren zur Herstellung von transgenen Pflanzen mit im Vergleich zu Kontrollpflanzen verstärkten mit dem Samenertrag im Zusammenhang stehenden Merkmalen, das Folgendes umfasst:
(i) Einführen und Exprimieren in einer Nukleinsäuresequenz, die für ein wie in einem der Ansprüche 1 bis 3 definiertes PHDf-HD-Polypeptid codiert, unter der Kontrolle eines pflanzlichen konstitutiven Promoters in eine(r) Pflanze, einen/einem Pflanzenteil oder eine(r) Pflanzenzelle; und
(ii) Kultivieren der Pflanzenzelle unter Bedingungen, die das Pflanzenwachstum und die Pflanzenentwicklung fördern.

13. Transgene Pflanze mit im Vergleich zu Kontrollpflanzen verstärkten mit dem Samenertrag in Zusammenhang stehenden Merkmalen aufgrund einer erhöhten Expression eines Nukleinsäuretransgens, das für ein wie in einem der Ansprüche 1 bis 3 definiertes PHDf-HD-Polypeptid codiert, in operativer Verknüpfung mit einem GOS2-Promoter, oder transgene Pflanzenzelle, die von der transgenen Pflanze, die das Nukleinsäuretransgen umfasst, abstammt; oder Erntegut der Pflanze, die das Nukleinsäuretransgen, das für ein PHDf-HD-Polypeptid codiert, umfasst, wobei es sich bei dem Erntegut vorzugsweise um Samen handelt.

14. Transgene Pflanze nach Anspruch 8, 11 oder 13, wobei es sich bei der Pflanze um eine Kulturpflanze oder eine monokotyle Pflanze oder ein Getreide, wie Reis, Mais, Weizen, Gerste, Hirse, Roggen, Triticale, Sorghum und Hafer handelt, oder transgene Pflanzenzelle, die von der transgenen Pflanze abstammt.

## Revendications

1. Procédé d'amélioration de caractères associés au rendement en graines, chez des plantes par rapport à des plantes témoins, comprenant l'augmentation de l'expression dans une plante d'une séquence d'acide nucléique codant pour un polypeptide d'homéodomaine de doigt d'homéodomaine géant (PHDf-HD), ledit polypeptide PHDf-HD comprenant :
(i) un domaine ayant au moins 25 %, 30 %, 35 %, 40 %, 45 %, 50 %, 55 %, 60 %, 65 %, 70 %, 75 %, 80 %, 85 %, 90 %, 95 %, 98 %, 99 % ou plus d'identité de séquence d'acides aminés avec une glissière à leucine/domaine de doigt d'homéodomaine de plante (ZIP/PHDf) tel que représenté par SEQ ID NO: 233 ; et
(ii) un domaine ayant au moins 25 %, 30 %, 35 %, 40 %, 45 %, 50 %, 55 %, 60 %, 65 %, 70 %, 75 %, 80 %, 85 %, 90 %, 95 %, 98 %, 99 % ou plus d'identité de séquence d'acides aminés avec un homéodomaine (HD) tel que représenté par SEQ ID NO: 234 ; et
(iii) dans lequel ledit polypeptide PHDf-HD, lorsqu'il est utilisé dans la construction d'un arbre phylogénétique HD, tel que celui décrit sur la figure 1 s'agrège avec le groupe PHDf-HD de polypeptides comprenant la séquence de polypeptide telle que représentée par SEQ ID NO: 180, plutôt qu'avec tout autre groupe HD ; et/ou dans lequel ledit polypeptide PHDf-HD possède, par ordre croissant de préférence, au moins 30 %, 35 %, 40 %, 45 %, 50 %, 55 %, 60 %, 65 %, 70 %, 75 %, 80 %, 85 %, 90 %, 95 %, 98 %, 99 % ou plus d'identité de séquence avec le polypeptide PHDf-HD tel que représenté par SEQ ID NO: 180 ou avec l'une quelconque des séquences polypeptidiques présentées dans le tableau D1 présentement, et facultativement la sélection de plantes ayant des caractères associés au rendement améliorés.

2. Procédé selon la revendication 1, dans lequel ledit polypeptide PHDf-HD comprend : (i) un domaine PHD tel que représenté par PFAM00628 ; et (ii) un HD tel que représenté par PFAM00046.

3. Procédé selon la revendication 1 ou 2, dans lequel ladite séquence d'acide nucléique codant pour un polypeptide PHDf-HD est représentée par l'un quelconque des SEQ ID NO de séquences d'acide nucléique présentés dans le tableau D1 ou une partie de ceux-ci, ou une séquence capable de s'hybrider avec l'un quelconque des SEQ ID NO de séquences d'acide nucléique présentés dans le tableau D1 ; et/ou dans lequel ladite séquence d'acide nucléique code pour un orthologue ou un paralogue de l'un quelconque des SEQ ID NO présentés dans le tableau D1.

4. Procédé selon l'une quelconque des revendications 1 à 3, dans lequel ladite expression augmentée est obtenue par l'un quelconque ou plusieurs de : l'étiquetage d'activation d'ADN-T, le TILLING ou une recombinaison homologue.

5. Procédé selon l'une quelconque des revendications 1 à 4, dans lequel ladite expression augmentée est effectuée par introduction et expression dans une plante d'une séquence d'acide nucléique codant pour un polypeptide PHDf-HD.

6. Procédé selon l'une quelconque des revendications 1 à 5, dans lequel lesdits caractères associés au rendement en graines, comprenant l'un ou plusieurs de : (i) un nombre de panicules primaires augmenté ; (ii) un poids total de graines par plante augmenté ; (iii) un nombre de graines (remplies) augmenté ; (iv) TKW augmenté ; ou (v) un indice de récolte augmenté.

7. Procédé selon l'une quelconque des revendications 1 à 6, dans lequel ladite séquence d'acide nucléique est fonctionnellement liée à un promoteur constitutif, de préférence à un promoteur constitutif de plante, plus préférablement un promoteur GOS2, de manière préférée entre toutes un promoteur GOS2 de riz ; et/ou dans lequel ladite séquence d'acide nucléique codant pour un polypeptide PHDf-HD est d'origine végétale, de préférence d'une plante monocotylédone, plus préférablement de la famille Poacae, plus préférablement du genre Oryza, de manière préférée entre toutes d'*Oryza sativa.*

8. Plantes ayant des caractères associés au rendement en graines améliorés ou parties de celles-ci (comprenant les graines), ou cellules de plante pouvant être obtenues par un procédé selon l'une quelconque des revendications 1 à 7, **caractérisées en ce que** la plante, partie ou cellule de celle-ci comprend un transgène d'acide nucléique isolé codant pour un polypeptide PHDf-HD fonctionnellement lié à un promoteur GOS2.

9. Construction comprenant :
(i) une séquence d'acide nucléique codant pour un polypeptide PHDf-HD tel que défini dans l'une quelconque des revendications 1 à 3 ;
(ii) une ou plusieurs séquences de contrôle capables de contrôler l'expression de la séquence d'acide nucléique de (i) ; et facultativement
(iii) une séquence de terminaison de transcription,
dans laquelle au moins une des séquences de contrôle est un promoteur GOS2.

10. Utilisation d'une construction selon la revendication 9 dans un procédé de fabrication de plantes ayant des caractères associés au rendement en graines amélioré, de préférence un ou plusieurs de : (i) un nombre de panicules primaires augmenté ; (ii) un poids total de graines par plante augmenté ; (iii) un nombre de graines (remplies) augmenté ; (iv) TKW augmenté ; ou (v) un indice de récolte augmenté ; ou utilisation d'une séquence d'acide nucléique codant pour un polypeptide PHDf-HD tel que défini dans l'une quelconque des revendications 1 à 4 dans l'amélioration de caractères associés au rendement en graines, comprenant un ou plusieurs de : (i) un nombre de panicules primaires augmenté ; (ii) un poids total de graines par plante augmenté ; (iii) un nombre de graines (remplies) augmenté ; (iv) TKW augmenté ; ou (v) un indice de récolte augmenté.

11. Plante ayant des caractères associés au rendement en graines améliorés par rapport à des plantes témoins ou partie de plante ou cellule de plante de celle-ci transformée avec une construction selon la revendication 9.

12. Procédé de production de plantes transgéniques ayant des caractères associés au rendement en graines augmenté par rapport à des plantes témoins, comprenant :
(i) l'introduction et l'expression dans une plante, partie de plante, ou cellule de plante, une séquence d'acide nucléique codant pour un polypeptide PHDf-HD tel que défini dans l'une quelconque des revendications 1 à 3, sous le contrôle d'un promoteur constitutif de plante ; et
(ii) la culture de la cellule de plante dans des conditions stimulant la croissance et le développement de plante.

13. Plantes transgéniques ayant des caractères associés au rendement en graines améliorés par rapport à des plantes témoins, résultant de l'expression accrue d'un transgène d'acide nucléique codant pour un polypeptide PHDf-HD tel que défini dans l'une quelconque des revendications 1 à 3, fonctionnellement lié à un promoteur GOS2, ou une cellule de plante transgénique dérivée de ladite plante transgénique comprenant ledit transgène d'acide nucléique ; ou des parties récoltables de ladite plante comprenant ledit transgène d'acide nucléique codant pour un polypeptide PHDf-HD, lesdites parties récoltables étant de préférence des graines.

14. Plante transgénique selon la revendication 8, 11 ou 13, ladite plante étant une plante cultivée ou une monocotylédone ou une céréale, telle que le riz, le maïs, le blé, l'orge, le millet, le seigle, la triticale, le sorgho et l'avoine, ou une cellule de plante transgénique dérivée de ladite plante transgénique.


### Sequence Listings, SEQ ID NO 179-243

<210> 179
   <211> 2046
   <212> DNA
   <213> Oryza sativa
<400> 179
   atgaataccc cagaaaagaa accactgtgc tatacttcta gaagagcact gcaacagagg     60
   acagaatcct cttcggaatt gatctctgta tcaaaaagag caacccgcca gaatactcca    120
   cgcaagccgg attctcctcc caaacgaaca acaagaagta gcgctaacct ggcgaaatgt    180
   atagagaaca aacaccatag cagtcctctg aagcggcggc ggggttcaga tgcggctacc 240
   ggaaagtctg caacagggcc aacaaggagg aagcataaac agaaaaggaa aaatgatgag 300
   agtgatgagg tcagtcgcat ggaaaaaaga gcaagatatt tacttatcaa aataaaacag 360
   gagcagaatt tgctagatgc atactctgga gatggttgga atgggcacag tcgagagaaa 420
   ataaagccag agaaggagct acagcgtgct aagaaacaga tcatgaaata caaaatcgct 480
   atccgtgacg tcattcacca acttgatttg tgtagttcca gtgggagcaa ggatgactct 540
   gtgattccac cagatgggtg tcacgagtct gtcaatcctg aacatacaat atgttcaagg 600
   tgcaaatccc acgagtcatt tcctgacaat aacattatat tctgtgaggg gggctgcaaa 660
   ctggcgtgtc atcagaaatg tttggagcct ccttttgata aaattcttcc aactacccgc 720
   catgggcggc tttgcaaaca ctgttcttcc aagatgaaaa ttttagacgc cattaatgcc 780
   catctgggga caagttttac agtgaagtgt ccctccagtg atattttcaa ggaagcagct 840
   gaacatttca actctgatga tggactgggc caagattggc tatctgagta ttcaggtgat 900
   gaggactatg accctgaaga aaatgaggcc agcagcagtg gtgaagagaa taaatctgcc 960
   gattccaatt gttcagggag tccactttat tctccaaatg atgatattcc agacttcata 1020
   tcagcagatt tcaatgatgc tgaaggattc tgtcgtgaaa gttcaaatct aggaatcgat 1080
   tttggtgaag atggtttggc tgagattctt acccaccaaa ggccaagaag agatgttgac 1140
   tatacacaac ttaatgagca aatgtttgga gagcctattg gcaatgacga acagagtgaa 1200
   gatgaagatt ggggtcttaa caagagaaag aaaagaagaa ctggttcaac tggtgttggg 1260
   actaattccg tagagggtcg atcagatgtc aaatccaata agaaagccca acctcggaga 1320
   aaacttttca ggattcctcc tgcggcagtt gaggtgctcc gtaaagcttt tgctgaaaat 1380
   gagcttcctg cccggagtgt caaagaaaat ctctcaacag agctgggtat ttcttttgaa 1440
   aagattgata agtggttcaa aaatacacgg tgtgctgctc tcagggatag gaagggtgaa 1500
   agtcgttatt ctggtcccag caaaaggtca agaacaagca tagaaaaggc tgaaacttca 1560
   gctaaagtgg atcaaatgga caactcatgc tttcttccct tatctgaaat aatcaacgtg 1620
   cccacacgtc tgcagaaagg tcttgataag aagccaaaat caattaacag ccctccaaga 1680
   cctcaggaca atgaaacttg cttgtcgcca actgataaaa ccaaggaggg tacaccgcct 1740
   acgatcaagc cttccatcac agattctagt caactgatga ataacgacat cggcactgag 1800
   gagactgctg tttcttgggt ggacacttgg gcctctgacg ctctgcattt cttggacgtg 1860
   agcgatgatg aacatttctt cgatgtgatc gagaaggtgt gcggtctcga gaatcggctg 1920
   cagcggttga aggagaacat gctatcatcg tcatcgtcaa ctgacaacaa tgtggctgct 1980
   gagagtggct tgcaaaacga agttgtgctt gtaccagctg ctgagctcaa ggataaagca 2040
   tcttaa 2046
<210> 180
   <211> 681
   <212> PRT
   <213> Oryza sativa
<400> 180
   Met Asn Thr Pro Glu Lys Lys Pro Leu Cys Tyr Thr Ser Arg Arg Ala
   1               5                   10                  15
   Leu Gln Gln Arg Thr Glu Ser Ser Ser Glu Leu Ile Ser Val Ser Lys
               20                  25                  30
   Arg Ala Thr Arg Gln Asn Thr Pro Arg Lys Pro Asp Ser Pro Pro Lys
           35                  40                  45
   Arg Thr Thr Arg Ser Ser Ala Asn Leu Ala Lys Cys Ile Glu Asn Lys
       50                  55                  60
   His His Ser Ser Pro Leu Lys Arg Arg Arg Gly Ser Asp Ala Ala Thr
   65                  70                  75                  80
   Gly Lys Ser Ala Thr Gly Pro Thr Arg Arg Lys His Lys Gln Lys Arg
                   85                  90                  95
   Lys Asn Asp Glu Ser Asp Glu Val Ser Arg Met Glu Lys Arg Ala Arg
               100                 105                 110
   Tyr Leu Leu Ile Lys Ile Lys Gln Glu Gln Asn Leu Leu Asp Ala Tyr
           115                 120                 125
   Ser Gly Asp Gly Trp Asn Gly His Ser Arg Glu Lys Ile Lys Pro Glu
       130                 135                 140
   Lys Glu Leu Gln Arg Ala Lys Lys Gln Ile Met Lys Tyr Lys Ile Ala
   145                 150                 155                 160
   Ile Arg Asp Val Ile His Gln Leu Asp Leu Cys Ser Ser Ser Gly Ser
                   165                 170                 175
   Lys Asp Asp Ser Val Ile Pro Pro Asp Gly Cys His Glu Ser Val Asn
               180                 185                 190
   Pro Glu His Thr Ile Cys Ser Arg Cys Lys Ser His Glu Ser Phe Pro
           195                 200                 205
   Asp Asn Asn Ile Ile Phe Cys Glu Gly Gly Cys Lys Leu Ala Cys His
       210                 215                 220
   Gln Lys Cys Leu Glu Pro Pro Phe Asp Lys Ile Leu Pro Thr Thr Arg
   225                 230                 235                 240
   His Gly Arg Leu Cys Lys His Cys Ser Ser Lys Met Lys Ile Leu Asp
                   245                 250                 255
   Ala Ile Asn Ala His Leu Gly Thr Ser Phe Thr Val Lys Cys Pro Ser
               260                 265                 270
   Ser Asp Ile Phe Lys Glu Ala Ala Glu His Phe Asn Ser Asp Asp Gly
           275                 280                 285
   Leu Gly Gln Asp Trp Leu Ser Glu Tyr Ser Gly Asp Glu Asp Tyr Asp
       290                 295                 300
   Pro Glu Glu Asn Glu Ala Ser Ser Ser Gly Glu Glu Asn Lys Ser Ala
   305                 310                 315                 320
   Asp Ser Asn Cys Ser Gly Ser Pro Leu Tyr Ser Pro Asn Asp Asp Ile
                   325                 330                 335
   Pro Asp Phe Ile Ser Ala Asp Phe Asn Asp Ala Glu Gly Phe Cys Arg
               340                 345                 350
   Glu Ser Ser Asn Leu Gly Ile Asp Phe Gly Glu Asp Gly Leu Ala Glu
           355                 360                 365
   Ile Leu Thr His Gln Arg Pro Arg Arg Asp Val Asp Tyr Thr Gln Leu
       370                 375                 380
   Asn Glu Gln Met Phe Gly Glu Pro Ile Gly Asn Asp Glu Gln Ser Glu
   385                 390                 395                 400
   Asp Glu Asp Trp Gly Leu Asn Lys Arg Lys Lys Arg Arg Thr Gly Ser
                   405                 410                 415
   Thr Gly Val Gly Thr Asn Ser Val Glu Gly Arg Ser Asp Val Lys Ser
               420                 425                 430
   Asn Lys Lys Ala Gln Pro Arg Arg Lys Leu Phe Arg Ile Pro Pro Ala
           435                 440                 445
   Ala Val Glu Val Leu Arg Lys Ala Phe Ala Glu Asn Glu Leu Pro Ala
       450                 455                 460
   Arg Ser Val Lys Glu Asn Leu Ser Thr Glu Leu Gly Ile Ser Phe Glu
   465                 470                 475                 480
   Lys Ile Asp Lys Trp Phe Lys Asn Thr Arg Cys Ala Ala Leu Arg Asp
                   485                 490                 495
   Arg Lys Gly Glu Ser Arg Tyr Ser Gly Pro Ser Lys Arg Ser Arg Thr
               500                 505                 510
   Ser Ile Glu Lys Ala Glu Thr Ser Ala Lys Val Asp Gln Met Asp Asn
           515                 520                 525
   Ser Cys Phe Leu Pro Leu Ser Glu Ile Ile Asn Val Pro Thr Arg Leu
       530                 535                 540
   Gln Lys Gly Leu Asp Lys Lys Pro Lys Ser Ile Asn Ser Pro Pro Arg
   545                 550                 555                 560
   Pro Gln Asp Asn Glu Thr Cys Leu Ser Pro Thr Asp Lys Thr Lys Glu
                   565                 570                 575
   Gly Thr Pro Pro Thr Ile Lys Pro Ser Ile Thr Asp Ser Ser Gln Leu
               580                 585                 590
   Met Asn Asn Asp Ile Gly Thr Glu Glu Thr Ala Val Ser Trp Val Asp
           595                 600                 605
   Thr Trp Ala Ser Asp Ala Leu His Phe Leu Asp Val Ser Asp Asp Glu
       610                 615                 620
   His Phe Phe Asp Val Ile Glu Lys Val Cys Gly Leu Glu Asn Arg Leu
   625                 630                 635                 640
   Gln Arg Leu Lys Glu Asn Met Leu Ser Ser Ser Ser Ser Thr Asp Asn
                   645                 650                 655
   Asn Val Ala Ala Glu Ser Gly Leu Gln Asn Glu Val Val Leu Val Pro
               660                 665                 670
   Ala Ala Glu Leu Lys Asp Lys Ala Ser
           675                 680
<210> 181
   <211> 2391
   <212> DNA
   <213> Arabidopsis thaliana
<400> 181
   atggaggaga gtgaaacaaa ggggagaatc agtcaagaaa ctgataaggc ttgtgtttca     60
   gttgagagaa ttgggtctac tttactctca tcatttgtga agaaaggtaa agaagtatca    120
   aataagagaa attctaagca gaataagcga aaagcggaag aagaactctg ttcgaaatca    180
   agaaccaaga agtactctcg gggatgggtc cgttgtgagg aaatggagga ggagaaggtt    240
   aagaaaacaa gaaagaggaa aagtaagaga cagcaaaagg ataataaggt tgaggttgat    300
   gattctttaa ggttgcaaag gaggacaagg tatttgctca ttaagatgaa gatgcagcag    360
   aatcttatcg atgcatacgc aactgaaggt tggaaaggtc agagccggga aaagataaga    420
   ccagacaagg agcttgagag agcccggaaa gagatcttaa actgcaagct tggactacgc    480
   gacgccattc gccaactgga tctgcttagt tctgtgggaa gcatggaaga gaaagtgata    540
   gcttcagatg gatctattca tcatgatcat atattttgtg cagaatgcaa ttctcgagaa    600
   gcttttccag acaatgatat aatactttgt gatggaacgt gtaatcgagc atttcaccag    660
   aaatgccttg accctccttt ggaaacagaa agcatacctc ccggagatca gggctggttt    720
   tgcaaatttt gtgattgcaa aattgaaatt atcgacacaa tgaatgcaca aattggaact    780
   catttccctg tggacagcaa ctggcaggat atcttcaatg aagaagctag tcttcctatt     840
   ggatctgaag ctacagttaa caatgaagca gattggcctt cggatgactc caaggatgat     900
   gattatgacc ctgaaatgag ggaaaacggt ggaggaaaca gcagcaacgt tagtggtgat     960
   ggtggtggtg ataacgatga agaaagcatt tcaactagct tgagcttatc atctgatggt    1020
   gttgcccttt caacgggatc atgggaaggc catagactca gcaatatggt ggagcagtgc    1080
   gaaacaagta atgaagaaac tgtatgtggg ccaagacagc gaaggactgt tgactataca    1140
   cagttgtact atgaaatgtt tggaaaggat gcagttttgc aagagcaagg tagtgaagat    1200
   gaagactggg gtccgaatga cagaaggaag agaaaaaggg aatctgatgc aggaagcaca    1260
   cttgtaacta tgtgtgaaag cagtaagaaa gatcaagatg ttgtagaaac gctagaacag    1320
   agtgagagag attctgtctc tgtggaaaat aaaggaggtc gaaggcggat gttcagactc    1380
   ccaagaaatg cagttgagaa gctacgtcaa gtatttgcag agactgagct tccctcaaaa    1440
   gctgtgaggg atcgtcttgc aaaagagctg agtcttgatc cagagaaggt caataaatgg    1500
   tttaaaaata ctcggtatat ggcactgcgg aataggaaga ctgagagcgt gaaacaacct    1560
   ggggattcta agacagtctc tggaggagat tctggaccag aagcagtcat ggagaacaac    1620
   actgaaacaa atgaagttca agatactttg gatgacactg ttccacccgg atttgacgct    1680
   acaaatcaaa acatactctc cccatgtaat aacaaccagg aggaatttca acaggaaaat    1740
   gtctcttttc cttcacccac agatgaaagt caacagtatc tggaacaaaa cgattcttct    1800
   ttcgttctag tgccacatga aaagcaaagc agcgaaatta gcttaaagac ggctgtagag    1860
   gagaatgaga cagaaagcaa gatgatgaaa gagccacatg aagagctaag cagtgaaatg    1920
   agcctaaaga cggctgctga ggagaaggag acagagagca agatgataga agagccacat    1980
   gaagagctaa gcagagaaat gagcttaaag acggctgtag aggagaagga gacagagagc    2040
   aagatgatgg aagagccaca tgatgagcta aacagcgaaa tgagcttaag tacagcggta    2100
   gaggagaagg agacagggag caagatgacg gaagagtcac atgaagagct aagcaacgaa    2160
   atgagcttag aagagaagga gacggggaga aagatgacgg aagaggaaga attggaagca    2220
   gtaatggaga tgctttgtag aacagaaaac aagttactgg acgtgacgca gaggcttgat    2280
   agatttaaaa caccaaaagg ccgaaaaaag ttaggcaatt cttcttctcc tttacttgaa    2340
   gaagactcag tagtgtatgt tccaatagca gagatcaagg agaaaaggta a             2391
<210> 182
   <211> 796
   <212> PRT
   <213> Arabidopsis thaliana
<400> 182
   Met Glu Glu Ser Glu Thr Lys Gly Arg Ile Ser Gln Glu Thr Asp Lys
   1               5                   10                  15
   Ala Cys Val Ser Val Glu Arg Ile Gly Ser Thr Leu Leu Ser Ser Phe
               20                  25                  30
   Val Lys Lys Gly Lys Glu Val Ser Asn Lys Arg Asn Ser Lys Gln Asn
           35                  40                  45
   Lys Arg Lys Ala Glu Glu Glu Leu Cys Ser Lys Ser Arg Thr Lys Lys
       50                  55                  60
   Tyr Ser Arg Gly Trp Val Arg Cys Glu Glu Met Glu Glu Glu Lys Val
   65                  70                  75                  80
   Lys Lys Thr Arg Lys Arg Lys Ser Lys Arg Gln Gln Lys Asp Asn Lys
                   85                  90                  95
   Val Glu Val Asp Asp Ser Leu Arg Leu Gln Arg Arg Thr Arg Tyr Leu
               100                 105                 110
   Leu Ile Lys Met Lys Met Gln Gln Asn Leu Ile Asp Ala Tyr Ala Thr
           115                 120                 125
   Glu Gly Trp Lys Gly Gln Ser Arg Glu Lys Ile Arg Pro Asp Lys Glu
       130                 135                 140
   Leu Glu Arg Ala Arg Lys Glu Ile Leu Asn Cys Lys Leu Gly Leu Arg
   145                 150                 155                 160
   Asp Ala Ile Arg Gln Leu Asp Leu Leu Ser Ser Val Gly Ser Met Glu
                   165                 170                 175
   Glu Lys Val Ile Ala Ser Asp Gly Ser Ile His His Asp His Ile Phe
               180                 185                 190
   Cys Ala Glu Cys Asn Ser Arg Glu Ala Phe Pro Asp Asn Asp Ile Ile
           195                 200                 205
   Leu Cys Asp Gly Thr Cys Asn Arg Ala Phe His Gln Lys Cys Leu Asp
       210                 215                 220
   Pro Pro Leu Glu Thr Glu Ser Ile Pro Pro Gly Asp Gln Gly Trp Phe
   225                 230                 235                 240
   Cys Lys Phe Cys Asp Cys Lys Ile Glu Ile Ile Asp Thr Met Asn Ala
                   245                 250                 255
   Gln Ile Gly Thr His Phe Pro Val Asp Ser Asn Trp Gln Asp Ile Phe
               260                 265                 270
   Asn Glu Glu Ala Ser Leu Pro Ile Gly Ser Glu Ala Thr Val Asn Asn
           275                 280                 285
   Glu Ala Asp Trp Pro Ser Asp Asp Ser Lys Asp Asp Asp Tyr Asp Pro
       290                 295                 300
   Glu Met Arg Glu Asn Gly Gly Gly Asn Ser Ser Asn Val Ser Gly Asp
   305                 310                 315                 320
   Gly Gly Gly Asp Asn Asp Glu Glu Ser Ile Ser Thr Ser Leu Ser Leu
                   325                 330                 335
   Ser Ser Asp Gly Val Ala Leu Ser Thr Gly Ser Trp Glu Gly His Arg
               340                 345                 350
   Leu Ser Asn Met Val Glu Gln Cys Glu Thr Ser Asn Glu Glu Thr Val
           355                 360                 365
   Cys Gly Pro Arg Gln Arg Arg Thr Val Asp Tyr Thr Gln Leu Tyr Tyr
       370                 375                 380
   Glu Met Phe Gly Lys Asp Ala Val Leu Gln Glu Gln Gly Ser Glu Asp
   385                 390                 395                 400
   Glu Asp Trp Gly Pro Asn Asp Arg Arg Lys Arg Lys Arg Glu Ser Asp
                   405                 410                 415
   Ala Gly Ser Thr Leu Val Thr Met Cys Glu Ser Ser Lys Lys Asp Gln
               420                 425                 430
   Asp Val Val Glu Thr Leu Glu Gln Ser Glu Arg Asp Ser Val Ser Val
           435                 440                 445
   Glu Asn Lys Gly Gly Arg Arg Arg Met Phe Arg Leu Pro Arg Asn Ala
       450                 455                 460
   Val Glu Lys Leu Arg Gln Val Phe Ala Glu Thr Glu Leu Pro Ser Lys
   465                 470                 475                 480
   Ala Val Arg Asp Arg Leu Ala Lys Glu Leu Ser Leu Asp Pro Glu Lys
                   485                 490                 495
   Val Asn Lys Trp Phe Lys Asn Thr Arg Tyr Met Ala Leu Arg Asn Arg
               500                 505                 510
   Lys Thr Glu Ser Val Lys Gln Pro Gly Asp Ser Lys Thr Val Ser Gly
           515                 520                 525
   Gly Asp Ser Gly Pro Glu Ala Val Met Glu Asn Asn Thr Glu Thr Asn
       530                 535                 540
   Glu Val Gln Asp Thr Leu Asp Asp Thr Val Pro Pro Gly Phe Asp Ala
   545                 550                 555                 560
   Thr Asn Gln Asn Ile Leu Ser Pro Cys Asn Asn Asn Gln Glu Glu Phe
                   565                 570                 575
   Gln Gln Glu Asn Val Ser Phe Pro Ser Pro Thr Asp Glu Ser Gln Gln
   580 585 590
   Tyr Leu Glu Gln Asn Asp Ser Ser Phe Val Leu Val Pro His Glu Lys
   595 600 605
   Gln Ser Ser Glu Ile Ser Leu Lys Thr Ala Val Glu Glu Asn Glu Thr
   610 615 620
   Glu Ser Lys Met Met Lys Glu Pro His Glu Glu Leu Ser Ser Glu Met
   625 630 635 640
   Ser Leu Lys Thr Ala Ala Glu Glu Lys Glu Thr Glu Ser Lys Met Ile
   645 650 655
   Glu Glu Pro His Glu Glu Leu Ser Arg Glu Met Ser Leu Lys Thr Ala
   660 665 670
   Val Glu Glu Lys Glu Thr Glu Ser Lys Met Met Glu Glu Pro His Asp
   675 680 685
   Glu Leu Asn Ser Glu Met Ser Leu Ser Thr Ala Val Glu Glu Lys Glu
   690 695 700
   Thr Gly Ser Lys Met Thr Glu Glu Ser His Glu Glu Leu Ser Asn Glu
   705 710 715 720
   Met Ser Leu Glu Glu Lys Glu Thr Gly Arg Lys Met Thr Glu Glu Glu
   725 730 735
   Glu Leu Glu Ala Val Met Glu Met Leu Cys Arg Thr Glu Asn Lys Leu
   740 745 750
   Leu Asp Val Thr Gln Arg Leu Asp Arg Phe Lys Thr Pro Lys Gly Arg
   755 760 765
   Lys Lys Leu Gly Asn Ser Ser Ser Pro Leu Leu Glu Glu Asp Ser Val
   770 775 780
   Val Tyr Val Pro Ile Ala Glu Ile Lys Glu Lys Arg
   785 790 795
<210> 183
   <211> 2151
   <212> DNA
   <213> Eucalyptus grandis
<400> 183
   atgcattcca gaaagaatct tgcccaaaaa gaagactttt tgaaggcaga atctggatcg     60
   gaggttcttg cctcgctgat attgagaaat aggagaagga aaaagcacaa acagaaacta    120
   caatcttctg taaaagcaag aggctcaacc cttgccaaga agagggccaa taactccatc    180
   agaaagagac tggtgcgtag gaacagctct gataaagaca aactcataaa tttaaaagct    240
   tttcataata agaaaattga aatagttccc gagaagcttt catcccctcc atcttcagaa     300
   agagagctgc ctattgcttc tgacgaaaat gtgcaaaaca ccgatgagga tgtcaaagtt     360
   aacaagctga gaaggaagag gaagaagaaa atgagaaagc atttttcgaa gttagatgag     420
   gcttcacgat tgcagaggag aacaagatac cttcttatta aaatgaagct ggaacagaac     480
   cttattgatg cttactctgg tgaaggctgg aaaggccaga gtcgggaaaa gatcaagcca     540
   acaaaggaat tacaaagggc caggaagcag atactgaatt gcaaactcgg aatacgtgat     600
   gctattcgac agctagactc cattagttca gtgggcagca ttgaagactc tgtgattgct     660
   cctgatggtt ctgttcacca tgaacatata ttctgtgcaa aatgcaagac aaatgaaact     720
   tcctcagata atgatatcat actctgcgat gggacatgca attgtgcttt tcatcaaaaa     780
   tgtcttgatc ctccactgga aactgaaagc attcccccag gagatcaggg ctggttttgc     840
   agactctgcg agtgtaagat ggaaattata gagtcaacaa atgcacatct ggggacccac     900
   ttttctcttg acagcaactg gcaggatata ttcgaggaag aagctgcatt gcctgatggg     960
   ggaattcagg tgcttgacct agacgaaaag tggccttcag atgactctga agatgatgac    1020
   tatgacccag aaaagatgac gaacaccggc atcaactgtg ccggcaatga tgattctcaa    1080
   tcatacgaca caagtagctc taccagcctg agttggtcat tagatggtga aattttgtca    1140
   ggaccaacaa tatcaggcaa ggaagggttg aactatcctg agagcattgc agggtctgat    1200
   gtattaatgg acacggatac tgatactgta tgtggcccac gacagcgaaa tgctgttgat    1260
   tataagcagc tttataatga aatgttcggt aaggatactc ctgcctgtga acaagccagt    1320
   gaagatgaag actggggtcc taataaaaga agaagaagag aaagggaaac agatgcagcc    1380
   agcaccctca tgactctctg tgaaagtgag aaaaacaatc cattcattga ccaagcctca    1440
   gaaggtgata agaaactctc tgcagaagca agaatgagaa ggcggatttt cagaattcct    1500
   ccaactgcag ttgagaagct tcgccaagta tttgcagaga atgaacttcc ctcaagatct    1560
   gtcaaagaaa atcttgctaa agagtttggt cttgaacctg agaaggttag caaatggttc    1620
   aagaatgcac gatacatggc actcaaaaca agaaaggctg ggggggctga tcaacctcca    1680
   aaatccccgc aaaagcataa agaatctgaa ctagcaaaaa tgatgaaagg ggctgcgcct    1740
   attttatcta aggatgcctc aacagacatt gaattccatt cctctctaaa tactgagaaa    1800
   gtcatccagc aaaagagtac gaagccaata cacgggaagg gttcacctat ggtacctgaa    1860
   catagctata tggagaattt tgagttcgat gacaatatgt ccttgaaaca aataaagcta    1920
   ttgaaaggaa aaccaaagga caggaagagg gttgacttcg cagccagtga gggattacga    1980
   gaaagagaag ctcagttgcg ggagctatgt aaaattagaa gtaggctaga tagtctgagg    2040
   aagagaaccc ttacatttca gtcgagtgaa tccaaggctt cagacgaatt caacacttgt    2100
   agatcacgtg tagtatatgt tccagtagca gaaataaagg agagagcttg a             2151
<210> 184
   <211> 716
   <212> PRT
   <213> Eucalyptus grandis
<400> 184
   Met His Ser Arg Lys Asn Leu Ala Gln Lys Glu Asp Phe Leu Lys Ala
   1               5                   10                  15
   Glu Ser Gly Ser Glu Val Leu Ala Ser Leu Ile Leu Arg Asn Arg Arg
               20                  25                  30
   Arg Lys Lys His Lys Gln Lys Leu Gln Ser Ser Val Lys Ala Arg Gly
           35                  40                  45
   Ser Thr Leu Ala Lys Lys Arg Ala Asn Asn Ser Ile Arg Lys Arg Leu
       50                  55                  60
   Val Arg Arg Asn Ser Ser Asp Lys Asp Lys Leu Ile Asn Leu Lys Ala
   65                  70                  75                  80
   Phe His Asn Lys Lys Ile Glu Ile Val Pro Glu Lys Leu Ser Ser Pro
                   85                  90                  95
   Pro Ser Ser Glu Arg Glu Leu Pro Ile Ala Ser Asp Glu Asn Val Gln
               100                 105                 110
   Asn Thr Asp Glu Asp Val Lys Val Asn Lys Leu Arg Arg Lys Arg Lys
           115                 120                 125
   Lys Lys Met Arg Lys His Phe Ser Lys Leu Asp Glu Ala Ser Arg Leu
       130                 135                 140
   Gln Arg Arg Thr Arg Tyr Leu Leu Ile Lys Met Lys Leu Glu Gln Asn
   145                 150                 155                 160
   Leu Ile Asp Ala Tyr Ser Gly Glu Gly Trp Lys Gly Gln Ser Arg Glu
                   165                 170                 175
   Lys Ile Lys Pro Thr Lys Glu Leu Gln Arg Ala Arg Lys Gln Ile Leu
               180                 185                 190
   Asn Cys Lys Leu Gly Ile Arg Asp Ala Ile Arg Gln Leu Asp Ser Ile
           195                 200                 205
   Ser Ser Val Gly Ser Ile Glu Asp Ser Val Ile Ala Pro Asp Gly Ser
       210                 215                 220
   Val His His Glu His Ile Phe Cys Ala Lys Cys Lys Thr Asn Glu Thr
   225                 230                 235                 240
   Ser Ser Asp Asn Asp Ile Ile Leu Cys Asp Gly Thr Cys Asn Cys Ala
                   245                 250                 255
   Phe His Gln Lys Cys Leu Asp Pro Pro Leu Glu Thr Glu Ser Ile Pro
               260                 265                 270
   Pro Gly Asp Gln Gly Trp Phe Cys Arg Leu Cys Glu Cys Lys Met Glu
           275                 280                 285
   Ile Ile Glu Ser Thr Asn Ala His Leu Gly Thr His Phe Ser Leu Asp
       290                 295                 300
   Ser Asn Trp Gln Asp Ile Phe Glu Glu Glu Ala Ala Leu Pro Asp Gly
   305                 310                 315                 320
   Gly Ile Gln Val Leu Asp Leu Asp Glu Lys Trp Pro Ser Asp Asp Ser
                   325                 330                 335
   Glu Asp Asp Asp Tyr Asp Pro Glu Lys Met Thr Asn Thr Gly Ile Asn
               340                 345                 350
   Cys Ala Gly Asn Asp Asp Ser Gln Ser Tyr Asp Thr Ser Ser Ser Thr
           355                 360                 365
   Ser Leu Ser Trp Ser Leu Asp Gly Glu Ile Leu Ser Gly Pro Thr Ile
       370                 375                 380
   Ser Gly Lys Glu Gly Leu Asn Tyr Pro Glu Ser Ile Ala Gly Ser Asp
   385                 390                 395                 400
   Val Leu Met Asp Thr Asp Thr Asp Thr Val Cys Gly Pro Arg Gln Arg
                   405                 410                 415
   Asn Ala Val Asp Tyr Lys Gln Leu Tyr Asn Glu Met Phe Gly Lys Asp
               420                 425                 430
   Thr Pro Ala Cys Glu Gln Ala Ser Glu Asp Glu Asp Trp Gly Pro Asn
           435                 440                 445
   Lys Arg Arg Arg Arg Glu Arg Glu Thr Asp Ala Ala Ser Thr Leu Met
       450                 455                 460
   Thr Leu Cys Glu Ser Glu Lys Asn Asn Pro Phe Ile Asp Gln Ala Ser
   465                 470                 475                 480
   Glu Gly Asp Lys Lys Leu Ser Ala Glu Ala Arg Met Arg Arg Arg Ile
                   485                 490                 495
   Phe Arg Ile Pro Pro Thr Ala Val Glu Lys Leu Arg Gln Val Phe Ala
               500                 505                 510
   Glu Asn Glu Leu Pro Ser Arg Ser Val Lys Glu Asn Leu Ala Lys Glu
           515                 520                 525
   Phe Gly Leu Glu Pro Glu Lys Val Ser Lys Trp Phe Lys Asn Ala Arg
       530                 535                 540
   Tyr Met Ala Leu Lys Thr Arg Lys Ala Gly Gly Ala Asp Gln Pro Pro
   545 550 555 560
   Lys Ser Pro Gln Lys His Lys Glu Ser Glu Leu Ala Lys Met Met Lys
   565 570 575
   Gly Ala Ala Pro Ile Leu Ser Lys Asp Ala Ser Thr Asp Ile Glu Phe
   580 585 590
   His Ser Ser Leu Asn Thr Glu Lys Val Ile Gln Gln Lys Ser Thr Lys
   595 600 605
   Pro Ile His Gly Lys Gly Ser Pro Met Val Pro Glu His Ser Tyr Met
   610 615 620
   Glu Asn Phe Glu Phe Asp Asp Asn Met Ser Leu Lys Gln Ile Lys Leu
   625 630 635 640
   Leu Lys Gly Lys Pro Lys Asp Arg Lys Arg Val Asp Phe Ala Ala Ser
   645 650 655
   Glu Gly Leu Arg Glu Arg Glu Ala Gln Leu Arg Glu Leu Cys Lys Ile
   660 665 670
   Arg Ser Arg Leu Asp Ser Leu Arg Lys Arg Thr Leu Thr Phe Gln Ser
   675 680 685
   Ser Glu Ser Lys Ala Ser Asp Glu Phe Asn Thr Cys Arg Ser Arg Val
   690 695 700
   Val Tyr Val Pro Val Ala Glu Ile Lys Glu Arg Ala
   705 710 715
<210> 185
   <211> 2124
   <212> DNA
   <213> Medicago truncatula
<400> 185
   atgcgggata ctgagaggtt aaataaccaa ggatctacaa aaccgagtaa cacaaaggaa     60
   catatcaagt tgaaggtaga ttccccacag ttcaaaacaa gtagcacaaa acaccaaagg    120
   aagaaacaca gactgaaatc aaaatctcat aaactcggag gttgcacaaa tgcgtcagtg    180
   aggacagcta ctgattcttc caacaaggcg tcaaccaagg attcttcaaa taaaacggat    240
   aggaattcaa cacaggttca gccttcaaaa aagatacaag gaggaaaaac ttctcttaat    300
   aatgatagga aaggtgagaa agatgttgtt gatcaagagg gaaatattca aaaacgtaag    360
   agaaggagaa agaagaaaag acaaagacat aatgtagatc ttgatgatac tgtacgcctc    420
   cagagaagaa caaggaatat cttaatcaga atgaagcaag aacagaatct tatcgatgct    480
   tacgctggag aaggttggaa aggtcagagt cgagagaaga ttaggccaga aatggagcta    540
   caaagagcaa aaaagcagat tttaaaatgc aagcttagta tccgtgatgc cattcaccaa     600
   ctggattctc ttagttcagt tggtagtatt gaaggttctg tcattgctac agatggatct     660
   gtttctcatg aacatatatt ctgtgcaaat tgcaagataa acgaagtttc tccagataat     720
   gatattatac tctgtgatgg cacatgcaat cgtgcgtttc accagagatg tcttgatcct     780
   cctttggaga ctgaagacat tcctcctgaa gaccaaggct ggttttgcaa gtattgtgac     840
   tgtaagatag aaatattgga ggcaacaaac gcgcatcttg ggactcgttt ccccttagac     900
   agtacttggc aggatgtatt caaggaagaa gctgctattc ctgacggcga tgctgcatta     960
   ctaaatcaag aagaagaatg gccgtcagat gatcctgaag acgatgacta taatccagag    1020
   aggaaagaag aaagccacgg tggcttcaac acagaaggaa atgataaaaa cgcatctgat    1080
   gattcaagca gttcttccag catgtggtct ctaaatggag aatgctctct gttagatgaa    1140
   ggcatcaacc ttgaatatta ttctaacgac cacatagatt ctgatgaatc cggggaaata    1200
   gcatgcggtc gtaggcagcg tagagctgtt gactacaaga aactttatga tgaaatgttt    1260
   ggaaaggatg ctccaccttg tgaacaagtg agtgaagatg aagattgggg tcctcgaaaa    1320
   agacggcgaa gagaaaagga gtctgaggct gttaatactc ttatgactct acatgagagt    1380
   gaaaataaat atccgaataa taagaacaat gatagaataa gagggaattc ttcaggcata    1440
   aaaagacctt gttttaggtt ttcacatgag gcagttgaga agcttcgcca agtttttgca    1500
   gagaacgagc ttcctccaaa atccgtgaag gatgcccttt caaaagaatt gggacttgat    1560
   gctgcaaagg ttaacaaatg gttcaaaaat gcacgttaca tggcacttaa aataagaaag    1620
   cttcaagaag gaggacaaca gcttcagagt atcacttcta agacctcaaa ggattctaca    1680
   tcacaacatg tgcaggaaga tgaggttttg aacccgaagt ccgcgaaaat tactgtgatt    1740
   agttccctga agaagtgtga aaatgttact ggcaaagaaa aaacaaaagc atctagcaat    1800
   cctttgaaga aaaagcgacc agaaatacct ccatgcttgg gagaaaatgg caacaaggat    1860
   tccatggagg tcagtgatga tgttagctta atgaaactat tacaagacag gaaaaagaag    1920
   gtaagttttg catttgaagg agactctgag gcagcagagt tggaatttga aagactcagc    1980
   aaagtgaaga cgaaaataga tagattgaag caaagattaa ctggagttca aaattgcaga    2040
   tcaaagggtt cagatgaagt tcatttgaat gagccatcta ttatgtatgt tcctatagct    2100
   gagttaaggg aaaaggtgaa atag                                           2124
<210> 186
   <211> 707
   <212> PRT
   <213> Medicago truncatula
<400> 186
   Met Arg Asp Thr Glu Arg Leu Asn Asn Gln Gly Ser Thr Lys Pro Ser
   1               5                   10                  15
   Asn Thr Lys Glu His Ile Lys Leu Lys Val Asp Ser Pro Gln Phe Lys
               20                  25                  30
   Thr Ser Ser Thr Lys His Gln Arg Lys Lys His Arg Leu Lys Ser Lys
   35 40 45
   Ser His Lys Leu Gly Gly Cys Thr Asn Ala Ser Val Arg Thr Ala Thr
   50 55 60
   Asp Ser Ser Asn Lys Ala Ser Thr Lys Asp Ser Ser Asn Lys Thr Asp
   65 70 75 80
   Arg Asn Ser Thr Gln Val Gln Pro Ser Lys Lys Ile Gln Gly Gly Lys
   85 90 95
   Thr Ser Leu Asn Asn Asp Arg Lys Gly Glu Lys Asp Val Val Asp Gln
   100 105 110
   Glu Gly Asn Ile Gln Lys Arg Lys Arg Arg Arg Lys Lys Lys Arg Gln
   115 120 125
   Arg His Asn Val Asp Leu Asp Asp Thr Val Arg Leu Gln Arg Arg Thr
   130 135 140
   Arg Asn Ile Leu Ile Arg Met Lys Gln Glu Gln Asn Leu Ile Asp Ala
   145 150 155 160
   Tyr Ala Gly Glu Gly Trp Lys Gly Gln Ser Arg Glu Lys Ile Arg Pro
   165 170 175
   Glu Met Glu Leu Gln Arg Ala Lys Lys Gln Ile Leu Lys Cys Lys Leu
   180 185 190
   Ser Ile Arg Asp Ala Ile His Gln Leu Asp Ser Leu Ser Ser Val Gly
   195 200 205
   Ser Ile Glu Gly Ser Val Ile Ala Thr Asp Gly Ser Val Ser His Glu
   210 215 220
   His Ile Phe Cys Ala Asn Cys Lys Ile Asn Glu Val Ser Pro Asp Asn
   225 230 235 240
   Asp Ile Ile Leu Cys Asp Gly Thr Cys Asn Arg Ala Phe His Gln Arg
   245 250 255
   Cys Leu Asp Pro Pro Leu Glu Thr Glu Asp Ile Pro Pro Glu Asp Gln
   260 265 270
   Gly Trp Phe Cys Lys Tyr Cys Asp Cys Lys Ile Glu Ile Leu Glu Ala
   275 280 285
   Thr Asn Ala His Leu Gly Thr Arg Phe Pro Leu Asp Ser Thr Trp Gln
   290 295 300
   Asp Val Phe Lys Glu Glu Ala Ala Ile Pro Asp Gly Asp Ala Ala Leu
   305 310 315 320
   Leu Asn Gln Glu Glu Glu Trp Pro Ser Asp Asp Pro Glu Asp Asp Asp
   325 330 335
   Tyr Asn Pro Glu Arg Lys Glu Glu Ser His Gly Gly Phe Asn Thr Glu
   340 345 350
   Gly Asn Asp Lys Asn Ala Ser Asp Asp Ser Ser Ser Ser Ser Ser Met
   355 360 365
   Trp Ser Leu Asn Gly Glu Cys Ser Leu Leu Asp Glu Gly Ile Asn Leu
   370 375 380
   Glu Tyr Tyr Ser Asn Asp His Ile Asp Ser Asp Glu Ser Gly Glu Ile
   385 390 395 400
   Ala Cys Gly Arg Arg Gln Arg Arg Ala Val Asp Tyr Lys Lys Leu Tyr
   405 410 415
   Asp Glu Met Phe Gly Lys Asp Ala Pro Pro Cys Glu Gln Val Ser Glu
   420 425 430
   Asp Glu Asp Trp Gly Pro Arg Lys Arg Arg Arg Arg Glu Lys Glu Ser
   435 440 445
   Glu Ala Val Asn Thr Leu Met Thr Leu His Glu Ser Glu Asn Lys Tyr
   450 455 460
   Pro Asn Asn Lys Asn Asn Asp Arg Ile Arg Gly Asn Ser Ser Gly Ile
   465 470 475 480
   Lys Arg Pro Cys Phe Arg Phe Ser His Glu Ala Val Glu Lys Leu Arg
   485 490 495
   Gln Val Phe Ala Glu Asn Glu Leu Pro Pro Lys Ser Val Lys Asp Ala
   500 505 510
   Leu Ser Lys Glu Leu Gly Leu Asp Ala Ala Lys Val Asn Lys Trp Phe
   515 520 525
   Lys Asn Ala Arg Tyr Met Ala Leu Lys Ile Arg Lys Leu Gln Glu Gly
   530 535 540
   Gly Gln Gln Leu Gln Ser Ile Thr Ser Lys Thr Ser Lys Asp Ser Thr
   545 550 555 560
   Ser Gln His Val Gln Glu Asp Glu Val Leu Asn Pro Lys Ser Ala Lys
   565 570 575
   Ile Thr Val Ile Ser Ser Leu Lys Lys Cys Glu Asn Val Thr Gly Lys
                   580                 585                 590
   Glu Lys Thr Lys Ala Ser Ser Asn Pro Leu Lys Lys Lys Arg Pro Glu
           595                 600                 605
   Ile Pro Pro Cys Leu Gly Glu Asn Gly Asn Lys Asp Ser Met Glu Val
       610                 615                 620
   Ser Asp Asp Val Ser Leu Met Lys Leu Leu Gln Asp Arg Lys Lys Lys
   625                 630                 635                 640
   Val Ser Phe Ala Phe Glu Gly Asp Ser Glu Ala Ala Glu Leu Glu Phe
                   645                 650                 655
   Glu Arg Leu Ser Lys Val Lys Thr Lys Ile Asp Arg Leu Lys Gln Arg
               660                 665                 670
   Leu Thr Gly Val Gln Asn Cys Arg Ser Lys Gly Ser Asp Glu Val His
           675                 680                 685
   Leu Asn Glu Pro Ser Ile Met Tyr Val Pro Ile Ala Glu Leu Arg Glu
       690                 695                 700
   Lys Val Lys
   705
<210> 187
   <211> 2556
   <212> DNA
   <213> Pinus radiata
<400> 187
   atgccgaaag caggaaaagg ctcttccggt tcactccgaa agaagcaagc gttgtcaaat 60
   acagcatcag agctaaggcc taaagctgca attcagaaga ctaacaatgc cgggagtatt 120
   tctggcagga tgagggaaag gaaaccacgg ttacatgtac agatgattgc ctcaattctc 180
   acgaaaaaaa ggacaggtga agcatctgaa tcgagaaatg attctgagct agaagacaaa 240
   caatatctga gcaatagttc aaaaacaaat gccaattttg tagcggggaa gttagttaaa 300
   aagactttga aaaaagcccc aagattaaac ttgattaaga atgacagtca gaaaaaaccc 360
   agtaagagtt ctgcttcgag gtcttatgct gcacagaaac ctgaacggcc acaacagaga 420
   aaaattagga ggacaaagtg gaagggtgat tctaaaaata taacaaaggt gaaaactgtg 480
   agaactagta atatagatgg cttgaagagg aagcagcaaa ggagaaagaa gaaaaagaag 540
   gcgcagaatg ttccacagga tgaagtatca cgtgcaaaga gaaggatcaa atatcttttg 600
   ctcaaaatga aacttgagca gaatcttatt gatgcatatt caggggaggg ctggaaaggc 660
   cagagtcggg agaaaataaa gccagagaag gagttacaga gagctgaaaa gcagattctg 720
   caatgcaaac ttggaattcg tgaagcagtg catcagcttg atcttctaag ttcggaagga 780
   tctatagcag atgccgttat tgatccagaa gggcgtgtat ttcatgagca tatattttgt 840
   gcaaaatgca aaatgacaga tgctcttccg gataatgaca taatattgtg tgatggagct     900
   tgcaatcgtg gcttccatca gaaatgtctg gatccacctt tggctactga aaacattcct     960
   cctggagatc agggttggct ctgtaaagtt tgcgaatgta agctagagag tttggaagca    1020
   ataaatgctc atttgggcac ccagtttaca gttgataata gctgggaggg aatttttgct    1080
   gatgcagcta gaattgcgaa tggtgaaagt acagctactg ggaatgggga agaatggcca    1140
   tcagatgatt cagaagatga tgactatgat ccagagaaac aagagattag agatgaaacg    1200
   ggctatgaga ataatatgag tgattctgga aagtcaagtg gatggtcaga atcatataat    1260
   gaaactgaca atgaatcaga tgcttcctta acagataatc taaatgaata catctctaag    1320
   ggaaagaaga ggagcagaga cactgatgaa aaaaacggac aagaagctat aatagattta    1380
   gatactgatg agaatgatgg ctcagacatg ccagtttcag ggagaaggca gcgaagagat    1440
   gttgattata aaaagttgca tgatgaaatg tttggaaaag atggaccttg tgaagatgaa    1500
   gtcagtgaag atgaagactg gggacccaat aggaggcgta gaagagcgaa acagccggat    1560
   acaaatacca ctaaggattc ttcaaatgaa gagaaagagc atgttagaaa ggaagcagat    1620
   gagaaagtag cagagcattc acaaagtagc ataaaggata agaaacatat tgtcagactg    1680
   ccagctaatg cagttgagaa gctgcgcaaa gtctttgctg aaaatgagct accgtcaagg    1740
   tcttacaagg aaaatctgtc acagcaactg ggccttacat ttcgaaaggt ccatatgtgg    1800
   ttcaaaaatg cccgttatat gtctctgaag agccgaaagg aaatgcctag aaatgagaac    1860
   tgcaatgaaa ctaatctcat gggagaaggt gatatggggc caaagaaaaa aataccaaaa    1920
   gagaagaaag cagaatcaac atgtctttta tcttctgtta gagcagaata taaatctaga    1980
   aatacaaaaa ggaagcatag gagaaaaaac atcaaagcac ccttatctat ctcttccaga    2040
   agtgagagaa aaagaactat gtgcagactg cctgccaatg cagttgagag gtttcgccaa    2100
   gtctttgctg agaatgagtt gccttcaatg tcctgtaaaa tggcattatc aaagcagttg    2160
   gatctacctt atcggcaggt gcatatgtgg ttcaaaaatg ctcgttatat gtctttgaag    2220
   aagaaaaaga tgaaccgttt aaaagaatca gctcccgtta tttcctcaca taccagaaga    2280
   cttgaagtag aaagtagcaa acccaatcca caaatcaaag agccagatga tgctggcaat    2340
   tcatacttaa cagagatgga aaaattgggt aatattgagt tgaaacttga aaatatgaga    2400
   aagattttag aaacagtctg tcctaaaaga ggtactgatt tttccaatga taagaatcaa    2460
   gaaggaatcg ccaaccttct tagtgaggag cttcttgtgt atgtaccagt ggtggagttg    2520
   agggagaaga gcttacaggt tgctcctgtc aggtga                              2556
<210> 188
   <211> 851
   <212> PRT
   <213> Pinus radiata

<400> 188
   Met Pro Lys Ala Gly Lys Gly Ser Ser Gly Ser Leu Arg Lys Lys Gln
   1               5                   10                  15
   Ala Leu Ser Asn Thr Ala Ser Glu Leu Arg Pro Lys Ala Ala Ile Gln
               20                  25                  30
   Lys Thr Asn Asn Ala Gly Ser Ile Ser Gly Arg Met Arg Glu Arg Lys
           35                  40                  45
   Pro Arg Leu His Val Gln Met Ile Ala Ser Ile Leu Thr Lys Lys Arg
       50                  55                  60
   Thr Gly Glu Ala Ser Glu Ser Arg Asn Asp Ser Glu Leu Glu Asp Lys
   65                  70                  75                  80
   Gln Tyr Leu Ser Asn Ser Ser Lys Thr Asn Ala Asn Phe Val Ala Gly
                   85                  90                  95
   Lys Leu Val Lys Lys Thr Leu Lys Lys Ala Pro Arg Leu Asn Leu Ile
               100                 105                 110
   Lys Asn Asp Ser Gln Lys Lys Pro Ser Lys Ser Ser Ala Ser Arg Ser
           115                 120                 125
   Tyr Ala Ala Gln Lys Pro Glu Arg Pro Gln Gln Arg Lys Ile Arg Arg
       130                 135                 140
   Thr Lys Trp Lys Gly Asp Ser Lys Asn Ile Thr Lys Val Lys Thr Val
   145                 150                 155                 160
   Arg Thr Ser Asn Ile Asp Gly Leu Lys Arg Lys Gln Gln Arg Arg Lys
                   165                 170                 175
   Lys Lys Lys Lys Ala Gln Asn Val Pro Gln Asp Glu Val Ser Arg Ala
               180                 185                 190
   Lys Arg Arg Ile Lys Tyr Leu Leu Leu Lys Met Lys Leu Glu Gln Asn
           195                 200                 205
   Leu Ile Asp Ala Tyr Ser Gly Glu Gly Trp Lys Gly Gln Ser Arg Glu
       210                 215                 220
   Lys Ile Lys Pro Glu Lys Glu Leu Gln Arg Ala Glu Lys Gln Ile Leu
   225                 230                 235                 240
   Gln Cys Lys Leu Gly Ile Arg Glu Ala Val His Gln Leu Asp Leu Leu
                   245                 250                 255
   Ser Ser Glu Gly Ser Ile Ala Asp Ala Val Ile Asp Pro Glu Gly Arg
               260                 265                 270
   Val Phe His Glu His Ile Phe Cys Ala Lys Cys Lys Met Thr Asp Ala
           275                 280                 285
   Leu Pro Asp Asn Asp Ile Ile Leu Cys Asp Gly Ala Cys Asn Arg Gly
   290 295 300
   Phe His Gln Lys Cys Leu Asp Pro Pro Leu Ala Thr Glu Asn Ile Pro
   305 310 315 320
   Pro Gly Asp Gln Gly Trp Leu Cys Lys Val Cys Glu Cys Lys Leu Glu
   325 330 335
   Ser Leu Glu Ala Ile Asn Ala His Leu Gly Thr Gln Phe Thr Val Asp
   340 345 350
   Asn Ser Trp Glu Gly Ile Phe Ala Asp Ala Ala Arg Ile Ala Asn Gly
   355 360 365
   Glu Ser Thr Ala Thr Gly Asn Gly Glu Glu Trp Pro Ser Asp Asp Ser
   370 375 380
   Glu Asp Asp Asp Tyr Asp Pro Glu Lys Gln Glu Ile Arg Asp Glu Thr
   385 390 395 400
   Gly Tyr Glu Asn Asn Met Ser Asp Ser Gly Lys Ser Ser Gly Trp Ser
   405 410 415
   Glu Ser Tyr Asn Glu Thr Asp Asn Glu Ser Asp Ala Ser Leu Thr Asp
   420 425 430
   Asn Leu Asn Glu Tyr Ile Ser Lys Gly Lys Lys Arg Ser Arg Asp Thr
   435 440 445
   Asp Glu Lys Asn Gly Gln Glu Ala Ile Ile Asp Leu Asp Thr Asp Glu
   450 455 460
   Asn Asp Gly Ser Asp Met Pro Val Ser Gly Arg Arg Gln Arg Arg Asp
   465 470 475 480
   Val Asp Tyr Lys Lys Leu His Asp Glu Met Phe Gly Lys Asp Gly Pro
   485 490 495
   Cys Glu Asp Glu Val Ser Glu Asp Glu Asp Trp Gly Pro Asn Arg Arg
   500 505 510
   Arg Arg Arg Ala Lys Gln Pro Asp Thr Asn Thr Thr Lys Asp Ser Ser
   515 520 525
   Asn Glu Glu Lys Glu His Val Arg Lys Glu Ala Asp Glu Lys Val Ala
   530 535 540
   Glu His Ser Gln Ser Ser Ile Lys Asp Lys Lys His Ile Val Arg Leu
   545 550 555 560
   Pro Ala Asn Ala Val Glu Lys Leu Arg Lys Val Phe Ala Glu Asn Glu
                   565                 570                 575
   Leu Pro Ser Arg Ser Tyr Lys Glu Asn Leu Ser Gln Gln Leu Gly Leu
               580                 585                 590
   Thr Phe Arg Lys Val His Met Trp Phe Lys Asn Ala Arg Tyr Met Ser
           595                 600                 605
   Leu Lys Ser Arg Lys Glu Met Pro Arg Asn Glu Asn Cys Asn Glu Thr
       610                 615                 620
   Asn Leu Met Gly Glu Gly Asp Met Gly Pro Lys Lys Lys Ile Pro Lys
   625                 630                 635                 640
   Glu Lys Lys Ala Glu Ser Thr Cys Leu Leu Ser Ser Val Arg Ala Glu
                   645                 650                 655
   Tyr Lys Ser Arg Asn Thr Lys Arg Lys His Arg Arg Lys Asn Ile Lys
               660                 665                 670
   Ala Pro Leu Ser Ile Ser Ser Arg Ser Glu Arg Lys Arg Thr Met Cys
           675                 680                 685
   Arg Leu Pro Ala Asn Ala Val Glu Arg Phe Arg Gln Val Phe Ala Glu
       690                 695                 700
   Asn Glu Leu Pro Ser Met Ser Cys Lys Met Ala Leu Ser Lys Gln Leu
   705                 710                 715                 720
   Asp Leu Pro Tyr Arg Gln Val His Met Trp Phe Lys Asn Ala Arg Tyr
                   725                 730                 735
   Met Ser Leu Lys Lys Lys Lys Met Asn Arg Leu Lys Glu Ser Ala Pro
               740                 745                 750
   Val Ile Ser Ser His Thr Arg Arg Leu Glu Val Glu Ser Ser Lys Pro
           755                 760                 765
   Asn Pro Gln Ile Lys Glu Pro Asp Asp Ala Gly Asn Ser Tyr Leu Thr
       770                 775                 780
   Glu Met Glu Lys Leu Gly Asn Ile Glu Leu Lys Leu Glu Asn Met Arg
   785                 790                 795                 800
   Lys Ile Leu Glu Thr Val Cys Pro Lys Arg Gly Thr Asp Phe Ser Asn
                   805                 810                 815
   Asp Lys Asn Gln Glu Gly Ile Ala Asn Leu Leu Ser Glu Glu Leu Leu
               820                 825                 830
   Val Tyr Val Pro Val Val Glu Leu Arg Glu Lys Ser Leu Gln Val Ala
           835                 840                 845
   Pro Val Arg
   850
<210> 189
   <211> 2178
   <212> DNA
   <213> Populus tremuloides
<400> 189
   atgggtgatt ctggaaagac gtcaaagctg caagactcgc acaaatgttc accttctgac     60
   acagtgactg ggtcgttgct gattaaatca ttgaagataa agaaggacag caaaatatct    120
   cctagaaaag gccaaaaaac gaaaacaaaa tcaaaaccaa aaccaatacc tcatctaaaa    180
   actatcatca gttcagctgt atcaaaaaga aaagtttctc ctaagggcat tgggaatggc    240
   tctaccagta gaaaactgat tcataggaaa attctgcata aagcacttga taagaaggcc    300
   tcaaggaagg gggcttcctc agggctacaa ttgtcaacta ttgattctaa gggaaatgga    360
   aaaaatggtg atgaaggtgc aattaaaaaa ctcaagaaga ggaagcctaa gaaaaggcaa    420
   agggacaagg taaagctaga tgaaccatca cgattgcaga ggagagcaag gtacctgatg    480
   attaaaatga agctggacca gaaccttata gatgcttact ctggagaagg ctggaaaggt    540
   caaagtcgag agaagatcag gccagaaaag gaactactgc gagcaaggaa gcagattttg    600
   aagtgtaaac ttggtttacg tgatataatt cggcaggtgg attctcttag tacagtagga    660
   tgtattgaag agacagttat ggctccagat ggatctgttt cacatgaaca tatattctgt    720
   gcgaagtgca aattgaatga agtttcccca gataatgata ttgtactctg tgatggtaca    780
   tgcaactgtg ctttccacca aaaatgcctt gaacctccct tggatactga aagtatacct    840
   ccaggagatc agggatggtt ttgcaagttc tgtgaatgca ggatggatat tatagaagct    900
   atgaatgctc atttgggaac ccatttctca gaggacagca gttggcagga tattttcaca    960
   gaagaagcag caattcctga tgctggaaat gtgctattaa accccgaaga agaatggcct   1020
   tcagatgatt ctgaagatga taattacgat ccagagagga gagacaacat catgagtgaa   1080
   gcgggtactg atgatgatgc atctgatgac atcagcagtt caaccagctt gggttggtct   1140
   tcagatggtg aggttttttt gggatccagg aggtgggaga tgcatggctt ggacttcaga   1200
   aacaattcta tttatagcag tttagattct gatgaaacca gtgatgggga aattgtttgt   1260
   ggccgtaggc agagaagagc aattgactat aagaagttat atgatgaggt gtttggaaag   1320
   gatgctcctg cacacgagca agccagtgaa gacgaagatt ggggtcctgg caaaagaaag   1380
   cgacgggaga aggagtcaaa tgcagccagc acccttatga cactgtgtga aagtaaaaaa   1440
   aaaagtaaaa gtgatgagac cattgaaggt atgatgaacc ttccccctca aactcgaagg   1500
   ccaattttca gactcccccc tgatgcagtt gagaaacttc gccaagtatt tgtagaaaat   1560
   gaacttccat ctagaactgt caaggagaat ctgtcaaaag aattgggcct tgaacctggg   1620
   aaggttagca aatggttcaa gaattcccgt tatttagctc tgaagtcccg gaaggtagag   1680
   aaaggagaac aacttcataa ctctagttcc aaagtctctg ctgaacccac attaaatgtc   1740
   atggagggca aaactgctga tctttcacag gattcttggg aagaaactga ggtatgtatc   1800
   ccaaagaatt tgaaaaggat ccttcagagg aagaagctga agtcaataag cagaagttta   1860
   aggaaaaatg aacagaaaag aggttcctac gaatcaccta ctaaaagcaa tgagatgaat   1920
   gctgagtgca gtgatgatgt gagcttgaag aagttgttaa aagcaaaacc aaagggagta   1980
   aagaaaaagg ttaatcctat ttcagtagcg gctgaatatg atatggagag actctgtaga   2040
   gcgaaggcta tattagagaa gatgaagcag aaagtggtga agctacaaaa tggcaaagct   2100
   aggaagtcat ccaaaacccg tccacttgaa gaatttatcg tttacattcc cattgctgag   2160
   ttgaggggaa aaaattga                                                  2178
<210> 190
   <211> 725
   <212> PRT
   <213> Populus tremuloides
<400> 190
   Met Gly Asp Ser Gly Lys Thr Ser Lys Leu Gln Asp Ser His Lys Cys
   1               5                   10                  15
   Ser Pro Ser Asp Thr Val Thr Gly Ser Leu Leu Ile Lys Ser Leu Lys
               20                  25                  30
   Ile Lys Lys Asp Ser Lys Ile Ser Pro Arg Lys Gly Gln Lys Thr Lys
           35                  40                  45
   Thr Lys Ser Lys Pro Lys Pro Ile Pro His Leu Lys Thr Ile Ile Ser
       50                  55                  60
   Ser Ala Val Ser Lys Arg Lys Val Ser Pro Lys Gly Ile Gly Asn Gly
   65                  70                  75                  80
   Ser Thr Ser Arg Lys Leu Ile His Arg Lys Ile Leu His Lys Ala Leu
                   85                  90                  95
   Asp Lys Lys Ala Ser Arg Lys Gly Ala Ser Ser Gly Leu Gln Leu Ser
               100                 105                 110
   Thr Ile Asp Ser Lys Gly Asn Gly Lys Asn Gly Asp Glu Gly Ala Ile
           115                 120                 125
   Lys Lys Leu Lys Lys Arg Lys Pro Lys Lys Arg Gln Arg Asp Lys Val
       130                 135                 140
   Lys Leu Asp Glu Pro Ser Arg Leu Gln Arg Arg Ala Arg Tyr Leu Met
   145                 150                 155                 160
   Ile Lys Met Lys Leu Asp Gln Asn Leu Ile Asp Ala Tyr Ser Gly Glu
                   165                 170                 175
   Gly Trp Lys Gly Gln Ser Arg Glu Lys Ile Arg Pro Glu Lys Glu Leu
               180                 185                 190
   Leu Arg Ala Arg Lys Gln Ile Leu Lys Cys Lys Leu Gly Leu Arg Asp
           195                 200                 205
   Ile Ile Arg Gln Val Asp Ser Leu Ser Thr Val Gly Cys Ile Glu Glu
       210                 215                 220
   Thr Val Met Ala Pro Asp Gly Ser Val Ser His Glu His Ile Phe Cys
   225                 230                 235                 240
   Ala Lys Cys Lys Leu Asn Glu Val Ser Pro Asp Asn Asp Ile Val Leu
                   245                 250                 255
   Cys Asp Gly Thr Cys Asn Cys Ala Phe His Gln Lys Cys Leu Glu Pro
               260                 265                 270
   Pro Leu Asp Thr Glu Ser Ile Pro Pro Gly Asp Gln Gly Trp Phe Cys
           275                 280                 285
   Lys Phe Cys Glu Cys Arg Met Asp Ile Ile Glu Ala Met Asn Ala His
       290                 295                 300
   Leu Gly Thr His Phe Ser Glu Asp Ser Ser Trp Gln Asp Ile Phe Thr
   305                 310                 315                 320
   Glu Glu Ala Ala Ile Pro Asp Ala Gly Asn Val Leu Leu Asn Pro Glu
                   325                 330                 335
   Glu Glu Trp Pro Ser Asp Asp Ser Glu Asp Asp Asn Tyr Asp Pro Glu
               340                 345                 350
   Arg Arg Asp Asn Ile Met Ser Glu Ala Gly Thr Asp Asp Asp Ala Ser
           355                 360                 365
   Asp Asp Ile Ser Ser Ser Thr Ser Leu Gly Trp Ser Ser Asp Gly Glu
       370                 375                 380
   Val Phe Leu Gly Ser Arg Arg Trp Glu Met His Gly Leu Asp Phe Arg
   385                 390                 395                 400
   Asn Asn Ser Ile Tyr Ser Ser Leu Asp Ser Asp Glu Thr Ser Asp Gly
                   405                 410                 415
   Glu Ile Val Cys Gly Arg Arg Gln Arg Arg Ala Ile Asp Tyr Lys Lys
               420                 425                 430
   Leu Tyr Asp Glu Val Phe Gly Lys Asp Ala Pro Ala His Glu Gln Ala
           435                 440                 445
   Ser Glu Asp Glu Asp Trp Gly Pro Gly Lys Arg Lys Arg Arg Glu Lys
   450 455 460
   Glu Ser Asn Ala Ala Ser Thr Leu Met Thr Leu Cys Glu Ser Lys Lys
   465 470 475 480
   Lys Ser Lys Ser Asp Glu Thr Ile Glu Gly Met Met Asn Leu Pro Pro
   485 490 495
   Gln Thr Arg Arg Pro Ile Phe Arg Leu Pro Pro Asp Ala Val Glu Lys
   500 505 510
   Leu Arg Gln Val Phe Val Glu Asn Glu Leu Pro Ser Arg Thr Val Lys
   515 520 525
   Glu Asn Leu Ser Lys Glu Leu Gly Leu Glu Pro Gly Lys Val Ser Lys
   530 535 540
   Trp Phe Lys Asn Ser Arg Tyr Leu Ala Leu Lys Ser Arg Lys Val Glu
   545 550 555 560
   Lys Gly Glu Gln Leu His Asn Ser Ser Ser Lys Val Ser Ala Glu Pro
   565 570 575
   Thr Leu Asn Val Met Glu Gly Lys Thr Ala Asp Leu Ser Gln Asp Ser
   580 585 590
   Trp Glu Glu Thr Glu Val Cys Ile Pro Lys Asn Leu Lys Arg Ile Leu
   595 600 605
   Gln Arg Lys Lys Leu Lys Ser Ile Ser Arg Ser Leu Arg Lys Asn Glu
   610 615 620
   Gln Lys Arg Gly Ser Tyr Glu Ser Pro Thr Lys Ser Asn Glu Met Asn
   625 630 635 640
   Ala Glu Cys Ser Asp Asp Val Ser Leu Lys Lys Leu Leu Lys Ala Lys
   645 650 655
   Pro Lys Gly Val Lys Lys Lys Val Asn Pro Ile Ser Val Ala Ala Glu
   660 665 670
   Tyr Asp Met Glu Arg Leu Cys Arg Ala Lys Ala Ile Leu Glu Lys Met
   675 680 685
   Lys Gln Lys Val Val Lys Leu Gln Asn Gly Lys Ala Arg Lys Ser Ser
   690 695 700
   Lys Thr Arg Pro Leu Glu Glu Phe Ile Val Tyr Ile Pro Ile Ala Glu
   705 710 715 720
   Leu Arg Gly Lys Asn
   725
<210> 191
   <211> 2256
   <212> DNA
   <213> Saccharum officinarum
<400> 191
   atgcattctt cggaaaataa actggtatgc tccaactcgg gaagatcatc aaaagggaat      60
   gagacctcta tggagttggt tcctgtacca aaaagaccaa ctagacatga tgcttcccgc     120
   caatgcaaat ctgattctcc tttgaagcga tcaccaagga aagctagaaa tgctactctg     180
   gcaaaaagca taaagaataa atatcattgt agtcccctca aacagcggag ggcttcagat     240
   tctgtttctg gaaaagttgc gacaggacta actgtgagga ggaggaagaa aagaaaaatg     300
   caaaatactg atgaggcaac tcgcttggaa cgaagagcga gatattttct aatcaagata     360
   aaattggagc agaatttgct agatgcttac tctggagatg gatggaatgg gcaaagccgg     420
   gaaaaaataa agccagataa ggatctgcgg cgtgccagga aacaaatcat aaaatgcaaa     480
   attgctatac gtgatatcat tcgccaactt ggtttgtata cttctactgg gagtcttaat     540
   gacccagcaa tgcctccaga tcagtctacc aatccggaac ataccatgtg ctcaacatgc     600
   aagtctcatg aatcatttcc cagcaataaa tttatcttct gtgaagggcc ctgcaaaagg     660
   gcatatcatg agaaatgttt ggaacctccc ttaaacaaaa gcgtacttcc aacaagtagc     720
   catggatggc tttgcaaatt ctgtttgtgc aaagtgaaaa ttttagaaac tattaatgca     780
   catctaggaa caagttttac agtgaagtgc cccttcgaag atatattcaa ggaagcaacg     840
   gaacaaatag actccgagga tgcactagat gaagattggc tttctgagta ttcaggtgat     900
   gaggactatg atcctgatga aaatgaggac agcggcaact gtatggacag cggggaggaa     960
   attatgtctg atgattccga tggttcagga agcccccttt attctccaaa tgacgatatt    1020
   cctgacttca tatcagcaga cttaaatgac gtggaagggt tttgtcacac caatttagac    1080
   ttaggcattg atgccgttga agatgatttg gcacagatcc ttacctacca aaggccaaga    1140
   agagatgttg attatagaag gcttaatgag gaaatgtttg gaaaaataat aggaaatgaa    1200
   ggacagagtg aggatgaaga ttggggccat gaaaggagaa agaaaaggac ttgttcaggt    1260
   ggtgctgggg ataattctgt gggtttctca aatgttatat ctgaggaaaa gagccaaaag    1320
   aaggggagaa aacttttcag gattcctcct gcagctgtcg aggtacttcg caaagctttt    1380
   gctgaaaatg agcttccacc ccgggatgtc aaagaaaatc tctcaagaga attgggaatt    1440
   tcttttgaaa agattgataa atggttcaaa aatacacggt gtgctgctct cagagatcgc    1500
   aaggctgaag ggaacagtca taatacggct cccagcaaaa gctcaagaaa taaaggaaaa    1560
   gctggaatct caagccaggc tgaaaggaat ggtcatgtta ctggtccctg caataactcg    1620
   agaacaaatg aagaaaaatc gttgacacta gctcctaatg cccagggaga gaagaatggg    1680
   attgcagcag attatgtctt acaaaaatct ggtatatcgg ggaaggttga ttcagtagac    1740
   aactcttgct tggttccctt gtctgaaagc atcaatgtgc atacacgatt gcaacagaat    1800
   cttgagatga ggaagatgga atcaactagc agtcctgtgt ggctgcacaa taaaggaggt    1860
   tgcttgtttc caacaggcca agccaaggag agtacattac ctacaggcaa gccttgtttg 1920
   cagtcagaaa taacccattc aacaattaat gaagtgagca ctttagtgca agctacttct 1980
   tggatggatg ctgggtcgtg cgctgaggtg caagaagcca ctccttgggt ggacatcggg 2040
   gcctcagatt accagccttt cctggacgtg atcgatgaga tgtgcggact tgagtgcagg 2100
   ctgcagagac tgaaggagaa catgctctca tctggcatag acggcaaaac cacaggtgag 2160
   agtgacatgg gaaaccaggc tgtggtgctc gtgccgaccg ccgagctcaa ggaaaaagcg 2220
   ccgcatggca gtttttttgg gcattattgc ccatag 2256
<210> 192
   <211> 751
   <212> PRT
   <213> Saccharum officinarum
<400> 192
   Met His Ser Ser Glu Asn Lys Leu Val Cys Ser Asn Ser Gly Arg Ser
   1               5                   10                  15
   Ser Lys Gly Asn Glu Thr Ser Met Glu Leu Val Pro Val Pro Lys Arg
               20                  25                  30
   Pro Thr Arg His Asp Ala Ser Arg Gln Cys Lys Ser Asp Ser Pro Leu
           35                  40                  45
   Lys Arg Ser Pro Arg Lys Ala Arg Asn Ala Thr Leu Ala Lys Ser Ile
       50                  55                  60
   Lys Asn Lys Tyr His Cys Ser Pro Leu Lys Gln Arg Arg Ala Ser Asp
   65                  70                  75                  80
   Ser Val Ser Gly Lys Val Ala Thr Gly Leu Thr Val Arg Arg Arg Lys
                   85                  90                  95
   Lys Arg Lys Met Gln Asn Thr Asp Glu Ala Thr Arg Leu Glu Arg Arg
               100                 105                 110
   Ala Arg Tyr Phe Leu Ile Lys Ile Lys Leu Glu Gln Asn Leu Leu Asp
           115                 120                 125
   Ala Tyr Ser Gly Asp Gly Trp Asn Gly Gln Ser Arg Glu Lys Ile Lys
       130                 135                 140
   Pro Asp Lys Asp Leu Arg Arg Ala Arg Lys Gln Ile Ile Lys Cys Lys
   145                 150                 155                 160
   Ile Ala Ile Arg Asp Ile Ile Arg Gln Leu Gly Leu Tyr Thr Ser Thr
                   165                 170                 175
   Gly Ser Leu Asn Asp Pro Ala Met Pro Pro Asp Gln Ser Thr Asn Pro
               180                 185                 190
   Glu His Thr Met Cys Ser Thr Cys Lys Ser His Glu Ser Phe Pro Ser
           195                 200                 205
   Asn Lys Phe Ile Phe Cys Glu Gly Pro Cys Lys Arg Ala Tyr His Glu
       210                 215                 220
   Lys Cys Leu Glu Pro Pro Leu Asn Lys Ser Val Leu Pro Thr Ser Ser
   225                 230                 235                 240
   His Gly Trp Leu Cys Lys Phe Cys Leu Cys Lys Val Lys Ile Leu Glu
                   245                 250                 255
   Thr Ile Asn Ala His Leu Gly Thr Ser Phe Thr Val Lys Cys Pro Phe
               260                 265                 270
   Glu Asp Ile Phe Lys Glu Ala Thr Glu Gln Ile Asp Ser Glu Asp Ala
           275                 280                 285
   Leu Asp Glu Asp Trp Leu Ser Glu Tyr Ser Gly Asp Glu Asp Tyr Asp
       290                 295                 300
   Pro Asp Glu Asn Glu Asp Ser Gly Asn Cys Met Asp Ser Gly Glu Glu
   305                 310                 315                 320
   Ile Met Ser Asp Asp Ser Asp Gly Ser Gly Ser Pro Leu Tyr Ser Pro
                   325                 330                 335
   Asn Asp Asp Ile Pro Asp Phe Ile Ser Ala Asp Leu Asn Asp Val Glu
               340                 345                 350
   Gly Phe Cys His Thr Asn Leu Asp Leu Gly Ile Asp Ala Val Glu Asp
           355                 360                 365
   Asp Leu Ala Gln Ile Leu Thr Tyr Gln Arg Pro Arg Arg Asp Val Asp
       370                 375                 380
   Tyr Arg Arg Leu Asn Glu Glu Met Phe Gly Lys Ile Ile Gly Asn Glu
   385                 390                 395                 400
   Gly Gln Ser Glu Asp Glu Asp Trp Gly His Glu Arg Arg Lys Lys Arg
                   405                 410                 415
   Thr Cys Ser Gly Gly Ala Gly Asp Asn Ser Val Gly Phe Ser Asn Val
               420                 425                 430
   Ile Ser Glu Glu Lys Ser Gln Lys Lys Gly Arg Lys Leu Phe Arg Ile
           435                 440                 445
   Pro Pro Ala Ala Val Glu Val Leu Arg Lys Ala Phe Ala Glu Asn Glu
       450                 455                 460
   Leu Pro Pro Arg Asp Val Lys Glu Asn Leu Ser Arg Glu Leu Gly Ile
   465                 470                 475                 480
   Ser Phe Glu Lys Ile Asp Lys Trp Phe Lys Asn Thr Arg Cys Ala Ala
                   485                 490                 495
   Leu Arg Asp Arg Lys Ala Glu Gly Asn Ser His Asn Thr Ala Pro Ser
               500                 505                 510
   Lys Ser Ser Arg Asn Lys Gly Lys Ala Gly Ile Ser Ser Gln Ala Glu
           515                 520                 525
   Arg Asn Gly His Val Thr Gly Pro Cys Asn Asn Ser Arg Thr Asn Glu
       530                 535                 540
   Glu Lys Ser Leu Thr Leu Ala Pro Asn Ala Gln Gly Glu Lys Asn Gly
   545                 550                 555                 560
   Ile Ala Ala Asp Tyr Val Leu Gln Lys Ser Gly Ile Ser Gly Lys Val
                   565                 570                 575
   Asp Ser Val Asp Asn Ser Cys Leu Val Pro Leu Ser Glu Ser Ile Asn
               580                 585                 590
   Val His Thr Arg Leu Gln Gln Asn Leu Glu Met Arg Lys Met Glu Ser
           595                 600                 605
   Thr Ser Ser Pro Val Trp Leu His Asn Lys Gly Gly Cys Leu Phe Pro
       610                 615                 620
   Thr Gly Gln Ala Lys Glu Ser Thr Leu Pro Thr Gly Lys Pro Cys Leu
   625                 630                 635                 640
   Gln Ser Glu Ile Thr His Ser Thr Ile Asn Glu Val Ser Thr Leu Val
                   645                 650                 655
   Gln Ala Thr Ser Trp Met Asp Ala Gly Ser Cys Ala Glu Val Gln Glu
               660                 665                 670
   Ala Thr Pro Trp Val Asp Ile Gly Ala Ser Asp Tyr Gln Pro Phe Leu
           675                 680                 685
   Asp Val Ile Asp Glu Met Cys Gly Leu Glu Cys Arg Leu Gln Arg Leu
       690                 695                 700
   Lys Glu Asn Met Leu Ser Ser Gly Ile Asp Gly Lys Thr Thr Gly Glu
   705                 710                 715                 720
   Ser Asp Met Gly Asn Gln Ala Val Val Leu Val Pro Thr Ala Glu Leu
                   725                 730                 735
   Lys Glu Lys Ala Pro His Gly Ser Phe Phe Gly His Tyr Cys Pro
   740 745 750
<210> 193
   <211> 2181
   <212> DNA
   <213> Vitis vinifera
<400> 193
   atgcgtggaa ctggaaagaa agcaggacat caggaatctg gaaagtcttg ctttcctaaa 60
   agaaatatcg ggcctaagct gaatgcagca ttgcagatta aaaatggtag taaaatatct 120
   cagaccagga aatgcaagcc aaaatcaaaa tctcatgcaa agacaattgg tgcaatcctg 180
   tctaagagaa caactactga ctctccaagc aagggaagca ggagtggatc cacaaccaga 240
   aaactgattc acaagaaaac cctgcataaa gccattgata ctgagtcttc caagaaggag 300
   tcttcatcaa agcttaaagg tgagaagccc ccacaaatta gcacaaacaa aaatggggaa 360
   actgtggaca agaatgttaa acctcaaaaa ttaaagaaaa gaggaaagcg gaagcggcga 420
   aaggataatt cagagctaga tgaagcttct cgcttgcaaa gaagaaccag gtacctcttg 480
   atcaaaatga aactggagca gaatcttatt gatgcttact ctggggaagg ttggaaaggt 540
   catagtcgag aaaagatcag gccagagaag gaactacaaa gagctacgaa gcaaatattg 600
   aaatgtaaac ttgggatccg tgatgcaatt cgccagctgg agtcactgag ctccatagga 660
   tgtattgaag atactgcaat tgcttcagat ggatctgttt atcatgaaca cataatctgt 720
   gcaaagtgca agttgcggga ggctttccca gataatgata ttatactctg tgatgggacg 780
   tgcaattgtg ctttccacca gaaatgtctt gatcctccat tggaaactga aaatattcct 840
   ccgggagacc aaggttggtt ttgcaagttc tgcgagtgta agatggaaat actagaagca 900
   atgaatgcac atcttggaac ccgcttctct gtggacagta cttggcagga tattttcaaa 960
   gaagaagctg ctttgcctga tggtgggagt gcactgccct atccagagga agattggcct 1020
   tctgatgatt cacaagatca tgattatgat cctgaaagaa atgaaaatag ctgcagcatc 1080
   agcactgctg gcactgaagg aaatgcttca gatgatacaa atagttcttt aagcttgagt 1140
   tggtcgtttg aagacgaaat cctttctgga tccaaaagat cagggattat cagtgcagat 1200
   tctgatgaaa catctgattg tgaaatcata agtggtcgta ggcaacgaag agctgttgat 1260
   tatagaaagt tatatgatga aatgtttgga aaggatgctc atgcaaatga acaagttagt 1320
   gaagatgaag actggggtcc tgctaacaaa aggcggagag aaaaggagtc tgatgcagcc 1380
   agcaccctca taactctata tgaaggtgag aaaaagttac ctaatgtgga aaccatggaa 1440
   gccaaacaaa aaatttcttc agatccacaa actaaaaggc cattttccag aattccactt 1500
   gatgcggttg agaagcttcg ccaagcattt ggggagaatg aacttccctc tagagatgtg 1560
   agggagaatc ttgcaaagca gttgggtctt gattatgaga aggtgaacaa atggttcaaa 1620
   aatgcacgat atatagcgct aaaaacaaga aaggcagaaa gagcaaaaca acttcagact 1680
   tctcccagaa tctccaagga atccagatca gaaatcgtga aggacaaaac tgttgatcta 1740
   gtggcatcaa gggataactc atcagcatca ctggtccgtg cactaaagaa tttgaaaaag 1800
   gtgcggagga gaaaaaatcc aaagccaata atgaccagcc ctgtaaagaa aaagcatcat 1860
   agaagggctt tacttgagtc acctacaaat gacaaggtta ctatggagtt tgacgatgat 1920
   gtgagcttga agaaacaact gaagcttttg aaagaaaaaa gtaagaggga caagcaaagg 1980
   gtcgacttca aggagggaac tggtgtacaa gatgcagaga aggagatgga gagactctgc 2040
   caaattaagg ataagataga gaagttgaag caggtaatac tgagacttca atgtgacaaa 2100
   actaatcaat ggcaagacca gtcagtgatc tatgttcctg tggcagagct aagggagaaa 2160
   ggtcgatttt gcatcacatc t 2181
<210> 194
   <211> 727
   <212> PRT
   <213> Vitis vinifera
<400> 194
   Met Arg Gly Thr Gly Lys Lys Ala Gly His Gln Glu Ser Gly Lys Ser
   1               5                   10                  15
   Cys Phe Pro Lys Arg Asn Ile Gly Pro Lys Leu Asn Ala Ala Leu Gln
               20                  25                  30
   Ile Lys Asn Gly Ser Lys Ile Ser Gln Thr Arg Lys Cys Lys Pro Lys
           35                  40                  45
   Ser Lys Ser His Ala Lys Thr Ile Gly Ala Ile Leu Ser Lys Arg Thr
       50                  55                  60
   Thr Thr Asp Ser Pro Ser Lys Gly Ser Arg Ser Gly Ser Thr Thr Arg
   65                  70                  75                  80
   Lys Leu Ile His Lys Lys Thr Leu His Lys Ala Ile Asp Thr Glu Ser
                   85                  90                  95
   Ser Lys Lys Glu Ser Ser Ser Lys Leu Lys Gly Glu Lys Pro Pro Gln
               100                 105                 110
   Ile Ser Thr Asn Lys Asn Gly Glu Thr Val Asp Lys Asn Val Lys Pro
           115                 120                 125
   Gln Lys Leu Lys Lys Arg Gly Lys Arg Lys Arg Arg Lys Asp Asn Ser
       130                 135                 140
   Glu Leu Asp Glu Ala Ser Arg Leu Gln Arg Arg Thr Arg Tyr Leu Leu
   145                 150                 155                 160
   Ile Lys Met Lys Leu Glu Gln Asn Leu Ile Asp Ala Tyr Ser Gly Glu
                   165                 170                 175
   Gly Trp Lys Gly His Ser Arg Glu Lys Ile Arg Pro Glu Lys Glu Leu
               180                 185                 190
   Gln Arg Ala Thr Lys Gln Ile Leu Lys Cys Lys Leu Gly Ile Arg Asp
           195                 200                 205
   Ala Ile Arg Gln Leu Glu Ser Leu Ser Ser Ile Gly Cys Ile Glu Asp
       210                 215                 220
   Thr Ala Ile Ala Ser Asp Gly Ser Val Tyr His Glu His Ile Ile Cys
   225                 230                 235                 240
   Ala Lys Cys Lys Leu Arg Glu Ala Phe Pro Asp Asn Asp Ile Ile Leu
                   245                 250                 255
   Cys Asp Gly Thr Cys Asn Cys Ala Phe His Gln Lys Cys Leu Asp Pro
               260                 265                 270
   Pro Leu Glu Thr Glu Asn Ile Pro Pro Gly Asp Gln Gly Trp Phe Cys
           275                 280                 285
   Lys Phe Cys Glu Cys Lys Met Glu Ile Leu Glu Ala Met Asn Ala His
       290                 295                 300
   Leu Gly Thr Arg Phe Ser Val Asp Ser Thr Trp Gln Asp Ile Phe Lys
   305                 310                 315                 320
   Glu Glu Ala Ala Leu Pro Asp Gly Gly Ser Ala Leu Pro Tyr Pro Glu
                   325                 330                 335
   Glu Asp Trp Pro Ser Asp Asp Ser Gln Asp His Asp Tyr Asp Pro Glu
               340                 345                 350
   Arg Asn Glu Asn Ser Cys Ser Ile Ser Thr Ala Gly Thr Glu Gly Asn
           355                 360                 365
   Ala Ser Asp Asp Thr Asn Ser Ser Leu Ser Leu Ser Trp Ser Phe Glu
       370                 375                 380
   Asp Glu Ile Leu Ser Gly Ser Lys Arg Ser Gly Ile Ile Ser Ala Asp
   385                 390                 395                 400
   Ser Asp Glu Thr Ser Asp Cys Glu Ile Ile Ser Gly Arg Arg Gln Arg
                   405                 410                 415
   Arg Ala Val Asp Tyr Arg Lys Leu Tyr Asp Glu Met Phe Gly Lys Asp
               420                 425                 430
   Ala His Ala Asn Glu Gln Val Ser Glu Asp Glu Asp Trp Gly Pro Ala
           435                 440                 445
   Asn Lys Arg Arg Arg Glu Lys Glu Ser Asp Ala Ala Ser Thr Leu Ile
       450                 455                 460
   Thr Leu Tyr Glu Gly Glu Lys Lys Leu Pro Asn Val Glu Thr Met Glu
   465                 470                 475                 480
   Ala Lys Gln Lys Ile Ser Ser Asp Pro Gln Thr Lys Arg Pro Phe Ser
                   485                 490                 495
   Arg Ile Pro Leu Asp Ala Val Glu Lys Leu Arg Gln Ala Phe Gly Glu
               500                 505                 510
   Asn Glu Leu Pro Ser Arg Asp Val Arg Glu Asn Leu Ala Lys Gln Leu
           515                 520                 525
   Gly Leu Asp Tyr Glu Lys Val Asn Lys Trp Phe Lys Asn Ala Arg Tyr
       530                 535                 540
   Ile Ala Leu Lys Thr Arg Lys Ala Glu Arg Ala Lys Gln Leu Gln Thr
   545                 550                 555                 560
   Ser Pro Arg Ile Ser Lys Glu Ser Arg Ser Glu Ile Val Lys Asp Lys
                   565                 570                 575
   Thr Val Asp Leu Val Ala Ser Arg Asp Asn Ser Ser Ala Ser Leu Val
               580                 585                 590
   Arg Ala Leu Lys Asn Leu Lys Lys Val Arg Arg Arg Lys Asn Pro Lys
           595                 600                 605
   Pro Ile Met Thr Ser Pro Val Lys Lys Lys His His Arg Arg Ala Leu
       610                 615                 620
   Leu Glu Ser Pro Thr Asn Asp Lys Val Thr Met Glu Phe Asp Asp Asp
   625                 630                 635                 640
   Val Ser Leu Lys Lys Gln Leu Lys Leu Leu Lys Glu Lys Ser Lys Arg
                   645                 650                 655
   Asp Lys Gln Arg Val Asp Phe Lys Glu Gly Thr Gly Val Gln Asp Ala
               660                 665                 670
   Glu Lys Glu Met Glu Arg Leu Cys Gln Ile Lys Asp Lys Ile Glu Lys
           675                 680                 685
   Leu Lys Gln Val Ile Leu Arg Leu Gln Cys Asp Lys Thr Asn Gln Trp
       690                 695                 700
   Gln Asp Gln Ser Val Ile Tyr Val Pro Val Ala Glu Leu Arg Glu Lys
   705                 710                 715                 720
   Gly Arg Phe Cys Ile Thr Ser
                   725
<210> 195
   <211> 2166
   <212> DNA
   <213> Zea mays
<400> 195
   atgcattctt cggaaaataa actgggttgg aatgagacct ctatggggtt ggttcctgta     60
   ccaaaaagac cagctagacc tgatgcttcc caccaatgca aatctgattc ctttatgaag    120
   cgatcaccaa ggaaagttag aaatgctact ctggcaaaaa gcataaagag caaataccac    180
   tatagtcccc tcaaacagcg gaagggttca gattctgttc ctgggaaaat cgtaacagga    240
   ctaaccgcaa ggaaaaagaa gaaaaggaaa atccaaatta cagacgaggc aactcgtttg    300
   gaacgaagag cgagatattt tctaatcaag ataaaactgg agcagaattt gctagatgct    360
   tactctggag atggatggaa tgggcaaagc cgagagaaaa taaagccaga gaaggaactg    420
   caacgtgcca ggaaacaaat cataaaatgc aaaattgcta tacgtgatat catccgccaa    480
   cttgatttgt atacttctac tgggagtgtt gatgacccac taatgcctac agatcagtcc    540
   accaatcccg aacataccat gtgctcaaca tgcaagtctc atgaatcatt tcccagcaat    600
   aaaattatct tctgcaaagg gccctgcaaa agggcatgtc atgagaaatg tttggaacct    660
   cccttaaaca aaagcgtact tccaacaagt agccatgggt ggctttgcaa attctgtttg    720
   tgcaaggtga ggattttaga aactattaat gcacatctag ggacgagttt tacagtgaag    780
   tgccactttg aagatatatt caaggaaact actgaactaa tagactctga ggatgcacta    840
   gatgaagatt ggctttctga gtattcaggt gatgaggact atgatcctga tgaaaatgag    900
   gccagtggcg actgtatgga cagcggggag aagattatgt ctgatgattc caatggttca    960
   ggaagccccc tttattctcc aaatgacgat attcctgact tcatatcagc agacttaaat   1020
   gttgtggaag ggttttgtca taccaattta gatttaggca ttgatgccgt tgaagatgat   1080
   tttgcacaga tcctcaccta ccaaaggcca agaagagatg ttgattatag aaggcttaac   1140
   gaggaaatgt ttgggaaaat aaccgggaat gaagaacaga gcgaggacga agattggggc   1200
   catgaaagga gaaagaaaag gacgcattca ggtgttgctg gggataattc tgttggtttc   1260
   ttgaacgtta tatctgatga aaagagccag aagaagggga gaaaactttt caggattcct   1320
   cctgcagctg ttgaggtact tcgcagagct tttgctgaaa atgagcttcc accccgggat   1380
   gttaaagaaa atctctcaag agaattggga atttcttttg aaaagattga taaatggttc   1440
   aaaaatacac ggtgtgctgc tctcagagat cggaaggctg aaggaaacag tcataataca   1500
   gctcccagca aaagctcaaa aaataaagga aaagctggaa tctcaggcaa gactggaagg   1560
   aatggtcatg ttactggtcc ctgcaataat ttgagaacaa atgaagaaaa aacaggtata   1620
   tcggggaagt ttgattcagg agacaactct tgtttggttc ccttctctga agtcatcaat   1680
   gtgcctacgc gattgccaca caactttgag atgaggaaga tggaatcaac tagaagtcct   1740
   gcgaggctac acaataaagg agggttcctg tctgcaacag tccaaattaa ggagagtacg   1800
   ttgttaccca caggcaagcc ttgttggcag tcagaaatga gccatccaac aactaatgaa   1860
   gtgagcactt cagcgcaagc tactacttgg atcgacgcgg gggcgtgtgc cgaggagcaa   1920
   gaacctactc cttgggtgga catcggggtc tcagactacc agcctttcct ggatgtgatc    1980
   gacgacatgt gcggacttga gtggaggctg cagagactga aggagaacat gctctcatct    2040
   agcatagacg gccaaaccgc cgccagtgag agtgacaaaa gaaaccagac cgtggtgcta    2100
   gtgccaaccg ccgagctcaa ggaaaagctg ccgcatgacg gtttattcgg gcattattgc    2160
   ccatag                                                               2166
<210> 196
   <211> 721
   <212> PRT
   <213> Zea mays
<400> 196
   Met His Ser Ser Glu Asn Lys Leu Gly Trp Asn Glu Thr Ser Met Gly
   1               5                   10                  15
   Leu Val Pro Val Pro Lys Arg Pro Ala Arg Pro Asp Ala Ser His Gln
               20                  25                  30
   Cys Lys Ser Asp Ser Phe Met Lys Arg Ser Pro Arg Lys Val Arg Asn
           35                  40                  45
   Ala Thr Leu Ala Lys Ser Ile Lys Ser Lys Tyr His Tyr Ser Pro Leu
       50                  55                  60
   Lys Gln Arg Lys Gly Ser Asp Ser Val Pro Gly Lys Ile Val Thr Gly
   65                  70                  75                  80
   Leu Thr Ala Arg Lys Lys Lys Lys Arg Lys Ile Gln Ile Thr Asp Glu
                   85                  90                  95
   Ala Thr Arg Leu Glu Arg Arg Ala Arg Tyr Phe Leu Ile Lys Ile Lys
               100                 105                 110
   Leu Glu Gln Asn Leu Leu Asp Ala Tyr Ser Gly Asp Gly Trp Asn Gly
           115                 120                 125
   Gln Ser Arg Glu Lys Ile Lys Pro Glu Lys Glu Leu Gln Arg Ala Arg
       130                 135                 140
   Lys Gln Ile Ile Lys Cys Lys Ile Ala Ile Arg Asp Ile Ile Arg Gln
   145                 150                 155                 160
   Leu Asp Leu Tyr Thr Ser Thr Gly Ser Val Asp Asp Pro Leu Met Pro
                   165                 170                 175
   Thr Asp Gln Ser Thr Asn Pro Glu His Thr Met Cys Ser Thr Cys Lys
               180                 185                 190
   Ser His Glu Ser Phe Pro Ser Asn Lys Ile Ile Phe Cys Lys Gly Pro
           195                 200                 205
   Cys Lys Arg Ala Cys His Glu Lys Cys Leu Glu Pro Pro Leu Asn Lys
       210                 215                 220
   Ser Val Leu Pro Thr Ser Ser His Gly Trp Leu Cys Lys Phe Cys Leu
   225                 230                 235                 240
   Cys Lys Val Arg Ile Leu Glu Thr Ile Asn Ala His Leu Gly Thr Ser
                   245                 250                 255
   Phe Thr Val Lys Cys His Phe Glu Asp Ile Phe Lys Glu Thr Thr Glu
               260                 265                 270
   Leu Ile Asp Ser Glu Asp Ala Leu Asp Glu Asp Trp Leu Ser Glu Tyr
           275                 280                 285
   Ser Gly Asp Glu Asp Tyr Asp Pro Asp Glu Asn Glu Ala Ser Gly Asp
       290                 295                 300
   Cys Met Asp Ser Gly Glu Lys Ile Met Ser Asp Asp Ser Asn Gly Ser
   305                 310                 315                 320
   Gly Ser Pro Leu Tyr Ser Pro Asn Asp Asp Ile Pro Asp Phe Ile Ser
                   325                 330                 335
   Ala Asp Leu Asn Val Val Glu Gly Phe Cys His Thr Asn Leu Asp Leu
               340                 345                 350
   Gly Ile Asp Ala Val Glu Asp Asp Phe Ala Gln Ile Leu Thr Tyr Gln
           355                 360                 365
   Arg Pro Arg Arg Asp Val Asp Tyr Arg Arg Leu Asn Glu Glu Met Phe
       370                 375                 380
   Gly Lys Ile Thr Gly Asn Glu Glu Gln Ser Glu Asp Glu Asp Trp Gly
   385                 390                 395                 400
   His Glu Arg Arg Lys Lys Arg Thr His Ser Gly Val Ala Gly Asp Asn
                   405                 410                 415
   Ser Val Gly Phe Leu Asn Val Ile Ser Asp Glu Lys Ser Gln Lys Lys
               420                 425                 430
   Gly Arg Lys Leu Phe Arg Ile Pro Pro Ala Ala Val Glu Val Leu Arg
           435                 440                 445
   Arg Ala Phe Ala Glu Asn Glu Leu Pro Pro Arg Asp Val Lys Glu Asn
       450                 455                 460
   Leu Ser Arg Glu Leu Gly Ile Ser Phe Glu Lys Ile Asp Lys Trp Phe
   465                 470                 475                 480
   Lys Asn Thr Arg Cys Ala Ala Leu Arg Asp Arg Lys Ala Glu Gly Asn
                   485                 490                 495
   Ser His Asn Thr Ala Pro Ser Lys Ser Ser Lys Asn Lys Gly Lys Ala
               500                 505                 510
   Gly Ile Ser Gly Lys Thr Gly Arg Asn Gly His Val Thr Gly Pro Cys
           515                 520                 525
   Asn Asn Leu Arg Thr Asn Glu Glu Lys Thr Gly Ile Ser Gly Lys Phe
       530                 535                 540
   Asp Ser Gly Asp Asn Ser Cys Leu Val Pro Phe Ser Glu Val Ile Asn
   545                 550                 555                 560
   Val Pro Thr Arg Leu Pro His Asn Phe Glu Met Arg Lys Met Glu Ser
                   565                 570                 575
   Thr Arg Ser Pro Ala Arg Leu His Asn Lys Gly Gly Phe Leu Ser Ala
               580                 585                 590
   Thr Val Gln Ile Lys Glu Ser Thr Leu Leu Pro Thr Gly Lys Pro Cys
           595                 600                 605
   Trp Gln Ser Glu Met Ser His Pro Thr Thr Asn Glu Val Ser Thr Ser
       610                 615                 620
   Ala Gln Ala Thr Thr Trp Ile Asp Ala Gly Ala Cys Ala Glu Glu Gln
   625                 630                 635                 640
   Glu Pro Thr Pro Trp Val Asp Ile Gly Val Ser Asp Tyr Gln Pro Phe
                   645                 650                 655
   Leu Asp Val Ile Asp Asp Met Cys Gly Leu Glu Trp Arg Leu Gln Arg
               660                 665                 670
   Leu Lys Glu Asn Met Leu Ser Ser Ser Ile Asp Gly Gln Thr Ala Ala
           675                 680                 685
   Ser Glu Ser Asp Lys Arg Asn Gln Thr Val Val Leu Val Pro Thr Ala
       690                 695                 700
   Glu Leu Lys Glu Lys Leu Pro His Asp Gly Leu Phe Gly His Tyr Cys
   705                 710                 715                 720
   Pro
<210> 197
   <211> 2172
   <212> DNA
   <213> Arabidopsis thaliana
<400> 197
   atgtataaag cagtaagtaa gcgggtaact agaagtagtg gatctggttt gaagcagaca      60
   aatgtagata atggaggaga gattagtcca actgttgaca gagtatctga acaaggcaaa     120
   tcatcagagg cagggagtca catgccaact gatgcaaacg gaaatggaca tttgcatcat     180
   gagattatgg atcatggaaa ggggaatgaa gaacagaaac caacccctca aaccgtgaag     240
   aaggattcta ataccaatac caaattttca gggtctcatc gtgagcttgt catcggtctt     300
   ccttgccgtg gacaatttga aatccacaac cggtccaggg catctacgag ttcgaagagg     360
   ttaggaggag gtggagaaag gaatgtgttg tttgcaagtc acaagagagc tcagagatct     420
   aaggaagatg ctggaccatc ttcggttgtt gctaattcca cacctgtagg tcgaccaaag     480
   aaaaagaata agactatgaa caaaggacaa gttagagaag atgatgagta cacaagaatc     540
   aaaaagaaac ttcgatactt tctaaaccgg attaactatg agcaaagcct aattgacgcc     600
   tattcgttag aaggctggaa aggttcaagc ctggaaaaga taaggccaga gaaagagctt     660
   gaacgagcca caaaggaaat tttgcggcgc aagcttaaaa ttagagatct ttttcagcac     720
   cttgacacac tctgtgctga aggaagcctt ccagagtctt tatttgatac tgatggagag     780
   atttctagcg aggatatctt ctgtgcaaaa tgtggttcaa aggacttgtc tgttgataat     840
   gatatcatac tatgcgatgg gttttgcgat cgaggctttc accaatactg tcttgaacca     900
   cctctgcgga aagaagatat tcctccggat gatgagggtt ggctatgccc cggatgtgat     960
   tgcaaagatg acagcttgga cttgcttaat gattctttag ggacaaaatt ttcggtcagc    1020
   gacagttggg agaaaatatt tcctgaggca gcagcagcgc tggttggtgg aggtcaaaat    1080
   cttgactgtg atcttccttc agatgattct gatgatgaag agtatgatcc agattgtttg    1140
   aatgataatg aaaatgatga agatggcagt gatgacaatg aagaatctga gaacgaagat    1200
   ggtagttctg atgaaactga attcacatct gcatctgacg aaatgattga gtcatttaaa    1260
   gaagggaaag atataatgaa agatgtaatg gcccttccat ctgatgattc tgaggatgat    1320
   gactatgatc ctgatgcccc gacttgtgat gatgataaag aaagttcaaa ttctgattgt    1380
   acatcagaca ctgaagatct tgaaacttcc ttcaaaggag atgagactaa tcagcaagct    1440
   gaagatacac cacttgagga tcctggcaga cagacgtctc agctccaagg tgatgcaatt    1500
   ttagagtcag atgttgggct tgatgatggt cctgccggtg tatccagaag gaggaatgtt    1560
   gaaaggctgg attacaaaaa gttgtatgat gaggaatatg acaatgttcc gacttcgtca    1620
   agtgatgatg acgattggga taaaactgca agaatgggaa aagaagattc cgagtcggaa    1680
   gatgaagggg acactgtacc tctgaaacag tcttcaaatg cagaagatca tacttctaag    1740
   aaacttatac ggaagtcgaa aagggcagat aaaaaggata ccttagaaat gccacaagaa    1800
   ggtcctggag aaaatggtgg ttctggtgaa attgaaaaaa gcagctcttc agcatgtaaa    1860
   cagacagatc ccaaaactca gagactgtac atatcttttc aagagaatca atatccagac    1920
   aaagccacga aggagagctt agccaaagag ctacaaatga cagttaagca agttaataac    1980
   tggtttaagc ataggcgttg gtcgataaac agcaaacctt tagtttcaga ggaaaatgtc    2040
   gagaagttaa aaacaggcaa ggaaggtgaa tgtgagacat ctgttgctgg aagtagcaaa 2100
   caaactatgg aaaccgagtc agttgcagag aaaccaacca atactggatc acggaaaaga 2160
   agaagaaagt ag 2172
<210> 198
   <211> 723
   <212> PRT
   <213> Arabidopsis thaliana
<400> 198
   Met Tyr Lys Ala Val Ser Lys Arg Val Thr Arg Ser Ser Gly Ser Gly
   1               5                   10                  15
   Leu Lys Gln Thr Asn Val Asp Asn Gly Gly Glu Ile Ser Pro Thr Val
               20                  25                  30
   Asp Arg Val Ser Glu Gln Gly Lys Ser Ser Glu Ala Gly Ser His Met
           35                  40                  45
   Pro Thr Asp Ala Asn Gly Asn Gly His Leu His His Glu Ile Met Asp
       50                  55                  60
   His Gly Lys Gly Asn Glu Glu Gln Lys Pro Thr Pro Gln Thr Val Lys
   65                  70                  75                  80
   Lys Asp Ser Asn Thr Asn Thr Lys Phe Ser Gly Ser His Arg Glu Leu
                   85                  90                  95
   Val Ile Gly Leu Pro Cys Arg Gly Gln Phe Glu Ile His Asn Arg Ser
               100                 105                 110
   Arg Ala Ser Thr Ser Ser Lys Arg Leu Gly Gly Gly Gly Glu Arg Asn
           115                 120                 125
   Val Leu Phe Ala Ser His Lys Arg Ala Gln Arg Ser Lys Glu Asp Ala
       130                 135                 140
   Gly Pro Ser Ser Val Val Ala Asn Ser Thr Pro Val Gly Arg Pro Lys
   145                 150                 155                 160
   Lys Lys Asn Lys Thr Met Asn Lys Gly Gln Val Arg Glu Asp Asp Glu
                   165                 170                 175
   Tyr Thr Arg Ile Lys Lys Lys Leu Arg Tyr Phe Leu Asn Arg Ile Asn
               180                 185                 190
   Tyr Glu Gln Ser Leu Ile Asp Ala Tyr Ser Leu Glu Gly Trp Lys Gly
           195                 200                 205
   Ser Ser Leu Glu Lys Ile Arg Pro Glu Lys Glu Leu Glu Arg Ala Thr
       210                 215                 220
   Lys Glu Ile Leu Arg Arg Lys Leu Lys Ile Arg Asp Leu Phe Gln His
   225 230 235 240
   Leu Asp Thr Leu Cys Ala Glu Gly Ser Leu Pro Glu Ser Leu Phe Asp
   245 250 255
   Thr Asp Gly Glu Ile Ser Ser Glu Asp Ile Phe Cys Ala Lys Cys Gly
   260 265 270
   Ser Lys Asp Leu Ser Val Asp Asn Asp Ile Ile Leu Cys Asp Gly Phe
   275 280 285
   Cys Asp Arg Gly Phe His Gln Tyr Cys Leu Glu Pro Pro Leu Arg Lys
   290 295 300
   Glu Asp Ile Pro Pro Asp Asp Glu Gly Trp Leu Cys Pro Gly Cys Asp
   305 310 315 320
   Cys Lys Asp Asp Ser Leu Asp Leu Leu Asn Asp Ser Leu Gly Thr Lys
   325 330 335
   Phe Ser Val Ser Asp Ser Trp Glu Lys Ile Phe Pro Glu Ala Ala Ala
   340 345 350
   Ala Leu Val Gly Gly Gly Gln Asn Leu Asp Cys Asp Leu Pro Ser Asp
   355 360 365
   Asp Ser Asp Asp Glu Glu Tyr Asp Pro Asp Cys Leu Asn Asp Asn Glu
   370 375 380
   Asn Asp Glu Asp Gly Ser Asp Asp Asn Glu Glu Ser Glu Asn Glu Asp
   385 390 395 400
   Gly Ser Ser Asp Glu Thr Glu Phe Thr Ser Ala Ser Asp Glu Met Ile
   405 410 415
   Glu Ser Phe Lys Glu Gly Lys Asp Ile Met Lys Asp Val Met Ala Leu
   420 425 430
   Pro Ser Asp Asp Ser Glu Asp Asp Asp Tyr Asp Pro Asp Ala Pro Thr
   435 440 445
   Cys Asp Asp Asp Lys Glu Ser Ser Asn Ser Asp Cys Thr Ser Asp Thr
   450 455 460
   Glu Asp Leu Glu Thr Ser Phe Lys Gly Asp Glu Thr Asn Gln Gln Ala
   465 470 475 480
   Glu Asp Thr Pro Leu Glu Asp Pro Gly Arg Gln Thr Ser Gln Leu Gln
   485 490 495
   Gly Asp Ala Ile Leu Glu Ser Asp Val Gly Leu Asp Asp Gly Pro Ala
                   500                 505                 510
   Gly Val Ser Arg Arg Arg Asn Val Glu Arg Leu Asp Tyr Lys Lys Leu
           515                 520                 525
   Tyr Asp Glu Glu Tyr Asp Asn Val Pro Thr Ser Ser Ser Asp Asp Asp
       530                 535                 540
   Asp Trp Asp Lys Thr Ala Arg Met Gly Lys Glu Asp Ser Glu Ser Glu
   545                 550                 555                 560
   Asp Glu Gly Asp Thr Val Pro Leu Lys Gln Ser Ser Asn Ala Glu Asp
                   565                 570                 575
   His Thr Ser Lys Lys Leu Ile Arg Lys Ser Lys Arg Ala Asp Lys Lys
                   580                 585                 590
   Asp Thr Leu Glu Met Pro Gln Glu Gly Pro Gly Glu Asn Gly Gly Ser
           595                 600                 605
   Gly Glu Ile Glu Lys Ser Ser Ser Ser Ala Cys Lys Gln Thr Asp Pro
       610                 615                 620
   Lys Thr Gln Arg Leu Tyr Ile Ser Phe Gln Glu Asn Gln Tyr Pro Asp
   625                 630                 635                 640
   Lys Ala Thr Lys Glu Ser Leu Ala Lys Glu Leu Gln Met Thr Val Lys
                   645                 650                 655
   Gln Val Asn Asn Trp Phe Lys His Arg Arg Trp Ser Ile Asn Ser Lys
                   660                 665                 670
   Pro Leu Val Ser Glu Glu Asn Val Glu Lys Leu Lys Thr Gly Lys Glu
           675                 680                 685
   Gly Glu Cys Glu Thr Ser Val Ala Gly Ser Ser Lys Gln Thr Met Glu
       690                 695                 700
   Thr Glu Ser Val Ala Glu Lys Pro Thr Asn Thr Gly Ser Arg Lys Arg
   705                 710                 715                 720
   Arg Arg Lys
<210> 199
   <211> 2901
   <212> DNA
   <213> Lotus japonicus
<400> 199
   atgattagtt caactggaga attgacaagc cataacaaca gtagcattga gcacatggaa      60
   actgagcaac ctgaattgtc tgaaaaaaca cctcagatag gctctgaatg tctggacggt    120
   gaacaaaggg tgcttggtac tgtgtcaacc agttctgtta ataatgaaaa ttcaaatcaa    180
   gtttctgcca atgtgaccga gaattctgtt tttcaactac cagcaacacc tcaacattgc    240
   ttagaaaaga gtggtcagac tgtaacaagt ccacccctgg aagaaagcac cttgaaacaa    300
   gttgctacta atgtatctaa tggtaaatca gaaaacaaat gtcagacatc ctctcaagat    360
   gttcaaaacg aacttgtgga agcaagtgat gtaataactg gctcttttgt tgcggaccaa    420
   acacaatcca ttcctgctca agtgaatacg agttctgtga atgaactatt ggatattcct    480
   tccggagatg ttgcagtaga agttggttct gatagtttag aaaaaaagtc taaagggtgc    540
   ttggtagaca gcgaacaaag ggtacttggt actgtgttaa ccaattctat taatgatgga    600
   aattcaaatc aagtttctgc caatgtgacc gagaattctg ttattcaact gccagcacca    660
   cctcaacatt gctttgaaaa gagtggacag actgtaaaaa gttcatctct ggaagaaagt    720
   accttaaaac aagtctccac taatttatct aacagtaaat cagaaaacat atgtcagaca    780
   tcctctcaag atgttcaaaa cgaacttgtg gaaacaagtg atgcagtaac tggctctgtt    840
   gttgcagaca aaacacaatc cattcctgct caagtgaata caagttctgt gaatgaacta    900
   ttggatattc cttccagaga tgttgcagaa gaagttggtt ctgatagttc agaaaggaag    960
   tctaaaagca caactccttc acaattgaga catataggta aaagtaactc aaagttatcg   1020
   aaaaagaagt atatcctaag gtcgttagga agcagtgaca gagctttgcg gtccaggaca   1080
   aaagaaaaac ctaaagcgcc tgaaccaagt agtaactcag ttgacgttaa taatgatgga   1140
   gtgaagaagg aaaaaaggag gaagaagaaa aaaacaggag gggaggaaaa gaaggatcaa   1200
   ttttataaaa tcaaagctca cctaagatat ttattaaaca gagtaagcta tgagcaaaac   1260
   ttaattgatg cttactctgg tgacggctgg aaaggataca gtatggacaa gttaaagcct   1320
   gataaggaga ttcaacgggc taaatctgaa attcttcgac gtaaattgaa aattagggat   1380
   ctatttcaaa acttggattc cttgtgtgct gaaggaaagt ttccagaatc tttgtttgat   1440
   tctgagggag agattgacag tgaggatata ttctgttcaa tttgccagac caaagagttg   1500
   agcactgata atgatataat tctctgtgat ggtgcttgtg accgtggatt tcaccagcac   1560
   tgcttggatc ctccattgtt aactgaagac attccacctg gcgatgaggg ttggttatgc   1620
   cccggatgtg attgcaaaga tgactgcgtt gaccttctta acgactcctt aggaacacgt   1680
   ctctctctta gtgacacctg ggagaaggtt tttcctgaag cagcagctgt tactggaaat   1740
   aatatggatc agggacttcc ttctgatgat tctgatgatg atgattataa ccctaatggt   1800
   aaagaggatg tggaggttga aggaggtgaa tcaagttctg atgaatctga atatgcttct   1860
   gcttctgaga aattggaaga ttcacaccat gaggatccat acttaggcct tccttctgaa   1920
   gattcagagg acaatgatta tgatcctagt gctccagatc ttgacaataa agtcacagaa   1980
   gaaagttcaa gctctgattt cacatctgac tctgaggatc ttgctgctgc tattgaggat   2040
   aacacgtccc ctggacatgt taaacagact aaaagcagac aaaaaagtaa agttggtaag   2100
   aacccatcaa tggttgatga agtgtcatct ttacgagagc cagaacttga ggaagagggt   2160
   ttcactccag tttctgggaa aagaaatgtg gaaaggctgg actacaaaaa gctgtatgat   2220
   gagacataca aaagtgatac tagtgaagat gaagaatgga ctgccagtgc cactccaagt   2280
   agaaaaaaga aactctgtgg caaaatgact ccagtgtcat cagatggaaa ggcctcaaat   2340
   aattctagac atactcctga aaggaacacc caacaagata aagttgaaaa tacaaataat   2400
   tcacccacta aatcacttga aggctgtctg gaatctggct caagggataa aaagcctagg   2460
   tcttcaacac gtcaaagact tggagacgtt gtagtgcaga gacttcacaa atcttttaaa   2520
   gacaatcagt atccggatcg agccacaaaa gaaagcttgg cagaagaact ggggctcact   2580
   tttttccagg ttgacaaatg gtttggcaat gctcgatggg gctttcggcg ttcatcgcgt   2640
   atgggagcta gtccaggcga atatgcttcg ccacaggcca ctggcagcgg acctgaaaat   2700
   actggggaga gggaacgcga attagcttcc caggaagtcg gtgaagaaaa attgaaaacc   2760
   cccagtccta gaaaaaggaa gcatttgtct gaaccgcagg catctgaagc accgcagatt   2820
   attgttcttg gcttagcagc atctcctggc tcaccacggg ctcatgcagg caataaaaag   2880
   aagacagtga aaaggaaatg a                                              2901
<210> 200
   <211> 966
   <212> PRT
   <213> Lotus japonicus

<400> 200
   Met Ile Ser Ser Thr Gly Glu Leu Thr Ser His Asn Asn Ser Ser Ile
   1               5                   10                  15
   Glu His Met Glu Thr Glu Gln Pro Glu Leu Ser Glu Lys Thr Pro Gln
               20                  25                  30
   Ile Gly Ser Glu Cys Leu Asp Gly Glu Gln Arg Val Leu Gly Thr Val
           35                  40                  45
   Ser Thr Ser Ser Val Asn Asn Glu Asn Ser Asn Gln Val Ser Ala Asn
       50                  55                  60
   Val Thr Glu Asn Ser Val Phe Gln Leu Pro Ala Thr Pro Gln His Cys
   65                  70                  75                  80
   Leu Glu Lys Ser Gly Gln Thr Val Thr Ser Pro Pro Leu Glu Glu Ser
                   85                  90                  95
   Thr Leu Lys Gln Val Ala Thr Asn Val Ser Asn Gly Lys Ser Glu Asn
               100                 105                 110
   Lys Cys Gln Thr Ser Ser Gln Asp Val Gln Asn Glu Leu Val Glu Ala
           115                 120                 125
   Ser Asp Val Ile Thr Gly Ser Phe Val Ala Asp Gln Thr Gln Ser Ile
       130                 135                 140
   Pro Ala Gln Val Asn Thr Ser Ser Val Asn Glu Leu Leu Asp Ile Pro
   145                 150                 155                 160
   Ser Gly Asp Val Ala Val Glu Val Gly Ser Asp Ser Leu Glu Lys Lys
                   165                 170                 175
   Ser Lys Gly Cys Leu Val Asp Ser Glu Gln Arg Val Leu Gly Thr Val
               180                 185                 190
   Leu Thr Asn Ser Ile Asn Asp Gly Asn Ser Asn Gln Val Ser Ala Asn
           195                 200                 205
   Val Thr Glu Asn Ser Val Ile Gln Leu Pro Ala Pro Pro Gln His Cys
       210                 215                 220
   Phe Glu Lys Ser Gly Gln Thr Val Lys Ser Ser Ser Leu Glu Glu Ser
   225                 230                 235                 240
   Thr Leu Lys Gln Val Ser Thr Asn Leu Ser Asn Ser Lys Ser Glu Asn
                   245                 250                 255
   Ile Cys Gln Thr Ser Ser Gln Asp Val Gln Asn Glu Leu Val Glu Thr
               260                 265                 270
   Ser Asp Ala Val Thr Gly Ser Val Val Ala Asp Lys Thr Gln Ser Ile
           275                 280                 285
   Pro Ala Gln Val Asn Thr Ser Ser Val Asn Glu Leu Leu Asp Ile Pro
       290                 295                 300
   Ser Arg Asp Val Ala Glu Glu Val Gly Ser Asp Ser Ser Glu Arg Lys
   305                 310                 315                 320
   Ser Lys Ser Thr Thr Pro Ser Gln Leu Arg His Ile Gly Lys Ser Asn
                   325                 330                 335
   Ser Lys Leu Ser Lys Lys Lys Tyr Ile Leu Arg Ser Leu Gly Ser Ser
               340                 345                 350
   Asp Arg Ala Leu Arg Ser Arg Thr Lys Glu Lys Pro Lys Ala Pro Glu
           355                 360                 365
   Pro Ser Ser Asn Ser Val Asp Val Asn Asn Asp Gly Val Lys Lys Glu
       370                 375                 380
   Lys Arg Arg Lys Lys Lys Lys Thr Gly Gly Glu Glu Lys Lys Asp Gln
   385                 390                 395                 400
   Phe Tyr Lys Ile Lys Ala His Leu Arg Tyr Leu Leu Asn Arg Val Ser
                   405                 410                 415
   Tyr Glu Gln Asn Leu Ile Asp Ala Tyr Ser Gly Asp Gly Trp Lys Gly
   420 425 430
   Tyr Ser Met Asp Lys Leu Lys Pro Asp Lys Glu Ile Gln Arg Ala Lys
   435 440 445
   Ser Glu Ile Leu Arg Arg Lys Leu Lys Ile Arg Asp Leu Phe Gln Asn
   450 455 460
   Leu Asp Ser Leu Cys Ala Glu Gly Lys Phe Pro Glu Ser Leu Phe Asp
   465 470 475 480
   Ser Glu Gly Glu Ile Asp Ser Glu Asp Ile Phe Cys Ser Ile Cys Gln
   485 490 495
   Thr Lys Glu Leu Ser Thr Asp Asn Asp Ile Ile Leu Cys Asp Gly Ala
   500 505 510
   Cys Asp Arg Gly Phe His Gln His Cys Leu Asp Pro Pro Leu Leu Thr
   515 520 525
   Glu Asp Ile Pro Pro Gly Asp Glu Gly Trp Leu Cys Pro Gly Cys Asp
   530 535 540
   Cys Lys Asp Asp Cys Val Asp Leu Leu Asn Asp Ser Leu Gly Thr Arg
   545 550 555 560
   Leu Ser Leu Ser Asp Thr Trp Glu Lys Val Phe Pro Glu Ala Ala Ala
   565 570 575
   Val Thr Gly Asn Asn Met Asp Gln Gly Leu Pro Ser Asp Asp Ser Asp
   580 585 590
   Asp Asp Asp Tyr Asn Pro Asn Gly Lys Glu Asp Val Glu Val Glu Gly
   595 600 605
   Gly Glu Ser Ser Ser Asp Glu Ser Glu Tyr Ala Ser Ala Ser Glu Lys
   610 615 620
   Leu Glu Asp Ser His His Glu Asp Pro Tyr Leu Gly Leu Pro Ser Glu
   625 630 635 640
   Asp Ser Glu Asp Asn Asp Tyr Asp Pro Ser Ala Pro Asp Leu Asp Asn
   645 650 655
   Lys Val Thr Glu Glu Ser Ser Ser Ser Asp Phe Thr Ser Asp Ser Glu
   660 665 670
   Asp Leu Ala Ala Ala Ile Glu Asp Asn Thr Ser Pro Gly His Val Lys
   675 680 685
   Gln Thr Lys Ser Arg Gln Lys Ser Lys Val Gly Lys Asn Pro Ser Met
       690                 695                 700
   Val Asp Glu Val Ser Ser Leu Arg Glu Pro Glu Leu Glu Glu Glu Gly
   705                 710                 715                 720
   Phe Thr Pro Val Ser Gly Lys Arg Asn Val Glu Arg Leu Asp Tyr Lys
                   725                 730                 735
   Lys Leu Tyr Asp Glu Thr Tyr Lys Ser Asp Thr Ser Glu Asp Glu Glu
               740                 745                 750
   Trp Thr Ala Ser Ala Thr Pro Ser Arg Lys Lys Lys Leu Cys Gly Lys
           755                 760                 765
   Met Thr Pro Val Ser Ser Asp Gly Lys Ala Ser Asn Asn Ser Arg His
       770                 775                 780
   Thr Pro Glu Arg Asn Thr Gln Gln Asp Lys Val Glu Asn Thr Asn Asn
   785                 790                 795                 800
   Ser Pro Thr Lys Ser Leu Glu Gly Cys Leu Glu Ser Gly Ser Arg Asp
                   805                 810                 815
   Lys Lys Pro Arg Ser Ser Thr Arg Gln Arg Leu Gly Asp Val Val Val
               820                 825                 830
   Gln Arg Leu His Lys Ser Phe Lys Asp Asn Gln Tyr Pro Asp Arg Ala
           835                 840                 845
   Thr Lys Glu Ser Leu Ala Glu Glu Leu Gly Leu Thr Phe Phe Gln Val
       850                 855                 860
   Asp Lys Trp Phe Gly Asn Ala Arg Trp Gly Phe Arg Arg Ser Ser Arg
   865                 870                 875                 880
   Met Gly Ala Ser Pro Gly Glu Tyr Ala Ser Pro Gln Ala Thr Gly Ser
                   885                 890                 895
   Gly Pro Glu Asn Thr Gly Glu Arg Glu Arg Glu Leu Ala Ser Gln Glu
               900                 905                 910
   Val Gly Glu Glu Lys Leu Lys Thr Pro Ser Pro Arg Lys Arg Lys His
           915                 920                 925
   Leu Ser Glu Pro Gln Ala Ser Glu Ala Pro Gln Ile Ile Val Leu Gly
       930                 935                 940
   Leu Ala Ala Ser Pro Gly Ser Pro Arg Ala His Ala Gly Asn Lys Lys
   945                 950                 955                 960
   Lys Thr Val Lys Arg Lys
   965
<210> 201
   <211> 2379
   <212> DNA
   <213> Oryza sativa
<400> 201
   atggataaga caactacttc tgatcttgtt ttggacaacg acaacattgg gagtaatgct      60
   ggttctgcac aggagcctct tactaccaat ggtaagacta gtggggtcag aaatagatac     120
   aaacaaactg ttaaaagggg aaggaaaggc tcccaaatat caccaagtaa aacatatccc     180
   ctgagatctt cacatagtaa tgtcagggtg cttcgctctg cctcaaaaaa gaagaatgag     240
   acacctattg tgcccacaaa tgataatact gctgttcaac gagttgcgaa gaaaaggaaa     300
   agaagcaaac ccttgagacc tgcacctagc agggtgcttc gctctacctc ggaaaagaag     360
   aataaggcac ataatgagct tctaaatgat ggtgctggtg ttcaaccagc cgaaaagaaa     420
   agaaaggtag gcagaccccc aaaaggagga actcccaagg acgattacct catgatccga     480
   aagcgagtta gatatgtttt gaatcgaatg aactatgaac aaagtttaat tcaagcctat     540
   gccagtgaag gttggaaagg tcaaagcttg gaaaaaataa gacctgagaa ggagcttgaa     600
   cgagccaagg tggaaattct gcgatgcaag tcaagaatac gggaagcttt tcggaatttg     660
   gattccctcc tatctgaggg aaagctggat gaatctatgt ttgattctgc tggagaaata     720
   tctagtgaag atattttctg tgctgcatgt ggttcaaagg atgttacatt aaaaaatgac     780
   ataattcttt gtgatggaat ctgcgacaga gggttccacc agtattgttt aaaccctcct     840
   ttgcttgctg aagatattcc acagggggat gaaggatggc tttgccctgc atgtgattgc     900
   aaaattgact gcatagatgt attaaatgaa ctccaagggg tcaaactctc tatccatgac     960
   tcttgggaga aagtttttcc tgaggcagca tcctttttga atggttctaa gcaaattgat    1020
   gcgtccgatc ttccatcaga tgattcagca gacaatgatt atgaccctac tttggctcaa    1080
   gggcacaagg tggatgaaga aaaatcttct ggagaagatg gtggtgaagg attagattct    1140
   gatgactcat cttctgagga ttctgaatct tctgagaagg aaaagtctaa aacttcacag    1200
   aatggaagga cagttgatga tcttggtttg ccttctgagg attcagagga tggtgacttt    1260
   gatccagcag gtccagattc cgacaaagaa caaaatgatg aatcaaactc agatcagtca    1320
   gatgaatcag attttacatc tgactctgat gatttttgtg ctgagattgc taaatcctgt    1380
   ggtcaggatg aaatctcagg cccttcatca tcacaaatca gaacagtcga tcgcaccgat    1440
   ggaagtggtt ttgatggtga gcctaatgca gaaaattcga atcttgcatt tatggagaca    1500
   gagctagagc aagacatggt gctaccaatt tctagtaaac gacaagttga acgtttggat    1560
   tacaaaaaac tgtataatga ggcttatggt aaagcatcat ctgattcaag tgacgatgaa    1620
   gagtggtatg gaaatagtac acccgaaaaa ggtaatttag aagacagtga aacagattcg    1680
   cttgctgaat cacctcaggg tggaaaagga ttctctagaa gagcaccagt taggtaccat    1740
   aataatgaac atactccaca gaatgtaagg cccggtggtt cagtaagcga ccagcaaact    1800
   gaagtacttt gctccaatag caatggtagc acagccaaaa acagacattt tggtcctgca 1860
   attaatcaga aactgaaggc acatttcaag gaagatccat accctagtcg tgcaacaaaa 1920
   gaaaatctgg cacaagagtt aggcctcaca tttaatcagg tcactaaatg gttttccagt 1980
   actcgtcatt acgcaagagt ggcagccaca aaaaaggaga acaacattga aaatcatacg 2040
   gctgagaata ataacaatac caacactgtt gatagcatac aactgagagg atccaatgat 2100
   attgttagtg tagatagaaa tgatatggtc tccgaggaaa ggacaggaca aagtaatctc 2160
   aatgaaggta ctccattaag atcagatacc agctgtgggc agagtgtggc tgtgactcct 2220
   atggtacacc cagaaaacca gggcaatgat tcctcaagta atgttagaac accaaatgct 2280
   aagtctgcgg agaaattgat ccctggactg gaaaattcag atgaggctag gcgaaaggcg 2340
   gtacaacggg aactgagaaa gatgaagact ggcaggtga 2379
<210> 202
   <211> 792
   <212> PRT
   <213> Oryza sativa
<400> 202
   Met Asp Lys Thr Thr Thr Ser Asp Leu Val Leu Asp Asn Asp Asn Ile
   1               5                   10                  15
   Gly Ser Asn Ala Gly Ser Ala Gln Glu Pro Leu Thr Thr Asn Gly Lys
               20                  25                  30
   Thr Ser Gly Val Arg Asn Arg Tyr Lys Gln Thr Val Lys Arg Gly Arg
           35                  40                  45
   Lys Gly Ser Gln Ile Ser Pro Ser Lys Thr Tyr Pro Leu Arg Ser Ser
       50                  55                  60
   His Ser Asn Val Arg Val Leu Arg Ser Ala Ser Lys Lys Lys Asn Glu
   65                  70                  75                  80
   Thr Pro Ile Val Pro Thr Asn Asp Asn Thr Ala Val Gln Arg Val Ala
                   85                  90                  95
   Lys Lys Arg Lys Arg Ser Lys Pro Leu Arg Pro Ala Pro Ser Arg Val
               100                 105                 110
   Leu Arg Ser Thr Ser Glu Lys Lys Asn Lys Ala His Asn Glu Leu Leu
           115                 120                 125
   Asn Asp Gly Ala Gly Val Gln Pro Ala Glu Lys Lys Arg Lys Val Gly
       130                 135                 140
   Arg Pro Pro Lys Gly Gly Thr Pro Lys Asp Asp Tyr Leu Met Ile Arg
   145                 150                 155                 160
   Lys Arg Val Arg Tyr Val Leu Asn Arg Met Asn Tyr Glu Gln Ser Leu
   165 170 175
   Ile Gln Ala Tyr Ala Ser Glu Gly Trp Lys Gly Gln Ser Leu Glu Lys
   180 185 190
   Ile Arg Pro Glu Lys Glu Leu Glu Arg Ala Lys Val Glu Ile Leu Arg
   195 200 205
   Cys Lys Ser Arg Ile Arg Glu Ala Phe Arg Asn Leu Asp Ser Leu Leu
   210 215 220
   Ser Glu Gly Lys Leu Asp Glu Ser Met Phe Asp Ser Ala Gly Glu Ile
   225 230 235 240
   Ser Ser Glu Asp Ile Phe Cys Ala Ala Cys Gly Ser Lys Asp Val Thr
   245 250 255
   Leu Lys Asn Asp Ile Ile Leu Cys Asp Gly Ile Cys Asp Arg Gly Phe
   260 265 270
   His Gln Tyr Cys Leu Asn Pro Pro Leu Leu Ala Glu Asp Ile Pro Gln
   275 280 285
   Gly Asp Glu Gly Trp Leu Cys Pro Ala Cys Asp Cys Lys Ile Asp Cys
   290 295 300
   Ile Asp Val Leu Asn Glu Leu Gln Gly Val Lys Leu Ser Ile His Asp
   305 310 315 320
   Ser Trp Glu Lys Val Phe Pro Glu Ala Ala Ser Phe Leu Asn Gly Ser
   325 330 335
   Lys Gln Ile Asp Ala Ser Asp Leu Pro Ser Asp Asp Ser Ala Asp Asn
   340 345 350
   Asp Tyr Asp Pro Thr Leu Ala Gln Gly His Lys Val Asp Glu Glu Lys
   355 360 365
   Ser Ser Gly Glu Asp Gly Gly Glu Gly Leu Asp Ser Asp Asp Ser Ser
   370 375 380
   Ser Glu Asp Ser Glu Ser Ser Glu Lys Glu Lys Ser Lys Thr Ser Gln
   385 390 395 400
   Asn Gly Arg Thr Val Asp Asp Leu Gly Leu Pro Ser Glu Asp Ser Glu
   405 410 415
   Asp Gly Asp Phe Asp Pro Ala Gly Pro Asp Ser Asp Lys Glu Gln Asn
   420 425 430
   Asp Glu Ser Asn Ser Asp Gln Ser Asp Glu Ser Asp Phe Thr Ser Asp
   435 440 445
   Ser Asp Asp Phe Cys Ala Glu Ile Ala Lys Ser Cys Gly Gln Asp Glu
       450                 455                 460
   Ile Ser Gly Pro Ser Ser Ser Gln Ile Arg Thr Val Asp Arg Thr Asp
   465                 470                 475                 480
   Gly Ser Gly Phe Asp Gly Glu Pro Asn Ala Glu Asn Ser Asn Leu Ala
                   485                 490                 495
   Phe Met Glu Thr Glu Leu Glu Gln Asp Met Val Leu Pro Ile Ser Ser
               500                 505                 510
   Lys Arg Gln Val Glu Arg Leu Asp Tyr Lys Lys Leu Tyr Asn Glu Ala
           515                 520                 525
   Tyr Gly Lys Ala Ser Ser Asp Ser Ser Asp Asp Glu Glu Trp Tyr Gly
       530                 535                 540
   Asn Ser Thr Pro Glu Lys Gly Asn Leu Glu Asp Ser Glu Thr Asp Ser
   545                 550                 555                 560
   Leu Ala Glu Ser Pro Gln Gly Gly Lys Gly Phe Ser Arg Arg Ala Pro
                   565                 570                 575
   Val Arg Tyr His Asn Asn Glu His Thr Pro Gln Asn Val Arg Pro Gly
               580                 585                 590
   Gly Ser Val Ser Asp Gln Gln Thr Glu Val Leu Cys Ser Asn Ser Asn
           595                 600                 605
   Gly Ser Thr Ala Lys Asn Arg His Phe Gly Pro Ala Ile Asn Gln Lys
       610                 615                 620
   Leu Lys Ala His Phe Lys Glu Asp Pro Tyr Pro Ser Arg Ala Thr Lys
   625                 630                 635                 640
   Glu Asn Leu Ala Gln Glu Leu Gly Leu Thr Phe Asn Gln Val Thr Lys
                   645                 650                 655
   Trp Phe Ser Ser Thr Arg His Tyr Ala Arg Val Ala Ala Thr Lys Lys
               660                 665                 670
   Glu Asn Asn Ile Glu Asn His Thr Ala Glu Asn Asn Asn Asn Thr Asn
           675                 680                 685
   Thr Val Asp Ser Ile Gln Leu Arg Gly Ser Asn Asp Ile Val Ser Val
       690                 695                 700
   Asp Arg Asn Asp Met Val Ser Glu Glu Arg Thr Gly Gln Ser Asn Leu
   705                 710                 715                 720
   Asn Glu Gly Thr Pro Leu Arg Ser Asp Thr Ser Cys Gly Gln Ser Val
                   725                 730                 735
   Ala Val Thr Pro Met Val His Pro Glu Asn Gln Gly Asn Asp Ser Ser
               740                 745                 750
   Ser Asn Val Arg Thr Pro Asn Ala Lys Ser Ala Glu Lys Leu Ile Pro
           755                 760                 765
   Gly Leu Glu Asn Ser Asp Glu Ala Arg Arg Lys Ala Val Gln Arg Glu
       770                 775                 780
   Leu Arg Lys Met Lys Thr Gly Arg
   785                 790
<210> 203
   <211> 3267
   <212> DNA
   <213> Petroselinum crispum
<400> 203
   atggaagaga tctcagaccc taaaccaaat gcattagagc aagtacttcc tactgtccca     60
   aatggtaaat gtactgcacc ggtgcaaatg gaaagtttgg ctgttgatgt tcaaaaagta    120
   tcaggtgaag caaaggtgag gatatgttcc tgttggtgcg agatagtacg ttctccagaa    180
   gatttaacta aacttgtgcc ttgtaatgat tttgcagaag atataaaatt gtttgattct    240
   gatcccatgc aacaagaagc tgaaagctcc attggaatac cattgatccc taaacaagtt    300
   acaatgtccc ataaccatga ccatgaatct ggttctgaaa tggtcagtaa cgaagtaatg    360
   caagaaaacc atgtaattgc cacagaaaat acttaccaga aatctgattt tgaccgtata    420
   aatatgggac aaaaagaaac catgccagaa gaagtgatcc ataagtcttt cttggaatcc    480
   tcaacttcct cgatagatat actattaaat aatcataaca gttatcaatc aggactgcca    540
   ccagagaatg cagtaacaga ttgcaagcaa gtccaattgg gacaccgaag tgatgatgct    600
   ataaagaatt ctggtcttgt ggaattggta ataggtcaga aaaatgttgc caagagtcct    660
   agccagttgg tagaaacggg gaaaagaggt cgtggtcggc ccaggaaagt acaaactggc    720
   cttgagcaat tggtaatagg tcagaaaact gctgccaaga gctctagcca gttaggtgac    780
   acagggaaaa gatctcgtgg tcggcccaga aaagtacaaa attctccaac ttctttcttg    840
   gagaatataa atatggaaca aaaagaaaca ataccagaac aagtgaccca aaattctatc    900
   ttggagtcct tgactatccc gacagacaat cagtcaagaa cttataacag tgatcaatca    960
   gaactgccac cagagaatgc agcaaaaaat tgcaatcacg cccaatttgg acatcaaagt   1020
   gatgatacta caaagatttc tggattcaag gaattggtaa taggtcagga aacagttgca   1080
   aagagtccta gccagttggt agacgcagga aaaagaggtc gtggtcggcc cagaaaagta   1140
   caaactggtc ttgagcaatt ggtaccagtt caggaaactg cggccaagag ctctagccag   1200
   ttgggagaca cagggaaaag atctcgcggc cggcccagga aagtacaaga ttctccaact   1260
   tctttagggg gtaatgttaa agtggtacca gagaaaggaa aagattctca ggaattgtcg 1320
   gtaaacagca gcagatcatt gcgctcaagg tcacaagaga aatctatcga acccgatgtc 1380
   aataatattg tggcagatga aggtgctgac agagaaaaac caaggaagaa gaggaaaaag 1440
   cgaatggagg aaaatagggt tgatgaattc tgtagaataa ggacacacct aaggtactta 1500
   ctacacagaa taaaatacga gaagaatttc cttgatgctt actctgggga gggatggaag 1560
   ggacaaagcc tagacaaaat aaaacctgag aaggagctta aacgagcaaa agctgaaatt 1620
   tttggacgca aattgaaaat tagagatcta tttcagcgct tagatttagc acgttccgag 1680
   ggaaggcttc cagaaattct atttgattct cgaggagaaa ttgacagcga ggatatattc 1740
   tgcgcgaaat gtggatccaa ggatgtgaca ctcagtaatg atatcatcct ttgcgatggt 1800
   gcatgtgatc gtggatttca ccaattttgt ttggacccac cattgctgaa agaatatatt 1860
   cctcctgacg acgagggttg gctttgcccg ggatgcgagt gcaaaattga ctgcatcaag 1920
   ttacttaatg attcccaaga gacaaatatt ttactcggtg acagctggga gaaagttttt 1980
   gctgaggagg cagcagcagc agcttccgga aaaaatctag atgataattc tggactgcct 2040
   tcagatgatt ccgaggatga tgattatgat cctgggggcc ctgatttgga tgagaaggtg 2100
   cagggagatg attcgagtac ggatgaatct gattatcagt cggaatcaga tgatatgcaa 2160
   gttatacgtc aaaaaaattc gcgcgggctt ccttcagatg attccgaaga tgatgaatac 2220
   gatcctagtg gcttagttac tgatcagatg tataaggaca gctcatgttc tgattttaca 2280
   tctgattctg aggattttac aggtgtgttt gacgattata aggatactgg taaagctcaa 2340
   gggccactgg catcgacccc agatcacgtc aggaacaatg aggaagggtg tgggcatcct 2400
   gagcaaggtg atactgcccc cctgtatccg agaagacaag tagagagttt ggactacaaa 2460
   aagctaaatg atattgagtt ctctaaaatg tgtgacatcc ttgatattct ttcaagtcaa 2520
   cttgatgtaa ttatttgtac tggtaaccag gaagagtatg ggaacacatc ttctgattca 2580
   agtgatgaag attatatggt tacttcttca ccagataaaa ataattctga taaagaagct 2640
   acagcaatgg agcgtggaag agagagcggt gatttggagc tagaccaaaa agcgagagaa 2700
   tccactcata ataggagata tataaagaag tttgctgttg aaggcacaga ttcttttctt 2760
   tctagatcgt gtgaagactc tgcagcacct gttgctggta gtaaaagtac ttcaaaaaca 2820
   ttgcacgggg aacatgcaac tcagagactc ctccaatcct tcaaggaaaa tcaataccct 2880
   caacgagctg tgaaagagag tttggctgca gaattagcac tttcagtccg acaggttagc 2940
   aactggttta ataatagacg ttggagcttt cgccactcat cacgtattgg atcggatgta 3000
   gctaaatttg attcaaacga tactccccgt cagaaaagca ttgacatgtc tggacccagc 3060
   ttgaaatcgg tcttggacag tgctacttac agtgaaattg aaaagaagga acaagatacg 3120
   gcaagccttg gtttgacaga aggttgtgac agatacatga cgctgaatat ggttgcagat 3180
   gaaggcaatg tgcacactcc ttgtattgca gaaactaggg aagaaaaaac cgaagtcggt 3240
   ataaagccac aacagaaccc actataa 3267
<210> 204
   <211> 1088
   <212> PRT
   <213> Petroselinum crispum
<400> 204
   Met Glu Glu Ile Ser Asp Pro Lys Pro Asn Ala Leu Glu Gln Val Leu
   1 5 10 15
   Pro Thr Val Pro Asn Gly Lys Cys Thr Ala Pro Val Gln Met Glu Ser
   20 25 30
   Leu Ala Val Asp Val Gln Lys Val Ser Gly Glu Ala Lys Val Arg Ile
   35 40 45
   Cys Ser Cys Trp Cys Glu Ile Val Arg Ser Pro Glu Asp Leu Thr Lys
   50 55 60
   Leu Val Pro Cys Asn Asp Phe Ala Glu Asp Ile Lys Leu Phe Asp Ser
   65 70 75 80
   Asp Pro Met Gln Gln Glu Ala Glu Ser Ser Ile Gly Ile Pro Leu Ile
   85 90 95
   Pro Lys Gln Val Thr Met Ser His Asn His Asp His Glu Ser Gly Ser
   100 105 110
   Glu Met Val Ser Asn Glu Val Met Gln Glu Asn His Val Ile Ala Thr
   115 120 125
   Glu Asn Thr Tyr Gln Lys Ser Asp Phe Asp Arg Ile Asn Met Gly Gln
   130 135 140
   Lys Glu Thr Met Pro Glu Glu Val Ile His Lys Ser Phe Leu Glu Ser
   145 150 155 160
   Ser Thr Ser Ser Ile Asp Ile Leu Leu Asn Asn His Asn Ser Tyr Gln
   165 170 175
   Ser Gly Leu Pro Pro Glu Asn Ala Val Thr Asp Cys Lys Gln Val Gln
   180 185 190
   Leu Gly His Arg Ser Asp Asp Ala Ile Lys Asn Ser Gly Leu Val Glu
   195 200 205
   Leu Val Ile Gly Gln Lys Asn Val Ala Lys Ser Pro Ser Gln Leu Val
   210 215 220
   Glu Thr Gly Lys Arg Gly Arg Gly Arg Pro Arg Lys Val Gln Thr Gly
   225 230 235 240
   Leu Glu Gln Leu Val Ile Gly Gln Lys Thr Ala Ala Lys Ser Ser Ser
   245 250 255
   Gln Leu Gly Asp Thr Gly Lys Arg Ser Arg Gly Arg Pro Arg Lys Val
   260 265 270
   Gln Asn Ser Pro Thr Ser Phe Leu Glu Asn Ile Asn Met Glu Gln Lys
   275 280 285
   Glu Thr Ile Pro Glu Gln Val Thr Gln Asn Ser Ile Leu Glu Ser Leu
   290 295 300
   Thr Ile Pro Thr Asp Asn Gln Ser Arg Thr Tyr Asn Ser Asp Gln Ser
   305 310 315 320
   Glu Leu Pro Pro Glu Asn Ala Ala Lys Asn Cys Asn His Ala Gln Phe
   325 330 335
   Gly His Gln Ser Asp Asp Thr Thr Lys Ile Ser Gly Phe Lys Glu Leu
   340 345 350
   Val Ile Gly Gln Glu Thr Val Ala Lys Ser Pro Ser Gln Leu Val Asp
   355 360 365
   Ala Gly Lys Arg Gly Arg Gly Arg Pro Arg Lys Val Gln Thr Gly Leu
   370 375 380
   Glu Gln Leu Val Pro Val Gln Glu Thr Ala Ala Lys Ser Ser Ser Gln
   385 390 395 400
   Leu Gly Asp Thr Gly Lys Arg Ser Arg Gly Arg Pro Arg Lys Val Gln
   405 410 415
   Asp Ser Pro Thr Ser Leu Gly Gly Asn Val Lys Val Val Pro Glu Lys
   420 425 430
   Gly Lys Asp Ser Gln Glu Leu Ser Val Asn Ser Ser Arg Ser Leu Arg
   435 440 445
   Ser Arg Ser Gln Glu Lys Ser Ile Glu Pro Asp Val Asn Asn Ile Val
   450 455 460
   Ala Asp Glu Gly Ala Asp Arg Glu Lys Pro Arg Lys Lys Arg Lys Lys
   465 470 475 480
   Arg Met Glu Glu Asn Arg Val Asp Glu Phe Cys Arg Ile Arg Thr His
   485 490 495
   Leu Arg Tyr Leu Leu His Arg Ile Lys Tyr Glu Lys Asn Phe Leu Asp
   500 505 510
   Ala Tyr Ser Gly Glu Gly Trp Lys Gly Gln Ser Leu Asp Lys Ile Lys
   515 520 525
   Pro Glu Lys Glu Leu Lys Arg Ala Lys Ala Glu Ile Phe Gly Arg Lys
   530 535 540
   Leu Lys Ile Arg Asp Leu Phe Gln Arg Leu Asp Leu Ala Arg Ser Glu
   545 550 555 560
   Gly Arg Leu Pro Glu Ile Leu Phe Asp Ser Arg Gly Glu Ile Asp Ser
   565 570 575
   Glu Asp Ile Phe Cys Ala Lys Cys Gly Ser Lys Asp Val Thr Leu Ser
   580 585 590
   Asn Asp Ile Ile Leu Cys Asp Gly Ala Cys Asp Arg Gly Phe His Gln
   595 600 605
   Phe Cys Leu Asp Pro Pro Leu Leu Lys Glu Tyr Ile Pro Pro Asp Asp
   610 615 620
   Glu Gly Trp Leu Cys Pro Gly Cys Glu Cys Lys Ile Asp Cys Ile Lys
   625 630 635 640
   Leu Leu Asn Asp Ser Gln Glu Thr Asn Ile Leu Leu Gly Asp Ser Trp
   645 650 655
   Glu Lys Val Phe Ala Glu Glu Ala Ala Ala Ala Ala Ser Gly Lys Asn
   660 665 670
   Leu Asp Asp Asn Ser Gly Leu Pro Ser Asp Asp Ser Glu Asp Asp Asp
   675 680 685
   Tyr Asp Pro Gly Gly Pro Asp Leu Asp Glu Lys Val Gln Gly Asp Asp
   690 695 700
   Ser Ser Thr Asp Glu Ser Asp Tyr Gln Ser Glu Ser Asp Asp Met Gln
   705 710 715 720
   Val Ile Arg Gln Lys Asn Ser Arg Gly Leu Pro Ser Asp Asp Ser Glu
   725 730 735
   Asp Asp Glu Tyr Asp Pro Ser Gly Leu Val Thr Asp Gln Met Tyr Lys
   740 745 750
   Asp Ser Ser Cys Ser Asp Phe Thr Ser Asp Ser Glu Asp Phe Thr Gly
   755 760 765
   Val Phe Asp Asp Tyr Lys Asp Thr Gly Lys Ala Gln Gly Pro Leu Ala
   770 775 780
   Ser Thr Pro Asp His Val Arg Asn Asn Glu Glu Gly Cys Gly His Pro
   785 790 795 800
   Glu Gln Gly Asp Thr Ala Pro Leu Tyr Pro Arg Arg Gln Val Glu Ser
                   805                 810                 815
   Leu Asp Tyr Lys Lys Leu Asn Asp Ile Glu Phe Ser Lys Met Cys Asp
               820                 825                 830
   Ile Leu Asp Ile Leu Ser Ser Gln Leu Asp Val Ile Ile Cys Thr Gly
           835                 840                 845
   Asn Gln Glu Glu Tyr Gly Asn Thr Ser Ser Asp Ser Ser Asp Glu Asp
       850                 855                 860
   Tyr Met Val Thr Ser Ser Pro Asp Lys Asn Asn Ser Asp Lys Glu Ala
   865                 870                 875                 880
   Thr Ala Met Glu Arg Gly Arg Glu Ser Gly Asp Leu Glu Leu Asp Gln
                   885                 890                 895
   Lys Ala Arg Glu Ser Thr His Asn Arg Arg Tyr Ile Lys Lys Phe Ala
               900                 905                 910
   Val Glu Gly Thr Asp Ser Phe Leu Ser Arg Ser Cys Glu Asp Ser Ala
           915                 920                 925
   Ala Pro Val Ala Gly Ser Lys Ser Thr Ser Lys Thr Leu His Gly Glu
       930                 935                 940
   His Ala Thr Gln Arg Leu Leu Gln Ser Phe Lys Glu Asn Gln Tyr Pro
   945                 950                 955                 960
   Gln Arg Ala Val Lys Glu Ser Leu Ala Ala Glu Leu Ala Leu Ser Val
                   965                 970                 975
   Arg Gln Val Ser Asn Trp Phe Asn Asn Arg Arg Trp Ser Phe Arg His
               980                 985                 990
   Ser Ser Arg Ile Gly Ser Asp Val  Ala Lys Phe Asp Ser  Asn Asp Thr
           995                 1000                 1005
   Pro Arg  Gln Lys Ser Ile Asp  Met Ser Gly Pro Ser  Leu Lys Ser
       1010                 1015                 1020
   Val Leu  Asp Ser Ala Thr Tyr  Ser Glu Ile Glu Lys  Lys Glu Gln
       1025                 1030                 1035
   Asp Thr  Ala Ser Leu Gly Leu  Thr Glu Gly Cys Asp  Arg Tyr Met
       1040                 1045                 1050
   Thr Leu  Asn Met Val Ala Asp  Glu Gly Asn Val His  Thr Pro Cys
       1055                 1060                 1065
   Ile Ala  Glu Thr Arg Glu Glu  Lys Thr Glu Val Gly  Ile Lys Pro
   1070 1075 1080
   Gln Gln Asn Pro Leu
   1085
<210> 205
   <211> 2160
   <212> DNA
   <213> Zea mays
<400> 205
   atggaaaaaa atatagctca ctgtcctgtt gagggcaatg gtgagattga aaatggtgcg     60
   agctctagtc agaatcctga gtctcttgag cattcagttt tgctgtctac atctcaaacg    120
   atgccaaata acttggggat aaggaaaaac tataaaaggg cagcaaacag aggtaaaaaa    180
   ggttcccaag gactaacagg ccaagcatat actttgatgt catctaatag tgatgtcagg    240
   gtgcttcgct ccacatcaag ttcaaagaca acatctactg agcatgttca ggctccagta    300
   caaccggctg ccaagagaag aaaaatgagc agagcctcaa acaaaagttc gactgatgag    360
   ttttcacaaa ttcgtaaacg agttagatat attttgaacc gaatgaatta tgagcaaagc    420
   ctaattgaag cttatgctag cgaaggctgg aaaaatcaaa gtttggataa gataagacct    480
   gagaaggagc ttgaacgagc caaatcagaa atcttacgat gcaaattgag aatacgagaa    540
   gtcttccgga atattgattc tcttctctcc aaggggaaaa ttgacgaaac tttatttgat    600
   tctgaaggag aaatatcttg cgaagatatc ttttgttcca cttgtggttc gaatgacgct    660
   acattgggta atgatatcat tctctgcgat ggagcttgtg acagagggtt ccaccagaac    720
   tgtttaaatc ctcccctgcg tactgaagat atccccatgg gagatgaagg atggctctgc    780
   ccagcatgtg attgcaagat agattgtata gatctaataa atgaactcca tgggagtaac    840
   atctccattg aggactcttg ggagaaagtt tttccagatg cagctgctat ggcaaatgac    900
   tctaaacaag atgacgcatt tgatcttcca tcagatgact cagacgacaa tgactttgac    960
   cccaatatgc ctgaagagca tgtggttggt aaggatgaag aatcgtctga agaggatgag   1020
   gatggaggat cagactctga tgactcagac tttttgacat gttctgatga ttcggaacct   1080
   ttgatagaca agaaggttga tgacctcaga ttaccttctg aagattcaga ggatgatgac   1140
   tatgatccag caggtcctga ttcagataaa gatgtcgaaa agaagtcaag ttctgatgaa   1200
   tctgacttca catctgactc agatgatttt tgtaaggaga tttcaaaatc tggacatgat   1260
   gaagtttcat cacctctatt acccgatgcc aaggtgggtg atatggaaaa gattactgct   1320
   caagctaaaa caacaagttc tgctgacgac cctatggaga ctgaaataga ccagggtgtg   1380
   gttttaccag attcaaggag acggcaagct gaacggttgg actacaaaaa gttgtatgat   1440
   gaggcatatg gtgaagcatc atctgattct agtgatgatg aagaatggtc tgggaagaac   1500
   acgccaataa taaaaagcaa tgaagagggt gaagccaatt ctcctgcggg aaaaggctcc   1560
   agagttgtgc accataatga tgaactgact acacaaagca ctaaaaaaag cttgcattct   1620
   attcatggtt cagtagatga gaaacctgga gaccttactt ctaatggcag caacagcaca   1680
   gccaggaaag gacattttgg cccagtaatt aatcagaagc tacatgaaca ttttaagaca   1740
   caaccatacc ctagtcgttc tgttaaagaa agtctggcag aagaactagg gttaacattt   1800
   cgtcaggtta acaaatggtt tgaaactagg cgtcattccg caagggtagc ttcttccagg   1860
   aaaggcatca gtctagataa gcatagcccc caaaatacta atagtcaagt gacagctagt   1920
   atggaaccta aagaacctga ggggactgtg gtggaagaat ctaatgtatg cctaaatggg   1980
   ggtactacca tctctaaaga agcagtgtct tcaaaagttg gatcaagaac ccctggaagt   2040
   gatgtgggag ggtcaaaagt tgattctgct gaggatcaga atccggggcc tgatcttgca   2100
   gagaaggcaa ggcaaaaggc aatacagcag gagttgagga agaaaaaaat gggacgatga   2160
<210> 206
   <211> 719
   <212> PRT
   <213> Zea mays
<400> 206
   Met Glu Lys Asn Ile Ala His Cys Pro Val Glu Gly Asn Gly Glu Ile
   1               5                   10                  15
   Glu Asn Gly Ala Ser Ser Ser Gln Asn Pro Glu Ser Leu Glu His Ser
               20                  25                  30
   Val Leu Leu Ser Thr Ser Gln Thr Met Pro Asn Asn Leu Gly Ile Arg
           35                  40                  45
   Lys Asn Tyr Lys Arg Ala Ala Asn Arg Gly Lys Lys Gly Ser Gln Gly
       50                  55                  60
   Leu Thr Gly Gln Ala Tyr Thr Leu Met Ser Ser Asn Ser Asp Val Arg
   65                  70                  75                  80
   Val Leu Arg Ser Thr Ser Ser Ser Lys Thr Thr Ser Thr Glu His Val
                   85                  90                  95
   Gln Ala Pro Val Gln Pro Ala Ala Lys Arg Arg Lys Met Ser Arg Ala
               100                 105                 110
   Ser Asn Lys Ser Ser Thr Asp Glu Phe Ser Gln Ile Arg Lys Arg Val
           115                 120                 125
   Arg Tyr Ile Leu Asn Arg Met Asn Tyr Glu Gln Ser Leu Ile Glu Ala
       130                 135                 140
   Tyr Ala Ser Glu Gly Trp Lys Asn Gln Ser Leu Asp Lys Ile Arg Pro
   145                 150                 155                 160
   Glu Lys Glu Leu Glu Arg Ala Lys Ser Glu Ile Leu Arg Cys Lys Leu
                   165                 170                 175
   Arg Ile Arg Glu Val Phe Arg Asn Ile Asp Ser Leu Leu Ser Lys Gly
   180 185 190
   Lys Ile Asp Glu Thr Leu Phe Asp Ser Glu Gly Glu Ile Ser Cys Glu
   195 200 205
   Asp Ile Phe Cys Ser Thr Cys Gly Ser Asn Asp Ala Thr Leu Gly Asn
   210 215 220
   Asp Ile Ile Leu Cys Asp Gly Ala Cys Asp Arg Gly Phe His Gln Asn
   225 230 235 240
   Cys Leu Asn Pro Pro Leu Arg Thr Glu Asp Ile Pro Met Gly Asp Glu
   245 250 255
   Gly Trp Leu Cys Pro Ala Cys Asp Cys Lys Ile Asp Cys Ile Asp Leu
   260 265 270
   Ile Asn Glu Leu His Gly Ser Asn Ile Ser Ile Glu Asp Ser Trp Glu
   275 280 285
   Lys Val Phe Pro Asp Ala Ala Ala Met Ala Asn Asp Ser Lys Gln Asp
   290 295 300
   Asp Ala Phe Asp Leu Pro Ser Asp Asp Ser Asp Asp Asn Asp Phe Asp
   305 310 315 320
   Pro Asn Met Pro Glu Glu His Val Val Gly Lys Asp Glu Glu Ser Ser
   325 330 335
   Glu Glu Asp Glu Asp Gly Gly Ser Asp Ser Asp Asp Ser Asp Phe Leu
   340 345 350
   Thr Cys Ser Asp Asp Ser Glu Pro Leu Ile Asp Lys Lys Val Asp Asp
   355 360 365
   Leu Arg Leu Pro Ser Glu Asp Ser Glu Asp Asp Asp Tyr Asp Pro Ala
   370 375 380
   Gly Pro Asp Ser Asp Lys Asp Val Glu Lys Lys Ser Ser Ser Asp Glu
   385 390 395 400
   Ser Asp Phe Thr Ser Asp Ser Asp Asp Phe Cys Lys Glu Ile Ser Lys
   405 410 415
   Ser Gly His Asp Glu Val Ser Ser Pro Leu Leu Pro Asp Ala Lys Val
   420 425 430
   Gly Asp Met Glu Lys Ile Thr Ala Gln Ala Lys Thr Thr Ser Ser Ala
   435 440 445
   Asp Asp Pro Met Glu Thr Glu Ile Asp Gln Gly Val Val Leu Pro Asp
   450 455 460
   Ser Arg Arg Arg Gln Ala Glu Arg Leu Asp Tyr Lys Lys Leu Tyr Asp
   465                 470                 475                 480
   Glu Ala Tyr Gly Glu Ala Ser Ser Asp Ser Ser Asp Asp Glu Glu Trp
                   485                 490                 495
   Ser Gly Lys Asn Thr Pro Ile Ile Lys Ser Asn Glu Glu Gly Glu Ala
               500                 505                 510
   Asn Ser Pro Ala Gly Lys Gly Ser Arg Val Val His His Asn Asp Glu
           515                 520                 525
   Leu Thr Thr Gln Ser Thr Lys Lys Ser Leu His Ser Ile His Gly Ser
       530                 535                 540
   Val Asp Glu Lys Pro Gly Asp Leu Thr Ser Asn Gly Ser Asn Ser Thr
   545                 550                 555                 560
   Ala Arg Lys Gly His Phe Gly Pro Val Ile Asn Gln Lys Leu His Glu
                   565                 570                 575
   His Phe Lys Thr Gln Pro Tyr Pro Ser Arg Ser Val Lys Glu Ser Leu
               580                 585                 590
   Ala Glu Glu Leu Gly Leu Thr Phe Arg Gln Val Asn Lys Trp Phe Glu
           595                 600                 605
   Thr Arg Arg His Ser Ala Arg Val Ala Ser Ser Arg Lys Gly Ile Ser
       610                 615                 620
   Leu Asp Lys His Ser Pro Gln Asn Thr Asn Ser Gln Val Thr Ala Ser
   625                 630                 635                 640
   Met Glu Pro Lys Glu Pro Glu Gly Thr Val Val Glu Glu Ser Asn Val
                   645                 650                 655
   Cys Leu Asn Gly Gly Thr Thr Ile Ser Lys Glu Ala Val Ser Ser Lys
               660                 665                 670
   Val Gly Ser Arg Thr Pro Gly Ser Asp Val Gly Gly Ser Lys Val Asp
           675                 680                 685
   Ser Ala Glu Asp Gln Asn Pro Gly Pro Asp Leu Ala Glu Lys Ala Arg
       690                 695                 700
   Gln Lys Ala Ile Gln Gln Glu Leu Arg Lys Lys Lys Met Gly Arg
   705                 710                 715
<210> 207
   <211> 2079
   <212> DNA
   <213> Zea mays
<400> 207
   atggaaaaaa agacagttca cgttctggct gagggtaatg gtgagattga aaatggtacg      60
   agctctagtc agaatcctga gacccttgag catccagttt tgctgtctgc atctcaaact     120
   gtgccaaata acttggggat aaggaaaaac tataaaaggg cagcaaacag aggtaaaaag     180
   ggttcccaag gactaacagg tcaggcatat acattgaggt catctgatat tgatgtcagg     240
   gtgcttcgct ctacatcagg ttcaaagaca acatctactg agcatgtgca gcctccagga     300
   caaccagctg ccaagagaag aaaaaggagc agagcctcaa acaaaaattc gacagatgag     360
   ttctcacaaa ttcgtaaacg ggttagatac attttgaacc gaatgaatta tgagcaaagc     420
   ctaattgaag cttatgctag tgaaggctgg aaaaatcaaa gtttggataa gataagacct     480
   gagaaggagc ttgaacgagc caaatcagaa atcttacgat gcaaattgag aatacgggaa     540
   gtcttccaaa atattgattc tcttctgtcc aaagggaaaa ttgacgaatc tttatttgat     600
   tctgaaggag aaatatcttg tgaagatatc ttttgtgcta cttgtggttc gaaaaatgct     660
   acattgggta atgatatcat tctctgtgat ggagcttgtg acagagggtt ccaccagaac     720
   tgtctaaatc ctcctctgcg tactgaagat attcccatgg gagatgaagg atggctctgc     780
   ccagcatgtg attgcaagtt agactgtata gatctaataa atgaactcca gggcagtgac     840
   atctccattg aggacccttg ggagaaagtt tttccagagg cagctgctat gaccaatggc     900
   tctaagcaag atgaacgatt tgatcttcca tctgatgatt cggatgacaa tgattttgac     960
   cccaatatgc ctgaagagca tgtggctagt aaggaagaag gatcttctga agaggaagag    1020
   gatgatgacg gaggatcaga ctctgacgac tctgacttct taacctgttc tgatgatttg    1080
   gaacctttga tagataaaaa gaaggttgat gacctgggat tatcttctga agattcagag    1140
   gatgatgact atgatccagc aggtcctgat tcggataaag atgtcgaaaa gaagtcaaat    1200
   tctgatgagt ctgatttcac gtctgactcg gatgattttt gtaaggagat taaaaaatct    1260
   ggtggacatg atgaagtttc atcgcctcca ttacccgatg tcaaggtggg tgatatggaa    1320
   aagaacactg ctcagtctaa cacaacaagt tctgctgacg accctatgga gactgaaata    1380
   gaccagagtg tggttttacc agtatcaagg agacgtcaag ctgaacggtt ggactacaaa    1440
   aggctgtatg atgaggcata tggtgaagca tcatctgatt ctagtgatga agaagaatgg    1500
   tctgggaaga acacaccaat aaaaagcaat gaagagggtg aagttggttc tcctgcagga    1560
   aaaggctcta gagttgcgca ccataatgaa ctgactacac aaaacactaa agaaagcttg    1620
   cattctcttc atggttcagt agatgagaaa catggagatc ttacttctaa tggcagcaat    1680
   atcaaagata ggaaaggaca ttttggtcca gtgattagtc agaagctaca tgaacatttt   1740
   aagacacaac cataccctag tcgttctctt aaagaaagct tggcagaaga actagggcta   1800
   acatttcatc aggtcaacag atggtttgaa aataggcgtc attttgcaag gttagcttct    1860
   tccaggaaag gcatcagtcc agataagcat agcccccaaa atactaatag cccagtgaca    1920
   cctagtatgc aacctaaaga accagagggg actgtgatgg aagaatctaa tgtatgcata    1980
   aacagggatg ctaccatctc taaaaaagcg gtttcttcaa aagtcggatc aagaaagaac 2040
   cttagcaaga gttcccctaa aagccgggtc aaaagttga 2079
<210> 208
   <211> 692
   <212> PRT
   <213> Zea mays
<400> 208
   Met Glu Lys Lys Thr Val His Val Leu Ala Glu Gly Asn Gly Glu Ile
   1               5                   10                  15
   Glu Asn Gly Thr Ser Ser Ser Gln Asn Pro Glu Thr Leu Glu His Pro
               20                  25                  30
   Val Leu Leu Ser Ala Ser Gln Thr Val Pro Asn Asn Leu Gly Ile Arg
           35                  40                  45
   Lys Asn Tyr Lys Arg Ala Ala Asn Arg Gly Lys Lys Gly Ser Gln Gly
       50                  55                  60
   Leu Thr Gly Gln Ala Tyr Thr Leu Arg Ser Ser Asp Ile Asp Val Arg
   65                  70                  75                  80
   Val Leu Arg Ser Thr Ser Gly Ser Lys Thr Thr Ser Thr Glu His Val
                   85                  90                  95
   Gln Pro Pro Gly Gln Pro Ala Ala Lys Arg Arg Lys Arg Ser Arg Ala
               100                 105                 110
   Ser Asn Lys Asn Ser Thr Asp Glu Phe Ser Gln Ile Arg Lys Arg Val
           115                 120                 125
   Arg Tyr Ile Leu Asn Arg Met Asn Tyr Glu Gln Ser Leu Ile Glu Ala
       130                 135                 140
   Tyr Ala Ser Glu Gly Trp Lys Asn Gln Ser Leu Asp Lys Ile Arg Pro
   145                 150                 155                 160
   Glu Lys Glu Leu Glu Arg Ala Lys Ser Glu Ile Leu Arg Cys Lys Leu
                   165                 170                 175
   Arg Ile Arg Glu Val Phe Gln Asn Ile Asp Ser Leu Leu Ser Lys Gly
               180                 185                 190
   Lys Ile Asp Glu Ser Leu Phe Asp Ser Glu Gly Glu Ile Ser Cys Glu
           195                 200                 205
   Asp Ile Phe Cys Ala Thr Cys Gly Ser Lys Asn Ala Thr Leu Gly Asn
       210                 215                 220
   Asp Ile Ile Leu Cys Asp Gly Ala Cys Asp Arg Gly Phe His Gln Asn
   225 230 235 240
   Cys Leu Asn Pro Pro Leu Arg Thr Glu Asp Ile Pro Met Gly Asp Glu
   245 250 255
   Gly Trp Leu Cys Pro Ala Cys Asp Cys Lys Leu Asp Cys Ile Asp Leu
   260 265 270
   Ile Asn Glu Leu Gln Gly Ser Asp Ile Ser Ile Glu Asp Pro Trp Glu
   275 280 285
   Lys Val Phe Pro Glu Ala Ala Ala Met Thr Asn Gly Ser Lys Gln Asp
   290 295 300
   Glu Arg Phe Asp Leu Pro Ser Asp Asp Ser Asp Asp Asn Asp Phe Asp
   305 310 315 320
   Pro Asn Met Pro Glu Glu His Val Ala Ser Lys Glu Glu Gly Ser Ser
   325 330 335
   Glu Glu Glu Glu Asp Asp Asp Gly Gly Ser Asp Ser Asp Asp Ser Asp
   340 345 350
   Phe Leu Thr Cys Ser Asp Asp Leu Glu Pro Leu Ile Asp Lys Lys Lys
   355 360 365
   Val Asp Asp Leu Gly Leu Ser Ser Glu Asp Ser Glu Asp Asp Asp Tyr
   370 375 380
   Asp Pro Ala Gly Pro Asp Ser Asp Lys Asp Val Glu Lys Lys Ser Asn
   385 390 395 400
   Ser Asp Glu Ser Asp Phe Thr Ser Asp Ser Asp Asp Phe Cys Lys Glu
   405 410 415
   Ile Lys Lys Ser Gly Gly His Asp Glu Val Ser Ser Pro Pro Leu Pro
   420 425 430
   Asp Val Lys Val Gly Asp Met Glu Lys Asn Thr Ala Gln Ser Asn Thr
   435 440 445
   Thr Ser Ser Ala Asp Asp Pro Met Glu Thr Glu Ile Asp Gln Ser Val
   450 455 460
   Val Leu Pro Val Ser Arg Arg Arg Gln Ala Glu Arg Leu Asp Tyr Lys
   465 470 475 480
   Arg Leu Tyr Asp Glu Ala Tyr Gly Glu Ala Ser Ser Asp Ser Ser Asp
   485 490 495
   Glu Glu Glu Trp Ser Gly Lys Asn Thr Pro Ile Lys Ser Asn Glu Glu
   500 505 510
   Gly Glu Val Gly Ser Pro Ala Gly Lys Gly Ser Arg Val Ala His His
           515                 520                 525
   Asn Glu Leu Thr Thr Gln Asn Thr Lys Glu Ser Leu His Ser Leu His
       530                 535                 540
   Gly Ser Val Asp Glu Lys His Gly Asp Leu Thr Ser Asn Gly Ser Asn
   545                 550                 555                 560
   Ile Lys Asp Arg Lys Gly His Phe Gly Pro Val Ile Ser Gln Lys Leu
                   565                 570                 575
   His Glu His Phe Lys Thr Gln Pro Tyr Pro Ser Arg Ser Leu Lys Glu
               580                 585                 590
   Ser Leu Ala Glu Glu Leu Gly Leu Thr Phe His Gln Val Asn Arg Trp
           595                 600                 605
   Phe Glu Asn Arg Arg His Phe Ala Arg Leu Ala Ser Ser Arg Lys Gly
       610                 615                 620
   Ile Ser Pro Asp Lys His Ser Pro Gln Asn Thr Asn Ser Pro Val Thr
   625                 630                 635                 640
   Pro Ser Met Gln Pro Lys Glu Pro Glu Gly Thr Val Met Glu Glu Ser
                   645                 650                 655
   Asn Val Cys Ile Asn Arg Asp Ala Thr Ile Ser Lys Lys Ala Val Ser
               660                 665                 670
   Ser Lys Val Gly Ser Arg Lys Asn Leu Ser Lys Ser Ser Pro Lys Ser
           675                 680                 685
   Arg Val Lys Ser
       690
<210> 209
   <211> 4731
   <212> DNA
   <213> Zea mays
<400> 209
   atgggaagga aagggtcaga agtatcacca agcaaaaggt atcccttgag gtctgcacac     60
   agtagtggta gagttcttcg ctctgcctta gccaataata ataaggacag tgagcctctg    120
   aatgcggctg ctgctaccca agcagctgtg aaaaaaagaa gtggcaacca gctagatagt    180
   cctaatagca ctgtcaggct acttcgttct acgtcaaaaa ataaggacga ggcacatagc    240
   gggcctctta atgacaggat cattgataag ccagctgcaa ataaaagaaa acatgccaat    300
   ccctcacagg ttggaagtcc cagcagcagt gtcagggtgc ttcgctctgc aataaaaaat    360
   aaagatatgg cgcgcagtga gcctcaaagt gacaggaccg ctggtgaggc agctacaaat    420
   aaaagaaaaa acgccaattc ctcaaaggtg gggagtctga gctctggtct cagtgtgctt 480
   cggtctgctt caaaagctaa ggatgagaca tgtatcaaac ctttaaatga tagtcctgct 540
   gttgaaccag cttcaagtaa aagaaaatgc accagtccgt caaaggtggg aagtcccagc 600
   aacagtgtca gggtgcttcg atctacttca aaatataaga atgagacatg taccgagcct 660
   ttaaatgata gtggtgctgt tgaaccagct gcaaatagaa ggaaaggtgt cagtcgttca 720
   aaggagggaa gtcccaatag cagtgtcagg gtgcttcgct ctgcctcaaa taataagatt 780
   gatgtatcta ttgagccact gaataatagt actactggtc aaccggctgc gaaaagaaga 840
   aaaaatggca gttcctttaa gagcagtgcc agggtgcttt gctctacctc agaaaggaaa 900
   aatgaggcat ctagtgagcc tttaaatgat agtactgctg ctcagccagc tgcgaggaaa 960
   aagaaaggtg gtgcaatgcc aaagactgat aacccaaaga ttggtgtcag ggttcttcgc 1020
   tctgcctcag gaaagaagaa tgagacatgt actgagcctg taaatcggag tacttctcgt 1080
   gaaccaactg tgacaaaaaa aagaaattgc aagccttcaa aggatcgcgg tcccaagaag 1140
   gactacttaa aaatttgtca aagaattaaa tatattttga atcgaatgaa gtatgaacaa 1200
   gcctttattc aggcttatgc aagtgaaggt tggaaaggcc aaagtttgga aaaaataaga 1260
   cccgagaagg agcttgaacg agccaaggca gaaatccttc aatgcaagtt aagaatccgg 1320
   gaagcttttc gaaatatgga ctctctttta tcgaagggga agcttgaaga atctttgttt 1380
   gattcagcag gacaaatttc aagcgaagat atattttgtg ccatatgtgg ctcaaaggat 1440
   gttacttcac aaaatgacat cattctttgt gatggagcct gtgacagagg attccaccag 1500
   aattgtttga gccctccttt gctaactgaa gaaattcctc cgggggatga aggatggctt 1560
   tgccctgcat gtgtgtgcaa agcagactat atagatgcgt taaatgaact tcaaggaagc 1620
   aaactctcca ttcatgactc atgggtgaaa gttttccctg aggcagcttc aactgccaat 1680
   ggttctaaac aagttgatgc ttctgatctg ctgccagatc atataaagga cagtgctaac 1740
   cttgcattgg ttggaacaca catggtgaat gaaatcaggt tttctgaaga ggatgatagc 1800
   aaagcggatg acctcggctt gccttcggag gactcagggg atggtgactt cgatccagca 1860
   ggtccagatt ctagtgaaga ccaaaatgat ggattgaact cagaagagtc agatttcaca 1920
   tctgactctg attatttttg tgctgagatt gctaaatctt gcggccagga tgaagtctcg 1980
   gcatctccat tatcaaatgt gataaaccgt actgatagaa tgaaactcag ggcctacagt 2040
   agacgctcta atgaagaaaa ccacaatcat gcatttatgg acatggagct agagcaagac 2100
   atagtgctac cagttccaag caggcggcag gttgaacggt tggattacaa aaaactatac 2160
   gatgaggcat atgaggaaga atcatctaat tcaagtgatg aagaagagtg gtctggaaaa 2220
   gaactcttag aaggcagtgg aacagattca cttgatgagc cttcgtcctg taaaaggctc 2280
   tctagaagag caccagcagg gcagcagaat aatgaactta caccacagag ccggcctcat 2340
   ggttctaaca gtgagcagaa aactgaagtt cttcgctcca atggcagtag tagcacagga 2400
   cggaagtatg atcctgaagt tacccagaaa ttgaaggtgc attttgagaa agatccatat 2460
   cctagccgtg aaacaaaaga aaatctatca gaagaactag gcttgacatt taatcaggtc 2520
   tccaaatggt tctctagtac tcgtcactat tcaagggttg cttctgccaa aaaagaaatg 2580
   catcctgaca gccatacttg tgagaataat gatgagacaa ctgtagacag catgcaagca 2640
   aaacaaccca atgctggggt gatggaaaag ttgacggggg atagaaatgg cattgttcct 2700
   gagaaaccga tggtgcagga caatcttaac caaggagcga tggaaaagtt aactagggat 2760
   aaaaatgaca ttgttcctga gaaaccggtg atgcagagca gtcttaacca atgtaataat 2820
   gaagatatac cgctttctgg aacagaaatt gagatggagt cttacgaaca agaatcatct 2880
   gactcaagtg atgaagagtg gtccatactt agcacaccac gaaaaacaag attacaagac 2940
   catggaaaat catcagttac tgaatcactt cgttctgcaa aacggtgttc cagaagagca 3000
   ccagctaggg agcagaataa tgaacgtatt cagagtgagc agcttcatgg ttctgcaagt 3060
   gagcagcaaa ctgaatttct tcgctccaat gtcagcagtg gcaaggtctc aaaatatcat 3120
   tttggcccta tagttagtca gaagcttaag gaacattttg aaaaagatcc atatccttgc 3180
   cgtgcaacaa aagagagtct ggcacaagag ctaggactga catttaatca gatcagtaag 3240
   tggttctctg gcactcgtca ttattcaaga gatgctgttg ccaagaatca gaaacgcccg 3300
   ggagaaaata ctactgtgaa taataatagc acaacctttg atgacataca agtaatagaa 3360
   cccaatgttg ggttgataga taaaccagct gcagatgtaa atgacatgat ttctgaaaag 3420
   ttgatggtcc aaataaatct taacgaaggc attgaagaag atatcccacc gagccaatat 3480
   gccaggtgtg aagagaaaat aactatgact ccagctgcca tctcaaggga agctggtctt 3540
   ccaggatatg gtcctggtcc tggagaaaac tttcttcagg tcagttcaag gaatactagc 3600
   tgtgagcaga gtgtgatact ggctcctacc gctatcgcaa gagaagttgg tccaccagga 3660
   tatgcgactg gagaaaacca gggcaatggg gctccatgga atacaagtta tgagcgaaga 3720
   ctatttatga atcctgtgac cagctcaaga gcagtcagtc ctccaggata tgggcctgaa 3780
   gaaagccgag gcagtggcat ttcctggaat acaagcagtg aacaaatatt gtttatgagt 3840
   cctacaacca tctcaagaga acttggtcca ccaggatatg ggcctagaga agaccagtgc 3900
   agtggcactt catggaatgc aagctgcgag ttgggagtat ttatgagtcc tgtgaccacc 3960
   tcaagagaag acagtccgcc aggatatggg cctcgagaaa accaggagaa tgacaatatg 4020
   agatgcgagt tgagaatgtt tacaagtcct acaaccatct ccagagaagt tggcccacca 4080
   ggatatgagc ttactggaaa aaacaagggc aacggcgctt catgtactac gaattaccag 4140
   cagggagcgt ttacgagtcc tgaagccttc ccaagagaag cctgtcctcc aggatttgtg 4200
   tctggagaga accagggtaa cgacactcca tggtatatga gcagcaaaca gatggcgttt 4260
   acgagtccta cgaccaaccc tagagtaggt ccgccagggt atggacagga aaaccagggc 4320
   agtcgtgttt cttggaatac gagttatgag cagggagtgt ttatgagttc tacaaacatc 4380
   tcaagagaag ccggtccgcc aggttatggg cctggggaaa gtcagggcaa tatcacttca 4440
   tggatttcga caggcgggca gagaatgttt acaggtcctt caactatccc gaacgaagtc 4500
   gggcagcaag gatatggccc ttcaggaatc ccgggaacgg atggctcaag gagtatggac 4560
   ttgaaagcct ttccaccagg atatggacct gctggagaaa accagggcgg tggtgcttca   4620
   gggagcgtca taagccccca tgctcggtct gcagagaacg tagagttttc agacgacgct   4680
   agaaaaaagg ctatacagcg ggagctgaga cggcggcaga agttcaggtg a            4731
<210> 210
   <211> 1576
   <212> PRT
   <213> Zea mays

<400> 210
   Met Gly Arg Lys Gly Ser Glu Val Ser Pro Ser Lys Arg Tyr Pro Leu
   1               5                   10                  15
   Arg Ser Ala His Ser Ser Gly Arg Val Leu Arg Ser Ala Leu Ala Asn
               20                  25                  30
   Asn Asn Lys Asp Ser Glu Pro Leu Asn Ala Ala Ala Ala Thr Gln Ala
           35                  40                  45
   Ala Val Lys Lys Arg Ser Gly Asn Gln Leu Asp Ser Pro Asn Ser Thr
       50                  55                  60
   Val Arg Leu Leu Arg Ser Thr Ser Lys Asn Lys Asp Glu Ala His Ser
   65                  70                  75                  80
   Gly Pro Leu Asn Asp Arg Ile Ile Asp Lys Pro Ala Ala Asn Lys Arg
                   85                  90                  95
   Lys His Ala Asn Pro Ser Gln Val Gly Ser Pro Ser Ser Ser Val Arg
               100                 105                 110
   Val Leu Arg Ser Ala Ile Lys Asn Lys Asp Met Ala Arg Ser Glu Pro
           115                 120                 125
   Gln Ser Asp Arg Thr Ala Gly Glu Ala Ala Thr Asn Lys Arg Lys Asn
       130                 135                 140
   Ala Asn Ser Ser Lys Val Gly Ser Leu Ser Ser Gly Leu Ser Val Leu
   145                 150                 155                 160
   Arg Ser Ala Ser Lys Ala Lys Asp Glu Thr Cys Ile Lys Pro Leu Asn
                   165                 170                 175
   Asp Ser Pro Ala Val Glu Pro Ala Ser Ser Lys Arg Lys Cys Thr Ser
               180                 185                 190
   Pro Ser Lys Val Gly Ser Pro Ser Asn Ser Val Arg Val Leu Arg Ser
           195                 200                 205
   Thr Ser Lys Tyr Lys Asn Glu Thr Cys Thr Glu Pro Leu Asn Asp Ser
       210                 215                 220
   Gly Ala Val Glu Pro Ala Ala Asn Arg Arg Lys Gly Val Ser Arg Ser
   225 230 235 240
   Lys Glu Gly Ser Pro Asn Ser Ser Val Arg Val Leu Arg Ser Ala Ser
   245 250 255
   Asn Asn Lys Ile Asp Val Ser Ile Glu Pro Leu Asn Asn Ser Thr Thr
   260 265 270
   Gly Gln Pro Ala Ala Lys Arg Arg Lys Asn Gly Ser Ser Phe Lys Ser
   275 280 285
   Ser Ala Arg Val Leu Cys Ser Thr Ser Glu Arg Lys Asn Glu Ala Ser
   290 295 300
   Ser Glu Pro Leu Asn Asp Ser Thr Ala Ala Gln Pro Ala Ala Arg Lys
   305 310 315 320
   Lys Lys Gly Gly Ala Met Pro Lys Thr Asp Asn Pro Lys Ile Gly Val
   325 330 335
   Arg Val Leu Arg Ser Ala Ser Gly Lys Lys Asn Glu Thr Cys Thr Glu
   340 345 350
   Pro Val Asn Arg Ser Thr Ser Arg Glu Pro Thr Val Thr Lys Lys Arg
   355 360 365
   Asn Cys Lys Pro Ser Lys Asp Arg Gly Pro Lys Lys Asp Tyr Leu Lys
   370 375 380
   Ile Cys Gln Arg Ile Lys Tyr Ile Leu Asn Arg Met Lys Tyr Glu Gln
   385 390 395 400
   Ala Phe Ile Gln Ala Tyr Ala Ser Glu Gly Trp Lys Gly Gln Ser Leu
   405 410 415
   Glu Lys Ile Arg Pro Glu Lys Glu Leu Glu Arg Ala Lys Ala Glu Ile
   420 425 430
   Leu Gln Cys Lys Leu Arg Ile Arg Glu Ala Phe Arg Asn Met Asp Ser
   435 440 445
   Leu Leu Ser Lys Gly Lys Leu Glu Glu Ser Leu Phe Asp Ser Ala Gly
   450 455 460
   Gln Ile Ser Ser Glu Asp Ile Phe Cys Ala Ile Cys Gly Ser Lys Asp
   465 470 475 480
   Val Thr Ser Gln Asn Asp Ile Ile Leu Cys Asp Gly Ala Cys Asp Arg
   485 490 495
   Gly Phe His Gln Asn Cys Leu Ser Pro Pro Leu Leu Thr Glu Glu Ile
               500                 505                 510
   Pro Pro Gly Asp Glu Gly Trp Leu Cys Pro Ala Cys Val Cys Lys Ala
           515                 520                 525
   Asp Tyr Ile Asp Ala Leu Asn Glu Leu Gln Gly Ser Lys Leu Ser Ile
       530                 535                 540
   His Asp Ser Trp Val Lys Val Phe Pro Glu Ala Ala Ser Thr Ala Asn
   545                 550                 555                 560
   Gly Ser Lys Gln Val Asp Ala Ser Asp Leu Leu Pro Asp His Ile Lys
                   565                 570                 575
   Asp Ser Ala Asn Leu Ala Leu Val Gly Thr His Met Val Asn Glu Ile
               580                 585                 590
   Arg Phe Ser Glu Glu Asp Asp Ser Lys Ala Asp Asp Leu Gly Leu Pro
           595                 600                 605
   Ser Glu Asp Ser Gly Asp Gly Asp Phe Asp Pro Ala Gly Pro Asp Ser
       610                 615                 620
   Ser Glu Asp Gln Asn Asp Gly Leu Asn Ser Glu Glu Ser Asp Phe Thr
   625                 630                 635                 640
   Ser Asp Ser Asp Tyr Phe Cys Ala Glu Ile Ala Lys Ser Cys Gly Gln
                   645                 650                 655
   Asp Glu Val Ser Ala Ser Pro Leu Ser Asn Val Ile Asn Arg Thr Asp
               660                 665                 670
   Arg Met Lys Leu Arg Ala Tyr Ser Arg Arg Ser Asn Glu Glu Asn His
           675                 680                 685
   Asn His Ala Phe Met Asp Met Glu Leu Glu Gln Asp Ile Val Leu Pro
       690                 695                 700
   Val Pro Ser Arg Arg Gln Val Glu Arg Leu Asp Tyr Lys Lys Leu Tyr
   705                 710                 715                 720
   Asp Glu Ala Tyr Glu Glu Glu Ser Ser Asn Ser Ser Asp Glu Glu Glu
                   725                 730                 735
   Trp Ser Gly Lys Glu Leu Leu Glu Gly Ser Gly Thr Asp Ser Leu Asp
               740                 745                 750
   Glu Pro Ser Ser Cys Lys Arg Leu Ser Arg Arg Ala Pro Ala Gly Gln
           755                 760                 765
   Gln Asn Asn Glu Leu Thr Pro Gln Ser Arg Pro His Gly Ser Asn Ser
       770                 775                 780
   Glu Gln Lys Thr Glu Val Leu Arg Ser Asn Gly Ser Ser Ser Thr Gly
   785                 790                 795                 800
   Arg Lys Tyr Asp Pro Glu Val Thr Gln Lys Leu Lys Val His Phe Glu
                   805                 810                 815
   Lys Asp Pro Tyr Pro Ser Arg Glu Thr Lys Glu Asn Leu Ser Glu Glu
               820                 825                 830
   Leu Gly Leu Thr Phe Asn Gln Val Ser Lys Trp Phe Ser Ser Thr Arg
           835                 840                 845
   His Tyr Ser Arg Val Ala Ser Ala Lys Lys Glu Met His Pro Asp Ser
       850                 855                 860
   His Thr Cys Glu Asn Asn Asp Glu Thr Thr Val Asp Ser Met Gln Ala
   865                 870                 875                 880
   Lys Gln Pro Asn Ala Gly Val Met Glu Lys Leu Thr Gly Asp Arg Asn
                   885                 890                 895
   Gly Ile Val Pro Glu Lys Pro Met Val Gln Asp Asn Leu Asn Gln Gly
               900                 905                 910
   Ala Met Glu Lys Leu Thr Arg Asp Lys Asn Asp Ile Val Pro Glu Lys
           915                 920                 925
   Pro Val Met Gln Ser Ser Leu Asn Gln Cys Asn Asn Glu Asp Ile Pro
       930                 935                 940
   Leu Ser Gly Thr Glu Ile Glu Met Glu Ser Tyr Glu Gln Glu Ser Ser
   945                 950                 955                 960
   Asp Ser Ser Asp Glu Glu Trp Ser Ile Leu Ser Thr Pro Arg Lys Thr
                   965                 970                 975
   Arg Leu Gln Asp His Gly Lys Ser Ser Val Thr Glu Ser Leu Arg Ser
               980                 985                 990
   Ala Lys Arg Cys Ser Arg Arg Ala  Pro Ala Arg Glu Gln  Asn Asn Glu
           995                 1000                1005
   Arg Ile  Gln Ser Glu Gln Leu  His Gly Ser Ala Ser  Glu Gln Gln
       1010                1015                1020
   Thr Glu  Phe Leu Arg Ser Asn  Val Ser Ser Gly Lys  Val Ser Lys
       1025                1030                1035
   Tyr His  Phe Gly Pro Ile Val  Ser Gln Lys Leu Lys  Glu His Phe
   1040 1045 1050
   Glu Lys Asp Pro Tyr Pro Cys Arg Ala Thr Lys Glu Ser Leu Ala
   1055 1060 1065
   Gln Glu Leu Gly Leu Thr Phe Asn Gln Ile Ser Lys Trp Phe Ser
   1070 1075 1080
   Gly Thr Arg His Tyr Ser Arg Asp Ala Val Ala Lys Asn Gln Lys
   1085 1090 1095
   Arg Pro Gly Glu Asn Thr Thr Val Asn Asn Asn Ser Thr Thr Phe
   1100 1105 1110
   Asp Asp Ile Gln Val Ile Glu Pro Asn Val Gly Leu Ile Asp Lys
   1115 1120 1125
   Pro Ala Ala Asp Val Asn Asp Met Ile Ser Glu Lys Leu Met Val
   1130 1135 1140
   Gln Ile Asn Leu Asn Glu Gly Ile Glu Glu Asp Ile Pro Pro Ser
   1145 1150 1155
   Gln Tyr Ala Arg Cys Glu Glu Lys Ile Thr Met Thr Pro Ala Ala
   1160 1165 1170
   Ile Ser Arg Glu Ala Gly Leu Pro Gly Tyr Gly Pro Gly Pro Gly
   1175 1180 1185
   Glu Asn Phe Leu Gln Val Ser Ser Arg Asn Thr Ser Cys Glu Gln
   1190 1195 1200
   Ser Val Ile Leu Ala Pro Thr Ala Ile Ala Arg Glu Val Gly Pro
   1205 1210 1215
   Pro Gly Tyr Ala Thr Gly Glu Asn Gln Gly Asn Gly Ala Pro Trp
   1220 1225 1230
   Asn Thr Ser Tyr Glu Arg Arg Leu Phe Met Asn Pro Val Thr Ser
   1235 1240 1245
   Ser Arg Ala Val Ser Pro Pro Gly Tyr Gly Pro Glu Glu Ser Arg
   1250 1255 1260
   Gly Ser Gly Ile Ser Trp Asn Thr Ser Ser Glu Gln Ile Leu Phe
   1265 1270 1275
   Met Ser Pro Thr Thr Ile Ser Arg Glu Leu Gly Pro Pro Gly Tyr
   1280 1285 1290
   Gly Pro Arg Glu Asp Gln Cys Ser Gly Thr Ser Trp Asn Ala Ser
   1295 1300 1305
   Cys Glu Leu Gly Val Phe Met Ser Pro Val Thr Thr Ser Arg Glu
   1310 1315 1320
   Asp Ser Pro Pro Gly Tyr Gly Pro Arg Glu Asn Gln Glu Asn Asp
   1325 1330 1335
   Asn Met Arg Cys Glu Leu Arg Met Phe Thr Ser Pro Thr Thr Ile
   1340 1345 1350
   Ser Arg Glu Val Gly Pro Pro Gly Tyr Glu Leu Thr Gly Lys Asn
   1355 1360 1365
   Lys Gly Asn Gly Ala Ser Cys Thr Thr Asn Tyr Gln Gln Gly Ala
   1370 1375 1380
   Phe Thr Ser Pro Glu Ala Phe Pro Arg Glu Ala Cys Pro Pro Gly
   1385 1390 1395
   Phe Val Ser Gly Glu Asn Gln Gly Asn Asp Thr Pro Trp Tyr Met
   1400 1405 1410
   Ser Ser Lys Gln Met Ala Phe Thr Ser Pro Thr Thr Asn Pro Arg
   1415 1420 1425
   Val Gly Pro Pro Gly Tyr Gly Gln Glu Asn Gln Gly Ser Arg Val
   1430 1435 1440
   Ser Trp Asn Thr Ser Tyr Glu Gln Gly Val Phe Met Ser Ser Thr
   1445 1450 1455
   Asn Ile Ser Arg Glu Ala Gly Pro Pro Gly Tyr Gly Pro Gly Glu
   1460 1465 1470
   Ser Gln Gly Asn Ile Thr Ser Trp Ile Ser Thr Gly Gly Gln Arg
   1475 1480 1485
   Met Phe Thr Gly Pro Ser Thr Ile Pro Asn Glu Val Gly Gln Gln
   1490 1495 1500
   Gly Tyr Gly Pro Ser Gly Ile Pro Gly Thr Asp Gly Ser Arg Ser
   1505 1510 1515
   Met Asp Leu Lys Ala Phe Pro Pro Gly Tyr Gly Pro Ala Gly Glu
   1520 1525 1530
   Asn Gln Gly Gly Gly Ala Ser Gly Ser Val Ile Ser Pro His Ala
   1535 1540 1545
   Arg Ser Ala Glu Asn Val Glu Phe Ser Asp Asp Ala Arg Lys Lys
   1550 1555 1560
   Ala Ile Gln Arg Glu Leu Arg Arg Arg Gln Lys Phe Arg
   1565 1570 1575
<210> 211
   <211> 4620
   <212> DNA
   <213> Zea mays
<400> 211
   atgggaagga aaggctcaga aatgtcacca agtaaaaggt atcccttgag gtctccacat 60
   agtagtggta gagttcttcg ctctgcctta gccaatgata ataaggacag tgagcctctg 120
   aatgctgctg ctgctaccca agccgctgtg aaaaaaagaa gtggcagtca gttggatagt 180
   cctaatagca gtgtcaggtt gcttcactct acatcaaaaa ataagaacga ggtatgtagc 240
   gggcctctaa atgacaggat ccctgataag ccagctgcaa ataaaagaaa acgtgccagt 300
   ccttcacagg tgggaagttc cagcaacagt gtcagggtcg ttcgctctgc aataaaaaat 360
   aaagatgagg catgcagtaa atctctaagt gacaggaccg ctggtgagcc agctgcaaat 420
   aaaagaaaaa acgtcaatcg ctcaaagacg gggagtctga gctgtggtgt cagggtgctt 480
   ctatctgctt caaaagctaa ggatgagaca tctaccaagc ctttaaatga tagtgctact 540
   gttgaaccag cttcaggtaa aaggaaaggc accagtccgt caaaggtggg aagtgccagc 600
   aatagtgtca gggtgcttcg atctacttca aaatataaga atgagacatg tactgagcct 660
   ttaaatgata gtggtgctgt tgaaccagct gcaaataaaa ggaaaggtgt cagtcgttca 720
   aaggagagaa gtctcaatag cagtgtcagg gtcgttgctc tggcctcaaa taataagatt 780
   gaggtatcta ttaagcctct aggtaatagt actgctgggg aaccagctgc gaaaagaaga 840
   aaaagcggca gttccttcga ggcaggaagc cctgtgagca gtgccagggt gcttcgccct 900
   acctcagaaa ggaagaatga ggcatctaag cctttaaatg aaagtactgc tgctcaacca 960
   gctgcgagga aaaaaaaagc tggggtaatt tcaaagaccg acaatccaaa gattggtctc 1020
   agggttcttc gctctgcctc aggaaagaag aatgaagcat gtattgggca tgtaaatgat 1080
   agtacttctg ctgaaccaac tgtgacaaaa agaaaccgca agccttcaat ggatcgcagt 1140
   cccaagaagg actacttaaa aatttgtcaa agagttagat atattttgaa tcgaatgaac 1200
   tatcaacaaa cctttattca agcttatgca agtgaaggtt ggaaaggcca aagtttggaa 1260
   aaaataagac ctgaaaagga gcttgaacga gccaaggcag aaatcctcca atgcaagtta 1320
   agaatccgtg aagcttttcg aaatatggac tctcttttat ccaaggggaa gcttgaagaa 1380
   tctttgtttg attccgcagg agaaatttca agcgaagata tattttgtgc tgtatgtggc 1440
   tcaaaggatg ttactttaca aaatgacatc attctttgtg atggagcctg tgacagagga 1500
   ttccaccaga actgtttgaa ccctcctttg ctaactgaag atatacctcc gggggatcaa 1560
   agatggcttt gccctgcatg tgtctgcaaa gcagactcta tagatgcact aaatgaactt 1620
   caagggagca agctctccat tcatgactca tgggagaaag ttttccctga ggcagcttca 1680
   attgccaacg gttctaaaca agttgatact tctgatctgc tgccagatca tataaagcac 1740
   agtgacaacc ctgcattggt tgaagggctg atggtggatg aagtcaggct ttctgcagag 1800
   gatgacagca aagcggatga ccttcgcttg tcttcggagg actcagggga tggtgacttc 1860
   gatccgtcag gtccagattc tagtgaagac caaaaagatg gattgaactc agaagagtca 1920
   gatttcacat cagactctga tgatttttgt gctgagattg ccaaatcttg tggccaggat 1980
   gaagtctcgg catctccatt atcaaatgtg ataaaccata cttatagaat gaaactcagg 2040
   gccagcaaca accgctctaa tgaagaaaac catgatcatg tatttatgga catggagcta 2100
   gggcaagaca tggtgctacc agtttcaagc aggcggcagg tggaacggtt ggattacaaa 2160
   aaactatatg atgaggcata tgggaaagaa tcatctaatt caagtgatga caaagagtgg 2220
   tctggaaaag aactattaga aggcagtgaa acagattcac ttagtgagcg acctcatcct 2280
   gtaaaacggt gctctagaag agcacaagca gagcagcaga ataatgaaca tacaccacag 2340
   agggagcgcc ttcatggttc tgaaagtgag cagaaaactg gaattcttcg atccaatggc 2400
   agcagtagca caggacggaa gtttggtcct gtagctaccc agaaattgaa ggtacatttt 2460
   gagaaagatc catatcctag ccgtgaaacg aaagagaata tatcagaaga actaggcttg 2520
   acatttaatc aggtctccag atggttctct agcactcgtc actattcaag ggttgcttct 2580
   gccagaaaag aaatgcatcc tgacggccat actagcgaga ataatgatac cacgactgta 2640
   gatagcatgc aagcaaggca acccaacact gtggtgatgg aaaagttgac tgggaataga 2700
   aatgacattg tttctgagaa accgatggtg cagaacaatc ttaaccaagg ggtggcagaa 2760
   aagttgacta gggatagaaa tgacattgtt cctgagaaac aggtggtgca gagcaatctt 2820
   aaccaatgta ataatgaaga tatcccgctt tctggaacag aaattgagat ggagtcttac 2880
   gaacaagaat catctgaaag cagtgatgaa gagtggtcca cacttagcac accacgaaaa 2940
   acaaaattac aaggcaacga aaaagtatca cttactgaat cactacgttc tgcaaaacgg 3000
   tgttctagaa gagcaccagc tagggagcag aataatgaac atactcagag tgagcaactt 3060
   catggttctg taagtaaaca gcaaactgaa gttcttcgct ccaatgtcag cagtggcgat 3120
   gcctcaaaat gtcattttgg ccctatagtt actcagaagc tgaaggaaca ttttgaaaaa 3180
   gatccatatc cttgccgtgc aacaaaagag ggtctggcac aagagctagg actgacattt 3240
   aatcagatca gtaagtggtt ctctgccacc catcattatt caagagatgc tgttgccaag 3300
   aaccaaaaat acccaggaga aaatactact gagaataata gtagcacaat ctttgatggt 3360
   atacaagtaa tagaacccaa ttttggtttg atagataaac cagatgcaga tacaaatgac 3420
   atgatttctg aaaagttgat ggtacaaatt aatcttaacg aaggcattga agaggatatc 3480
   ccaccgagcc aatatactac caggtgtgaa gagaaattaa ctatgactca agctgccatc 3540
   tcaagggaag ctggacctcc aggttatggt cctggagaaa actttcttca ggtcagttca 3600
   aggattacta gctgtgagca gagtgtgata atggctccta gcgctatcgc aagaggagtt 3660
   ggtccaccag gatatacgcc tggagaacac cagggcaatg gggctccatg gaatacaagc 3720
   tatgagcgaa gaccgtttac gagtcctgcg actagctcaa gagaagtcgg acctccagga 3780
   tatgggcctg aagaaaacca aggcagtggc atttcctgta atacaagcag agagcaaata 3840
   ttgtttatga gccctacaac catctcgaga gaacttggtc caccaggata tttgcccaga 3900
   gaaaaccagc gcagtgacac ttcatggaat acaagctgcg agtcgggact atttatgagt 3960
   cctgcgacca cctcaataga acatggtcca ccaggatatg ggcctggaga aaaccaggag 4020
   aatgacagta caagctgcga gttgagaatg tttaccagtc ctacaatcgt ctccagagaa 4080
   gttggcccac caggatacca gcagggagtg tttatgagtc ctaaaaccat ctcaagagaa 4140
   gtctgtcctc caggatttgg gtctggagag aaccagggca acgacactac atggtacaaa 4200
   cagagcgcgt ttacaagtcc tacaaccatc tcgagagaag tcggtccgcc agggtatgga 4260
   caggaagatc agggcaatgg tggttcttgg aacacgagtt gtgagcaggg agtgtttacg 4320
   agtcctacta gaggcatctc aagagaagtc ggtccgccag gatacgggcc cggggaaagt 4380
   cagggcaata ccacttcgtg gatttcgacc tgccagcaga gaatgcttgc aggaatccag 4440
   agaactggtg gctcaaggag tatggacttg gaagcctttc caccgggata cggacctggt 4500
   ggtgcttcag ggagtgtcat aagcccccaa gctcggtctg cagagaatgt agaattttcg 4560
   gacgaggcta gaaaaaaggc tatacagcgg gagctgagac ggcggcagaa gttcaggtga 4620
<210> 212
   <211> 1539
   <212> PRT
   <213> Zea mays
<400> 212
   Met Gly Arg Lys Gly Ser Glu Met Ser Pro Ser Lys Arg Tyr Pro Leu
   1               5                   10                  15
   Arg Ser Pro His Ser Ser Gly Arg Val Leu Arg Ser Ala Leu Ala Asn
               20                  25                  30
   Asp Asn Lys Asp Ser Glu Pro Leu Asn Ala Ala Ala Ala Thr Gln Ala
           35                  40                  45
   Ala Val Lys Lys Arg Ser Gly Ser Gln Leu Asp Ser Pro Asn Ser Ser
       50                  55                  60
   Val Arg Leu Leu His Ser Thr Ser Lys Asn Lys Asn Glu Val Cys Ser
   65                  70                  75                  80
   Gly Pro Leu Asn Asp Arg Ile Pro Asp Lys Pro Ala Ala Asn Lys Arg
                   85                  90                  95
   Lys Arg Ala Ser Pro Ser Gln Val Gly Ser Ser Ser Asn Ser Val Arg
               100                 105                 110
   Val Val Arg Ser Ala Ile Lys Asn Lys Asp Glu Ala Cys Ser Lys Ser
           115                 120                 125
   Leu Ser Asp Arg Thr Ala Gly Glu Pro Ala Ala Asn Lys Arg Lys Asn
       130                 135                 140
   Val Asn Arg Ser Lys Thr Gly Ser Leu Ser Cys Gly Val Arg Val Leu
   145                 150                 155                 160
   Leu Ser Ala Ser Lys Ala Lys Asp Glu Thr Ser Thr Lys Pro Leu Asn
                   165                 170                 175
   Asp Ser Ala Thr Val Glu Pro Ala Ser Gly Lys Arg Lys Gly Thr Ser
               180                 185                 190
   Pro Ser Lys Val Gly Ser Ala Ser Asn Ser Val Arg Val Leu Arg Ser
           195                 200                 205
   Thr Ser Lys Tyr Lys Asn Glu Thr Cys Thr Glu Pro Leu Asn Asp Ser
       210                 215                 220
   Gly Ala Val Glu Pro Ala Ala Asn Lys Arg Lys Gly Val Ser Arg Ser
   225                 230                 235                 240
   Lys Glu Arg Ser Leu Asn Ser Ser Val Arg Val Val Ala Leu Ala Ser
                   245                 250                 255
   Asn Asn Lys Ile Glu Val Ser Ile Lys Pro Leu Gly Asn Ser Thr Ala
               260                 265                 270
   Gly Glu Pro Ala Ala Lys Arg Arg Lys Ser Gly Ser Ser Phe Glu Ala
           275                 280                 285
   Gly Ser Pro Val Ser Ser Ala Arg Val Leu Arg Pro Thr Ser Glu Arg
       290                 295                 300
   Lys Asn Glu Ala Ser Lys Pro Leu Asn Glu Ser Thr Ala Ala Gln Pro
   305                 310                 315                 320
   Ala Ala Arg Lys Lys Lys Ala Gly Val Ile Ser Lys Thr Asp Asn Pro
                   325                 330                 335
   Lys Ile Gly Leu Arg Val Leu Arg Ser Ala Ser Gly Lys Lys Asn Glu
               340                 345                 350
   Ala Cys Ile Gly His Val Asn Asp Ser Thr Ser Ala Glu Pro Thr Val
           355                 360                 365
   Thr Lys Arg Asn Arg Lys Pro Ser Met Asp Arg Ser Pro Lys Lys Asp
       370                 375                 380
   Tyr Leu Lys Ile Cys Gln Arg Val Arg Tyr Ile Leu Asn Arg Met Asn
   385                 390                 395                 400
   Tyr Gln Gln Thr Phe Ile Gln Ala Tyr Ala Ser Glu Gly Trp Lys Gly
                   405                 410                 415
   Gln Ser Leu Glu Lys Ile Arg Pro Glu Lys Glu Leu Glu Arg Ala Lys
   420 425 430
   Ala Glu Ile Leu Gln Cys Lys Leu Arg Ile Arg Glu Ala Phe Arg Asn
   435 440 445
   Met Asp Ser Leu Leu Ser Lys Gly Lys Leu Glu Glu Ser Leu Phe Asp
   450 455 460
   Ser Ala Gly Glu Ile Ser Ser Glu Asp Ile Phe Cys Ala Val Cys Gly
   465 470 475 480
   Ser Lys Asp Val Thr Leu Gln Asn Asp Ile Ile Leu Cys Asp Gly Ala
   485 490 495
   Cys Asp Arg Gly Phe His Gln Asn Cys Leu Asn Pro Pro Leu Leu Thr
   500 505 510
   Glu Asp Ile Pro Pro Gly Asp Gln Arg Trp Leu Cys Pro Ala Cys Val
   515 520 525
   Cys Lys Ala Asp Ser Ile Asp Ala Leu Asn Glu Leu Gln Gly Ser Lys
   530 535 540
   Leu Ser Ile His Asp Ser Trp Glu Lys Val Phe Pro Glu Ala Ala Ser
   545 550 555 560
   Ile Ala Asn Gly Ser Lys Gln Val Asp Thr Ser Asp Leu Leu Pro Asp
   565 570 575
   His Ile Lys His Ser Asp Asn Pro Ala Leu Val Glu Gly Leu Met Val
   580 585 590
   Asp Glu Val Arg Leu Ser Ala Glu Asp Asp Ser Lys Ala Asp Asp Leu
   595 600 605
   Arg Leu Ser Ser Glu Asp Ser Gly Asp Gly Asp Phe Asp Pro Ser Gly
   610 615 620
   Pro Asp Ser Ser Glu Asp Gln Lys Asp Gly Leu Asn Ser Glu Glu Ser
   625 630 635 640
   Asp Phe Thr Ser Asp Ser Asp Asp Phe Cys Ala Glu Ile Ala Lys Ser
   645 650 655
   Cys Gly Gln Asp Glu Val Ser Ala Ser Pro Leu Ser Asn Val Ile Asn
   660 665 670
   His Thr Tyr Arg Met Lys Leu Arg Ala Ser Asn Asn Arg Ser Asn Glu
   675 680 685
   Glu Asn His Asp His Val Phe Met Asp Met Glu Leu Gly Gln Asp Met
       690                 695                 700
   Val Leu Pro Val Ser Ser Arg Arg Gln Val Glu Arg Leu Asp Tyr Lys
   705                 710                 715                 720
   Lys Leu Tyr Asp Glu Ala Tyr Gly Lys Glu Ser Ser Asn Ser Ser Asp
                   725                 730                 735
   Asp Lys Glu Trp Ser Gly Lys Glu Leu Leu Glu Gly Ser Glu Thr Asp
               740                 745                 750
   Ser Leu Ser Glu Arg Pro His Pro Val Lys Arg Cys Ser Arg Arg Ala
           755                 760                 765
   Gln Ala Glu Gln Gln Asn Asn Glu His Thr Pro Gln Arg Glu Arg Leu
       770                 775                 780
   His Gly Ser Glu Ser Glu Gln Lys Thr Gly Ile Leu Arg Ser Asn Gly
   785                 790                 795                 800
   Ser Ser Ser Thr Gly Arg Lys Phe Gly Pro Val Ala Thr Gln Lys Leu
                   805                 810                 815
   Lys Val His Phe Glu Lys Asp Pro Tyr Pro Ser Arg Glu Thr Lys Glu
               820                 825                 830
   Asn Ile Ser Glu Glu Leu Gly Leu Thr Phe Asn Gln Val Ser Arg Trp
           835                 840                 845
   Phe Ser Ser Thr Arg His Tyr Ser Arg Val Ala Ser Ala Arg Lys Glu
       850                 855                 860
   Met His Pro Asp Gly His Thr Ser Glu Asn Asn Asp Thr Thr Thr Val
   865                 870                 875                 880
   Asp Ser Met Gln Ala Arg Gln Pro Asn Thr Val Val Met Glu Lys Leu
                   885                 890                 895
   Thr Gly Asn Arg Asn Asp Ile Val Ser Glu Lys Pro Met Val Gln Asn
               900                 905                 910
   Asn Leu Asn Gln Gly Val Ala Glu Lys Leu Thr Arg Asp Arg Asn Asp
           915                 920                 925
   Ile Val Pro Glu Lys Gln Val Val Gln Ser Asn Leu Asn Gln Cys Asn
       930                 935                 940
   Asn Glu Asp Ile Pro Leu Ser Gly Thr Glu Ile Glu Met Glu Ser Tyr
   945                 950                 955                 960
   Glu Gln Glu Ser Ser Glu Ser Ser Asp Glu Glu Trp Ser Thr Leu Ser
                   965                 970                 975
   Thr Pro Arg Lys Thr Lys Leu Gln Gly Asn Glu Lys Val Ser Leu Thr
               980                 985                 990
   Glu Ser Leu Arg Ser Ala Lys Arg  Cys Ser Arg Arg Ala  Pro Ala Arg
           995                 1000                 1005
   Glu Gln  Asn Asn Glu His Thr  Gln Ser Glu Gln Leu  His Gly Ser
       1010                 1015                 1020
   Val Ser  Lys Gln Gln Thr Glu  Val Leu Arg Ser Asn  Val Ser Ser
       1025                 1030                 1035
   Gly Asp  Ala Ser Lys Cys His  Phe Gly Pro Ile Val  Thr Gln Lys
       1040                 1045                 1050
   Leu Lys  Glu His Phe Glu Lys  Asp Pro Tyr Pro Cys  Arg Ala Thr
       1055                 1060                 1065
   Lys Glu  Gly Leu Ala Gln Glu  Leu Gly Leu Thr Phe  Asn Gln Ile
       1070                 1075                 1080
   Ser Lys  Trp Phe Ser Ala Thr  His His Tyr Ser Arg  Asp Ala Val
       1085                 1090                 1095
   Ala Lys  Asn Gln Lys Tyr Pro  Gly Glu Asn Thr Thr  Glu Asn Asn
       1100                 1105                 1110
   Ser Ser  Thr Ile Phe Asp Gly  Ile Gln Val Ile Glu  Pro Asn Phe
       1115                 1120                 1125
   Gly Leu  Ile Asp Lys Pro Asp  Ala Asp Thr Asn Asp  Met Ile Ser
       1130                 1135                 1140
   Glu Lys  Leu Met Val Gln Ile  Asn Leu Asn Glu Gly  Ile Glu Glu
       1145                 1150                 1155
   Asp Ile  Pro Pro Ser Gln Tyr  Thr Thr Arg Cys Glu  Glu Lys Leu
       1160                 1165                 1170
   Thr Met  Thr Gln Ala Ala Ile  Ser Arg Glu Ala Gly  Pro Pro Gly
       1175                 1180                 1185
   Tyr Gly  Pro Gly Glu Asn Phe  Leu Gln Val Ser Ser  Arg Ile Thr
       1190                 1195                 1200
   Ser Cys  Glu Gln Ser Val Ile  Met Ala Pro Ser Ala  Ile Ala Arg
       1205                 1210                 1215
   Gly Val  Gly Pro Pro Gly Tyr  Thr Pro Gly Glu His  Gln Gly Asn
       1220                 1225                 1230
   Gly Ala Pro Trp Asn Thr Ser Tyr Glu Arg Arg Pro Phe Thr Ser
   1235 1240 1245
   Pro Ala Thr Ser Ser Arg Glu Val Gly Pro Pro Gly Tyr Gly Pro
   1250 1255 1260
   Glu Glu Asn Gln Gly Ser Gly Ile Ser Cys Asn Thr Ser Arg Glu
   1265 1270 1275
   Gln Ile Leu Phe Met Ser Pro Thr Thr Ile Ser Arg Glu Leu Gly
   1280 1285 1290
   Pro Pro Gly Tyr Leu Pro Arg Glu Asn Gln Arg Ser Asp Thr Ser
   1295 1300 1305
   Trp Asn Thr Ser Cys Glu Ser Gly Leu Phe Met Ser Pro Ala Thr
   1310 1315 1320
   Thr Ser Ile Glu His Gly Pro Pro Gly Tyr Gly Pro Gly Glu Asn
   1325 1330 1335
   Gln Glu Asn Asp Ser Thr Ser Cys Glu Leu Arg Met Phe Thr Ser
   1340 1345 1350
   Pro Thr Ile Val Ser Arg Glu Val Gly Pro Pro Gly Tyr Gln Gln
   1355 1360 1365
   Gly Val Phe Met Ser Pro Lys Thr Ile Ser Arg Glu Val Cys Pro
   1370 1375 1380
   Pro Gly Phe Gly Ser Gly Glu Asn Gln Gly Asn Asp Thr Thr Trp
   1385 1390 1395
   Tyr Lys Gln Ser Ala Phe Thr Ser Pro Thr Thr Ile Ser Arg Glu
   1400 1405 1410
   Val Gly Pro Pro Gly Tyr Gly Gln Glu Asp Gln Gly Asn Gly Gly
   1415 1420 1425
   Ser Trp Asn Thr Ser Cys Glu Gln Gly Val Phe Thr Ser Pro Thr
   1430 1435 1440
   Arg Gly Ile Ser Arg Glu Val Gly Pro Pro Gly Tyr Gly Pro Gly
   1445 1450 1455
   Glu Ser Gln Gly Asn Thr Thr Ser Trp Ile Ser Thr Cys Gln Gln
   1460 1465 1470
   Arg Met Leu Ala Gly Ile Gln Arg Thr Gly Gly Ser Arg Ser Met
   1475 1480 1485
   Asp Leu Glu Ala Phe Pro Pro Gly Tyr Gly Pro Gly Gly Ala Ser
   1490 1495 1500
   Gly Ser Val Ile Ser Pro Gln Ala Arg Ser Ala Glu Asn Val Glu
   1505 1510 1515
   Phe Ser Asp Glu Ala Arg Lys Lys Ala Ile Gln Arg Glu Leu Arg
   1520 1525 1530
   Arg Arg Gln Lys Phe Arg
   1535
<210> 213
   <211> 2907
   <212> DNA
   <213> Vitis vinifera
<400> 213
   atggatgctt cccctgccca agagagtaat cggactagga aaagttctag tcctaaacag      60
   aatattttag aagaggccag aaaactttct gaaagtgtgt gtagcgaatc gtcagagcag     120
   aaacgtccct ctgaaaatgg acagcatgag ccagcagaaa tcagccctgt gctatccaat     180
   tgtattgtta ctgaacagtc agaactgcct ccagaagatg tgggtgatac aatattagga     240
   ttgcctccag cagatgtgac caagaattct cttactgaac atttgggatt gcctccagaa     300
   gatgcaatca agaatgatgg aactgaacaa ttggggtttt ttcctgaagt tgtgaccaag     360
   agttctatta ttgaaaaatt aggacaatca gagccacctc ctgaaaatgt ggcccggtat     420
   tctggtcttg accagtcagg gtcagcaccc aaagatttgg ccaataaaag aactgcaaaa     480
   ctagttaaaa gaaaatataa attgagatct tcagtaagcg gttcccgggt tttacgctca     540
   aggtcacaag agaaacctaa agcttcacag ccaagtgata attttgtaaa tgccagtgct     600
   agcagagaaa ggaaaggaag aaagaagaaa aggatgaata aaacaactgc agatgaattt     660
   gcgagaatca ggaaacatct tagatattta ctgaacagaa tgagctatga gcaaaatctg     720
   attgatgctt attctgccga aggttggaaa ggacaaagtg tggaaaaatt aaagccagag     780
   aaggaacttc aacgtgcctc atctgaaatt tctcgtcgca aactgmaaat acgtgatcta     840
   tttcaacatc ttgattcatt atgtgctgaa ggaaggtttc cagaatcttt atttgattct     900
   gaagggcaga ttgacagtga ggatatattc tgtgctaaat gtgagtccaa agacatgtct     960
   gctgataatg acataatact ctgtgacggt gcttgtgatc gtggatttca ccagttttgt    1020
   ctggaaccgc cattgttaaa agaagaaatt cctcctgatg acgagggttg gctgtgccct    1080
   gcatgtgatt gcaaagttga ctgcatggac ctgcttaatg actctcaagg aacaaaactt    1140
   tctgtaattg atagctggga gaaggttttt cctgaggcag ctgcagctgg gaataaccag    1200
   gataacaact ctggattttc atcagatgat tctgaggata atgattatga ccccgattgt    1260
   ccagaggttg atgagaaggg tcagggagat aagtcaagtt ctgataaatt tgatgaatct    1320
   gatgaatttg acgaatctga tgaatctgat ttcacttctg catctgatga tatggtggtc    1380
   tcaccraata atgagcagtg tttggggctt ccttctgatg attcagagga tgatgatttt   1440
   gatcctgatg ctccagaaat tgatgaacag gttaatcagg ggagttcaag ttctgatttt   1500
   acatctgact ccgaggattt tactgccact ttagatcgca gaaacttctc tgacaacgag   1560
   gatggtcttg atgagcaaag aagatttggt aggaagaaga aagatacttt aaaggatgag   1620
   ctcttatccg tgttagagtc aaattctggt caagataatg cacctctgtc tgcaaagaga   1680
   catgtagaaa ggctggatta caaaaagctg catgatgagg catatggaaa tgtttcttct   1740
   gattcaagtg atgatgaaga ctggacagag aatgttatac caagaaagag gaagaatctt   1800
   agtggtaatg ttgcctcagt gtcaccaaat ggaaacactt caatcactga gaatgggaca   1860
   aataccaagg acataaagca cgacttggaa gcagctggat gcactcccaa gcgaagaact   1920
   cgtcagaagc tgaactttga aagcacaaat aattcacttg ctgagtcaca taaaggttct   1980
   cgaagtcctg gttctactgg tgaaaaaagt gggcaatcat catataaaaa acttggagaa   2040
   gctgtaactg agagactcta caaatccttc caggaaaatc agtaccctga tcgtgctatg   2100
   aaagaaaaat tggcagaaga gctgggaata acgagccggc aggttagcaa atggtttgag   2160
   aatgcccgtt ggagctttcg ccatcgacca cccaaggaag ccagtgcggg taaaagtgct   2220
   gtgaaaaagg atgcatccac gtctcaaaca gatcaaaagc ctgagcaaga agtggttctt   2280
   agagaaagtt ctcacaatgg agtgggaaaa aaggagtcgc ccaaagcagg tgcttcgaag   2340
   gtagaccgca gcaaggaagc caatgcaggt aaaagtgctg tgaaaaagga tgcatccacg   2400
   tctcaaacag atcaaaagcc tgagcaagaa gtggttatta aagaaagttc tcacaatgga   2460
   gtgggaaaaa aggagtcgac caaagcaggt gcttcgaagg tagaccgctg cagtggagct   2520
   aagaggagaa ggaaattggc aactgatgga agccatagac agaaatcttc aactcccaac   2580
   tctacaagac aaaagacaaa gtcaaatcat gaagcatctg aggcaaccaa tggaagcaga   2640
   aggcagaatt cttctactcc caagtctaga aggcaaaaga ctaagttagc tggtgaagcg   2700
   tctgaagcaa ctggtggaag cagtagacag aattcgtcaa ccccaaattc taaaaggcga   2760
   aaaaccaagt cagatcatga agcatctaat cccgtcctta gcggtaagaa gatagcaaag   2820
   acggcaaaga gctcatctgg cacaccaaaa acaaaggaaa agctaagtga cagaatccaa   2880
   acaagaagta ggaaatctat tgcttga                                       2907
<210> 214
   <211> 968
   <212> PRT
   <213> Vitis vinifera
<220>
   <221> UNSURE
   <222> (276)..(276)
   <223> Xaa can be any naturally occurring amino acid
<400> 214
   Met Asp Ala Ser Pro Ala Gln Glu Ser Asn Arg Thr Arg Lys Ser Ser
   1               5                   10                  15
   Ser Pro Lys Gln Asn Ile Leu Glu Glu Ala Arg Lys Leu Ser Glu Ser
               20                  25                  30
   Val Cys Ser Glu Ser Ser Glu Gln Lys Arg Pro Ser Glu Asn Gly Gln
           35                  40                  45
   His Glu Pro Ala Glu Ile Ser Pro Val Leu Ser Asn Cys Ile Val Thr
       50                  55                  60
   Glu Gln Ser Glu Leu Pro Pro Glu Asp Val Gly Asp Thr Ile Leu Gly
   65                  70                  75                  80
   Leu Pro Pro Ala Asp Val Thr Lys Asn Ser Leu Thr Glu His Leu Gly
                   85                  90                  95
   Leu Pro Pro Glu Asp Ala Ile Lys Asn Asp Gly Thr Glu Gln Leu Gly
               100                 105                 110
   Phe Phe Pro Glu Val Val Thr Lys Ser Ser Ile Ile Glu Lys Leu Gly
           115                 120                 125
   Gln Ser Glu Pro Pro Pro Glu Asn Val Ala Arg Tyr Ser Gly Leu Asp
       130                 135                 140
   Gln Ser Gly Ser Ala Pro Lys Asp Leu Ala Asn Lys Arg Thr Ala Lys
   145                 150                 155                 160
   Leu Val Lys Arg Lys Tyr Lys Leu Arg Ser Ser Val Ser Gly Ser Arg
                   165                 170                 175
   Val Leu Arg Ser Arg Ser Gln Glu Lys Pro Lys Ala Ser Gln Pro Ser
               180                 185                 190
   Asp Asn Phe Val Asn Ala Ser Ala Ser Arg Glu Arg Lys Gly Arg Lys
           195                 200                 205
   Lys Lys Arg Met Asn Lys Thr Thr Ala Asp Glu Phe Ala Arg Ile Arg
       210                 215                 220
   Lys His Leu Arg Tyr Leu Leu Asn Arg Met Ser Tyr Glu Gln Asn Leu
   225                 230                 235                 240
   Ile Asp Ala Tyr Ser Ala Glu Gly Trp Lys Gly Gln Ser Val Glu Lys
                   245                 250                 255
   Leu Lys Pro Glu Lys Glu Leu Gln Arg Ala Ser Ser Glu Ile Ser Arg
               260                 265                 270
   Arg Lys Leu Xaa Ile Arg Asp Leu Phe Gln His Leu Asp Ser Leu Cys
           275                 280                 285
   Ala Glu Gly Arg Phe Pro Glu Ser Leu Phe Asp Ser Glu Gly Gln Ile
       290                 295                 300
   Asp Ser Glu Asp Ile Phe Cys Ala Lys Cys Glu Ser Lys Asp Met Ser
   305                 310                 315                 320
   Ala Asp Asn Asp Ile Ile Leu Cys Asp Gly Ala Cys Asp Arg Gly Phe
                   325                 330                 335
   His Gln Phe Cys Leu Glu Pro Pro Leu Leu Lys Glu Glu Ile Pro Pro
               340                 345                 350
   Asp Asp Glu Gly Trp Leu Cys Pro Ala Cys Asp Cys Lys Val Asp Cys
           355                 360                 365
   Met Asp Leu Leu Asn Asp Ser Gln Gly Thr Lys Leu Ser Val Ile Asp
       370                 375                 380
   Ser Trp Glu Lys Val Phe Pro Glu Ala Ala Ala Ala Gly Asn Asn Gln
   385                 390                 395                 400
   Asp Asn Asn Ser Gly Phe Ser Ser Asp Asp Ser Glu Asp Asn Asp Tyr
                   405                 410                 415
   Asp Pro Asp Cys Pro Glu Val Asp Glu Lys Gly Gln Gly Asp Lys Ser
               420                 425                 430
   Ser Ser Asp Lys Phe Asp Glu Ser Asp Glu Phe Asp Glu Ser Asp Glu
           435                 440                 445
   Ser Asp Phe Thr Ser Ala Ser Asp Asp Met Val Val Ser Pro Asn Asn
       450                 455                 460
   Glu Gln Cys Leu Gly Leu Pro Ser Asp Asp Ser Glu Asp Asp Asp Phe
   465                 470                 475                 480
   Asp Pro Asp Ala Pro Glu Ile Asp Glu Gln Val Asn Gln Gly Ser Ser
                   485                 490                 495
   Ser Ser Asp Phe Thr Ser Asp Ser Glu Asp Phe Thr Ala Thr Leu Asp
               500                 505                 510
   Arg Arg Asn Phe Ser Asp Asn Glu Asp Gly Leu Asp Glu Gln Arg Arg
           515                 520                 525
   Phe Gly Arg Lys Lys Lys Asp Thr Leu Lys Asp Glu Leu Leu Ser Val
       530                 535                 540
   Leu Glu Ser Asn Ser Gly Gln Asp Asn Ala Pro Leu Ser Ala Lys Arg
   545                 550                 555                 560
   His Val Glu Arg Leu Asp Tyr Lys Lys Leu His Asp Glu Ala Tyr Gly
   565 570 575
   Asn Val Ser Ser Asp Ser Ser Asp Asp Glu Asp Trp Thr Glu Asn Val
   580 585 590
   Ile Pro Arg Lys Arg Lys Asn Leu Ser Gly Asn Val Ala Ser Val Ser
   595 600 605
   Pro Asn Gly Asn Thr Ser Ile Thr Glu Asn Gly Thr Asn Thr Lys Asp
   610 615 620
   Ile Lys His Asp Leu Glu Ala Ala Gly Cys Thr Pro Lys Arg Arg Thr
   625 630 635 640
   Arg Gln Lys Leu Asn Phe Glu Ser Thr Asn Asn Ser Leu Ala Glu Ser
   645 650 655
   His Lys Gly Ser Arg Ser Pro Gly Ser Thr Gly Glu Lys Ser Gly Gln
   660 665 670
   Ser Ser Tyr Lys Lys Leu Gly Glu Ala Val Thr Glu Arg Leu Tyr Lys
   675 680 685
   Ser Phe Gln Glu Asn Gln Tyr Pro Asp Arg Ala Met Lys Glu Lys Leu
   690 695 700
   Ala Glu Glu Leu Gly Ile Thr Ser Arg Gln Val Ser Lys Trp Phe Glu
   705 710 715 720
   Asn Ala Arg Trp Ser Phe Arg His Arg Pro Pro Lys Glu Ala Ser Ala
   725 730 735
   Gly Lys Ser Ala Val Lys Lys Asp Ala Ser Thr Ser Gln Thr Asp Gln
   740 745 750
   Lys Pro Glu Gln Glu Val Val Leu Arg Glu Ser Ser His Asn Gly Val
   755 760 765
   Gly Lys Lys Glu Ser Pro Lys Ala Gly Ala Ser Lys Val Asp Arg Ser
   770 775 780
   Lys Glu Ala Asn Ala Gly Lys Ser Ala Val Lys Lys Asp Ala Ser Thr
   785 790 795 800
   Ser Gln Thr Asp Gln Lys Pro Glu Gln Glu Val Val Ile Lys Glu Ser
   805 810 815
   Ser His Asn Gly Val Gly Lys Lys Glu Ser Thr Lys Ala Gly Ala Ser
   820 825 830
   Lys Val Asp Arg Cys Ser Gly Ala Lys Arg Arg Arg Lys Leu Ala Thr
   835 840 845
   Asp Gly Ser His Arg Gln Lys Ser Ser Thr Pro Asn Ser Thr Arg Gln
       850                 855                 860
   Lys Thr Lys Ser Asn His Glu Ala Ser Glu Ala Thr Asn Gly Ser Arg
   865                 870                 875                 880
   Arg Gln Asn Ser Ser Thr Pro Lys Ser Arg Arg Gln Lys Thr Lys Leu
                   885                 890                 895
   Ala Gly Glu Ala Ser Glu Ala Thr Gly Gly Ser Ser Arg Gln Asn Ser
               900                 905                 910
   Ser Thr Pro Asn Ser Lys Arg Arg Lys Thr Lys Ser Asp His Glu Ala
           915                 920                 925
   Ser Asn Pro Val Leu Ser Gly Lys Lys Ile Ala Lys Thr Ala Lys Ser
       930                 935                 940
   Ser Ser Gly Thr Pro Lys Thr Lys Glu Lys Leu Ser Asp Arg Ile Gln
   945                 950                 955                 960
   Thr Arg Ser Arg Lys Ser Ile Ala
                   965
<210> 215
   <211> 2805
   <212> DNA
   <213> Populus tremuloides
<400> 215
   atgtctgagg ctgagaacat gggtgtttct ccatcacaag ttagttctca aactaaaagt     60
   tactcatgcc cttcacaaac taaactggaa aatactcatg gatttactgc tgaatacaac    120
   tgtggtggat attcggaaga gaaacataag cttgagtctg aaatcataca aactgaagcc    180
   ggagatagcg gtactgcagt tctccaatct ggtgctgggg aaactgtgga accatctact    240
   gaagatgtaa caaacaattc ttttactgat ttggatccac ctcccgaaga tgccaggggt    300
   gctacttttg atgaaagttc aagacctata ctaactgcta tagatcagaa actcgagcca    360
   ggtgctagaa gtgtgaatac tgcgtgtacg catggtgaat catcaaaagc aactgactct    420
   ggtattctac aggatgaacc tggaaatacc aatgctgcat catcaagctg cattgctaat    480
   gaaacctcac aagcatcact tgaaaatcta gccaataatt cttgtactga agatgtgggt    540
   ccgccgtatg gagatgcaag caagggtaac cagattgata aaagttcata ccctcaacaa    600
   actatatctg gacatacact tgagttactt tctgacagag cttgttgtga acgatcagaa    660
   gaaagacaga agcctggatc tgaactttca gaaaatgaat caacgggaat tgacactgaa    720
   ttatatagtg gcattgctat cgagaattca gagccgctta ctcaactagt gaccaagagt    780
   tctcctatca aacatgtagg cttgcttcct ggtgacagca tcatcattcc tgcaaatgaa    840
   caaacaagac caactcatga tgatgaggac aaaggcccag atcatgaaca tttggaaaca    900
   ccatctagag ttgcgattgg tattactaga cgcggtagac ctagaggtaa gagtgcttca    960
   agattgtcaa ggaagatata tatgttaaga tctttgagaa gtagtgatag agttctccgg   1020
   tcaagatcac aagagaaacc taaagctcct gaatcaagta ataattcagg taatgttaat   1080
   tccactggtg ataaaaaagg gaaaagaagg aaaaagagaa gaggaaagaa tattgtggct   1140
   gatgaatatt ccaaaatcag ggcacatctt aggtacttat tgaatcggat gagctatgag   1200
   caaagcttga tcactgctta ttctggggaa ggctggaaag gacttagcct agaaaagtta   1260
   aagcccgaga aggagttgca gcgagccaca tctgaaatta ccagacgcaa agtgaaaata   1320
   agggatttat ttcaacatat tgattctctg tgcagcgaag gaaggttccc atcatcttta   1380
   tttgattctg aaggacagat tgacagtgag gatgtattct gtgcaaaatg tgggtccaaa   1440
   gatttgaatg ctgataatga tattatacta tgtgatggtg cttgtgacag aggatttcat   1500
   cagttctgtt tgataccacc attgttaaga gaagacattc ctcctgatga tgagggctgg   1560
   ttatgccctg gatgtgattg caaagttgac tgcattggtt tgcttaacga ttctcaagga   1620
   acaaatatat ctatcagtga tagctgggag aaagttttcc ctgaggctgc cgccacagcg   1680
   tctggacaaa aactggatca caactttgga ccatcttctg atgattctga tgataatgat   1740
   tatgaacctg atggtccaga tattgataag aagagtcagg aggaggaatc gagctctgac   1800
   gaatctgact tcacttctgc atctgatgaa ttcaaggctc ccccggatgg caaagagtac   1860
   ttggggctct cttctgatga ttcagaggat gatgactatg atcctgatgc tccagttctt   1920
   gaggagaagc tgaagcagga aagttcaagt tctgatttta catcagactc tgaggatctt   1980
   gctgcaacta ttaatggtga tgggttgtct ttagaagatg aatgccacat gcctattgaa   2040
   cctcgtggag tttctaatgg gaggaaatct aaatttgatg gaaagaagat gcagtcttta   2100
   aacagcgagc ttttatccat gctagaacca gatctgtgcc aagatgagtc agcaactgtt   2160
   tctggaaaaa gaaatgttga cagattggac tacaagaagc tctatgatga aacgtatgga   2220
   aacatttcta cttcaagtga tgatgattac actgatactg ttgggccaag gaagaggagg   2280
   aaaaatactg gagatgttgc tacagtgaca gcaaacggag atgcctctgt taccgagaat   2340
   ggaatgaata gcaagaatat gaaccaggaa ttaaaggaga acaaacgtaa tcctgagaga   2400
   ggaacttgcc aaaactcgag ctttcaagaa acaaatgttt ccccggctaa atcatatgtt   2460
   ggtgcatctc tatctggttc tagtggtaaa agcgttaggc cctctgctta taaaaaactt   2520
   ggagaagctg taactcagag actgtacagt tacttcaggg aaaatcagta tccagaccga   2580
   gctgcaaaag caagcttggc agaagagcta ggaattactt ttgagcaggt taacaagtgg   2640
   tttgtgaatg cccgttggag cttcaaccat tcatcatcta caggtacaag taaagctgaa   2700
   agtgcttctg gaaaaggtag ctgtgatggc caagtgaggg atagtgaatc gaagaaccga   2760
   aagagcaata aacaaaaaac taacaccccg aaatctagga gataa                   2805
<210> 216
   <211> 934
   <212> PRT
   <213> Populus tremuloides
<400> 216
   Met Ser Glu Ala Glu Asn Met Gly Val Ser Pro Ser Gln Val Ser Ser
   1               5                   10                  15
   Gln Thr Lys Ser Tyr Ser Cys Pro Ser Gln Thr Lys Leu Glu Asn Thr
               20                  25                  30
   His Gly Phe Thr Ala Glu Tyr Asn Cys Gly Gly Tyr Ser Glu Glu Lys
           35                  40                  45
   His Lys Leu Glu Ser Glu Ile Ile Gln Thr Glu Ala Gly Asp Ser Gly
       50                  55                  60
   Thr Ala Val Leu Gln Ser Gly Ala Gly Glu Thr Val Glu Pro Ser Thr
   65                  70                  75                  80
   Glu Asp Val Thr Asn Asn Ser Phe Thr Asp Leu Asp Pro Pro Pro Glu
                   85                  90                  95
   Asp Ala Arg Gly Ala Thr Phe Asp Glu Ser Ser Arg Pro Ile Leu Thr
               100                 105                 110
   Ala Ile Asp Gln Lys Leu Glu Pro Gly Ala Arg Ser Val Asn Thr Ala
           115                 120                 125
   Cys Thr His Gly Glu Ser Ser Lys Ala Thr Asp Ser Gly Ile Leu Gln
       130                 135                 140
   Asp Glu Pro Gly Asn Thr Asn Ala Ala Ser Ser Ser Cys Ile Ala Asn
   145                 150                 155                 160
   Glu Thr Ser Gln Ala Ser Leu Glu Asn Leu Ala Asn Asn Ser Cys Thr
                   165                 170                 175
   Glu Asp Val Gly Pro Pro Tyr Gly Asp Ala Ser Lys Gly Asn Gln Ile
               180                 185                 190
   Asp Lys Ser Ser Tyr Pro Gln Gln Thr Ile Ser Gly His Thr Leu Glu
           195                 200                 205
   Leu Leu Ser Asp Arg Ala Cys Cys Glu Arg Ser Glu Glu Arg Gln Lys
       210                 215                 220
   Pro Gly Ser Glu Leu Ser Glu Asn Glu Ser Thr Gly Ile Asp Thr Glu
   225                 230                 235                 240
   Leu Tyr Ser Gly Ile Ala Ile Glu Asn Ser Glu Pro Leu Thr Gln Leu
                   245                 250                 255
   Val Thr Lys Ser Ser Pro Ile Lys His Val Gly Leu Leu Pro Gly Asp
               260                 265                 270
   Ser Ile Ile Ile Pro Ala Asn Glu Gln Thr Arg Pro Thr His Asp Asp
           275                 280                 285
   Glu Asp Lys Gly Pro Asp His Glu His Leu Glu Thr Pro Ser Arg Val
       290                 295                 300
   Ala Ile Gly Ile Thr Arg Arg Gly Arg Pro Arg Gly Lys Ser Ala Ser
   305                 310                 315                 320
   Arg Leu Ser Arg Lys Ile Tyr Met Leu Arg Ser Leu Arg Ser Ser Asp
                   325                 330                 335
   Arg Val Leu Arg Ser Arg Ser Gln Glu Lys Pro Lys Ala Pro Glu Ser
               340                 345                 350
   Ser Asn Asn Ser Gly Asn Val Asn Ser Thr Gly Asp Lys Lys Gly Lys
           355                 360                 365
   Arg Arg Lys Lys Arg Arg Gly Lys Asn Ile Val Ala Asp Glu Tyr Ser
       370                 375                 380
   Lys Ile Arg Ala His Leu Arg Tyr Leu Leu Asn Arg Met Ser Tyr Glu
   385                 390                 395                 400
   Gln Ser Leu Ile Thr Ala Tyr Ser Gly Glu Gly Trp Lys Gly Leu Ser
                   405                 410                 415
   Leu Glu Lys Leu Lys Pro Glu Lys Glu Leu Gln Arg Ala Thr Ser Glu
               420                 425                 430
   Ile Thr Arg Arg Lys Val Lys Ile Arg Asp Leu Phe Gln His Ile Asp
           435                 440                 445
   Ser Leu Cys Ser Glu Gly Arg Phe Pro Ser Ser Leu Phe Asp Ser Glu
       450                 455                 460
   Gly Gln Ile Asp Ser Glu Asp Val Phe Cys Ala Lys Cys Gly Ser Lys
   465                 470                 475                 480
   Asp Leu Asn Ala Asp Asn Asp Ile Ile Leu Cys Asp Gly Ala Cys Asp
                   485                 490                 495
   Arg Gly Phe His Gln Phe Cys Leu Ile Pro Pro Leu Leu Arg Glu Asp
               500                 505                 510
   Ile Pro Pro Asp Asp Glu Gly Trp Leu Cys Pro Gly Cys Asp Cys Lys
           515                 520                 525
   Val Asp Cys Ile Gly Leu Leu Asn Asp Ser Gln Gly Thr Asn Ile Ser
       530                 535                 540
   Ile Ser Asp Ser Trp Glu Lys Val Phe Pro Glu Ala Ala Ala Thr Ala
   545                 550                 555                 560
   Ser Gly Gln Lys Leu Asp His Asn Phe Gly Pro Ser Ser Asp Asp Ser
                   565                 570                 575
   Asp Asp Asn Asp Tyr Glu Pro Asp Gly Pro Asp Ile Asp Lys Lys Ser
               580                 585                 590
   Gln Glu Glu Glu Ser Ser Ser Asp Glu Ser Asp Phe Thr Ser Ala Ser
           595                 600                 605
   Asp Glu Phe Lys Ala Pro Pro Asp Gly Lys Glu Tyr Leu Gly Leu Ser
       610                 615                 620
   Ser Asp Asp Ser Glu Asp Asp Asp Tyr Asp Pro Asp Ala Pro Val Leu
   625                 630                 635                 640
   Glu Glu Lys Leu Lys Gln Glu Ser Ser Ser Ser Asp Phe Thr Ser Asp
                   645                 650                 655
   Ser Glu Asp Leu Ala Ala Thr Ile Asn Gly Asp Gly Leu Ser Leu Glu
               660                 665                 670
   Asp Glu Cys His Met Pro Ile Glu Pro Arg Gly Val Ser Asn Gly Arg
           675                 680                 685
   Lys Ser Lys Phe Asp Gly Lys Lys Met Gln Ser Leu Asn Ser Glu Leu
       690                 695                 700
   Leu Ser Met Leu Glu Pro Asp Leu Cys Gln Asp Glu Ser Ala Thr Val
   705                 710                 715                 720
   Ser Gly Lys Arg Asn Val Asp Arg Leu Asp Tyr Lys Lys Leu Tyr Asp
                   725                 730                 735
   Glu Thr Tyr Gly Asn Ile Ser Thr Ser Ser Asp Asp Asp Tyr Thr Asp
               740                 745                 750
   Thr Val Gly Pro Arg Lys Arg Arg Lys Asn Thr Gly Asp Val Ala Thr
           755                 760                 765
   Val Thr Ala Asn Gly Asp Ala Ser Val Thr Glu Asn Gly Met Asn Ser
       770                 775                 780
   Lys Asn Met Asn Gln Glu Leu Lys Glu Asn Lys Arg Asn Pro Glu Arg
   785                 790                 795                 800
   Gly Thr Cys Gln Asn Ser Ser Phe Gln Glu Thr Asn Val Ser Pro Ala
                   805                 810                 815
   Lys Ser Tyr Val Gly Ala Ser Leu Ser Gly Ser Ser Gly Lys Ser Val
   820 825 830
   Arg Pro Ser Ala Tyr Lys Lys Leu Gly Glu Ala Val Thr Gln Arg Leu
   835 840 845
   Tyr Ser Tyr Phe Arg Glu Asn Gln Tyr Pro Asp Arg Ala Ala Lys Ala
   850 855 860
   Ser Leu Ala Glu Glu Leu Gly Ile Thr Phe Glu Gln Val Asn Lys Trp
   865 870 875 880
   Phe Val Asn Ala Arg Trp Ser Phe Asn His Ser Ser Ser Thr Gly Thr
   885 890 895
   Ser Lys Ala Glu Ser Ala Ser Gly Lys Gly Ser Cys Asp Gly Gln Val
   900 905 910
   Arg Asp Ser Glu Ser Lys Asn Arg Lys Ser Asn Lys Gln Lys Thr Asn
   915 920 925
   Thr Pro Lys Ser Arg Arg
   930
<210> 217
   <211> 2793
   <212> DNA
   <213> Populus tremuloides
<400> 217
   atgtccgagg ctgagcacat gggtgtttct ccatcaaaag tttctcatac taaaagttat      60
   tcatgccctg cacaaactac actggaaaat acgcatgaac ccagtgctga atacaagttt     120
   ggtggatatc cggaagagag acataagctt gagtgtgaaa tcatacaaac tgaagctgga     180
   gataacaggg ctgcagttct ccaatcttgt tctggtgaag ttgtgcaacc atctactgac     240
   gatttaacaa aaagtcctct tattgatttg gacccacctc ctgacgatgc aaggagtgct     300
   ctgtttgata atagtccaag acctatatca accgctatgg atcagaaact tgagccaggt     360
   gctacaagtg tgaatactgc ttgtgtgcat agtgaatcat caaaagcaat tgactctagt     420
   attctactgg atgaacctag gaatagcaac acagaattgt caagctgcat tgcaaatgaa     480
   acctcacaag catcacttga aggtctagcc aatgattctc gtgctgaaga tgcaggactg     540
   tcacttgtag aggcaagcaa tagtgacctg attgatgaaa gttcatactc tcaacaaact     600
   acatctggac agacacgtga gtttcattct gacagagctt gttgtaaacc attggaagaa     660
   agacagaagc ccggctctga acttgcagaa aatgaatcaa tggaaattgg cattggatta     720
   cccagtggta ttgctattga gaatttggag ccgcttactg aacttgtgac caagagttgt     780
   cctatcaaac atataggctt gcctcctggt gatgacatca gcattcctgc aaatgaacaa     840
   ataagaccta ctcatgataa ggagtccaaa taccctgatt gtgaacattt ggaaaaacta     900
   tctggaattg tgattggtat tactagtcaa ggtgtaccca gtgttaagag aacttcaaaa     960
   ttgtcaggga agaaatatac aagttcttcg agaaaaagtg atagagttct ccggtcaaat    1020
   tcacaagaga aacctaaagc tcccgagccg agtaataatt ctactaatgt taattccact    1080
   ggtgaggaaa aagggaaaag gaggaaaaag agaagaggga aaagtatagt ggctgatgaa    1140
   tattccagaa tcagggcacg tcttaggtac ttgttgaatc ggatgagcta tgagcaaagc    1200
   ttgatcactg cttattccgg ggaaggctgg aaaggactta gtctagaaaa gttaaaacct    1260
   gagaaggagt tgcaacgggc cacatctgaa atcatcagac gcaaagtgaa aataagggat    1320
   ttatttcaac atattgattc actatgcggt gaaggaaggt tcccagcatc tttgtttgat    1380
   tctgaaggac aaattgacag tgaggatata ttctgtgcaa aatgtggatc caaagatttg    1440
   actgctgata atgatattat actatgtgat ggtgcttgcg accgagggtt tcatcaattc    1500
   tgtttggtac caccattgtt aagagaagac attcctcctg gtgatgaggg ctggttatgc    1560
   cctggatgtg attgcaaagt tgactgcatt gacttgctta atgactctca aggaacaaat    1620
   atatctatca gtgacagatg ggataatgtt ttccccgagg cagctgcagt agcatctgga    1680
   caaaaactcg actacaactt tggattgtct tctgatgatt ctgatgataa tgattatgat    1740
   cctgatggtc cagatattga cgagaagagt caggaggaat caagctctga cgaatctgac    1800
   ttcagttctg cgtctgatga atttgaggct ccacctgatg ataaacagta cttggggctc    1860
   ccttctgatg attcagagga tgatgactat gatcctgatg ctccagttct cgaggagaag    1920
   ctgaagcagg aaagttccag ttctgatttt acttcggact ctgaggatct tgatgccact    1980
   cttaatggtg atgggttgtc tttaggagat gaataccaca tgcctattga acctcatgaa    2040
   gattctaatg ggcgaagatc gagatttggt ggaaagaaga atcattcttt aaacagcaag    2100
   cttttatcca tgctagaacc agattctcac caagaaaagt ctgcacctgt ttctggaaaa    2160
   agaaatattg aaagattgga ctacaagaag ctctatgatg aaacatatgg aaacatttgt    2220
   acttcaagtg atgatgattt cactgatact gttgcaccaa ggaagaggag gaaaaacact    2280
   ggagatgttg ctatggggat agcaaatgga gatgcttctg ttactgagaa tggattgaat    2340
   agcaagaata tgaatcagga attaaagaag aatgaacata cttctgggag aactcaccaa    2400
   aactcgagct ttcaagatac aaatgtttcc ccagcaaaaa cacatgttgg cgaatctctc    2460
   tctggttcta gcagtaaaag agttaggccc tctgcttata aaaaacttgg agaagctgta    2520
   actcagaaac tgtacagttt cttcaaggaa aatcggtatc ctgaccaagc tgcaaaggca    2580
   agtttggcag aagagctagg aattactttt gagcaggtta acaagtggtt catgaatgct    2640
   cgttggagct tcaaccattc atcacctgaa ggtacaagta aagctgaaag tgcttcagga    2700
   aagggtagct gtgatgggca tgtgagggat agtgaatcga agaaccaaaa gagcaataaa    2760
   caaaaaacta gtaccccaaa atctaggaga tag                                 2793
<210> 218
   <211> 930
   <212> PRT
   <213> Populus tremuloides

<400> 218
   Met Ser Glu Ala Glu His Met Gly Val Ser Pro Ser Lys Val Ser His
   1               5                   10                  15
   Thr Lys Ser Tyr Ser Cys Pro Ala Gln Thr Thr Leu Glu Asn Thr His
               20                  25                  30
   Glu Pro Ser Ala Glu Tyr Lys Phe Gly Gly Tyr Pro Glu Glu Arg His
           35                  40                  45
   Lys Leu Glu Cys Glu Ile Ile Gln Thr Glu Ala Gly Asp Asn Arg Ala
       50                  55                  60
   Ala Val Leu Gln Ser Cys Ser Gly Glu Val Val Gln Pro Ser Thr Asp
   65                  70                  75                  80
   Asp Leu Thr Lys Ser Pro Leu Ile Asp Leu Asp Pro Pro Pro Asp Asp
                   85                  90                  95
   Ala Arg Ser Ala Leu Phe Asp Asn Ser Pro Arg Pro Ile Ser Thr Ala
               100                 105                 110
   Met Asp Gln Lys Leu Glu Pro Gly Ala Thr Ser Val Asn Thr Ala Cys
           115                 120                 125
   Val His Ser Glu Ser Ser Lys Ala Ile Asp Ser Ser Ile Leu Leu Asp
       130                 135                 140
   Glu Pro Arg Asn Ser Asn Thr Glu Leu Ser Ser Cys Ile Ala Asn Glu
   145                 150                 155                 160
   Thr Ser Gln Ala Ser Leu Glu Gly Leu Ala Asn Asp Ser Arg Ala Glu
                   165                 170                 175
   Asp Ala Gly Leu Ser Leu Val Glu Ala Ser Asn Ser Asp Leu Ile Asp
               180                 185                 190
   Glu Ser Ser Tyr Ser Gln Gln Thr Thr Ser Gly Gln Thr Arg Glu Phe
           195                 200                 205
   His Ser Asp Arg Ala Cys Cys Lys Pro Leu Glu Glu Arg Gln Lys Pro
       210                 215                 220
   Gly Ser Glu Leu Ala Glu Asn Glu Ser Met Glu Ile Gly Ile Gly Leu
   225                 230                 235                 240
   Pro Ser Gly Ile Ala Ile Glu Asn Leu Glu Pro Leu Thr Glu Leu Val
                   245                 250                 255
   Thr Lys Ser Cys Pro Ile Lys His Ile Gly Leu Pro Pro Gly Asp Asp
               260                 265                 270
   Ile Ser Ile Pro Ala Asn Glu Gln Ile Arg Pro Thr His Asp Lys Glu
           275                 280                 285
   Ser Lys Tyr Pro Asp Cys Glu His Leu Glu Lys Leu Ser Gly Ile Val
       290                 295                 300
   Ile Gly Ile Thr Ser Gln Gly Val Pro Ser Val Lys Arg Thr Ser Lys
   305                 310                 315                 320
   Leu Ser Gly Lys Lys Tyr Thr Ser Ser Ser Arg Lys Ser Asp Arg Val
                   325                 330                 335
   Leu Arg Ser Asn Ser Gln Glu Lys Pro Lys Ala Pro Glu Pro Ser Asn
               340                 345                 350
   Asn Ser Thr Asn Val Asn Ser Thr Gly Glu Glu Lys Gly Lys Arg Arg
           355                 360                 365
   Lys Lys Arg Arg Gly Lys Ser Ile Val Ala Asp Glu Tyr Ser Arg Ile
       370                 375                 380
   Arg Ala Arg Leu Arg Tyr Leu Leu Asn Arg Met Ser Tyr Glu Gln Ser
   385                 390                 395                 400
   Leu Ile Thr Ala Tyr Ser Gly Glu Gly Trp Lys Gly Leu Ser Leu Glu
                   405                 410                 415
   Lys Leu Lys Pro Glu Lys Glu Leu Gln Arg Ala Thr Ser Glu Ile Ile
               420                 425                 430
   Arg Arg Lys Val Lys Ile Arg Asp Leu Phe Gln His Ile Asp Ser Leu
           435                 440                 445
   Cys Gly Glu Gly Arg Phe Pro Ala Ser Leu Phe Asp Ser Glu Gly Gln
       450                 455                 460
   Ile Asp Ser Glu Asp Ile Phe Cys Ala Lys Cys Gly Ser Lys Asp Leu
   465                 470                 475                 480
   Thr Ala Asp Asn Asp Ile Ile Leu Cys Asp Gly Ala Cys Asp Arg Gly
                   485                 490                 495
   Phe His Gln Phe Cys Leu Val Pro Pro Leu Leu Arg Glu Asp Ile Pro
               500                 505                 510
   Pro Gly Asp Glu Gly Trp Leu Cys Pro Gly Cys Asp Cys Lys Val Asp
           515                 520                 525
   Cys Ile Asp Leu Leu Asn Asp Ser Gln Gly Thr Asn Ile Ser Ile Ser
       530                 535                 540
   Asp Arg Trp Asp Asn Val Phe Pro Glu Ala Ala Ala Val Ala Ser Gly
   545 550 555 560
   Gln Lys Leu Asp Tyr Asn Phe Gly Leu Ser Ser Asp Asp Ser Asp Asp
   565 570 575
   Asn Asp Tyr Asp Pro Asp Gly Pro Asp Ile Asp Glu Lys Ser Gln Glu
   580 585 590
   Glu Ser Ser Ser Asp Glu Ser Asp Phe Ser Ser Ala Ser Asp Glu Phe
   595 600 605
   Glu Ala Pro Pro Asp Asp Lys Gln Tyr Leu Gly Leu Pro Ser Asp Asp
   610 615 620
   Ser Glu Asp Asp Asp Tyr Asp Pro Asp Ala Pro Val Leu Glu Glu Lys
   625 630 635 640
   Leu Lys Gln Glu Ser Ser Ser Ser Asp Phe Thr Ser Asp Ser Glu Asp
   645 650 655
   Leu Asp Ala Thr Leu Asn Gly Asp Gly Leu Ser Leu Gly Asp Glu Tyr
   660 665 670
   His Met Pro Ile Glu Pro His Glu Asp Ser Asn Gly Arg Arg Ser Arg
   675 680 685
   Phe Gly Gly Lys Lys Asn His Ser Leu Asn Ser Lys Leu Leu Ser Met
   690 695 700
   Leu Glu Pro Asp Ser His Gln Glu Lys Ser Ala Pro Val Ser Gly Lys
   705 710 715 720
   Arg Asn Ile Glu Arg Leu Asp Tyr Lys Lys Leu Tyr Asp Glu Thr Tyr
   725 730 735
   Gly Asn Ile Cys Thr Ser Ser Asp Asp Asp Phe Thr Asp Thr Val Ala
   740 745 750
   Pro Arg Lys Arg Arg Lys Asn Thr Gly Asp Val Ala Met Gly Ile Ala
   755 760 765
   Asn Gly Asp Ala Ser Val Thr Glu Asn Gly Leu Asn Ser Lys Asn Met
   770 775 780
   Asn Gln Glu Leu Lys Lys Asn Glu His Thr Ser Gly Arg Thr His Gln
   785 790 795 800
   Asn Ser Ser Phe Gln Asp Thr Asn Val Ser Pro Ala Lys Thr His Val
   805 810 815
   Gly Glu Ser Leu Ser Gly Ser Ser Ser Lys Arg Val Arg Pro Ser Ala
               820                 825                 830
   Tyr Lys Lys Leu Gly Glu Ala Val Thr Gln Lys Leu Tyr Ser Phe Phe
           835                 840                 845
   Lys Glu Asn Arg Tyr Pro Asp Gln Ala Ala Lys Ala Ser Leu Ala Glu
       850                 855                 860
   Glu Leu Gly Ile Thr Phe Glu Gln Val Asn Lys Trp Phe Met Asn Ala
   865                 870                 875                 880
   Arg Trp Ser Phe Asn His Ser Ser Pro Glu Gly Thr Ser Lys Ala Glu
                   885                 890                 895
   Ser Ala Ser Gly Lys Gly Ser Cys Asp Gly His Val Arg Asp Ser Glu
               900                 905                 910
   Ser Lys Asn Gln Lys Ser Asn Lys Gln Lys Thr Ser Thr Pro Lys Ser
           915                 920                 925
   Arg Arg
       930
<210> 219
   <211> 2184
   <212> DNA
   <213> Populus tremuloides
<400> 219
   atgggtgatt ctggaaagaa atcaaagcag caagacttgc acgaatcttc accatctgac 60
   acagtgaatg ggtcgttgct gattaaatca ttgaagataa agaagggtgg caaattatct 120
   catagaaaaa gcgaaaaacc taaaactaaa cctcatctga aaactatcat taattcatct 180
   gtatcaaaga aaaaggttac tcctaagaag ggcatcagga atggctctac cagtagaaga 240
   ttgattcaca ggaaaattct gcataaagca cttgataaga aggcctcaag aaatggggct 300
   tcctcagagc tccaaggcaa acagttgtca actattgatt ctgaggggaa tggaaaaaat 360
   gccgatgaag gtgcaattaa aaaagtcaag aagaggaagc ctaagaaaag gcaaaaggac 420
   aaggtaaagc tagatgaacc accacgcttg cagaggagag caaggtactt gatgattaaa 480
   atgaagctgg agcagaatct tatagatgct tactctggag aaggttggaa aggtaaaagt 540
   cgagagaaga ttcggccaga aaaggaacta ctgagagcca ggaaacagat tttgaaatgt 600
   aaacttggat tacgtgaaat aattcggcag gtggattctc taagtacagt aggatgtatt 660
   gaagatgctg ttatggctcc agatggatct gtttcccatg aacatatatt ctgtgcgaag 720
   tgcaaattga atgaagtttc ccaagataat gatattgtac tctgtgatgg gacatgcaac 780
   tgtgccttcc accaaaaatg ccttgatcct cctttggata ctgaaaatat acctccagga 840
   gatcagggat ggttttgcaa gttctgtgac tgtaggatgg aaattataga agccatgaat 900
   gctcatttgg ggacccactt ctcagaggac agcggttggc aggatatttt caaagaagaa 960
   gcagcagttc cagatggtgg aaatatgcta ttaaatccag aagaagaatg gccttctgat 1020
   gattctgaag atgatgatta tgatccagag aggagagaga acgtcatgag tggagcaggt 1080
   actgatgatg atgcatctga tgacaccagc aattcaaccc gcttgagttg gtcttcagat 1140
   ggtgaggttt tttcaggatc caggaggtgg gaggtggacg gcttggactt cagaaacaat 1200
   tctatttata gcagtttaga ttctgatgaa accagtgatg gggaaattat ttgtggccgt 1260
   aggcagagaa gagcggttga ttataagaag ttatacaatg agatgtttgg aaaggacgct 1320
   cctgcacatg agcaacccag cgaagatgaa gattggggtc ctagcaaaag aaagcgacga 1380
   gagaaggagt cagatgcagc cagcacccta atgactctat atgaaagtaa aagaagatgt 1440
   aaaaatgatg caaccattga aggcatgatg aagcttccac gagatcctca aattcgaagg 1500
   ccaattttca ggctcccccc tgatgctgtt gagaaacttc gccaagtatt tgcagagaat 1560
   gaacttccat ctagaactgt caaggagaat ctatcaaaag aattgggcct tgaacctggg 1620
   aaggttagca aatggttcaa gaattcccgt tatttagctc tgaagtctag aaaggtagag 1680
   aagggagaac aagttcatta ttctagttcc aaagtctctg ctgaacccac tttaaatgtc 1740
   atgaaggaca aaactgctga tctttcactg ttaaaggata gccaggcaga aactggggtg 1800
   tgtaccccag agaatttgaa aaggatcttg cagaggaaga agccgaggtc aataagtaaa 1860
   agtttaaaga aaaatgaaca gaaaagaggt tccttcgaat cacctactaa aagcaatgag 1920
   atgaatgttg agcacaatga tgatctgagc ttgaagaagc tgttaaaagc aaaaacaaag 1980
   ggagtaaaga aaaagggtaa tcgtatttca gcagctgctg aatctgatat ggagaaactt 2040
   tgtagagcaa agacgagagt agagaatctg aagcagaaac tggtgaaact acaaactggc 2100
   aaagctagga agtcttccaa aatccgtccg ctagacgaat ctgttgtcta cgttcctatt 2160
   gctgagctaa gagaaaagaa atga 2184
<210> 220
   <211> 727
   <212> PRT
   <213> Populus tremuloides
<400> 220
   Met Gly Asp Ser Gly Lys Lys Ser Lys Gln Gln Asp Leu His Glu Ser
   1               5                   10                  15
   Ser Pro Ser Asp Thr Val Asn Gly Ser Leu Leu Ile Lys Ser Leu Lys
               20                  25                  30
   Ile Lys Lys Gly Gly Lys Leu Ser His Arg Lys Ser Glu Lys Pro Lys
           35                  40                  45
   Thr Lys Pro His Leu Lys Thr Ile Ile Asn Ser Ser Val Ser Lys Lys
       50                  55                  60
   Lys Val Thr Pro Lys Lys Gly Ile Arg Asn Gly Ser Thr Ser Arg Arg
   65                  70                  75                  80
   Leu Ile His Arg Lys Ile Leu His Lys Ala Leu Asp Lys Lys Ala Ser
   85 90 95
   Arg Asn Gly Ala Ser Ser Glu Leu Gln Gly Lys Gln Leu Ser Thr Ile
   100 105 110
   Asp Ser Glu Gly Asn Gly Lys Asn Ala Asp Glu Gly Ala Ile Lys Lys
   115 120 125
   Val Lys Lys Arg Lys Pro Lys Lys Arg Gln Lys Asp Lys Val Lys Leu
   130 135 140
   Asp Glu Pro Pro Arg Leu Gln Arg Arg Ala Arg Tyr Leu Met Ile Lys
   145 150 155 160
   Met Lys Leu Glu Gln Asn Leu Ile Asp Ala Tyr Ser Gly Glu Gly Trp
   165 170 175
   Lys Gly Lys Ser Arg Glu Lys Ile Arg Pro Glu Lys Glu Leu Leu Arg
   180 185 190
   Ala Arg Lys Gln Ile Leu Lys Cys Lys Leu Gly Leu Arg Glu Ile Ile
   195 200 205
   Arg Gln Val Asp Ser Leu Ser Thr Val Gly Cys Ile Glu Asp Ala Val
   210 215 220
   Met Ala Pro Asp Gly Ser Val Ser His Glu His Ile Phe Cys Ala Lys
   225 230 235 240
   Cys Lys Leu Asn Glu Val Ser Gln Asp Asn Asp Ile Val Leu Cys Asp
   245 250 255
   Gly Thr Cys Asn Cys Ala Phe His Gln Lys Cys Leu Asp Pro Pro Leu
   260 265 270
   Asp Thr Glu Asn Ile Pro Pro Gly Asp Gln Gly Trp Phe Cys Lys Phe
   275 280 285
   Cys Asp Cys Arg Met Glu Ile Ile Glu Ala Met Asn Ala His Leu Gly
   290 295 300
   Thr His Phe Ser Glu Asp Ser Gly Trp Gln Asp Ile Phe Lys Glu Glu
   305 310 315 320
   Ala Ala Val Pro Asp Gly Gly Asn Met Leu Leu Asn Pro Glu Glu Glu
   325 330 335
   Trp Pro Ser Asp Asp Ser Glu Asp Asp Asp Tyr Asp Pro Glu Arg Arg
   340 345 350
   Glu Asn Val Met Ser Gly Ala Gly Thr Asp Asp Asp Ala Ser Asp Asp
           355                 360                 365
   Thr Ser Asn Ser Thr Arg Leu Ser Trp Ser Ser Asp Gly Glu Val Phe
       370                 375                 380
   Ser Gly Ser Arg Arg Trp Glu Val Asp Gly Leu Asp Phe Arg Asn Asn
   385                 390                 395                 400
   Ser Ile Tyr Ser Ser Leu Asp Ser Asp Glu Thr Ser Asp Gly Glu Ile
                   405                 410                 415
   Ile Cys Gly Arg Arg Gln Arg Arg Ala Val Asp Tyr Lys Lys Leu Tyr
               420                 425                 430
   Asn Glu Met Phe Gly Lys Asp Ala Pro Ala His Glu Gln Pro Ser Glu
           435                 440                 445
   Asp Glu Asp Trp Gly Pro Ser Lys Arg Lys Arg Arg Glu Lys Glu Ser
       450                 455                 460
   Asp Ala Ala Ser Thr Leu Met Thr Leu Tyr Glu Ser Lys Arg Arg Cys
   465                 470                 475                 480
   Lys Asn Asp Ala Thr Ile Glu Gly Met Met Lys Leu Pro Arg Asp Pro
                   485                 490                 495
   Gln Ile Arg Arg Pro Ile Phe Arg Leu Pro Pro Asp Ala Val Glu Lys
               500                 505                 510
   Leu Arg Gln Val Phe Ala Glu Asn Glu Leu Pro Ser Arg Thr Val Lys
           515                 520                 525
   Glu Asn Leu Ser Lys Glu Leu Gly Leu Glu Pro Gly Lys Val Ser Lys
       530                 535                 540
   Trp Phe Lys Asn Ser Arg Tyr Leu Ala Leu Lys Ser Arg Lys Val Glu
   545                 550                 555                 560
   Lys Gly Glu Gln Val His Tyr Ser Ser Ser Lys Val Ser Ala Glu Pro
                   565                 570                 575
   Thr Leu Asn Val Met Lys Asp Lys Thr Ala Asp Leu Ser Leu Leu Lys
               580                 585                 590
   Asp Ser Gln Ala Glu Thr Gly Val Cys Thr Pro Glu Asn Leu Lys Arg
           595                 600                 605
   Ile Leu Gln Arg Lys Lys Pro Arg Ser Ile Ser Lys Ser Leu Lys Lys
       610                 615                 620
   Asn Glu Gln Lys Arg Gly Ser Phe Glu Ser Pro Thr Lys Ser Asn Glu
   625                 630                 635                 640
   Met Asn Val Glu His Asn Asp Asp Leu Ser Leu Lys Lys Leu Leu Lys
                   645                 650                 655
   Ala Lys Thr Lys Gly Val Lys Lys Lys Gly Asn Arg Ile Ser Ala Ala
               660                 665                 670
   Ala Glu Ser Asp Met Glu Lys Leu Cys Arg Ala Lys Thr Arg Val Glu
           675                 680                 685
   Asn Leu Lys Gln Lys Leu Val Lys Leu Gln Thr Gly Lys Ala Arg Lys
       690                 695                 700
   Ser Ser Lys Ile Arg Pro Leu Asp Glu Ser Val Val Tyr Val Pro Ile
   705                 710                 715                 720
   Ala Glu Leu Arg Glu Lys Lys
                   725
<210> 221
   <211> 2460
   <212> DNA
   <213> Ostreococcus tauri
<220>
   <221> misc_feature
   <222> (178)..(178)
   <223> n is a, c, g, or t
<220>
   <221> misc_feature
   <222> (214)..(214)
   <223> n is a, c, g, or t
<220>
   <221> misc_feature
   <222> (1375)..(1375)
   <223> n is a, c, g, or t
<220>
   <221> misc_feature
   <222> (1381)..(1381)
   <223> n is a, c, g, or t
<220>
   <221> misc_feature
   <222> (1648)..(1648)
   <223> n is a, c, g, or t
<220>
   <221> misc_feature
   <222> (1684)..(1684)
   <223> n is a, c, g, or t
<400> 221
   atgaatgagg tgcgcgacgc ggaggaacga tgtaaacgtc gtgtgcgagc ggtcgcggac 60
   gcggtgcgga agggaggcgt ggtgtgggcg atcgtggggt ggaaagatag tcaatgcgcg 120
   cgcgcggcgt ccgacgcgcg cgttgttgga gattgggcgc gcgcgatgtg tgtgaganga 180
   ctgacgatgc atcgatcggt ggttgatgat acgnagtttg cgctcgcgcg atcgcggata 240
   cagtcgcagt tcgcgacgat tcgaagacat caggcgttgg tggaagcgta cgcgagcgac 300
   ggctggcgcg gacaggcggc tcaaaagccg aaaccagtca gggagattga aaaggcgcgc 360
   gagaagatat tcgagggtaa attgaagata cgggagtatt ttaaggtttt ggagttcgat 420
   gagcgcgagc gcgagatcac gacggtggcg gacgagtttg gggagtgcga cgccgcggat 480
   attttttgta gcaagtgcac cctcgcagac gacaggcatg acgacgatat tttgctctgc 540
   gatggcttct gcgatcgtgc gtatcatcaa tcatgcgtgg ctccaccggt tttggcggaa 600
   gacattccgc ccgaggacga aggttggctc tgtccgagat gcgacgcgcg agtggacgtc 660
   atttacgtct tgaacgacga gtacgatcaa aatttgggcc agaggtgtgt ttcagcggat 720
   attttcgttg cagaggcgga catgcgagat aaagggatcg ttccaggaac ggcgcagttc 780
   aaacacgcgc acgaagagga ttggccgagc gatgagagcg atgacgagga cttcgatcag 840
   ggcggacaca gcgacgatgg gcgagacgac gagcacgagg cgctgagcgg aagcgcacag 900
   tcatcgagcg atgaatcgag cagcgaatca gagtctgatc tcatcatcga gggcccacga 960
   aggcgcacga aggttgatta cgtcgccctg aacaacgcca tgttcggcga cagcgaggcg 1020
   tacgagggcg aagctgagga gctcggttgg aaacgcagca acaaaaccaa ggcggagatg 1080
   ctcgcggcgc tcaagggtga gagcgcgaac gggataacga acggtgccac gaagaaggat 1140
   ccaaaatcaa aacgctcgaa gcgagagcgc gtggatgaga aatctcccgc accgcgcgtc 1200
   aggttcactc ccgatcaaaa acttgagctc gagcgtgtct tctccgagtg cgcgtcgctc 1260
   gacattgacg cgcgcgacgc cctcgcgaag cgactcggga tacccggcgg cgcaaattcg 1320
   attaaaatat ggttcatgaa tcgccgtcgt agggctaaga ttttgggtga gaggnaatac 1380
   ngacagcacg ataatccacg tcccgctcgc tcgttcgacg cctcgtcaca aacaacctct 1440
   gaaaaacctt caagtcacct cagaagctca cgcgcgtctc aaacatgtcg atcgccccta 1500
   aacgcgcccc caaactcata tacttctccg cgtggttctg cccgttcgcc catcgagcga 1560
   cgctcgcgtt ggagcattac gcgcacggcg gagagatttc ctacgagtgg aaggaatcgc 1620
   tcggtgaggt cacgcttcgt tgacattnga cgctcgaatg cgacgaaact tgagcgcctc 1680
   gacngaccgg agacgaatcg ccgcgtagga tgggaaaaac gcaagtcaac ctcgagtgag 1740
   gtgaagacga aacacgagaa ttggtaccat tacaagtccc cagagctact gagacacaac 1800
   ccgctgggca tggtgccgac actcgtgacg ccggcgacgt atgaggataa agatggagat 1860
   ccatcgcgga acgtggtgac ggagagcttg gtgtgcgtac agtttatcga tgaactcgtt 1920
   cggggaaacg cgtacgccgg ggagacggcg gcgatcatgc cgagcgaccc gtacgcgcgc 1980
   gcgaaggcga gagtggacgc cgactgggtg aacaaaaacg tatgctcgag gtattatcac 2040
   gtcctggtgc gacaagaggc gagcgagcag agagaagcgt ttgaaaagct cgtggagggg 2100
   ctggagacat tcgcggcgtg gtgcggcagc ggagaaggga agttctatgg tggacaaacg 2160
   acgcccggat tggtggacta cgcgctcttt ccatgggcgt ggcgcctccc ggtgttcgag 2220
   cactaccgcg ggatggattt caagattcct cgcaccgcgg cactgaaatc gtatcacgat   2280
   tggatggaag ccatgctcgc gcgagagcaa gttcgcaaga cgcttccacc gtgggacgat   2340
   tacttggaac acattggacg atacgccgac gggagcgcgc gatcgaaagt cgccaacgca   2400
   gtccgcagcg gccgtgcggc gcacgattac gacgacaagg aggacaacac cgatagttga   2460
<210> 222
   <211> 819
   <212> PRT
   <213> Ostreococcus tauri
<220>
   <221> UNSURE
   <222> (60)..(60)
   <223> Xaa can be any naturally occurring amino acid
<220>
   <221> UNSURE
   <222> (72)..(72)
   <223> Xaa can be any naturally occurring amino acid
<220>
   <221> UNSURE
   <222> (459)..(459)
   <223> Xaa can be any naturally occurring amino acid
<220>
   <221> UNSURE
   <222> (461)..(461)
   <223> Xaa can be any naturally occurring amino acid
<220>
   <221> UNSURE
   <222> (550)..(550)
   <223> Xaa can be any naturally occurring amino acid
<220>
   <221> UNSURE
   <222> (562)..(562)
   <223> Xaa can be any naturally occurring amino acid
<400> 222
   Met Asn Glu Val Arg Asp Ala Glu Glu Arg Cys Lys Arg Arg Val Arg
   1               5                   10                  15
   Ala Val Ala Asp Ala Val Arg Lys Gly Gly Val Val Trp Ala Ile Val
               20                  25                  30
   Gly Trp Lys Asp Ser Gln Cys Ala Arg Ala Ala Ser Asp Ala Arg Val
           35                  40                  45
   Val Gly Asp Trp Ala Arg Ala Met Cys Val Arg Xaa Leu Thr Met His
       50                  55                  60
   Arg Ser Val Val Asp Asp Thr Xaa Phe Ala Leu Ala Arg Ser Arg Ile
   65                  70                  75                  80
   Gln Ser Gln Phe Ala Thr Ile Arg Arg His Gln Ala Leu Val Glu Ala
   85 90 95
   Tyr Ala Ser Asp Gly Trp Arg Gly Gln Ala Ala Gln Lys Pro Lys Pro
   100 105 110
   Val Arg Glu Ile Glu Lys Ala Arg Glu Lys Ile Phe Glu Gly Lys Leu
   115 120 125
   Lys Ile Arg Glu Tyr Phe Lys Val Leu Glu Phe Asp Glu Arg Glu Arg
   130 135 140
   Glu Ile Thr Thr Val Ala Asp Glu Phe Gly Glu Cys Asp Ala Ala Asp
   145 150 155 160
   Ile Phe Cys Ser Lys Cys Thr Leu Ala Asp Asp Arg His Asp Asp Asp
   165 170 175
   Ile Leu Leu Cys Asp Gly Phe Cys Asp Arg Ala Tyr His Gln Ser Cys
   180 185 190
   Val Ala Pro Pro Val Leu Ala Glu Asp Ile Pro Pro Glu Asp Glu Gly
   195 200 205
   Trp Leu Cys Pro Arg Cys Asp Ala Arg Val Asp Val Ile Tyr Val Leu
   210 215 220
   Asn Asp Glu Tyr Asp Gln Asn Leu Gly Gln Arg Cys Val Ser Ala Asp
   225 230 235 240
   Ile Phe Val Ala Glu Ala Asp Met Arg Asp Lys Gly Ile Val Pro Gly
   245 250 255
   Thr Ala Gln Phe Lys His Ala His Glu Glu Asp Trp Pro Ser Asp Glu
   260 265 270
   Ser Asp Asp Glu Asp Phe Asp Gln Gly Gly His Ser Asp Asp Gly Arg
   275 280 285
   Asp Asp Glu His Glu Ala Leu Ser Gly Ser Ala Gln Ser Ser Ser Asp
   290 295 300
   Glu Ser Ser Ser Glu Ser Glu Ser Asp Leu Ile Ile Glu Gly Pro Arg
   305 310 315 320
   Arg Arg Thr Lys Val Asp Tyr Val Ala Leu Asn Asn Ala Met Phe Gly
   325 330 335
   Asp Ser Glu Ala Tyr Glu Gly Glu Ala Glu Glu Leu Gly Trp Lys Arg
   340 345 350
   Ser Asn Lys Thr Lys Ala Glu Met Leu Ala Ala Leu Lys Gly Glu Ser
   355 360 365
   Ala Asn Gly Ile Thr Asn Gly Ala Thr Lys Lys Asp Pro Lys Ser Lys
   370 375 380
   Arg Ser Lys Arg Glu Arg Val Asp Glu Lys Ser Pro Ala Pro Arg Val
   385 390 395 400
   Arg Phe Thr Pro Asp Gln Lys Leu Glu Leu Glu Arg Val Phe Ser Glu
   405 410 415
   Cys Ala Ser Leu Asp Ile Asp Ala Arg Asp Ala Leu Ala Lys Arg Leu
   420 425 430
   Gly Ile Pro Gly Gly Ala Asn Ser Ile Lys Ile Trp Phe Met Asn Arg
   435 440 445
   Arg Arg Arg Ala Lys Ile Leu Gly Glu Arg Xaa Tyr Xaa Gln His Asp
   450 455 460
   Asn Pro Arg Pro Ala Arg Ser Phe Asp Ala Ser Ser Gln Thr Thr Ser
   465 470 475 480
   Glu Lys Pro Ser Ser His Leu Arg Ser Ser Arg Ala Ser Gln Thr Cys
   485 490 495
   Arg Ser Pro Leu Asn Ala Pro Pro Asn Ser Tyr Thr Ser Pro Arg Gly
   500 505 510
   Ser Ala Arg Ser Pro Ile Glu Arg Arg Ser Arg Trp Ser Ile Thr Arg
   515 520 525
   Thr Ala Glu Arg Phe Pro Thr Ser Gly Arg Asn Arg Ser Val Arg Ser
   530 535 540
   Arg Phe Val Asp Ile Xaa Arg Ser Asn Ala Thr Lys Leu Glu Arg Leu
   545 550 555 560
   Asp Xaa Pro Glu Thr Asn Arg Arg Val Gly Trp Glu Lys Arg Lys Ser
   565 570 575
   Thr Ser Ser Glu Val Lys Thr Lys His Glu Asn Trp Tyr His Tyr Lys
   580 585 590
   Ser Pro Glu Leu Leu Arg His Asn Pro Leu Gly Met Val Pro Thr Leu
   595 600 605
   Val Thr Pro Ala Thr Tyr Glu Asp Lys Asp Gly Asp Pro Ser Arg Asn
   610 615 620
   Val Val Thr Glu Ser Leu Val Cys Val Gln Phe Ile Asp Glu Leu Val
   625 630 635 640
   Arg Gly Asn Ala Tyr Ala Gly Glu Thr Ala Ala Ile Met Pro Ser Asp
                   645                 650                 655
   Pro Tyr Ala Arg Ala Lys Ala Arg Val Asp Ala Asp Trp Val Asn Lys
               660                 665                 670
   Asn Val Cys Ser Arg Tyr Tyr His Val Leu Val Arg Gln Glu Ala Ser
           675                 680                 685
   Glu Gln Arg Glu Ala Phe Glu Lys Leu Val Glu Gly Leu Glu Thr Phe
       690                 695                 700
   Ala Ala Trp Cys Gly Ser Gly Glu Gly Lys Phe Tyr Gly Gly Gln Thr
   705                 710                 715                 720
   Thr Pro Gly Leu Val Asp Tyr Ala Leu Phe Pro Trp Ala Trp Arg Leu
                   725                 730                 735
   Pro Val Phe Glu His Tyr Arg Gly Met Asp Phe Lys Ile Pro Arg Thr
               740                 745                 750
   Ala Ala Leu Lys Ser Tyr His Asp Trp Met Glu Ala Met Leu Ala Arg
           755                 760                 765
   Glu Gln Val Arg Lys Thr Leu Pro Pro Trp Asp Asp Tyr Leu Glu His
       770                 775                 780
   Ile Gly Arg Tyr Ala Asp Gly Ser Ala Arg Ser Lys Val Ala Asn Ala
   785                 790                 795                 800
   Val Arg Ser Gly Arg Ala Ala His Asp Tyr Asp Asp Lys Glu Asp Asn
                   805                 810                 815
   Thr Asp Ser
<210> 223
   <211> 2076
   <212> DNA
   <213> Aquilegia formosa x Aquilegia pubescens
<220>
   <221> misc_feature
   <222> (692)..(713)
   <223> n is a, c, g, or t
<220>
   <221> misc_feature
   <222> (1459)..(1462)
   <223> n is a, c, g, or t
<400> 223
   atgcaagatg gtgggaacct tggttttcca aagaaacgtg ttggatccca aataaatacc 60
   tcatttcaga agaagaatag ttgcaaaaat ccgcatatta ggagatggaa accaaagcca 120
   agatctcatg tcaagacaat tgcttcgttc ttgtcgaaga gaaagacatc tgagctctca     180
   agcaacagag ccagaaataa atgttccaat ggaaaacctt ttagaagaag agcgtcacag     240
   aaacccaatg ataccagtcc ttctgatagg ccactttcat caatgcttaa agatttcaaa     300
   tcgttaagca ataaatgtca gggacgtgaa aagacaactg acaaggatct tggaagtcaa     360
   aaggctcaca gaaggagaag gagcaagaag aggaagaatg aggagttaga tgaagccgct     420
   cgagtccaaa gaagaacaag gtacctcctg attaaaatga agctggagca gaaccttatt     480
   gaggcttact ctggggaggg ttggaaaggc cagagtcgtg agaaaattaa gccagaaaag     540
   gaactgcaaa gagccaagaa gcagatattg aagtgcaaac ttggaattcg tgatgctatt     600
   cgtcagctgg aatctcttag ttctgaggga agcattgaag attctgtgat aggttcagat     660
   ggatctgttt tccatgaaca tattttctgt tnnnnnnnnn nnnnnnnnnn nnnagcattt     720
   ccagataatg acattatact gtgtgatgga gcatgcaatt gtgctttcca ccagaaatgc     780
   ctggagcctc cattagccac tgaaaatatt cctcctggtg atcaagggtg gtattgcaaa     840
   ttttgtgagt gcaagatggc aatacttgat gcaatcaatg cacatcttgg gactcagttt     900
   tctctagaca gtaaatggca agatattttc atggaagcag ctaatgcacg cgataatgag     960
   gatatctcaa ttcatcctga tggtgactgg ccatctgatg actctgaaga tgttgattat    1020
   gacccagaaa aatatgaaat tgattgcagc tatagcagaa tgggctcgga gcacaacgtg    1080
   tctgacgatg caagtagttc cggcaccttg tattggactt ccgaagaaga tgatggtttg    1140
   cattctgagc gattcagaac tgatggtagt ctggaggggg gattttatgg ggataaaact    1200
   aaaatccaga gttctgatag catagattcc aatgacagta ctgatggtat ggcatttcat    1260
   cgtaggcagc gaagagatgt tgattataag aaactgcatg atgaaatgtt tggtaaagat    1320
   atgcctgaaa gtgaagaaat cagtgaagat gaagattggg gacctagaag aagaaagcgc    1380
   agaagaaatg agtctaatgc agccactact ttagtatctt cgtgtggtac tgagaatgaa    1440
   ttttcgaatg tggcacttnn nntaccatca agagtggtca gggagagtct ctcgaagcaa    1500
   ttgggtattg caattgagaa gatcaacaag tggttcaaga atgctcggta tacagcttta    1560
   aaaattagaa aggcagaaaa gactgaacaa tcccctggag gtggcaagat gcaaagtgct    1620
   gacgtattaa catcaaagga aaaccattat ttggtctcaa caggaacagc agtccaaaga    1680
   tccaaagttt cgagtgtcca tagaagtaaa aaccgtaaat tgacaaccac accctcaagg    1740
   gaagtacaag agaaagtgtc tgctgttgca ccatccgcta ctgcaaatga ggtgagtgta    1800
   cagttaagaa aagcagtgag cgtagagaag gaaatgggca gtgtaaaaag aaaatctgca    1860
   caagtaaaga ggatcaagcg cacatgctcc actaaggagc agcagctata catgattgag    1920
   ttggagagaa tttgccgtct tgaggagaag cttgaaaaga tgaagaaggt attgcttagt    1980
   atagatgaca atcatagtaa tgtatcagat ggaccccata tgaacgaaca gtctgccgta    2040
   tacattccag ttgcagagct aagagaaaag atgtga                              2076
<210> 224
   <211> 691
   <212> PRT
   <213> Aquilegia formosa x Aquilegia pubescens
<220>
   <221> UNSURE
   <222> (231)..(238)
   <223> Xaa can be any naturally occurring amino acid
<220>
   <221> UNSURE
   <222> (487)..(488)
   <223> Xaa can be any naturally occurring amino acid
<400> 224
   Met Gln Asp Gly Gly Asn Leu Gly Phe Pro Lys Lys Arg Val Gly Ser
   1               5                   10                  15
   Gln Ile Asn Thr Ser Phe Gln Lys Lys Asn Ser Cys Lys Asn Pro His
               20                  25                  30
   Ile Arg Arg Trp Lys Pro Lys Pro Arg Ser His Val Lys Thr Ile Ala
           35                  40                  45
   Ser Phe Leu Ser Lys Arg Lys Thr Ser Glu Leu Ser Ser Asn Arg Ala
       50                  55                  60
   Arg Asn Lys Cys Ser Asn Gly Lys Pro Phe Arg Arg Arg Ala Ser Gln
   65                  70                  75                  80
   Lys Pro Asn Asp Thr Ser Pro Ser Asp Arg Pro Leu Ser Ser Met Leu
                   85                  90                  95
   Lys Asp Phe Lys Ser Leu Ser Asn Lys Cys Gln Gly Arg Glu Lys Thr
               100                 105                 110
   Thr Asp Lys Asp Leu Gly Ser Gln Lys Ala His Arg Arg Arg Arg Ser
           115                 120                 125
   Lys Lys Arg Lys Asn Glu Glu Leu Asp Glu Ala Ala Arg Val Gln Arg
       130                 135                 140
   Arg Thr Arg Tyr Leu Leu Ile Lys Met Lys Leu Glu Gln Asn Leu Ile
   145                 150                 155                 160
   Glu Ala Tyr Ser Gly Glu Gly Trp Lys Gly Gln Ser Arg Glu Lys Ile
                   165                 170                 175
   Lys Pro Glu Lys Glu Leu Gln Arg Ala Lys Lys Gln Ile Leu Lys Cys
               180                 185                 190
   Lys Leu Gly Ile Arg Asp Ala Ile Arg Gln Leu Glu Ser Leu Ser Ser
           195                 200                 205
   Glu Gly Ser Ile Glu Asp Ser Val Ile Gly Ser Asp Gly Ser Val Phe
       210                 215                 220
   His Glu His Ile Phe Cys Xaa Xaa Xaa Xaa Xaa Xaa Xaa Xaa Ala Phe
   225                 230                 235                 240
   Pro Asp Asn Asp Ile Ile Leu Cys Asp Gly Ala Cys Asn Cys Ala Phe
                   245                 250                 255
   His Gln Lys Cys Leu Glu Pro Pro Leu Ala Thr Glu Asn Ile Pro Pro
               260                 265                 270
   Gly Asp Gln Gly Trp Tyr Cys Lys Phe Cys Glu Cys Lys Met Ala Ile
           275                 280                 285
   Leu Asp Ala Ile Asn Ala His Leu Gly Thr Gln Phe Ser Leu Asp Ser
       290                 295                 300
   Lys Trp Gln Asp Ile Phe Met Glu Ala Ala Asn Ala Arg Asp Asn Glu
   305                 310                 315                 320
   Asp Ile Ser Ile His Pro Asp Gly Asp Trp Pro Ser Asp Asp Ser Glu
                   325                 330                 335
   Asp Val Asp Tyr Asp Pro Glu Lys Tyr Glu Ile Asp Cys Ser Tyr Ser
               340                 345                 350
   Arg Met Gly Ser Glu His Asn Val Ser Asp Asp Ala Ser Ser Ser Gly
           355                 360                 365
   Thr Leu Tyr Trp Thr Ser Glu Glu Asp Asp Gly Leu His Ser Glu Arg
       370                 375                 380
   Phe Arg Thr Asp Gly Ser Leu Glu Gly Gly Phe Tyr Gly Asp Lys Thr
   385                 390                 395                 400
   Lys Ile Gln Ser Ser Asp Ser Ile Asp Ser Asn Asp Ser Thr Asp Gly
                   405                 410                 415
   Met Ala Phe His Arg Arg Gln Arg Arg Asp Val Asp Tyr Lys Lys Leu
               420                 425                 430
   His Asp Glu Met Phe Gly Lys Asp Met Pro Glu Ser Glu Glu Ile Ser
           435                 440                 445
   Glu Asp Glu Asp Trp Gly Pro Arg Arg Arg Lys Arg Arg Arg Asn Glu
       450                 455                 460
   Ser Asn Ala Ala Thr Thr Leu Val Ser Ser Cys Gly Thr Glu Asn Glu
   465                 470                 475                 480
   Phe Ser Asn Val Ala Leu Xaa Xaa Pro Ser Arg Val Val Arg Glu Ser
   485 490 495
   Leu Ser Lys Gln Leu Gly Ile Ala Ile Glu Lys Ile Asn Lys Trp Phe
   500 505 510
   Lys Asn Ala Arg Tyr Thr Ala Leu Lys Ile Arg Lys Ala Glu Lys Thr
   515 520 525
   Glu Gln Ser Pro Gly Gly Gly Lys Met Gln Ser Ala Asp Val Leu Thr
   530 535 540
   Ser Lys Glu Asn His Tyr Leu Val Ser Thr Gly Thr Ala Val Gln Arg
   545 550 555 560
   Ser Lys Val Ser Ser Val His Arg Ser Lys Asn Arg Lys Leu Thr Thr
   565 570 575
   Thr Pro Ser Arg Glu Val Gln Glu Lys Val Ser Ala Val Ala Pro Ser
   580 585 590
   Ala Thr Ala Asn Glu Val Ser Val Gln Leu Arg Lys Ala Val Ser Val
   595 600 605
   Glu Lys Glu Met Gly Ser Val Lys Arg Lys Ser Ala Gln Val Lys Arg
   610 615 620
   Ile Lys Arg Thr Cys Ser Thr Lys Glu Gln Gln Leu Tyr Met Ile Glu
   625 630 635 640
   Leu Glu Arg Ile Cys Arg Leu Glu Glu Lys Leu Glu Lys Met Lys Lys
   645 650 655
   Val Leu Leu Ser Ile Asp Asp Asn His Ser Asn Val Ser Asp Gly Pro
   660 665 670
   His Met Asn Glu Gln Ser Ala Val Tyr Ile Pro Val Ala Glu Leu Arg
   675 680 685
   Glu Lys Met
   690
<210> 225
   <211> 1743
   <212> DNA
   <213> Glycine max
<220>
   <221> misc_feature
   <222> (261)..(261)
   <223> n is a, c, g, or t
<400> 225
   aagaagaaaa gacaaaggaa taacatagat gttgatgatg cttcacgctt gcgaaggaga      60
   acaaggtacc tcttaatcaa aatgaagcta gagcagaacc ttattgatgc ttactctgga     120
   gaaggttgga aaggtcaaag tcgggaaaag attaggccag aaaaggagct actacgagca     180
   aaaaagcaga ttttaaaatg taaacttagt attcgcgatg ccatacacca gctggattct     240
   cttagttctg tgggtagcat ngaagattct gccattgctc cagatggatc tgtttatcat     300
   gaaaatatat tctgtgccaa ttgcaaacta catgaagctt tcccagataa tgatattata     360
   ctctgtgatg gcacttgcaa tcgtgctttt caccaaagat gtctcaatcc tcctttggat     420
   actgaaaata ttcctcctgg agatcaaggc tggttttgca agttttgtga atgtaagata     480
   gaaatactag aggcaacaaa tgcccatctt gggactcaat tctccctaga cagtaattgg     540
   caggatgtat tcaaggaaga ggctgctatg cctgatggtg acattgcatt actaaatcct     600
   gaagaagaat ggccatcaga cgatcctgaa gatgatgatt acaatccaga gaggaaagaa     660
   gacaaccaca actttgacac agaaggagct gatgaaaatg catccaatga ttcaaccagt     720
   tgttccagtc tgttgtcttt gaatggcgaa tgtcctccag tagatgaagg catctgtcat     780
   gaatattatt ctgttaatag ctgcttagat tctgatgaat ctggggaaat agcatgtggt     840
   cctaggcagc gtaaagctgt tgactacaag aaactctatg atgaaatgta tgggaaggat     900
   gctccacctt gtgaacaaat gagtgaagat gaagactggg gtcctggcaa aagaaagcga     960
   agagaaaagg agtctgatgc tgttgattct cttatgactt tgcatgaaag tgagaatagg    1020
   catcccaata atgagcacaa catgacaagt aaagattctt caagcataaa gatcaaaagg    1080
   cattgtttta ggattccacg tgatgcagtt gagaggcttc gccaagtttt tgctgagaac    1140
   gaacttcctc caagatctat aagagagggt ctttcaaaag agctgggcct tgacaccgag    1200
   aaggtcagca aatggttcaa aaatgcacgt tacttagcac ttaaaaacag aaagcatcaa    1260
   gcagaaggag gagcagatca gcttcagagt atcacttcta caaagaacag attacaaaaa    1320
   caggagaatg tcgatccttt gaaatcaaag accccaaaaa ttactaggac acattcccag    1380
   aaggatgtta aaaatgtcaa tggaagaaag aaaatgaagt catctaacaa gaaaagacaa    1440
   ccagaaattc ctccacctcc aggagaaaat ggcaagaagg actttatgga gatcagtgat    1500
   gatgtgagct tgaagaaact gttgaagaaa agaaagaaga ggttgataaa ttttacgttt    1560
   ggaggagact ctcagttggc agagttggaa tttgaaagac taagcgaatt gaagactaaa    1620
   gtagatagta tgaagcaaaa attaactgca attcaaaatt acagagttaa gggttcaccc    1680
   tattcgaatg aaccatccat tgtatatgta cccacagctg tgttaaggga aaaggttgaa    1740
   tga                                                                  1743
<210> 226
   <211> 580
   <212> PRT
   <213> Glycine max
<220>
   <221> UNSURE
   <222> (87)..(87)
   <223> Xaa can be any naturally occurring amino acid
<400> 226
   Lys Lys Lys Arg Gln Arg Asn Asn Ile Asp Val Asp Asp Ala Ser Arg
   1               5                   10                  15
   Leu Arg Arg Arg Thr Arg Tyr Leu Leu Ile Lys Met Lys Leu Glu Gln
               20                  25                  30
   Asn Leu Ile Asp Ala Tyr Ser Gly Glu Gly Trp Lys Gly Gln Ser Arg
           35                  40                  45
   Glu Lys Ile Arg Pro Glu Lys Glu Leu Leu Arg Ala Lys Lys Gln Ile
       50                  55                  60
   Leu Lys Cys Lys Leu Ser Ile Arg Asp Ala Ile His Gln Leu Asp Ser
   65                  70                  75                  80
   Leu Ser Ser Val Gly Ser Xaa Glu Asp Ser Ala Ile Ala Pro Asp Gly
                   85                  90                  95
   Ser Val Tyr His Glu Asn Ile Phe Cys Ala Asn Cys Lys Leu His Glu
               100                 105                 110
   Ala Phe Pro Asp Asn Asp Ile Ile Leu Cys Asp Gly Thr Cys Asn Arg
           115                 120                 125
   Ala Phe His Gln Arg Cys Leu Asn Pro Pro Leu Asp Thr Glu Asn Ile
       130                 135                 140
   Pro Pro Gly Asp Gln Gly Trp Phe Cys Lys Phe Cys Glu Cys Lys Ile
   145                 150                 155                 160
   Glu Ile Leu Glu Ala Thr Asn Ala His Leu Gly Thr Gln Phe Ser Leu
                   165                 170                 175
   Asp Ser Asn Trp Gln Asp Val Phe Lys Glu Glu Ala Ala Met Pro Asp
               180                 185                 190
   Gly Asp Ile Ala Leu Leu Asn Pro Glu Glu Glu Trp Pro Ser Asp Asp
           195                 200                 205
   Pro Glu Asp Asp Asp Tyr Asn Pro Glu Arg Lys Glu Asp Asn His Asn
       210                 215                 220
   Phe Asp Thr Glu Gly Ala Asp Glu Asn Ala Ser Asn Asp Ser Thr Ser
   225                 230                 235                 240
   Cys Ser Ser Leu Leu Ser Leu Asn Gly Glu Cys Pro Pro Val Asp Glu
                   245                 250                 255
   Gly Ile Cys His Glu Tyr Tyr Ser Val Asn Ser Cys Leu Asp Ser Asp
               260                 265                 270
   Glu Ser Gly Glu Ile Ala Cys Gly Pro Arg Gln Arg Lys Ala Val Asp
           275                 280                 285
   Tyr Lys Lys Leu Tyr Asp Glu Met Tyr Gly Lys Asp Ala Pro Pro Cys
       290                 295                 300
   Glu Gln Met Ser Glu Asp Glu Asp Trp Gly Pro Gly Lys Arg Lys Arg
   305                 310                 315                 320
   Arg Glu Lys Glu Ser Asp Ala Val Asp Ser Leu Met Thr Leu His Glu
                   325                 330                 335
   Ser Glu Asn Arg His Pro Asn Asn Glu His Asn Met Thr Ser Lys Asp
               340                 345                 350
   Ser Ser Ser Ile Lys Ile Lys Arg His Cys Phe Arg Ile Pro Arg Asp
           355                 360                 365
   Ala Val Glu Arg Leu Arg Gln Val Phe Ala Glu Asn Glu Leu Pro Pro
       370                 375                 380
   Arg Ser Ile Arg Glu Gly Leu Ser Lys Glu Leu Gly Leu Asp Thr Glu
   385                 390                 395                 400
   Lys Val Ser Lys Trp Phe Lys Asn Ala Arg Tyr Leu Ala Leu Lys Asn
                   405                 410                 415
   Arg Lys His Gln Ala Glu Gly Gly Ala Asp Gln Leu Gln Ser Ile Thr
               420                 425                 430
   Ser Thr Lys Asn Arg Leu Gln Lys Gln Glu Asn Val Asp Pro Leu Lys
           435                 440                 445
   Ser Lys Thr Pro Lys Ile Thr Arg Thr His Ser Gln Lys Asp Val Lys
       450                 455                 460
   Asn Val Asn Gly Arg Lys Lys Met Lys Ser Ser Asn Lys Lys Arg Gln
   465                 470                 475                 480
   Pro Glu Ile Pro Pro Pro Pro Gly Glu Asn Gly Lys Lys Asp Phe Met
                   485                 490                 495
   Glu Ile Ser Asp Asp Val Ser Leu Lys Lys Leu Leu Lys Lys Arg Lys
               500                 505                 510
   Lys Arg Leu Ile Asn Phe Thr Phe Gly Gly Asp Ser Gln Leu Ala Glu
           515                 520                 525
   Leu Glu Phe Glu Arg Leu Ser Glu Leu Lys Thr Lys Val Asp Ser Met
       530                 535                 540
   Lys Gln Lys Leu Thr Ala Ile Gln Asn Tyr Arg Val Lys Gly Ser Pro
   545                 550                 555                 560
   Tyr Ser Asn Glu Pro Ser Ile Val Tyr Val Pro Thr Ala Val Leu Arg
                   565                 570                 575
   Glu Lys Val Glu
               580
<210> 227
   <211> 1614
   <212> DNA
   <213> Lotus corniculatus
<400> 227
   atgcagggta ctgagaagtt aaatggcaca ggatctacaa aatccagtaa ctcagaggaa 60
   catgctgagt caaaggtaga ttcattgaga tacagaacaa atataacaaa atgccgaggg 120
   aagaaacaaa aactgaaatc aaaatctcat aaactcagcg gttgcttaag tgcatcaggg 180
   aggacagtta ccaattcttc catcaagaga caagtaaagg attcttctaa taaaaagatt 240
   attagacaaa gtctacataa aactgatggc aaatcttcac ggatgctgtc ttcaacaatg 300
   atacaaggag gaaaatcttc tcttggttct agaaaggagg gcaaagatgt tgatggggag 360
   gtaatggttc aaaaagttaa aagaaagaga aagaagaaaa ggagacagga tgatgttgat 420
   cttgatgatg cttcacgctt actgagaaga acaaggtacc tcttaatcaa aatgaagcta 480
   gagcagaatc ttattgatgc ttactctgga gaaggttgga aaggtcaaag tcgagagaag 540
   attaggccag aaaaggagct acaaagagcc aagaagcaga ttttgaattg taaacttagt 600
   attcgggatg ccattcgcca gttagattct cttagctcat tgggcagcat tgaagattct 660
   gtcattgatc cagatggatc tgtttatcat gaacatatat tctgtgcaaa ttgcaagttg 720
   cgtgaagctt tcccagataa tgatataata ctctgtgatg gcacatgcaa tcgtgctttt 780
   caccaacggt gtctcaaccc tcctttggat actgaaaata ttcctcctgg agaccaaggc 840
   tggttctgca atttttgtga atgtaagata gaaatattgg aggcaacaaa tgcccatctt 900
   gggactcaaa tctccttgga cagtacttgg caggatgtat tcaaggagga ggctgctata 960
   cccgatggtg ataatgcatt actgaatcca gaagaagaat ggccatcaga tgatcctgaa 1020
   gacgatgatt attatccaga gaggaaagaa gacaaccaca gcatcaaagc agaaggaact 1080
   gatgataatg catccaatga tttaagcagt tcttccagtc tagggtcttt gaatggtgaa 1140
   tgttctccag tagatgaagg cacgggtctt gaatattatt ctgttaattg ctgcatagat 1200
   tctgatgatt ctggggaaat agcatgtggc cgtaggcagc gtaaatctgt tgactacaag 1260
   aaactatatg atgaaatgtt tgggaaggat gctcctccat gtgaactagt gagtgaagat 1320
   gaagactggg gtactggcaa aagaaagcga agagaaaaag agtctgatgc tgttaatact 1380
   ctcatgacta tgcatgaaag tgagaacaaa aatcccaata atgagaatag tgatggaata 1440
   agagagggtt cttcaggcaa accaataaga aggtcttgtt tcaggattcc acatgatgca 1500
   cttgagaagc ttcgccaagt ttttgcggag aatgagcttc ctccaagatc tgtcaaagaa 1560
   ggtctttcga aagagttggg aattgatgct gcgaaggtta tgccttacaa cctc 1614
<210> 228
   <211> 538
   <212> PRT
   <213> Lotus corniculatus
<400> 228
   Met Gln Gly Thr Glu Lys Leu Asn Gly Thr Gly Ser Thr Lys Ser Ser
   1 5 10 15
   Asn Ser Glu Glu His Ala Glu Ser Lys Val Asp Ser Leu Arg Tyr Arg
   20 25 30
   Thr Asn Ile Thr Lys Cys Arg Gly Lys Lys Gln Lys Leu Lys Ser Lys
   35 40 45
   Ser His Lys Leu Ser Gly Cys Leu Ser Ala Ser Gly Arg Thr Val Thr
   50 55 60
   Asn Ser Ser Ile Lys Arg Gln Val Lys Asp Ser Ser Asn Lys Lys Ile
   65 70 75 80
   Ile Arg Gln Ser Leu His Lys Thr Asp Gly Lys Ser Ser Arg Met Leu
   85 90 95
   Ser Ser Thr Met Ile Gln Gly Gly Lys Ser Ser Leu Gly Ser Arg Lys
   100 105 110
   Glu Gly Lys Asp Val Asp Gly Glu Val Met Val Gln Lys Val Lys Arg
   115 120 125
   Lys Arg Lys Lys Lys Arg Arg Gln Asp Asp Val Asp Leu Asp Asp Ala
   130 135 140
   Ser Arg Leu Leu Arg Arg Thr Arg Tyr Leu Leu Ile Lys Met Lys Leu
   145 150 155 160
   Glu Gln Asn Leu Ile Asp Ala Tyr Ser Gly Glu Gly Trp Lys Gly Gln
   165 170 175
   Ser Arg Glu Lys Ile Arg Pro Glu Lys Glu Leu Gln Arg Ala Lys Lys
   180 185 190
   Gln Ile Leu Asn Cys Lys Leu Ser Ile Arg Asp Ala Ile Arg Gln Leu
   195 200 205
   Asp Ser Leu Ser Ser Leu Gly Ser Ile Glu Asp Ser Val Ile Asp Pro
   210 215 220
   Asp Gly Ser Val Tyr His Glu His Ile Phe Cys Ala Asn Cys Lys Leu
   225 230 235 240
   Arg Glu Ala Phe Pro Asp Asn Asp Ile Ile Leu Cys Asp Gly Thr Cys
   245 250 255
   Asn Arg Ala Phe His Gln Arg Cys Leu Asn Pro Pro Leu Asp Thr Glu
   260 265 270
   Asn Ile Pro Pro Gly Asp Gln Gly Trp Phe Cys Asn Phe Cys Glu Cys
   275 280 285
   Lys Ile Glu Ile Leu Glu Ala Thr Asn Ala His Leu Gly Thr Gln Ile
   290 295 300
   Ser Leu Asp Ser Thr Trp Gln Asp Val Phe Lys Glu Glu Ala Ala Ile
   305 310 315 320
   Pro Asp Gly Asp Asn Ala Leu Leu Asn Pro Glu Glu Glu Trp Pro Ser
   325 330 335
   Asp Asp Pro Glu Asp Asp Asp Tyr Tyr Pro Glu Arg Lys Glu Asp Asn
   340 345 350
   His Ser Ile Lys Ala Glu Gly Thr Asp Asp Asn Ala Ser Asn Asp Leu
   355 360 365
   Ser Ser Ser Ser Ser Leu Gly Ser Leu Asn Gly Glu Cys Ser Pro Val
   370 375 380
   Asp Glu Gly Thr Gly Leu Glu Tyr Tyr Ser Val Asn Cys Cys Ile Asp
   385 390 395 400
   Ser Asp Asp Ser Gly Glu Ile Ala Cys Gly Arg Arg Gln Arg Lys Ser
   405 410 415
   Val Asp Tyr Lys Lys Leu Tyr Asp Glu Met Phe Gly Lys Asp Ala Pro
   420 425 430
   Pro Cys Glu Leu Val Ser Glu Asp Glu Asp Trp Gly Thr Gly Lys Arg
   435 440 445
   Lys Arg Arg Glu Lys Glu Ser Asp Ala Val Asn Thr Leu Met Thr Met
   450 455 460
   His Glu Ser Glu Asn Lys Asn Pro Asn Asn Glu Asn Ser Asp Gly Ile
   465 470 475 480
   Arg Glu Gly Ser Ser Gly Lys Pro Ile Arg Arg Ser Cys Phe Arg Ile
   485 490 495
   Pro His Asp Ala Leu Glu Lys Leu Arg Gln Val Phe Ala Glu Asn Glu
   500 505 510
   Leu Pro Pro Arg Ser Val Lys Glu Gly Leu Ser Lys Glu Leu Gly Ile
           515                 520                 525
   Asp Ala Ala Lys Val Met Pro Tyr Asn Leu
       530                 535
<210> 229
   <211> 573
   <212> DNA
   <213> Sorghum propinquum
<400> 229
   aactcgagaa caaatgaaga aaaatctggt atatcaggga agattgattc agtagacaac     60
   tcttgcttgg ttcccttgtc tgaaatcatc aatgtgcata cacgagtgca ccacaatctt    120
   gagatgagga agatggaatc aactagcagt cctgtgtggc tgcacaatga aggaggctgc    180
   ttgtttccaa cactccaagc caaggagagt acattaccta caagcaagcc ttgtttgccg    240
   tcagaaataa cccatccaac aactaatgaa gtgagcactt tagtgcaagc tacttcttgg    300
   atggatgctg gggcgtgcat tgagcagcaa gaaaccactc cttgggtgga taccggggcc    360
   tcaggttacc agcctttcct ggacgtgatt gatgagatgt gcggacttga gtgcaggctg    420
   cagaggctga aggagaacat gttctcatct ggcatggacg gcagaaccgc cggtgtgagt    480
   gacatgggaa accaggctgt ggtgcttgtg ccgactgctg agctcaagga aaaagcgccg    540
   catggcggtt tatttgggca ctattgccca tag                                 573
<210> 230
   <211> 190
   <212> PRT
   <213> Sorghum propinquum
<400> 230
   Asn Ser Arg Thr Asn Glu Glu Lys Ser Gly Ile Ser Gly Lys Ile Asp
   1               5                   10                  15
   Ser Val Asp Asn Ser Cys Leu Val Pro Leu Ser Glu Ile Ile Asn Val
               20                  25                  30
   His Thr Arg Val His His Asn Leu Glu Met Arg Lys Met Glu Ser Thr
           35                  40                  45
   Ser Ser Pro Val Trp Leu His Asn Glu Gly Gly Cys Leu Phe Pro Thr
       50                  55                  60
   Leu Gln Ala Lys Glu Ser Thr Leu Pro Thr Ser Lys Pro Cys Leu Pro
   65                  70                  75                  80
   Ser Glu Ile Thr His Pro Thr Thr Asn Glu Val Ser Thr Leu Val Gln
                   85                  90                  95
   Ala Thr Ser Trp Met Asp Ala Gly Ala Cys Ile Glu Gln Gln Glu Thr
               100                 105                 110
   Thr Pro Trp Val Asp Thr Gly Ala Ser Gly Tyr Gln Pro Phe Leu Asp
           115                 120                 125
   Val Ile Asp Glu Met Cys Gly Leu Glu Cys Arg Leu Gln Arg Leu Lys
       130                 135                 140
   Glu Asn Met Phe Ser Ser Gly Met Asp Gly Arg Thr Ala Gly Val Ser
   145                 150                 155                 160
   Asp Met Gly Asn Gln Ala Val Val Leu Val Pro Thr Ala Glu Leu Lys
                   165                 170                 175
   Glu Lys Ala Pro His Gly Gly Leu Phe Gly His Tyr Cys Pro
               180                 185                 190
<210> 231
   <211> 532
   <212> DNA
   <213> Sorghum propinquum
<400> 231
   aagataaaat tggagcagaa tctactagat gcttactctg gagatggatg gaatgggcaa      60
   agccgggaga aaataaagcc agagaaggaa ctgcagcgtg ccaggaaaca aatcataaaa     120
   tgcaaaattg ctatacgtga tatcattcgc caactttgtt tgtatacttc tactgggagt     180
   gttgatgacc cagcaatgcc tccagatcag tttaccaatc ccgaacatac catgtgctca     240
   acatgcaagt ctcatgaatc atttcccagc aataaattta tcttctgtga agggccctgc     300
   aaaagggcat atcatgagaa atgtttggaa cctcccttaa acaaaggtgt acttccaaca     360
   agtagccatg gatggctttg caaattctgt ttgtgcaagg tgaaaatttt agaaactatt     420
   aatgcacatc taggaacaag ttttacagtg atgtgctcct ttgaagatat attcaaggaa     480
   gcaacagaac aaatagactc cgaggatgca ctagatgaag attggctctc tg             532
<210> 232
   <211> 177
   <212> PRT
   <213> Sorghum propinquum
<400> 232
   Lys Ile Lys Leu Glu Gln Asn Leu Leu Asp Ala Tyr Ser Gly Asp Gly
   1               5                   10                  15
   Trp Asn Gly Gln Ser Arg Glu Lys Ile Lys Pro Glu Lys Glu Leu Gln
               20                  25                  30
   Arg Ala Arg Lys Gln Ile Ile Lys Cys Lys Ile Ala Ile Arg Asp Ile
           35                  40                  45
   Ile Arg Gln Leu Cys Leu Tyr Thr Ser Thr Gly Ser Val Asp Asp Pro
       50                  55                  60
   Ala Met Pro Pro Asp Gln Phe Thr Asn Pro Glu His Thr Met Cys Ser
   65 70 75 80
   Thr Cys Lys Ser His Glu Ser Phe Pro Ser Asn Lys Phe Ile Phe Cys
   85 90 95
   Glu Gly Pro Cys Lys Arg Ala Tyr His Glu Lys Cys Leu Glu Pro Pro
   100 105 110
   Leu Asn Lys Gly Val Leu Pro Thr Ser Ser His Gly Trp Leu Cys Lys
   115 120 125
   Phe Cys Leu Cys Lys Val Lys Ile Leu Glu Thr Ile Asn Ala His Leu
   130 135 140
   Gly Thr Ser Phe Thr Val Met Cys Ser Phe Glu Asp Ile Phe Lys Glu
   145 150 155 160
   Ala Thr Glu Gln Ile Asp Ser Glu Asp Ala Leu Asp Glu Asp Trp Leu
   165 170 175
   Ser
<210> 233
   <211> 180
   <212> PRT
   <213> Oryza sativa
<400> 233
   Asp Glu Val Ser Arg Met Glu Lys Arg Ala Arg Tyr Leu Leu Ile Lys
   1 5 10 15
   Ile Lys Gln Glu Gln Asn Leu Leu Asp Ala Tyr Ser Gly Asp Gly Trp
   20 25 30
   Asn Gly His Ser Arg Glu Lys Ile Lys Pro Glu Lys Glu Leu Gln Arg
   35 40 45
   Ala Lys Lys Gln Ile Met Lys Tyr Lys Ile Ala Ile Arg Asp Val Ile
   50 55 60
   His Gln Leu Asp Leu Cys Ser Ser Ser Gly Ser Lys Asp Asp Ser Val
   65 70 75 80
   Ile Pro Pro Asp Gly Cys His Glu Ser Val Asn Pro Glu His Thr Ile
   85 90 95
   Cys Ser Arg Cys Lys Ser His Glu Ser Phe Pro Asp Asn Asn Ile Ile
   100 105 110
   Phe Cys Glu Gly Gly Cys Lys Leu Ala Cys His Gln Lys Cys Leu Glu
   115 120 125
   Pro Pro Phe Asp Lys Ile Leu Pro Thr Thr Arg His Gly Arg Leu Cys
   130 135 140
   Lys His Cys Ser Ser Lys Met Lys Ile Leu Asp Ala Ile Asn Ala His
   145 150 155 160
   Leu Gly Thr Ser Phe Thr Val Lys Cys Pro Ser Ser Asp Ile Phe Lys
   165 170 175
   Glu Ala Ala Glu
   180

<210> 234
   <211> 50
   <212> PRT
   <213> Oryza sativa
<400> 234
   Arg Ile Pro Pro Ala Ala Val Glu Val Leu Arg Lys Ala Phe Ala Glu
   1 5 10 15
   Asn Glu Leu Pro Ala Arg Ser Val Lys Glu Asn Leu Ser Thr Glu Leu
   20 25 30
   Gly Ile Ser Phe Glu Lys Ile Asp Lys Trp Phe Lys Asn Thr Arg Cys
   35 40 45
   Ala Ala
   50
<210> 235
   <211> 2194
   <212> DNA
   <213> Oryza sativa
<400> 235
   aatccgaaaa gtttctgcac cgttttcacc ccctaactaa caatataggg aacgtgtgct 60
   aaatataaaa tgagacctta tatatgtagc gctgataact agaactatgc aagaaaaact 120
   catccaccta ctttagtggc aatcgggcta aataaaaaag agtcgctaca ctagtttcgt 180
   tttccttagt aattaagtgg gaaaatgaaa tcattattgc ttagaatata cgttcacatc 240
   tctgtcatga agttaaatta ttcgaggtag ccataattgt catcaaactc ttcttgaata 300
   aaaaaatctt tctagctgaa ctcaatgggt aaagagagag atttttttta aaaaaataga 360
   atgaagatat tctgaacgta ttggcaaaga tttaaacata taattatata attttatagt 420
   ttgtgcattc gtcatatcgc acatcattaa ggacatgtct tactccatcc caatttttat 480
   ttagtaatta aagacaattg acttattttt attatttatc ttttttcgat tagatgcaag 540
   gtacttacgc acacactttg tgctcatgtg catgtgtgag tgcacctcct caatacacgt 600
   tcaactagca acacatctct aatatcactc gcctatttaa tacatttagg tagcaatatc 660
   tgaattcaag cactccacca tcaccagacc acttttaata atatctaaaa tacaaaaaat 720
   aattttacag aatagcatga aaagtatgaa acgaactatt taggtttttc acatacaaaa 780
   aaaaaaagaa ttttgctcgt gcgcgagcgc caatctccca tattgggcac acaggcaaca 840
   acagagtggc tgcccacaga acaacccaca aaaaacgatg atctaacgga ggacagcaag 900
   tccgcaacaa ccttttaaca gcaggctttg cggccaggag agaggaggag aggcaaagaa 960
   aaccaagcat cctccttctc ccatctataa attcctcccc ccttttcccc tctctatata 1020
   ggaggcatcc aagccaagaa gagggagagc accaaggaca cgcgactagc agaagccgag 1080
   cgaccgcctt ctcgatccat atcttccggt cgagttcttg gtcgatctct tccctcctcc 1140
   acctcctcct cacagggtat gtgcctccct tcggttgttc ttggatttat tgttctaggt 1200
   tgtgtagtac gggcgttgat gttaggaaag gggatctgta tctgtgatga ttcctgttct 1260
   tggatttggg atagaggggt tcttgatgtt gcatgttatc ggttcggttt gattagtagt 1320
   atggttttca atcgtctgga gagctctatg gaaatgaaat ggtttaggga tcggaatctt 1380
   gcgattttgt gagtaccttt tgtttgaggt aaaatcagag caccggtgat tttgcttggt 1440
   gtaataaagt acggttgttt ggtcctcgat tctggtagtg atgcttctcg atttgacgaa 1500
   gctatccttt gtttattccc tattgaacaa aaataatcca actttgaaga cggtcccgtt 1560
   gatgagattg aatgattgat tcttaagcct gtccaaaatt tcgcagctgg cttgtttaga 1620
   tacagtagtc cccatcacga aattcatgga aacagttata atcctcagga acaggggatt 1680
   ccctgttctt ccgatttgct ttagtcccag aatttttttt cccaaatatc ttaaaaagtc 1740
   actttctggt tcagttcaat gaattgattg ctacaaataa tgcttttata gcgttatcct 1800
   agctgtagtt cagttaatag gtaatacccc tatagtttag tcaggagaag aacttatccg 1860
   atttctgatc tccattttta attatatgaa atgaactgta gcataagcag tattcatttg 1920
   gattattttt tttattagct ctcacccctt cattattctg agctgaaagt ctggcatgaa 1980
   ctgtcctcaa ttttgttttc aaattcacat cgattatcta tgcattatcc tcttgtatct 2040
   acctgtagaa gtttcttttt ggttattcct tgactgcttg attacagaaa gaaatttatg 2100
   aagctgtaat cgggatagtt atactgcttg ttcttatgat tcatttcctt tgtgcagttc 2160
   ttggtgtagc ttgccacttt caccagcaaa gttc 2194
<210> 236
   <211> 56
   <212> DNA
   <213> Artificial sequence
<220>
   <223> primer: prm09687
<400> 236
   ggggacaagt ttgtacaaaa aagcaggctt aaacaatgaa taccccagaa aagaaa 56
<210> 237
   <211> 50
   <212> DNA
   <213> Artificial sequence
<220>
   <223> primer: prm09688
<400> 237
   ggggaccact ttgtacaaga aagctgggtg atgcaaggtt aagatgcttt 50
<210> 238
   <211> 2649
   <212> DNA
   <213> Physcomitrella patens
<400> 238
   atgatgaaga attaccgtcc tccatcacca cttccaagtg ctcgccaagc tttcttacaa      60
   gacccggcgt cgtcattcca aatgacagga cgacattggg cagacccaga gggaggtgga     120
   gaacggaagc ttcacacaca ctctcactca cactcactca ctcacgaagg aaacgaaggg     180
   caaaggaagg ggagccccgc cgtgagccga gagcggagag caagcattcc caacggaagt     240
   ctccgtcaat gcggtgccgg agccgacgac gacgacgatg atgggctgag cctgtcatcg     300
   caatggatgc attgccctgt aagtgggaag gtggaggaag gattggggaa gaggttgagg     360
   aagagaaaga gaaagagaaa gagtttgttg ttgttgttgg ggaaggggag ggggcgacgg     420
   atgatgccca tgggcgtagt ccgcgacgat gagtcccagg ccgctgctgg ctcgccgcct     480
   gcgcccaccc ctcccccaat cgccaagccg acgctgcgat cccactcgcg ctccagggcg     540
   cacaaggcgc gctcccaaca cccgccgctg ggcgatggcg atgcgccctt gcccccaccg     600
   caaggaacag caacaccagg aggaggagga gcagcagcag tgggcgcacc aggggccatg     660
   gatgccgagg ctatggcggc ggggcagcgg cggcgtcgac gacgccgacg acagcggggc     720
   aagaactcgg atgtgccgct tgacaatcgc acgcgaatta agaggcgtgt caagtacttg     780
   ctcatgaaaa tgcgagtcga ccagaacctg ctggatgcgt actccgggga gggttggaag     840
   gggcagagga attctctttt tggcatatcc agtcgggaaa agatcaggcc ggagcaagaa     900
   ctacagcgtg ctgaagcgca gatcctgcat tcgaaattgg ccatacggga ggctattcat     960
   gaattggata acttggggtt agaaggctcg ctcgacgaaa atgcatttga tgctgaaggc    1020
   cgtgtttacc atgaagagat attttgtgcc aagtgccgat ctcaagatgc tctacctgac    1080
   aacgacatta ttttatgtga cggcgcatgc aacagaggct tccatcagta ctgtttagac    1140
   ccacctttag ctacgatcaa cattcctcct ggagacgaag ggtggttgtg tcctgtatgt    1200
   gagtgcaaga tggagtgcat tgaagttatt aatgcatact tgggcacaca ctttgaggtg    1260
   gagaatagtt gggaggagtt gttttctgaa gaagctctcg tggctgcggg cggtagaact    1320
   caaggggtca cagatggtga ttttccttcc tcagactcag aagacgacga ttataaccct    1380
   gaaggagcgg agaagcggac agacagtgaa agcgaaggcg aaagtgatga aggtggggga    1440
   gatagtgatt caggaagcga ttttagggaa aatgaagcgt ctggtagtga caaagatgag    1500
   caagatccaa tgcaggcaac taatttagag agacttgcga gaacagcaaa gcggaccagc    1560
   aaggaaggaa gtggaggtcc agaatcttca ggcagtgatg actcagaagt ttttcttgtt     1620
   gatgggttta cggatggtgg agtcaaatta ggtttaagac gtagagactc acaagcagaa 1680
   gaatcagctt cagacgagga aaccatggtt atagcaggaa aaagacatcg aaaagcagta 1740
   gactacaaga gattacatga tgaaatgttt gggaacatgg agattgatgt ggacgatatc 1800
   atatccgaag atgaggattg gggtccgaaa cgccgtcgga gaagaaccat tcctgatgac 1860
   ccaagcaggt ctagacctcc tcgagttcga cggagaaagg cgcgaacctc aactggagat 1920
   gctttaaaag aagttgatgc agatggggta ccatcagatt taccctctgg gtcacaaggc 1980
   gagggagcag ccgatactct tgaagctgtt agagacaaac gaatgtggag gaagttgcct 2040
   gattctgctg ttgagacgtt acgactggct gcggcagtga cgacactgcc ctcaaaatcc 2100
   cgtaaagagg agttggcgac acagcttggc ttgagttttt cacaagtcca tggatggttt 2160
   aaaaatcagc ggtatttagc tctcagaaat gggttggcga tttcaaagcg accagcagct 2220
   gggcggatgg cggccactgt aaccatacaa tcggaacacg acaacaattc aagtgcctat 2280
   ttaactgaga agttggatga agtccaaatg cgactttttg aactgaagca aactttggaa 2340
   gatttcgcta agaattcatc tggtttaggc ggtggagaat cagataacag ttttgaaaat 2400
   ggaggaattg gacttcctgc aaatgggaaa cggattatct atgtcccagt ggctgaggtt 2460
   agagaacggc cttctctttt ctctgacagc tgtccgcttt cattattagc agtgttaaat 2520
   tgcgaggtga attacaagag gaatctacgt atattcatgt gttcatggcc tagctgctta 2580
   gcgtacgggg ttttgcatgc caactgccct tgtcatattg gatcatgttt cgaaggaata 2640
   ggtgggtga 2649
<210> 239
   <211> 882
   <212> PRT
   <213> Physcomitrella patens
<400> 239
   Met Met Lys Asn Tyr Arg Pro Pro Ser Pro Leu Pro Ser Ala Arg Gln
   1               5                   10                  15
   Ala Phe Leu Gln Asp Pro Ala Ser Ser Phe Gln Met Thr Gly Arg His
               20                  25                  30
   Trp Ala Asp Pro Glu Gly Gly Gly Glu Arg Lys Leu His Thr His Ser
           35                  40                  45
   His Ser His Ser Leu Thr His Glu Gly Asn Glu Gly Gln Arg Lys Gly
       50                  55                  60
   Ser Pro Ala Val Ser Arg Glu Arg Arg Ala Ser Ile Pro Asn Gly Ser
   65                  70                  75                  80
   Leu Arg Gln Cys Gly Ala Gly Ala Asp Asp Asp Asp Asp Asp Gly Leu
                   85                  90                  95
   Ser Leu Ser Ser Gln Trp Met His Cys Pro Val Ser Gly Lys Val Glu
               100                 105                 110
   Glu Gly Leu Gly Lys Arg Leu Arg Lys Arg Lys Arg Lys Arg Lys Ser
           115                 120                 125
   Leu Leu Leu Leu Leu Gly Lys Gly Arg Gly Arg Arg Met Met Pro Met
       130                 135                 140
   Gly Val Val Arg Asp Asp Glu Ser Gln Ala Ala Ala Gly Ser Pro Pro
   145                 150                 155                 160
   Ala Pro Thr Pro Pro Pro Ile Ala Lys Pro Thr Leu Arg Ser His Ser
                   165                 170                 175
   Arg Ser Arg Ala His Lys Ala Arg Ser Gln His Pro Pro Leu Gly Asp
               180                 185                 190
   Gly Asp Ala Pro Leu Pro Pro Pro Gln Gly Thr Ala Thr Pro Gly Gly
           195                 200                 205
   Gly Gly Ala Ala Ala Val Gly Ala Pro Gly Ala Met Asp Ala Glu Ala
       210                 215                 220
   Met Ala Ala Gly Gln Arg Arg Arg Arg Arg Arg Arg Arg Gln Arg Gly
   225                 230                 235                 240
   Lys Asn Ser Asp Val Pro Leu Asp Asn Arg Thr Arg Ile Lys Arg Arg
                   245                 250                 255
   Val Lys Tyr Leu Leu Met Lys Met Arg Val Asp Gln Asn Leu Leu Asp
               260                 265                 270
   Ala Tyr Ser Gly Glu Gly Trp Lys Gly Gln Arg Asn Ser Leu Phe Gly
           275                 280                 285
   Ile Ser Ser Arg Glu Lys Ile Arg Pro Glu Gln Glu Leu Gln Arg Ala
       290                 295                 300
   Glu Ala Gln Ile Leu His Ser Lys Leu Ala Ile Arg Glu Ala Ile His
   305                 310                 315                 320
   Glu Leu Asp Asn Leu Gly Leu Glu Gly Ser Leu Asp Glu Asn Ala Phe
                   325                 330                 335
   Asp Ala Glu Gly Arg Val Tyr His Glu Glu Ile Phe Cys Ala Lys Cys
               340                 345                 350
   Arg Ser Gln Asp Ala Leu Pro Asp Asn Asp Ile Ile Leu Cys Asp Gly
           355                 360                 365
   Ala Cys Asn Arg Gly Phe His Gln Tyr Cys Leu Asp Pro Pro Leu Ala
   370 375 380
   Thr Ile Asn Ile Pro Pro Gly Asp Glu Gly Trp Leu Cys Pro Val Cys
   385 390 395 400
   Glu Cys Lys Met Glu Cys Ile Glu Val Ile Asn Ala Tyr Leu Gly Thr
   405 410 415
   His Phe Glu Val Glu Asn Ser Trp Glu Glu Leu Phe Ser Glu Glu Ala
   420 425 430
   Leu Val Ala Ala Gly Gly Arg Thr Gln Gly Val Thr Asp Gly Asp Phe
   435 440 445
   Pro Ser Ser Asp Ser Glu Asp Asp Asp Tyr Asn Pro Glu Gly Ala Glu
   450 455 460
   Lys Arg Thr Asp Ser Glu Ser Glu Gly Glu Ser Asp Glu Gly Gly Gly
   465 470 475 480
   Asp Ser Asp Ser Gly Ser Asp Phe Arg Glu Asn Glu Ala Ser Gly Ser
   485 490 495
   Asp Lys Asp Glu Gln Asp Pro Met Gln Ala Thr Asn Leu Glu Arg Leu
   500 505 510
   Ala Arg Thr Ala Lys Arg Thr Ser Lys Glu Gly Ser Gly Gly Pro Glu
   515 520 525
   Ser Ser Gly Ser Asp Asp Ser Glu Val Phe Leu Val Asp Gly Phe Thr
   530 535 540
   Asp Gly Gly Val Lys Leu Gly Leu Arg Arg Arg Asp Ser Gln Ala Glu
   545 550 555 560
   Glu Ser Ala Ser Asp Glu Glu Thr Met Val Ile Ala Gly Lys Arg His
   565 570 575
   Arg Lys Ala Val Asp Tyr Lys Arg Leu His Asp Glu Met Phe Gly Asn
   580 585 590
   Met Glu Ile Asp Val Asp Asp Ile Ile Ser Glu Asp Glu Asp Trp Gly
   595 600 605
   Pro Lys Arg Arg Arg Arg Arg Thr Ile Pro Asp Asp Pro Ser Arg Ser
   610 615 620
   Arg Pro Pro Arg Val Arg Arg Arg Lys Ala Arg Thr Ser Thr Gly Asp
   625 630 635 640
   Ala Leu Lys Glu Val Asp Ala Asp Gly Val Pro Ser Asp Leu Pro Ser
   645 650 655
   Gly Ser Gln Gly Glu Gly Ala Ala Asp Thr Leu Glu Ala Val Arg Asp
   660 665 670
   Lys Arg Met Trp Arg Lys Leu Pro Asp Ser Ala Val Glu Thr Leu Arg
   675 680 685
   Leu Ala Ala Ala Val Thr Thr Leu Pro Ser Lys Ser Arg Lys Glu Glu
   690 695 700
   Leu Ala Thr Gln Leu Gly Leu Ser Phe Ser Gln Val His Gly Trp Phe
   705 710 715 720
   Lys Asn Gln Arg Tyr Leu Ala Leu Arg Asn Gly Leu Ala Ile Ser Lys
   725 730 735
   Arg Pro Ala Ala Gly Arg Met Ala Ala Thr Val Thr Ile Gln Ser Glu
   740 745 750
   His Asp Asn Asn Ser Ser Ala Tyr Leu Thr Glu Lys Leu Asp Glu Val
   755 760 765
   Gln Met Arg Leu Phe Glu Leu Lys Gln Thr Leu Glu Asp Phe Ala Lys
   770 775 780
   Asn Ser Ser Gly Leu Gly Gly Gly Glu Ser Asp Asn Ser Phe Glu Asn
   785 790 795 800
   Gly Gly Ile Gly Leu Pro Ala Asn Gly Lys Arg Ile Ile Tyr Val Pro
   805 810 815
   Val Ala Glu Val Arg Glu Arg Pro Ser Leu Phe Ser Asp Ser Cys Pro
   820 825 830
   Leu Ser Leu Leu Ala Val Leu Asn Cys Glu Val Asn Tyr Lys Arg Asn
   835 840 845
   Leu Arg Ile Phe Met Cys Ser Trp Pro Ser Cys Leu Ala Tyr Gly Val
   850 855 860
   Leu His Ala Asn Cys Pro Cys His Ile Gly Ser Cys Phe Glu Gly Ile
   865 870 875 880
   Gly Gly
<210> 240
   <211> 2379
   <212> DNA
   <213> Physcomitrella patens
<400> 240
   atggtgccga cgggcgtggt ggagcgggag gaccagcctg agcctgctgc tgccccccca 60
   gtgccggtga ttacacccaa gacgcttcga tcgcactcta tcaggacgca gaccacgcca 120
   ggtgatggcg atgctggtgc ggatcatgct cctggagcag tggcagcagc agcagcatca 180
   gcaggaggag gaggagcagc ggatgtcatg caaggtaacg atgcggggca ggaacggcga 240
   ggacgacggc gcgggcgacg gaagaagtca gacgtgccgc tcgataatcc cacgcgaatc 300
   ctaaagcatg tcaagtactt gctcattaaa atgcgagtcc accagaacct gctcgatgcg 360
   tacacagggg agggttggaa gggtcagagt cgcgagaaga tcaggccgga gcaagaacta 420
   caacgtgcga aggcgcagat actgcgatct aagttggcca tacgggaggc tattcatgaa 480
   ctcgatgacg tgggtttaga aggttctctc gacaaaaacg cttttgattc tgaaggtcgt 540
   atttaccatg aagagatttt ttgtgcgaag tgcaaatccc aagaagctct tcctgacaac 600
   gacattatct tgtgcgacgg ggcgtgcaac agaggcttcc atcagtattg cttagaccca 660
   cctttagcta cggaagacat tcctcctgga gatgaaggat ggttatgccc tgtatgcgac 720
   tgcaagatgg agtgtattga agcgataaat tcatattttg gcaccagttt tgaggtcgag 780
   aatagttggg agagcttttt ttcgaacgaa gctggtatag ctgcaggcgg tggaacgcaa 840
   gaaggggctg gtggtgattg gccttcctca ggcgatgaag atgatgagta tgatccagaa 900
   acagcggaag ggccttcttc agcttctgaa agtggagctc aagtaatagg gcttgctgtt 960
   gactggccgt cctcagacga cgaagatcac gactatgatc cggaagcagc ggaagaacct 1020
   cctggagctt ctgggagtgg aactcaaaca gtaggtcttg acgttgattg gccttccaca 1080
   gacgatgaag atgacgactt tgatccagaa ggtatcagaa agcaggcaaa cagtgacgaa 1140
   gaaagtagta gtgatgaaag tgaggaggac ggggaggata gtggttctgt aagcggcttc 1200
   agggaaaatg aggcgtcttc aggtgtaagt gacaaagatg agcaggagcc tagtgaaaaa 1260
   cataatctgg agctaactgc tgtcactaac atcaagaaac ttgcaagaaa aggaaaacgt 1320
   aacataatca gcaacaaagg aaaaagtgaa tatttagagt cttcagacag tgatgattct 1380
   gatgtctctc tttttgacga gttcaagggt ggtaatgcca atttaggctt aagacgtggg 1440
   gatccacagg cagcagtatc agcagcagac gaggaagccg tgattattgc tgggaaaaga 1500
   catcggaaag ctgtggacta caaaagacta catgatgaaa tgtatggtaa agcggaggcc 1560
   gataacgatg acgacgttat ctctgaggat gaagattggg gtcctgaacg tcgccgaaga 1620
   cgaaccattc ctgatgatcc taactcgcca aggtcccgaa ttcgaggtag gaaagtgaga 1680
   actccaagtg gagatatttc taaaggagcc gatgcagatt taccctctgg attaccagat 1740
   atgccatccc actttcccac tggttcacaa ggtgaagtag gctcaccaca agacattcca 1800
   gcactagatg gagcagctga cagtcctgga ggagaaaagc ggatgtggcg gcgattgcct 1860
   gattccgcag tcgaggcatt acggtgtatt cttgatgtta ataggctgcc ctcaaaatct 1920
   cggaaggaag aattagcaat taagcttggg ttgagctttt cacaggttca tggatggttt 1980
   aaaaatcagc ggcatcaagc tctgaggaaa gggttggcat gtcctaagcg gatacatcca 2040
   gtagcatcaa gctcaagaga tttcaatact gtcgaatctc catcgtctgt ggcgacttta 2100
   actgaaagca cattaccgca aaaagagaat gctccaaatt ctattttaac tgagaagttg 2160
   gacgaagttc aggtgcgact cctcgaattg aagcaagcgt tggacgattt taccaagaat 2220
   gcagcagtgg ttgcggatgg cggaagtatt gaattggata acaacgtgga aaacggagga 2280
   attggacttc ctgaaaatgg aaaacgtgtt gtctatgtgc cagttgtggc cgacgttagg 2340
   gaacaatcac caatgagtac gcatactagc gcatgttga 2379
<210> 241
   <211> 792
   <212> PRT
   <213> Physcomitrella patens
<400> 241
   Met Val Pro Thr Gly Val Val Glu Arg Glu Asp Gln Pro Glu Pro Ala
   1 5 10 15
   Ala Ala Pro Pro Val Pro Val Ile Thr Pro Lys Thr Leu Arg Ser His
   20 25 30
   Ser Ile Arg Thr Gln Thr Thr Pro Gly Asp Gly Asp Ala Gly Ala Asp
   35 40 45
   His Ala Pro Gly Ala Val Ala Ala Ala Ala Ala Ser Ala Gly Gly Gly
   50 55 60
   Gly Ala Ala Asp Val Met Gln Gly Asn Asp Ala Gly Gln Glu Arg Arg
   65 70 75 80
   Gly Arg Arg Arg Gly Arg Arg Lys Lys Ser Asp Val Pro Leu Asp Asn
   85 90 95
   Pro Thr Arg Ile Leu Lys His Val Lys Tyr Leu Leu Ile Lys Met Arg
   100 105 110
   Val His Gln Asn Leu Leu Asp Ala Tyr Thr Gly Glu Gly Trp Lys Gly
   115 120 125
   Gln Ser Arg Glu Lys Ile Arg Pro Glu Gln Glu Leu Gln Arg Ala Lys
   130 135 140
   Ala Gln Ile Leu Arg Ser Lys Leu Ala Ile Arg Glu Ala Ile His Glu
   145 150 155 160
   Leu Asp Asp Val Gly Leu Glu Gly Ser Leu Asp Lys Asn Ala Phe Asp
   165 170 175
   Ser Glu Gly Arg Ile Tyr His Glu Glu Ile Phe Cys Ala Lys Cys Lys
   180 185 190
   Ser Gln Glu Ala Leu Pro Asp Asn Asp Ile Ile Leu Cys Asp Gly Ala
   195 200 205
   Cys Asn Arg Gly Phe His Gln Tyr Cys Leu Asp Pro Pro Leu Ala Thr
       210                 215                 220
   Glu Asp Ile Pro Pro Gly Asp Glu Gly Trp Leu Cys Pro Val Cys Asp
   225                 230                 235                 240
   Cys Lys Met Glu Cys Ile Glu Ala Ile Asn Ser Tyr Phe Gly Thr Ser
                   245                 250                 255
   Phe Glu Val Glu Asn Ser Trp Glu Ser Phe Phe Ser Asn Glu Ala Gly
               260                 265                 270
   Ile Ala Ala Gly Gly Gly Thr Gln Glu Gly Ala Gly Gly Asp Trp Pro
           275                 280                 285
   Ser Ser Gly Asp Glu Asp Asp Glu Tyr Asp Pro Glu Thr Ala Glu Gly
       290                 295                 300
   Pro Ser Ser Ala Ser Glu Ser Gly Ala Gln Val Ile Gly Leu Ala Val
   305                 310                 315                 320
   Asp Trp Pro Ser Ser Asp Asp Glu Asp His Asp Tyr Asp Pro Glu Ala
                   325                 330                 335
   Ala Glu Glu Pro Pro Gly Ala Ser Gly Ser Gly Thr Gln Thr Val Gly
               340                 345                 350
   Leu Asp Val Asp Trp Pro Ser Thr Asp Asp Glu Asp Asp Asp Phe Asp
           355                 360                 365
   Pro Glu Gly Ile Arg Lys Gln Ala Asn Ser Asp Glu Glu Ser Ser Ser
       370                 375                 380
   Asp Glu Ser Glu Glu Asp Gly Glu Asp Ser Gly Ser Val Ser Gly Phe
   385                 390                 395                 400
   Arg Glu Asn Glu Ala Ser Ser Gly Val Ser Asp Lys Asp Glu Gln Glu
                   405                 410                 415
   Pro Ser Glu Lys His Asn Leu Glu Leu Thr Ala Val Thr Asn Ile Lys
               420                 425                 430
   Lys Leu Ala Arg Lys Gly Lys Arg Asn Ile Ile Ser Asn Lys Gly Lys
           435                 440                 445
   Ser Glu Tyr Leu Glu Ser Ser Asp Ser Asp Asp Ser Asp Val Ser Leu
       450                 455                 460
   Phe Asp Glu Phe Lys Gly Gly Asn Ala Asn Leu Gly Leu Arg Arg Gly
   465                 470                 475                 480
   Asp Pro Gln Ala Ala Val Ser Ala Ala Asp Glu Glu Ala Val Ile Ile
                   485                 490                 495
   Ala Gly Lys Arg His Arg Lys Ala Val Asp Tyr Lys Arg Leu His Asp
               500                 505                 510
   Glu Met Tyr Gly Lys Ala Glu Ala Asp Asn Asp Asp Asp Val Ile Ser
           515                 520                 525
   Glu Asp Glu Asp Trp Gly Pro Glu Arg Arg Arg Arg Arg Thr Ile Pro
       530                 535                 540
   Asp Asp Pro Asn Ser Pro Arg Ser Arg Ile Arg Gly Arg Lys Val Arg
   545                 550                 555                 560
   Thr Pro Ser Gly Asp Ile Ser Lys Gly Ala Asp Ala Asp Leu Pro Ser
                   565                 570                 575
   Gly Leu Pro Asp Met Pro Ser His Phe Pro Thr Gly Ser Gln Gly Glu
               580                 585                 590
   Val Gly Ser Pro Gln Asp Ile Pro Ala Leu Asp Gly Ala Ala Asp Ser
           595                 600                 605
   Pro Gly Gly Glu Lys Arg Met Trp Arg Arg Leu Pro Asp Ser Ala Val
       610                 615                 620
   Glu Ala Leu Arg Cys Ile Leu Asp Val Asn Arg Leu Pro Ser Lys Ser
   625                 630                 635                 640
   Arg Lys Glu Glu Leu Ala Ile Lys Leu Gly Leu Ser Phe Ser Gln Val
                   645                 650                 655
   His Gly Trp Phe Lys Asn Gln Arg His Gln Ala Leu Arg Lys Gly Leu
               660                 665                 670
   Ala Cys Pro Lys Arg Ile His Pro Val Ala Ser Ser Ser Arg Asp Phe
           675                 680                 685
   Asn Thr Val Glu Ser Pro Ser Ser Val Ala Thr Leu Thr Glu Ser Thr
       690                 695                 700
   Leu Pro Gln Lys Glu Asn Ala Pro Asn Ser Ile Leu Thr Glu Lys Leu
   705                 710                 715                 720
   Asp Glu Val Gln Val Arg Leu Leu Glu Leu Lys Gln Ala Leu Asp Asp
                   725                 730                 735
   Phe Thr Lys Asn Ala Ala Val Val Ala Asp Gly Gly Ser Ile Glu Leu
               740                 745                 750
   Asp Asn Asn Val Glu Asn Gly Gly Ile Gly Leu Pro Glu Asn Gly Lys
   755 760 765
   Arg Val Val Tyr Val Pro Val Val Ala Asp Val Arg Glu Gln Ser Pro
   770 775 780
   Met Ser Thr His Thr Ser Ala Cys
   785 790
<210> 242
   <211> 2166
   <212> DNA
   <213> Zea mays
<400> 242
   atgcattctt cggaaaataa actgggttgg aatgagacct ctatggggtt ggttcctgta     60
   ccaaaaagac cagctagacc tgatgcttcc caccaatgca aatctgattc ctttatgaag    120
   cgatcaccaa ggaaagttag aaatgctact ctggcaaaaa gcataaagag caaataccac    180
   tatagtcccc tcaaacagcg gaagggttca gattctgttc ctgggaaaat cgtaacagga    240
   ctaaccgcaa ggaaaaagaa gaaaaggaaa atccaaatta cagacgaggc aactcgtttg    300
   gaacgaagag cgagatattt tctaatcaag ataaaactgg agcagaattt gctagatgct    360
   tactctggag atggatggaa tgggcaaagc cgagagaaaa taaagccaga gaaggaactg    420
   caacgtgcca ggaaacaaat cataaaatgc aaaattgcta tacgtgatat catccgccaa    480
   cttgatttgt atacttctac tgggagtgtt gatgacccac taatgcctac agatcagtcc    540
   accaatcccg aacataccat gtgctcaaca tgcaagtctc atgaatcatt tcccagcaat    600
   aaaattatct tctgcaaagg gccctgcaaa agggcatgtc atgagaaatg tttggaacct    660
   cccttaaaca aaagcgtact tccaacaagt agccatgggt ggctttgcaa attctgtttg    720
   tgcaaggtga ggattttaga aactattaat gcacatctag ggacgagttt tacagtgaag    780
   tgccactttg aagatatatt caaggaaact actgaactaa tagactctga ggatgcacta    840
   gatgaagatt ggctttctga gtattcaggt gatgaggact atgatcctga tgaaaatgag    900
   gccagtggcg actgtatgga cagcggggag aagattatgt ctgatgattc caatggttca    960
   ggaagccccc tttattctcc aaatgacgat attcctgact tcatatcagc agacttaaat   1020
   gttgtggaag ggttttgtca taccaattta gatttaggca ttgatgccgt tgaagatgat   1080
   tttgcacaga tcctcaccta ccaaaggcca agaagagatg ttgattatag aaggcttaac   1140
   gaggaaatgt ttgggaaaat aaccgggaat gaagaacaga gcgaggacga agattggggc   1200
   catgaaagga gaaagaaaag gacgcattca ggtgttgctg gggataattc tgttggtttc   1260
   ttgaacgtta tatctgatga aaagagccag aagaagggga gaaaactttt caggattcct   1320
   cctgcagctg ttgaggtact tcgcagagct tttgctgaaa atgagcttcc accccgggat   1380
   gttaaagaaa atctctcaag agaattggga atttcttttg aaaagattga taaatggttc   1440
   aaaaatacac ggtgtgctgc tctcagagat cggaaggctg aaggaaacag tcataataca   1500
   gctcccagca aaatctcaaa aaataaagga aaagctggaa tctcaggcaa ggctggaagg   1560
   aatggtcatg ttactggtcc ctgcaataat ttgagaataa atgaagaaaa aacaggtata   1620
   tcggggaagt ttgattcagg agacaactct tgtttggttc ccttctctga agtcatcaat   1680
   gtgcctacgc gattgccaca caactttgag atgaggaaga tggaatcaac tagaagtcct   1740
   gcgaggctac acaataaagg agggtgcctg tctgcaacaa tccaaattaa ggagagtaca   1800
   ttgttaccca caggcaagcc ttgttggcag tcagaaatga gccatccaac aactaatgaa   1860
   gtgagcactt cagcgcaagc tactacttgg atcgacgcgg gggcgtgtgc cgaggagcaa   1920
   gaacctactc cttgggtgga catcggggcc tcagactacc agcctttcct ggatgtgatc   1980
   gacgacatgt gcggacttga gtggaggctg cagagactga aggagaacat gctctcatct   2040
   agcatagacg gccaaaccgc cgccagtgag agtgacaaaa gaaaccagac cgtggtgctc   2100
   gtgccaactg ccgagctcaa ggaaaaactg ccgcatgacg gtttattcgg gcattattgc   2160
   ccatag                                                              2166
<210> 243
   <211> 721
   <212> PRT
   <213> Zea mays
<400> 243
   Met His Ser Ser Glu Asn Lys Leu Gly Trp Asn Glu Thr Ser Met Gly
   1               5                   10                  15
   Leu Val Pro Val Pro Lys Arg Pro Ala Arg Pro Asp Ala Ser His Gln
               20                  25                  30
   Cys Lys Ser Asp Ser Phe Met Lys Arg Ser Pro Arg Lys Val Arg Asn
           35                  40                  45
   Ala Thr Leu Ala Lys Ser Ile Lys Ser Lys Tyr His Tyr Ser Pro Leu
       50                  55                  60
   Lys Gln Arg Lys Gly Ser Asp Ser Val Pro Gly Lys Ile Val Thr Gly
   65                  70                  75                  80
   Leu Thr Ala Arg Lys Lys Lys Lys Arg Lys Ile Gln Ile Thr Asp Glu
                   85                  90                  95
   Ala Thr Arg Leu Glu Arg Arg Ala Arg Tyr Phe Leu Ile Lys Ile Lys
               100                 105                 110
   Leu Glu Gln Asn Leu Leu Asp Ala Tyr Ser Gly Asp Gly Trp Asn Gly
           115                 120                 125
   Gln Ser Arg Glu Lys Ile Lys Pro Glu Lys Glu Leu Gln Arg Ala Arg
       130                 135                 140
   Lys Gln Ile Ile Lys Cys Lys Ile Ala Ile Arg Asp Ile Ile Arg Gln
   145                 150                 155                 160
   Leu Asp Leu Tyr Thr Ser Thr Gly Ser Val Asp Asp Pro Leu Met Pro
                   165                 170                 175
   Thr Asp Gln Ser Thr Asn Pro Glu His Thr Met Cys Ser Thr Cys Lys
               180                 185                 190
   Ser His Glu Ser Phe Pro Ser Asn Lys Ile Ile Phe Cys Lys Gly Pro
           195                 200                 205
   Cys Lys Arg Ala Cys His Glu Lys Cys Leu Glu Pro Pro Leu Asn Lys
       210                 215                 220
   Ser Val Leu Pro Thr Ser Ser His Gly Trp Leu Cys Lys Phe Cys Leu
   225                 230                 235                 240
   Cys Lys Val Arg Ile Leu Glu Thr Ile Asn Ala His Leu Gly Thr Ser
                   245                 250                 255
   Phe Thr Val Lys Cys His Phe Glu Asp Ile Phe Lys Glu Thr Thr Glu
               260                 265                 270
   Leu Ile Asp Ser Glu Asp Ala Leu Asp Glu Asp Trp Leu Ser Glu Tyr
           275                 280                 285
   Ser Gly Asp Glu Asp Tyr Asp Pro Asp Glu Asn Glu Ala Ser Gly Asp
       290                 295                 300
   Cys Met Asp Ser Gly Glu Lys Ile Met Ser Asp Asp Ser Asn Gly Ser
   305                 310                 315                 320
   Gly Ser Pro Leu Tyr Ser Pro Asn Asp Asp Ile Pro Asp Phe Ile Ser
                   325                 330                 335
   Ala Asp Leu Asn Val Val Glu Gly Phe Cys His Thr Asn Leu Asp Leu
               340                 345                 350
   Gly Ile Asp Ala Val Glu Asp Asp Phe Ala Gln Ile Leu Thr Tyr Gln
           355                 360                 365
   Arg Pro Arg Arg Asp Val Asp Tyr Arg Arg Leu Asn Glu Glu Met Phe
       370                 375                 380
   Gly Lys Ile Thr Gly Asn Glu Glu Gln Ser Glu Asp Glu Asp Trp Gly
   385                 390                 395                 400
   His Glu Arg Arg Lys Lys Arg Thr His Ser Gly Val Ala Gly Asp Asn
                   405                 410                 415
   Ser Val Gly Phe Leu Asn Val Ile Ser Asp Glu Lys Ser Gln Lys Lys
               420                 425                 430
   Gly Arg Lys Leu Phe Arg Ile Pro Pro Ala Ala Val Glu Val Leu Arg
   435 440 445
   Arg Ala Phe Ala Glu Asn Glu Leu Pro Pro Arg Asp Val Lys Glu Asn
   450 455 460
   Leu Ser Arg Glu Leu Gly Ile Ser Phe Glu Lys Ile Asp Lys Trp Phe
   465 470 475 480
   Lys Asn Thr Arg Cys Ala Ala Leu Arg Asp Arg Lys Ala Glu Gly Asn
   485 490 495
   Ser His Asn Thr Ala Pro Ser Lys Ile Ser Lys Asn Lys Gly Lys Ala
   500 505 510
   Gly Ile Ser Gly Lys Ala Gly Arg Asn Gly His Val Thr Gly Pro Cys
   515 520 525
   Asn Asn Leu Arg Ile Asn Glu Glu Lys Thr Gly Ile Ser Gly Lys Phe
   530 535 540
   Asp Ser Gly Asp Asn Ser Cys Leu Val Pro Phe Ser Glu Val Ile Asn
   545 550 555 560
   Val Pro Thr Arg Leu Pro His Asn Phe Glu Met Arg Lys Met Glu Ser
   565 570 575
   Thr Arg Ser Pro Ala Arg Leu His Asn Lys Gly Gly Cys Leu Ser Ala
   580 585 590
   Thr Ile Gln Ile Lys Glu Ser Thr Leu Leu Pro Thr Gly Lys Pro Cys
   595 600 605
   Trp Gln Ser Glu Met Ser His Pro Thr Thr Asn Glu Val Ser Thr Ser
   610 615 620
   Ala Gln Ala Thr Thr Trp Ile Asp Ala Gly Ala Cys Ala Glu Glu Gln
   625 630 635 640
   Glu Pro Thr Pro Trp Val Asp Ile Gly Ala Ser Asp Tyr Gln Pro Phe
   645 650 655
   Leu Asp Val Ile Asp Asp Met Cys Gly Leu Glu Trp Arg Leu Gln Arg
   660 665 670
   Leu Lys Glu Asn Met Leu Ser Ser Ser Ile Asp Gly Gln Thr Ala Ala
   675 680 685
   Ser Glu Ser Asp Lys Arg Asn Gln Thr Val Val Leu Val Pro Thr Ala
   690 695 700
   Glu Leu Lys Glu Lys Leu Pro His Asp Gly Leu Phe Gly His Tyr Cys
   705 710 715 720
   Pro